# EUROPEAN PATENT APPLICATION

(11) **EP 1 792 998 A2**
(43) Date of publication of application: **06.06.2007**
(21) Application number: 06114643.7
(22) Date of filing: 01.11.1999
(51) Int. Cl.: C12N 15/87

(54) **An unusual retrotransposon from the yeast Candida albicans**

(30) Priority: 30.10.1998 US 106342 P; 30.10.1998 CA 2249046
(62) Divisional of application: 99954511.4
(71) Applicant: JANSSEN PHARMACEUTICA N.V., 2340 Beerse (BE)
(72) Inventor: Luyten, Walter, Herman, Maria, Louis, 2340 Beerse (BE); De Backer, Marianne, Denise, 2340 Beerse (BE); Nelissen, Bart, Jozef, Maria, 2340 Beerse (BE); Poulter, Russell, Tony, Maskell, RD2, Dunedin (NZ)

(57) **Abstract**

TCa2 is a Tyl/*copia* retrotransposon from the pathogenic yeast *Candida albicans.* In contrast to other retrotransposons it can appear as an abundant, extrachromosomal double-stranded DNA molecule, called pCal. The invention relates to the isolation and characterisation of TCa2 and pCal together with its uses for inducing random mutagenesis in a genome, as a component of a transposable element and of an expression vector.

### Annex to the application documents - subsequently filed sequences listing

SEQUENCE LISTING

<110> Janssen Pharmaceutica

<120> An unusual retrotransposon from the yeast candida albicans

<130> JP801783/142

<140> PCT/NZ99/00179

<141> 1999-11-01

<150> US 60/106342

<151> 1998-10-30

<150> CA 2249046

<151> 1998-10-30

<160> 79

<170> PatentIn version 3.0

<210> 1

<211> 6426

<212> DNA

<213> Candida albicans

<400> 1
tgttggtttg tgcactattt tgtgtcagaa actgatcaat gaaaatgatg gttattatga 60

gaatggaaaa tttttccatc acacatcagg tgatgacaga actaaactat attgtgtagt 120

ataaataagg gtatgaaata ccaacatccc agaatatcaa cgagatagaa gggaggagtt 180

tcaatatata tcttgtgaat aataacttcg ttctaattca ctatacacaa ctagacgtgt 240

acacgctcaa tctcaggtaa agaaagttta tattccatca gattagaagt cgatagtgat 300
aatcatttcg tcccaaatta gcgttgtata aattcagtcc tcagatttgt attattgatt 360
gatagtttcg aagtttgaag gtacagaatt tcacaagatg agttccgcaa agaatgatga 420
taacgaaggg aaggtcatgg aaagtgttga tcaagctaat gctattagta aggtggatga 480
acatatcaag gctagattca atatgctttt cataaaattt aatgacttac ctaagttggc 540
cgtcggtaat cagaaaagcg tggataaatg gaatgaagaa tttaaatatt tccacgttgc 600
ttaccccgat gttttggaat ttttgcttga ctataatcct aaagataaat tcaaggttaa 660
aaaggtagaa ggtatttatt ttactggttg gtgtttacaa atgtgtttac agtccatttt 720
tgataggttc agattgatca tgatttctaa gctaccaaag cacttgcaaa aggaagcaaa 780
cttaatcaaa gctgcttatg atgctgttac taaatctaaa gattatacca ttactagtaa 840
gatcttgctg aagtttgtaa acgttgaaca tgagttagtg gtttgctata accttccata 900
tttgctgcag gtggaagaga aacttgagga aatactctac aacacttcaa acgttgtcga 960
tgagtatgtc cgtagtcttc caaatctcat aggtcaagtc ttgtacttca atcatgtgaa 1020
gaaatcagag gctttaagtt tgtttttgaa tattcatgcc tcatactact caaagtggat 1080
tcaagctgac aatgatacat cagtactccc aagttgctct accatagctg aagaaatgtg 1140
tgatcatcct gattatgcta gattggttga cattccaagc aacaaatatg aacttaatct 1200
tattgttagt ttaccagcac cagagaaacc aaaaggaaaa ccagaggaga actcactgga 1260
acaatctcaa aagaagaacc tgaaatcaag aaagagaaat aagaaacatc caaaatcaga 1320
taacgataaa ggtgaaaaag aaaaagaaaa agaaaaaact tcactggaat gaaaaacagg 1380
tgctgcttct attaattgtg taatgaatat acataattgc agcaaaacca cgtttccagt 1440
agaaaattct cattctctta atgcttcttt gaacgtaatg aattttaaag gtttaaggtt 1500
taacaagtat ctagtgtatg atactggtgc cacaatatct gttgtgaaca ataaagatat 1560
attgctgaat gttaaggacg caacaattga agtttctgtt gctgatggtg ctacattaga 1620
agcagattgt attggtgatc taattatcag agtcggtatt gtctcgatta cgttagagaa 1680
tacattgtat ttaccagaaa gttcctttaa tcttgtgagt ttgaaacaaa ttgaagaacg 1740
aggatttaat gttcttatta ctaaagaatc agtgattgta tttaaccaaa atgtggctcc 1800
tactattatt gcttcaagga agaatgctgc tgatctttat atgggtcctc aattcagtga 1860
agaatcttta gaatgtgatt ttgattatga tggtttggca gatatgttgt ccaatgctaa 1920
ccaagatgac aaagataaat caagtatgaa tgaaatgtca gaatatcaag aacatgatta 1980
tagttctcga gcattaataa attctttgac ggaggttgat gttttagatg ttgaaatttc 2040
cccatatgga gttgaacaat tgctaccaac tggagataag aacgatattt ataatttcca 2100

tttgatgtca aatcatatgt ccattgagaa aatcttgttg ttacaaaaat accagggtct 2160

cgtacttcac acttcaaaag agagtcttca aaagattgct gattgtaagg tatgtctatt 2220

atcgaatgcc aaacagagaa gtcacaatca tcattcagaa agaaaagcct cgagaagaca 2280

tgagagactt cattgtgata ctctcggtcc atttaggtcc gaaaataaca agtggtattt 2340

aacgtctgtt atagatgaac atacgggtta cattgaagga attattacta aagacagaaa 2400

ggtaaaggat ctcttaattc aacgattaaa gatctggaat aatcggttta acgataaggt 2460

ggcatacttc agaagtgata atgctcctga gttcccacaa ccttctgatt tagctgagtt 2520

cggtatttgg agggagacta tagcggcata tctgcctgag cttaatggtc tcgccgaggt 2580

tgttaataaa ttgattttac aacagattta caggatcgtt gtgacacttg gtccacaaat 2640

actcaagttg atttattatg tgattcaata ttctattaca atgatcaacc acactccacg 2700

tcgttcactc aagggacaaa ccccttatgg ttgctattat caattaagtg agggaaattt 2760

ctaccggttt ccttttgcca tcgattgtgt cgttacattt agtaatgcca tcgaaaagaa 2820

ccgttacgga gttacatcaa ctaaaggagc tccttcatcg atcatgggtg ctgtgattgg 2880

ctacgctagc gattgtttta gttattacgt gttgctaaaa aatatgcggt gtgatattat 2940

ccttagccct aatgtccgta tattgcgaag ctatgaggtt attaactcct atctcaaaaa 3000

cttatccact acacctatgt cacacattgt tcctatggct gaaggtatcc agggaaggca 3060

actgggcgct cagtacgagg tacgcggaac atatgtggaa agtgaatatg acaatacaaa 3120

tgacgtgatg cacatgccca aagagtcata ttcagttcag ccagcatcgt ttactttaac 3180

tacgggtaac agttctaacg aatatgttat aaatgatgat ccagtacaga ttaccattga 3240

gaatcccgat gatttttcta accctcttca actaactgaa gaatcacacg atatggtatc 3300

cgaagtaaaa tcggatgaga atcctaaacc cagtctccac gagctaacac ctggggataa 3360

tccggtgtct aaacctcctc aacttggtac cgagacttca gtaataggga agtctaaaga 3420

gcctattaca aaccacacaa aggacgcccc ttccatccag gggagggacc ataaacgcct 3480

ggaatctact gctcaggttg gactatcaca ccaaccccag actggtactc ccgcttcgga 3540

ggagtcaaaa ttgtcaggaa cagatcattt cggtgtcgac gttgttaaag aaacagtctc 3600

agaagattgg catacttctg actacccaga aactagtgct gaagatgaac agcaaaatcc 3660

ctcgttactg gctaataaga atcgggtaac tgaaaaaata gatgagggag aaaatatttc 3720

atttccgggg ggtgatgatg attctgtcgt gatcaactca aatgttgagc aatctaatgt 3780

tgaaacagag gatgctggta acagtccaat tcaagacgaa gtttctcaag agggaagaat 3840

acttaatgaa caaactgata tagttgatac tgttgctaaa gttattgaga atgaaaaaat 3900

ctctcctatt aattcattag atgatcatac tgaacttgct acagactcgg gaaatgatag 3960

caattcaaca gaatccgaca ttcaatcgaa aaatgaaata tcaccagtga ttaatgagaa 4020

aaatactgaa ataatccaaa aacacattga aagtatcctt gctgataaga gattggatga 4080

atttgaaacg tataatgttg atgaaattga gaatgtgatt aatgacgatg acattgctga 4140

agctaatcca ctaccagatg aaaataatga tgttcagatg aatgagagtt ttgataataa 4200

tcatagcatg tcacgagcaa agaagaaata cacatttgag aaagaagtta acgaaaaaat 4260

tgctggtact aaacattcac ttgatacaac tgatccaaga gaagcaatca gagtgttaaa 4320

tactggtgaa accaagagaa tcgaacccaa gaaaagagag gtgcctatca ctgtgaaatt 4380

aaacaaaaga tcgcaataca agtcaccata tgttacaaga agtggtagaa cggttataaa 4440

ccccaagagg tatttacatg cggtcgtcaa caaaatcgac tataatgatc cgggatggat 4500

aaagtcaatg aatgctgaac tagagaaatt tagatcaaaa gatgtttacg aagaagttcc 4560

aattcccacc ggtgtgaagc ctatatctat gggttgggta catactgaga aaattgattc 4620

tctcaaaggt gttgttcgga aatcacgttg tgttgtccat ggcaacagac aaaaggaaaa 4680

attggattat gaccctttta gtgttagttc acctgttata gatcttgtga ctataagatt 4740

attgacaata ataggttgtg aattaggaat gacaattcaa catttagacg tcgagtcggc 4800

gtatctaaat gcctctatta ctcattcaaa tccaatttat gtctttcctc ctaaatcagt 4860

acctttgaag aaaaaccatt gttggttatt gaaacgttct gtctatgggt taaaacagtc 4920

gggtttggaa tggtatcaca ctatcaaaag agtattggaa gacattggtt ttactcaagt 4980

tttacacaat gatggtttat ttcacattga atatgaagag ggatcagtaa tatatttagg 5040

tttatatgtt gatgatattc ttatggttgg aagttcacaa aaagttattg ataattttgt 5100

ggatcaattg agagatcatt ttgaagttaa agtgtttggt gaaatatcaa attatcttgg 5160

tattgaattt cgtaaaaccg aatctggtta tattttatct caagaaaaat ttctcaagaa 5220

attacttaag gatttcaaac tagatgactc atatgggaaa aacataccct ggattccgaa 5280

tgacaaatat gaaaaggttg caataattcg tgaaaacgtt aatccagaga atgattttga 5340

aaaggttccg aatgagacat tgcttgaccc tgatgctaaa aaactatacc aaagtggtgt 5400

tggcctgctt ttatgggctg ccacaaacac acgtccagat atatcggtcg tagtgaattc 5460

gttgggttct aaatctgcaa atccaaatgt ccatgattat gagaaattga tttattgtct 5520

taggtatatc aaaaatagca tgggatatca cattgagtac aaaagaaaca gattgaatat 5580

accaccaaaa tcatttgtta tcgaatgttt cagtgatgcg tcatttgcac caggattgga 5640

tagaaaatct attagtggaa ctttgattta tgtgaatgga aatttggtgc aatgggcgac 5700

caaaaaacaa acggtcatag cacaaagctc agcagcttgt gaaatgttgg ctctaaatta 5760

tacaatgttg aaagctatcg aaataaaaaa ccatttaatg gatttgggtt ttgaagtagg 5820

taagatacat tgtcatcaag acaaccaagc tgtgattaaa gttttgagaa ataactattg 5880

tcacccacat cgaccaatag atatctgcta taagtttcta cgccaattga tcaatgataa 5940

agtattttca atatcctatg tgaagacaaa tgataattac gccgattgta tgactaagtg 6000

tctaagtcgt gctaaattca aagcattcgt tgagggtatg ataaaacggt tagacctaga 6060

agataatcaa acactgatac aaaatgcaat aacggcagaa taagtggatt tatcattact 6120

attatcgtaa tgctcaatca ggggagtgtt ggtttgtgca ctattttgtg tcagaaactg 6180

atcaatgaaa atgatggtta ttatgagaat ggaaaatttt tccatcacac atcaggtgat 6240

gacagaacta aactatattg tgtagtataa ataagggtat gaaataccaa catcccagaa 6300

tatcaacgag atagaaggga ggagtttcaa tatatatctt gtgaataata acttcgttct 6360

aattcactat acacaactag acgtgtacac gctcaatctc aggtaaagaa agtttatatt 6420

ccatca 6426

<210> 2

<211> 1576

<212> PRT

<213> Candida albicans

<400> 2

Lys Thr Gly Ala Ala Ser Ile Asn Cys Val Met Asn Ile His Asn Cys
1               5                   10                  15

Ser Lys Thr Thr Phe Pro Val Glu Asn Ser His Ser Leu Asn Ala Ser
            20                  25                  30

Leu Asn Val Met Asn Phe Lys Gly Leu Arg Phe Asn Lys Tyr Leu Val
        35                  40                  45

Tyr Asp Thr Gly Ala Thr Ile Ser Val Val Asn Asn Lys Asp Ile Leu
    50                  55                  60

Ser Asn Val Lys Asp Ala Thr Ile Glu Val Ser Val Ala Asp Gly Ala
65                  70                  75                  80

Thr Leu Glu Ala Asp Cys Ile Gly Asp Leu Ile Ile Arg Val Gly Ile
                85                  90                  95

Val Ser Ile Thr Leu Glu Asn Thr Leu Tyr Leu Pro Glu Ser Ser Phe
            100                 105                 110

Asn Leu Val Ser Leu Lys Gln Ile Glu Glu Arg Gly Phe Asn Val Leu
        115                 120                 125

Ile Thr Lys Glu Ser Val Ile Val Phe Asn Gln Asn Val Ala Pro Thr
    130                 135                 140

Ile Ile Ala Ser Arg Lys Asn Ala Ala Asp Leu Tyr Met Gly Pro Gln
145                 150                 155                 160

Phe Ser Glu Glu Ser Leu Glu Cys Asp Phe Asp Tyr Asp Gly Leu Ala
                165                 170                 175

Asp Met Leu Ser Asn Ala Asn Gln Asp Asp Lys Asp Lys Ser Ser Met
            180                 185                 190

Asn Glu Met Ser Glu Tyr Gln Glu His Asp Tyr Ser Ser Arg Ala Leu
        195                 200                 205

Ile Asn Ser Leu Thr Glu Val Asp Val Leu Asp Val Glu Ile Ser Pro
    210                 215                 220

Tyr Gly Val Glu Gln Leu Leu Pro Thr Gly Asp Lys Asn Asp Ile Tyr
225                 230                 235                 240

Asn Phe His Leu Met Ser Asn His Met Ser Ile Glu Lys Ile Leu Leu
                245                 250                 255

Leu Gln Lys Tyr Gln Gly Leu Val Leu His Thr Ser Lys Glu Ser Leu
            260                 265                 270

Gln Lys Ile Ala Asp Cys Lys Val Cys Leu Leu Ser Asn Ala Lys Gln
        275                 280                 285

Arg Ser His Asn His His Ser Glu Arg Lys Ala Ser Arg Arg His Glu
    290                 295                 300

Arg Leu His Cys Asp Thr Leu Gly Pro Phe Arg Ser Glu Asn Asn Lys
305                 310                 315                 320

Trp Tyr Leu Thr Ser Val Ile Asp Glu His Thr Gly Tyr Ile Glu Gly
                325                 330                 335

Ile Ile Thr Lys Asp Arg Lys Val Lys Asp Leu Leu Ile Gln Arg Leu
            340                 345                 350

Lys Ile Trp Asn Asn Arg Phe Asn Asp Lys Val Ala Tyr Phe Arg Ser
        355                 360                 365

Asp Asn Ala Pro Glu Phe Pro Gln Pro Ser Asp Leu Ala Glu Phe Gly
    370                 375                 380

Ile Trp Arg Glu Thr Ile Ala Ala Tyr Ser Pro Glu Leu Asn Gly Leu
385                 390                 395                 400

Ala Glu Val Val Asn Lys Leu Ile Leu Gln Gln Ile Tyr Arg Ile Val
                405                 410                 415

Val Thr Leu Gly Pro Gln Ile Leu Lys Leu Ile Tyr Tyr Val Ile Gln
            420                 425                 430

Tyr Ser Ile Thr Met Ile Asn His Thr Pro Arg Arg Ser Leu Lys Gly
        435                 440                 445

Gln Thr Pro Tyr Gly Cys Tyr Tyr Gln Leu Ser Glu Gly Asn Phe Tyr
    450                 455                 460

Arg Phe Pro Phe Ala Ile Asp Cys Val Val Thr Phe Ser Asn Ala Ile
465                 470                 475                 480

Glu Lys Asn Arg Tyr Gly Val Thr Ser Thr Lys Gly Ala Pro Ser Ser
                485                 490                 495

Ile Met Gly Ala Val Ile Gly Tyr Ala Ser Asp Cys Phe Ser Tyr Tyr
            500                 505                 510

Val Leu Leu Lys Asn Met Arg Cys Asp Ile Ile Leu Ser Pro Asn Val
        515                 520                 525

Arg Ile Leu Arg Ser Tyr Glu Val Ile Asn Ser Tyr Leu Lys Asn Leu
    530                 535                 540

Ser Thr Thr Pro Met Ser His Ile Val Pro Met Ala Glu Gly Ile Gln
545                 550                 555                 560

Gly Arg Gln Ser Gly Ala Gln Tyr Glu Val Arg Gly Thr Tyr Val Glu
                565                 570                 575

Ser Glu Tyr Asp Asn Thr Asn Asp Val Met His Met Pro Lys Glu Ser
            580                 585                 590

Tyr Ser Val Gln Pro Ala Ser Phe Thr Leu Thr Thr Gly Asn Ser Ser
        595                 600                 605

Asn Glu Tyr Val Ile Asn Asp Asp Pro Val Gln Ile Thr Ile Glu Asn
    610                 615                 620

Pro Asp Asp Phe Ser Asn Pro Leu Gln Leu Thr Glu Glu Ser His Asp
625                 630                 635                 640

Met Val Ser Glu Val Lys Ser Asp Glu Asn Pro Lys Pro Ser Leu His
                645                 650                 655

Glu Leu Thr Pro Gly Asp Asn Pro Val Ser Lys Pro Pro Gln Leu Gly
            660                 665                 670

Thr Glu Thr Ser Val Ile Gly Lys Ser Lys Glu Pro Ile Thr Asn His
        675                 680                 685

Thr Lys Asp Ala Pro Ser Ile Gln Gly Arg Asp His Lys Arg Ser Glu
    690                 695                 700

Ser Thr Ala Gln Val Gly Leu Ser His Gln Pro Gln Thr Gly Thr Pro
705                 710                 715                 720

Ala Ser Glu Glu Ser Lys Leu Ser Gly Thr Asp His Phe Gly Val Asp
                725                 730                 735

Val Val Lys Glu Thr Val Ser Glu Asp Trp His Thr Ser Asp Tyr Pro
            740                 745                 750

Glu Thr Ser Ala Glu Asp Glu Gln Gln Asn Pro Ser Leu Ser Ala Asn
        755                 760                 765

Lys Asn Arg Val Thr Glu Lys Ile Asp Glu Gly Glu Asn Ile Ser Phe

770                 775                 780

Pro Gly Gly Asp Asp Asp Ser Val Val Ile Asn Ser Asn Val Glu Gln
785                 790                 795                 800

Ser Asn Val Glu Thr Glu Asp Ala Gly Asn Ser Pro Ile Gln Asp Glu
                805                 810                 815

Val Ser Gln Glu Gly Arg Ile Leu Asn Glu Gln Thr Asp Ile Val Asp
            820                 825                 830

Thr Val Ala Lys Val Ile Glu Asn Glu Lys Ile Ser Pro Ile Asn Ser
        835                 840                 845

Leu Asp Asp His Thr Glu Leu Ala Thr Asp Ser Gly Asn Asp Ser Asn
    850                 855                 860

Ser Thr Glu Ser Asp Ile Gln Ser Lys Asn Glu Ile Ser Pro Val Ile
865                 870                 875                 880

Asn Glu Lys Asn Thr Glu Ile Ile Gln Lys His Ile Glu Ser Ile Leu
                885                 890                 895

Ala Asp Lys Arg Leu Asp Glu Phe Glu Thr Tyr Asn Val Asp Glu Ile
            900                 905                 910

Glu Asn Val Ile Asn Asp Asp Asp Ile Ala Glu Ala Asn Pro Leu Pro
        915                 920                 925

Asp Glu Asn Asn Asp Val Gln Met Asn Glu Ser Phe Asp Asn Asn His
    930                 935                 940

Ser Met Ser Arg Ala Lys Lys Lys Tyr Thr Phe Glu Lys Glu Val Asn
945                 950                 955                 960

Glu Lys Ile Ala Gly Thr Lys His Ser Leu Asp Thr Thr Asp Pro Arg
                965                 970                 975

Glu Ala Ile Arg Val Leu Asn Thr Gly Glu Thr Lys Arg Ile Glu Pro
            980                 985                 990

Lys Lys Arg Glu Val Pro Ile Thr  Val Lys Leu Asn Lys  Arg Ser Gln
        995                 1000                 1005

Tyr Lys  Ser Pro Tyr Val Thr  Arg Ser Gly Arg Thr  Val Ile Asn
    1010                 1015                 1020

Pro Lys  Arg Tyr Leu His Ala  Val Val Asn Lys Ile  Asp Tyr Asn
    1025                 1030                 1035

Asp Pro  Gly Trp Ile Lys Ser  Met Asn Ala Glu Leu  Glu Lys Phe
    1040                 1045                 1050

Arg Ser  Lys Asp Val Tyr Glu  Glu Val Pro Ile Pro  Thr Gly Val
    1055                 1060                 1065

Lys Pro  Ile Ser Met Gly Trp  Val His Thr Glu Lys  Ile Asp Ser
    1070                 1075                 1080

Leu Lys  Gly Val Val Arg Lys  Ser Arg Cys Val Val  His Gly Asn
    1085                 1090                 1095

Arg Gln Lys Glu Lys Leu Asp Tyr Asp Pro Phe Ser Val Ser Ser
    1100                1105                1110

Pro Val Ile Asp Leu Val Thr Ile Arg Leu Leu Thr Ile Ile Gly
    1115                1120                1125

Cys Glu Leu Gly Met Thr Ile Gln His Leu Asp Val Glu Ser Ala
    1130                1135                1140

Tyr Leu Asn Ala Ser Ile Thr His Ser Asn Pro Ile Tyr Val Phe
    1145                1150                1155

Pro Pro Lys Ser Val Pro Leu Lys Lys Asn His Cys Trp Leu Leu
    1160                1165                1170

Lys Arg Ser Val Tyr Gly Leu Lys Gln Ser Gly Leu Glu Trp Tyr
    1175                1180                1185

His Thr Ile Lys Arg Val Leu Glu Asp Ile Gly Phe Thr Gln Val
    1190                1195                1200

Leu His Asn Asp Gly Leu Phe His Ile Glu Tyr Glu Glu Gly Ser
    1205                1210                1215

Val Ile Tyr Leu Gly Leu Tyr Val Asp Asp Ile Leu Met Val Gly
    1220                1225                1230

Ser Ser Gln Lys Val Ile Asp Asn Phe Val Asp Gln Leu Arg Asp
    1235                1240                1245

His Phe Glu Val Lys Val Phe Gly Glu Ile Ser Asn Tyr Leu Gly
    1250                1255                1260

Ile Glu Phe Arg Lys Thr Glu Ser Gly Tyr Ile Leu Ser Gln Glu
    1265                1270                1275

Lys Phe Leu Lys Lys Leu Leu Lys Asp Phe Lys Leu Asp Asp Ser
    1280                1285                1290

Tyr Gly Lys Asn Ile Pro Trp Ile Pro Asn Asp Lys Tyr Glu Lys
    1295                1300                1305

Val Ala Ile Ile Arg Glu Asn Val Asn Pro Glu Asn Asp Phe Glu
    1310                1315                1320

Lys Val Pro Asn Glu Thr Leu Leu Asp Pro Asp Ala Lys Lys Leu
    1325                1330                1335

Tyr Gln Ser Gly Val Gly Ser Leu Leu Trp Ala Ala Thr Asn Thr
    1340                1345                1350

Arg Pro Asp Ile Ser Val Val Val Asn Ser Leu Gly Ser Lys Ser
    1355                1360                1365

Ala Asn Pro Asn Val His Asp Tyr Glu Lys Leu Ile Tyr Cys Leu
    1370                1375                1380

Arg Tyr Ile Lys Asn Ser Met Gly Tyr His Ile Glu Tyr Lys Arg
    1385                1390                1395

Asn Arg Leu Asn Ile Pro Pro Lys Ser Phe Val Ile Glu Cys Phe
    1400                1405                1410

Ser Asp Ala Ser Phe Ala Pro Gly Leu Asp Arg Lys Ser Ile Ser
    1415                1420                1425

Gly Thr Leu Ile Tyr Val Asn Gly Asn Leu Val Gln Trp Ala Thr
    1430                1435                1440

Lys Lys Gln Thr Val Ile Ala Gln Ser Ser Ala Ala Cys Glu Met
    1445                1450                1455

Leu Ala Leu Asn Tyr Thr Met Leu Lys Ala Ile Glu Ile Lys Asn
    1460                1465                1470

His Leu Met Asp Leu Gly Phe Glu Val Gly Lys Ile His Cys His
    1475                1480                1485

Gln Asp Asn Gln Ala Val Ile Lys Val Leu Arg Asn Asn Tyr Cys
    1490                1495                1500

His Pro His Arg Pro Ile Asp Ile Cys Tyr Lys Phe Leu Arg Gln
    1505                1510                1515

Leu Ile Asn Asp Lys Val Phe Ser Ile Ser Tyr Val Lys Thr Asn
    1520                1525                1530

Asp Asn Tyr Ala Asp Cys Met Thr Lys Cys Leu Ser Arg Ala Lys
    1535                1540                1545

Phe Lys Ala Phe Val Glu Gly Met Ile Lys Arg Leu Asp Leu Glu
    1550                1555                1560

Asp Asn Gln Thr Ser Ile Gln Asn Ala Ile Thr Ala Glu
    1565                1570                1575

<210>  3

<211>  1309

<212>  DNA

<213>  Candida albicans

<220>

<221>  misc_feature

<222>  (1)..(1309)

<223>  "n" can be any one of a, g, c or t

<400>  3
ctggataaag aaatcagaaa agagatagca ggaaaaccag gaaaaggtga cgatgatgac      60

gacgacagtt ggggatctgt gcctgtttca attcgagtat ttgctgaagt tgaaaagaag     120

ttgaagcaaa agaaaagttt ggcatcaagc tagatattta tatatgtata tgattagacc 180

aacataaaac tagacgtcca aatatttatt tatttattta ttgatatata ttcttattta 240

ttactgttat gatcttttga ttcacacaga gatttaatcc aaatcaatac cttttgtttt 300

gtagaaatct tttgcttctt caatttgtat tttcaattct ttgtatttat gttctttgtc 360

tttgaatgta acaattcccc aacctaacgt tgataaggca taagacccaa atgtgactaa 420

tccccaccat ggcaagtatg gcaatatttc atcgtgtatt ttagctggag ttggaatcac 480

acctgtgata agagcaaaat aaatagctga taaggcaaaa attgttaatc ctgtttcagt 540

agctttagtc attcttatag ttagacttgt taaagggtag ttgtgttaat tgaagatatg 600

ctggaaaact atacttttcg ttgttttttt ttttcaatct aggtcgggtg tgctgttatt 660

ttttttctct cttcttggtt cttagtattg gattatatgt tggtttatgc gacgtttgtg 720

tcagggaaat aacaccttga tataagtcgt gcgtattagg tcaacattgg tgaaaaattt 780

gcactcatcg agagccagga attagtataa aaagaagaga aaagaaagat atttaggata 840

tttattatat agggaccgag tttcaggaga cacttttagt gggcgtaaac ttcattcact 900

ctgttttttg cttattacaa attatcacct atcgtgtact aggactaatt ctcacgaata 960

ttccgtgtat acaaacactt attgccaact tatggtgcgg aactttattt gtctgaacca 1020

aaatcaaagt cacatcattt aaatgaacgt tgacataaat agattcttta ttcaatagaa 1080

acaatttctt cctttntctt ttctttgtat tantggttag atttccattc catatacaca 1140

caagatgtca acgaaatcag caaattcaac tgctgtcaat tcatttaatg caaaccactc 1200

caactatgac gtttttagac cttcattcac cccagttttg gtcaatacat tcttagtaca 1260

tcttggatta gctacgaaaa acccagatga cactttcact tttgacata 1309

<210> 4

<211> 1340

<212> DNA

<213> Candida albicans

<220>

<221> misc_feature

<223> "n" can be any one of a, g, c or t

<400> 4
cccntttgtn tggtacatgt tagacaggcc caaaaaatgg tatcatttag aactgtatgg 60

agaacattag ttttggtcca acattgcgtg atgatggtat ntntttcgta ttatagtaca 120

atgatggctc aatgattnat tttaggttta tatgtggatg atatcttaat ggacagaatc 180

tcagatggaa tcgttatcag atttgttgaa caagagagag tttatttcgc gtnaaaatca 240

atttaggtct catgacagaa tatgtgagat aaaatgtcca cgtaagcaaa actgggtgat 300

actntgaatt aagagatact cctaaataag caaaccaagg atnttaaact acacaantcg 360

tatggtaaaa cgtgctttga gtnccaaatg atagatgcga gataccaaca aaatagnact 420

gtcgcaaatg ctgaanacaa tttcactgag gttcgaaatg naaaatnact taantcaatt 480

aaaaaattta taccaaaagg tggtctggaa gtgctgatat gaacacgaaa tttaangcat 540

tctgtggaaa attcgtttaa gctcacantc ggaaaatact accattctac atttgcagaa 600

aattaaaatt gtgttgtgaa atatctacat cctacaaagt tcaagacatt tattgatggt 660

atattcaaag gactcgatgt tgagaatgat aataacctga accaagacgc tacaaatgct 720

aattgagtaa ttcgtaattg ctaaacaacg ccatttcgaa tcaggggagt gttggtttat 780

gcgacgtttg tgtcagggaa ataacacctt gatataagtc gtgcgtatta ggtcaacatt 840

ggtgaaaaat ttgcactcat cgagagccag gaattagtat aaaaagaaga gaaaagaaag 900

atatttagga tatttattat atagggaccg agtttcagga gacactttta gtgggcgtaa 960

actncattac tntgtttttt gcttattgca aataatccct atcgtgtact aggactaatt 1020

ctcacgaata ttccgtgtat acaaacaaaa tcagacttct tggtaagccc agccgaaaca 1080

gccatacttc tagtggatct ttctatacta caacattcac actgcttgac ctacaactac 1140

acatattcct tgttataagg gcaatctatc acacaaaaga tttactgttg actcacaaga 1200

tatcaactgt actaataaag gagtgcattc tatgaccttt ggagaggaac tatgtataat 1260

ataagagaga agggactaaa gatctatata taatgagcag gatgggtaac ccggtggggt 1320

attagcacgc acacgacctg 1340

<210> 5

<211> 556

<212> DNA

<213> Candida albicans

<400> 5
caacattggg tgaaaaaatt tgcactcatc gagagccagg aattagtata aaaagaggag 60

aaagaaggta tttaggatat ttattatata gggaccgagt ttcaggagac acttttagtg 120

ggcgtaaact tcattcactc tgttttttgc ttattacaaa ttatcaccta tcgtgtacta 180

ggactaattc tcacgaatat tccgtgtata caaacattat acgtgtctgt aactacgcga 240

aactacttcg tctcagtttt ttgttacaaa caactttccg tatagacctg agattttgtc 300

agcttgattg aatggaagag tttactaaag taccagaaag gtgttttata gataacatgt 360

agatatataa aaatgttata ttacaaatga cttccaaaag aaactgtacg aattttgctg 420

tttattaaaa accagttcct gaaaactagt atcttagctt cagtacattt agcccaccta 480

aattggacct atgacaagtt ctactttccc gacaatgcta atatagagca gtttcttctt 540

cttcttcttc ctcgtc 556

<210> 6

<211> 2112

<212> DNA

<213> Candida albicans

<400> 6
atttaatatg ttggtattgg ctactgccaa cttcttagct gatgcagatg ccattgttaa 60

tattgttaaa ttgggtaaat agtatgaagg aagctttggc aggcgttgtt atttttttca 120

ccaattatta tcatcacctg cggaggttag tcaatttgag attgtgcgag ggaaaaaaaa 180

cgacctccat acactacctc aagtataagt ccagtccaat tgttcgctat agagagattt 240

cctagccgga atgcacgaca atcctgagac ggaagtcgat cgtcgatgcc catggtgcgt 300

ggtgaaaaat tttcttagaa aatttgttct ttccttcaac tgctttgaag agagggaggt 360

tcaagtggtt taagtacgac ggtcacaaag attgcggctt atgaggcccg aactgagttg 420

aaatacaaaa tcaagatata attatatacc ttacttgtct atattgtttt ataatacatt 480

cttcagatat ttaaatttct gtgtatcatc ctataaaaca gagatacatt cagtgcattt 540

agtatactga gtgaactggt acctgtgaca ttcaagataa ctgtttcacg cacgctggca 600

gacgaacacc aatagtatga tgaagaactg accatggtgt aagaggtttg atggagtttc 660

ttttttttag aagaggttga taagccaaca gatgaggagt aacaagtaac tcgcaacatt 720

gtataacata agtttacatc aaatcagaat ttactaagaa aatcaatcca ttcaaaaggc 780

actcaatcat tgaaaaaacg agcttaatga gtagacggtc tgttcatatg aaacaattga 840

aagggttgaa tattgtttgg aaaattatat aattcatgtc aaactgggag gcttaaatta 900

tggtcactcc acagattatg aaacgtagtt acacaattct tggacctgga aatcccacaa 960

gagagcgtta gttagtttgc actctcctca ccagttaaac tacccatgat tctccaatgt 1020

ggcttattta agtatcagac aacagataca tggtttccaa gtggtctcat ttttggttta 1080

ctggagtctg cattccccac aaaagtacct ttcaaaacta attaatgtag cttctatttg 1140

atagcctctg ttatggaaat agatttgctc tgcccagtgg gtgtaattat tcccagctgg 1200

aactattccg atagatatgt tttaatgtca atttaaatct tgtaataata gtaaggatgc 1260

ggtttatccg cgatcttctt aatacctgtg gagttactcc agaacagagg ttcaattttt 1320

tcttggttgg taaattatcc gagtaacacg gggtagcttg gttactccag ttgagaatgt 1380

aaactataga tgaagatttc aacacgcaat tattacccca ccttggcgaa ttactaatcg 1440

actatttgtt aatccagaaa aaattataca caaacactgc ctttttttaa aaaaagcgtt 1500

attttgatgg aacgataatt aacgatggtt ctgcacaaaa atgtggtcca aagccccaga 1560

ctattctgaa gtatgatttg ttacttaatt tagtgaataa ttaaacataa aatctggaga 1620

aaaatttttt ttttgctctc atgaccagtg gcaaattctt ggtaacgagg cttaacatta 1680

atccgcaaat tacctggcaa cagagaaaac acccagaaag ttctgtcgta tgagaaaacc 1740

tacagttgtt tccgatttct ccgagcacta aacataaaga gaccagtaat gctaaaaaaa 1800

tttttatttc tgcattactg tttttagcaa atacacgtct aatttattgt atttgttaaa 1860

cattcttttc ctgaaatttt aagaaaatgt tttggtttgt tggaattcca tttaaacggt 1920

actttggggt gcagacagca atccatttgg agagtggcaa gtctacacga atttagctaa 1980

ggttcactat atcgtgtaac aagaaatttc tataccaaat aaacagcact tgattgaact 2040

acaatatgta aaaacttgct tttattacca gtcttcatac ataccccggt cttctctttt 2100

caatattctg ta 2112

<210> 7

<211> 3742

<212> DNA

<213> Candida albicans

<400> 7
ttagaaaaca ggaaacagca atagagagca ataattgaaa aatagtgttg tcaacaatag 60

aacaaattgg tcaaacttta aatgcaaaac atgaaattcc caatttccag aataaataat 120

atcagcatac atggccccga aaactacttt accgtgtcgc tttaaccccc cccttcctaa 180

aacgagacaa ttagacatac attccacaat tatcataatc cccttttttt tccttacaaa 240

acactttatt tttgtcgttt tcgttatttg cttcgacgac attgtaaact ctttggattt 300

gcagtagtag tgctcctggt gtaaggtggg tttggttgta gagtaaaaga aacgacaatt 360
gattacacct cgatatgcat acgcatggca aagagaatac cgagttaata gtgagtctat 420
tagtgttgca ggaaaagtta tacgaacaac attttgttta gtgtggatat tccagatcaa 480
caacaatatg actaaaatca tagctctaat tttcagttta cctttgttta ttacgatact 540
gccacagtcg tgctgtacca gggtcagttt tagaaaaact attctagaaa tgatgagtag 600
aaatgtacta ttatgagcaa tatttcaaaa agtgaaatta taattgctgc tgacaacacc 660
aacaatacat acaaatttgg aaacgagcaa atcgagaaaa tttcaatccg tttagcaagt 720
tgttcgttgt cgtcattgtc gattagtttc agtttctaga ggtgaaattt tctatggcac 780
caaaaccaaa gcctcaattt taatttactc tgtgtggtac aaaatacatt agagaggatc 840
ctctccaaac aggattgcag gaagttttac acgagaatga tttactacac gacgttgaat 900
taaaaagctc aaccagtttg tcagcaattt tgttctatct gttcaatttc ttgtataaaa 960
taaagcaata tgagagagca tctaaatcaa taatgtcaac acaatattaa actttgagaa 1020
ggattgttca acaaaacaat ccgatgaata gaagaagaat aatatcaaat tgttcctgat 1080
tgattgttgt tatttatttt ttatctccga attcctgcac aatggctcaa caacagccaa 1140
cacggatcac acattaaatt tttttttcgt gcaggacccc gtggtggtgg ctgtggctgt 1200
gattgtgatc attgtagttt ctgccttgat gatgacaaaa aatgatagag ttcagtatga 1260
ggaagaaatt aagcgatatc ggtttatgat gtgtttagtt attaattgct ctcaatggtt 1320
ttcaacaacg tatacaaaac tggtggtgct tgaaacgaat gagtaataca gatctaatta 1380
agctgtgatt ttctaagttt gccttgtctc tacagttcaa aaaaaaagaa cagaacacct 1440
cagaggctgt tgtgatgcaa tttttaggaa cctcaacaac aaccactgac tgatctaagc 1500
cagcatctgt ttaatgggtt ttcaaaaaga atggggcaaa cggggaattg aaccccgggc 1560
ctcctcgaat tttgtgtttg gtgaacaacc caaacgagga atcataccac tagaccattc 1620
gcccaattcg atgacttgga attattctag ttatttttga catacaaagc tcagctttat 1680
tacagatagt catgtttgca tggatgaatt agtactacta ataatataag aaaactagtt 1740
aattggagtc aatgtcttat acatgtcttc tgatgggtta tgcattgatt aattatgaat 1800
ttcttttaaa tacaatctat tgctattatt tgtatgtaaa actttaccca aaaaccaaca 1860
aaaaagagtg gtcttggata aagattaaag taattccaaa aagatttggt aattagctat 1920
attgttttga cgtacatcta taactacaaa tagccattca gtttgattat gtatattgac 1980
atagttggat ttgtaatttc tgttaaaatg gaaaacccta atcaaatgta tatgttgaat 2040
aggtagttaa attgtacaac ctactacttg ttgtcaattg aattcagagc caatacttat 2100
atctcctgga aactgataca caaacgaatt gttaaactat aacactcgac gttcacatct 2160

aaggattcat cgtcgttaag atttatactc attagcaaac tcacttgcca tattaaacac 2220

ttctcaatct atttcccaca atccaattaa tcagcacgaa aactaagata ctatatatat 2280

ctgcctatac ctgatataca catggcacat ggcgtatccc acaaaaaacc gtcaagacaa 2340

caccaatatg acaatgccaa ttatacaatt gcatatacca cgtgacttca ttttatggtc 2400

atgagaaatt aacttatcat ggggttaggc gagaatatca actgttcgct atagagagat 2460

ttcctagccg gaatgcacga caatcctgag acggaagtcg atcgacgatg cccatggtgc 2520

gtggtgaaaa attttcttag aaaatttgtt ctttccttca actgctttga agaaagggag 2580

gttcaagtgg tttaagtacg acggtcacaa agattgcggc ttatgaggcc cgaactgagt 2640

tgaaatacaa aatcaagata taattatata ccttacttgt ctatattgtt ttataataca 2700

ttcttcagat atttaaattt ctgtgtatca ttctataaaa cagagataca ttcagtacat 2760

ttagtatact gagtgaactg gtacctgtga cattcaagat aactgtttcg cgcacgctgg 2820

cagacgaaca tcaacactga tcatttgttt tttttttatt tctccttttt ctcctttttc 2880

tttctttttt cttctttctt cagacgttgt tgatttattt tatcgacagc atccttttct 2940

ttggccacat atccaagcga tatactggcc aaagcgaagt ccttttataa agcaatgcta 3000

ccaaatgtaa cagttcgagg tcagaagatt aagcgggtat gttcacacgg atattttatg 3060

gggtatcact tgtaccaaac actttgatac gataagaata tttgtaatac taacttcagt 3120

gtctttcata atcagctcat aacctgttgg aatttaaatt cgtatgttgt tcattcaaaa 3180

ttttgataaa tgggacgaga aatcatcgtt gcctcctaat tagattatga cttagtacta 3240

actaaactgt ttatcatttt ttaaagcgtt gggctccatg ttagaataga ttattagggc 3300

ggtacgtatt tcataattta tatataggta cttattttta ctaatttatt gcacaggaaa 3360

agataaaagg tatcgattat acctatcagc aaggtttaag caaaatgaag tatttttacc 3420

atatttttcc atttttatat agatacatca agaggtttat tttaagttca cctggataaa 3480

ccattcaact aacccaattg aattgaatga caatttgatc tccaaagagg gattcatttc 3540

tattctggag agataaacgt cattgtttag gaaagagcaa gagataagaa atcttttgta 3600

tattgtatat atattattaa tgttatatta cactattgtt tgtttgtttg ttataattat 3660

atgtgagatt tcatatgtaa gatgttgtta tctctttcca ttatttagct tttttgaaaa 3720

agctatcaat ggctccacgt tt 3742

<210> 8

<211> 1438

<212>  DNA

<213>  Candida albicans

<220>

<221>  misc_feature

<223>  "n" may be any one of a, g, c or t

<400>  8
gtgtagatgc aataggtgta tgaaatgtat ctagattata tcatgaagcc cttgccaata     60

aaatctagcc aaaaatttgt gtactgcaat tgttcgctat agagagatat cctagccgga    120

atgcacgaca atcctgagac ggaagtcgat cgtcgatgcc catggtgcgt ggtgaaaaat    180

tntcttagaa aatttgttct ttccttcaac tgcttttaag agaagggagg ttcaagtggt    240

ttaagtacga cggtcacaaa gattgcggct tatgaggccc gaactgagtt gaaatacaaa    300

atcaagatat aattatatac cttacttgtc tatattgttt tataatacat tcttcagata    360

tttaaatttc tgtgtatcat cctataaaac agagatacat tcagtacatt tagtatactg    420

agtgaactgg tacctgtgac attcaagata actgtttcgc gcacgctggc agacgaacag    480

caattctgta attgtcgtag agtagcaaca aatcttcccg atgattggta cttgtgttag    540

tctacacgac atgtgttttg gtacacttga actgtatgtc caagaatgga aacatatgcg    600

ggaaggacgc gaaagatgag tttggtatag aagggataag aactgtaaaa tatattatgt    660

agttatatat tttaattatg ggaaattgag tgtttattct gttcaacaag tttcaaccgt    720

agagattaca tttaaagtct gtggtcgaaa tccacaagat acagcaaatt catgaattca    780

cctatttaaa tcaagtttac caagcaccat tgcctagaac ttgccatatc atcaattaag    840

tcagacatta ctaatttgag caaagctttt agcttaatgg gccaactaat ttaagtcgaa    900

ttggtaatgc aatctgttct tcatttgagt cgcttgctac ggctccatga cacatccatt    960

tgattgtttt aattcgagca attatccacc ataactctca gtaatatcat taacagtttt   1020

acgcttaata agcatagaaa gttgtatgaa gttgtctcct aggtatgcta gagagatttg   1080

tatatacgac cagtaaagag tgtgatgagg tgtttactgt agggtaaatt gcaattgact   1140

tgagttgata gcggttatta caaaagtata gattcaacaa attaagacaa gtaccaaacg   1200

ataggccgaa tgtgacttat accgttgaag ttcaagcgtt tttaacaaat agaaatgtga   1260

gattaatgag ttcgacaaat gttttactag atactattaa tttcgatgta ctatataagt   1320

ttaaccagct ataaccggca gagcagactt cctgaaactc aaattggttg tgtttggact   1380

tgagttacac cacaaagttt gacaatcgtg aggacatagc aacctatcaa gccactca     1438

<210>  9

<211>  1304

<212>  DNA

<213>  Candida albicans

<400>  9
tgaagatctg gctttggcca aagtatcagc tgcattagat actgtcattg gcattggctt      60

gaacccactg gctgtggatg taactgtgga gccaaaagct cgtaaagctt tggcgttcat     120

ggagaaaaat cttttaacag acattgtata aacgttgaag attaaagaaa aaaaaaacag     180

aaagattacg aataatttgt ttttaattgg tgggtatgag gtgttgcgca gtcgactcaa     240

caattctctt ttggtgcaca aagttggttt tatggtcaac aattacggag tactgtctgt     300

agtgatgttg aatctaagac ggaaatgcct cctttacatt tgtttctatt ctcttaaaat     360

acatattcaa ttgtgtgttt taattgaaaa tttgttcatc ttcatctgat gattgtgtaa     420

tctttgcggg gggggggcgt gtcatgaacc aatctctttg agtcatagga cgagtcatcc     480

tattgtgact catggctcat cttactctct tactaatctc ttacttcatc tgtttactat     540

aaatatgtct actactcctc tattttatta cctcgtttac tatttttatt caatatatga     600

tcttatcttt aaatttcttt tgacaaatac aatcaactta caaaacaaaa gaaaaaagac     660

taataaaata gaattaatga aaaaaaaaaa agactaataa aagaaaaaga aagaagacta     720

acaaaagaaa aaacaaaccg gagaaccctt cgctgtagag gaatttccta gccggattgc     780

acgacaatcc tgagacggaa ttcgatcgtt gatgaccgtg gtgcgaggtg aaaagttttc     840

gtagaaattt tgttctctct ttcaaactgc ttttaagaaa atgaggttca agtggtttaa     900

gtacgacggt cacaaagatt gcgacttatg aggaccgaac taagttgaaa tacaaaatca     960

agatataatt atatacctta cttgtctata ttgttttata atacattctt cagatattta    1020

aattcctgtg tatcatccta taaaacagac atacattcag tacatttaat atactgagtg    1080

agcttgtatc tgtgacattc aagatatgtt tcgcgcacgc tgacagacaa acatttggtt    1140

gtaaaaaaaa aaatattgaa gaacctcatc accaagatgt ttgaaaaaaa aaaaaatcaa    1200

atacttaatc gcaagctttt caatttattg attgtttgaa ttaattgaat ataaacaaaa    1260

aaaaaaagaa ttcaaattca tttgacatgt cagtggaagt taga                     1304

<210>  10

<211>  3604

<212>  DNA

<213>  Candida albicans

<220>

<221>  misc_feature

<223>  "n" may be any one of a, g, c or t

<400>  10
agccccaaaa tggttttcct agnggaggat ggaatggatg ggaccaccca ccaatttggt      60

tcccggaatt tggtttaaaa aaaagtttac ggggatgatt tatttccaaa cccagatgtt     120

tcctgctgct gaaagaattg gaaaagctct tttcagtnac aatctaactg agagaacttg     180

aaagggatca gcatttttgt tatgtcaaca tttaatgacc aatgaccacc agcacgatga     240

tattattctt aaatttctcg ttagcggtgt ctcaccatgg tacttacatc tgcaaattta     300

catgctgtca tataaacttg gattctcaaa tttgttttta gagatttatg ctcaacatta     360

tgaattgtat aaagcagatc ccatttacaa attgccagat agtatgacat tgttgaatga     420

aataagatca aatagagatt atcctaaagt ggtaaatgct gcaaaaaata cagtacaagt     480

caataatgtt tcatccaaga acaataaaaa gaaggatgaa tgacaacaat tagccaataa     540

aattgaggaa gtaggacgtt atagcgaaat aaacgcaaca tctacatatc atgaaattgg     600

cgataccaac aaaaaccaaa ggacaattaa tattgaattt gaaaaatcat acaaaattaa     660

gtgaacaaaa gaagaaaaca aacctattgg tatatgatct gggagccaca gtatccgtgg     720

tgaatgataa gactttactt aacgacatta aagaatcaaa tatcgaaatt gcaactgctg     780

aaggggagac atctacggct tatgctttag gtactctaac catatctgtg aatggattga     840

atgcgaaatt agatggtgtt ctatacttgc catctattca attaaactta atatctataa     900

aacaatttga agatttatgc tacgcaattt tgatttccga aaatttaatg tttctagttc     960

acagtgacca cgaacctacg gtcattgcga aatattcacc taaagatgac ttatactcag    1020

gcccaagatc gggaaacttt cttaagaaga atcataatga acaaaaccaa attttgcttg    1080

acactgctaa aaaactatta ggatcagaga acatatttct ggagaaatca ctgaaaaatc    1140

caatgattga tcaaggaaaa ttagatccgt tgaaaatgaa caataaagta gaaagagtta    1200

actatgtcag catacacaac atcaaacaag aagtggcaga caaatatatg ataaaagatc    1260

tttactacta tcatttatta attaatcacc tttcacatga aaaactacaa ttattagtaa    1320

aaaggggagt gattaaacca gtcaaatcta cttcggctga gtcggccatt ttaaattgtc 1380

agatatgtgt tgcagcccat gcaaaattag ctagccataa tcacactcaa caacgggaat 1440

tggagcgacc attacaacgc ctccatttgg ataccgccgg accatttacc tcaaataaaa 1500

ctaagagcta tcttacaacc gtgattgatc aattttccag atatactgaa gttattgtat 1560

ctgacaccaa agcagtcaaa caaagcatat tgcatagact tagggtctgg aacaatagat 1620

ttcagtttaa gatcgcggag ataagatatg ataatgcatt ggagtatcca tcggctgagg 1680

agttagagga gttaggaatt tataaacacc ttctcccaaa ctactctcct atgcttaacg 1740

gtacagctga agcaaccaac cgccccattg tccaaggtat ttataaggta gtgttaaatt 1800

ttagttgtca agtattaata cttttcccat ttatagtgga gtatgcggtt catatccgga 1860

atcatacacc tataaaagaa tttgatggtg ctactcctta tgaacgttac tatggtttat 1920

ctaaatacgt cataccattt tttcagtttg gaaccgacgt tttgataaaa tgtgctagtg 1980

tacaagaagc tatttcatta aaactaccat cttcaagaga taaagctttt cctacagtga 2040

tgtttggtgc ttttctcggt tacggctcag attcctttac cttcagagtt ttagtttcca 2100

cgaaaggata tccagttatt acaacatcaa acatccgtcc aatagcgacg atgcaagtac 2160

tcaatgacta tttggcatac atatcggaga atagctcaat aagctatgac gatacattct 2220

tatcaccttt gaatcaccca atgattcgca caaaccaaca tgatagacgt ggagacaata 2280

taaatgtcga atatgaaaac cgtccaaatg taccatttga atatcatgct gaacctcctc 2340

gtacaaattc atcgacggga attatcgatc gaccagatat tagacctaga gctgatccca 2400

cctggcaacg tatgcctgat gccaacatac atcaggaaac aacaactgta cagactcctg 2460

atcatgggga gttagatacc atgatcaaca acgaacacca actaccacga tctggggagg 2520

gtaattaccc cgggcaacag gtgcgcaccg atattattgg gcaatttcga gatcgcgggc 2580

ctaccactct aaacactccg atcgatctag gtgtacccga tgaaacagac gatattagta 2640

tgacatcaga gaatccaatt gattccccaa attccgagat gatcatatcc ccatctttac 2700

ccacaaatga attggaacat caaatcgata tcagttcagg ggagatgtcg ttattgcaaa 2760

cgaatatgga agcagataac gaattgaaaa caaatgaaat ggtattatac aaatcaaaaa 2820

atgatggtat tatcattcaa caacaacaat tcactgaaaa tttgtcagat gaaaatgaag 2880

aagattcatc aacagatgag gaaacattgg aagacaaaaa acaacagcga ttggaatata 2940

atatttcacc aaacgatgag tggataaata atgacgttca gaacgaagat gacacacaag 3000

tgccacatgt taaggaacca atcaattatg aaactcaaag tagaaatgga acaaacatgc 3060

cacgaattga aatgggcata atagaaaact taagtgatga tggaaagaat acaccacgtg 3120

aattacgtat ggtcacctac gataataata aaaaaattca aaagtaccaa aacagtaata 3180

tcgagatcct ggaacccaga aacgaaaata aaaaccacac attcattgaa agcaacttag 3240

aattacttga caatcaagaa atgtttcaag aagatcctca agttgaagat attcgattga 3300

caactccaaa aaaggacaaa tcgttatcac ctgatttcaa tcaaacccat aatgaaatac 3360

aactattcat ggcagatatc aatgaagata tgctagaaga atatgatgaa aatataaata 3420

tgaatgaagt gttagctgac tccacggaga cgttggacaa agaattagat ttagatgaag 3480

aaagtggaag gatcgaatat attgctgata gagttagaaa naagacagag gtactgatgg 3540

tgcgccacac ggggaattnt ttacagaaaa atggataaag atttttgggt ccattaaaaa 3600

ggcc 3604

<210> 11

<211> 995

<212> PRT

<213> Candida albicans

<220>

<221> Misc

<222> (1)..(995)

<223> "X" may be any amino acid

<400> 11

Met Lys Leu Ala Ile Pro Thr Lys Thr Lys Gly Gln Leu Ile Leu Asn
1               5                   10                  15

Leu Lys Asn His Thr Lys Leu Ser Glu Gln Lys Lys Lys Thr Asn Leu
            20                  25                  30

Leu Val Tyr Asp Ser Gly Ala Thr Val Ser Val Val Asn Asp Lys Thr
        35                  40                  45

Leu Leu Asn Asp Ile Lys Glu Ser Asn Ile Glu Ile Ala Thr Ala Glu
    50                  55                  60

Gly Glu Thr Ser Thr Ala Tyr Ala Leu Gly Thr Leu Thr Ile Ser Val
65                  70                  75                  80

Asn Gly Leu Asn Ala Lys Leu Asp Gly Val Leu Tyr Leu Pro Ser Ile
                85                  90                  95

Gln Leu Asn Leu Ile Ser Ile Lys Gln Phe Glu Asp Leu Cys Tyr Ala
            100                 105                 110

Ile Leu Ile Ser Glu Asn Leu Met Phe Leu Val His Ser Asp His Glu
        115                 120                 125

Pro Thr Val Ile Ala Lys Tyr Ser Pro Lys Asp Asp Leu Tyr Ser Gly
    130                 135                 140

Pro Arg Ser Gly Asn Phe Leu Lys Lys Asn His Asn Glu Gln Asn Gln
145                 150                 155                 160

Ile Leu Leu Asp Thr Ala Lys Lys Leu Leu Gly Ser Glu Asn Ile Phe
                165                 170                 175

Ser Glu Lys Ser Ser Lys Asn Pro Met Ile Asp Gln Gly Lys Leu Asp
            180                 185                 190

Pro Leu Lys Met Asn Asn Lys Val Glu Arg Val Asn Tyr Val Ser Ile
        195                 200                 205

His Asn Ile Lys Gln Glu Val Ala Asp Lys Tyr Met Ile Lys Asp Leu
    210                 215                 220

Tyr Tyr Tyr His Leu Leu Ile Asn His Leu Ser His Glu Lys Leu Gln
225                 230                 235                 240

Leu Leu Val Lys Arg Gly Val Ile Lys Pro Val Lys Ser Thr Ser Ala
                245                 250                 255

Glu Ser Ala Ile Leu Asn Cys Gln Ile Cys Val Ala Ala His Ala Lys
            260                 265                 270

Leu Ala Ser His Asn His Thr Gln Gln Arg Glu Leu Glu Arg Pro Leu
        275                 280                 285

Gln Arg Leu His Leu Asp Thr Ala Gly Pro Phe Thr Ser Asn Lys Thr
    290                 295                 300

Lys Ser Tyr Leu Thr Thr Val Ile Asp Gln Phe Ser Arg Tyr Thr Glu
305                 310                 315                 320

Val Ile Val Ser Asp Thr Lys Ala Val Lys Gln Ser Ile Leu His Arg
                325                 330                 335

Leu Arg Val Trp Asn Asn Arg Phe Gln Phe Lys Ile Ala Glu Ile Arg
            340                 345                 350

Tyr Asp Asn Ala Leu Glu Tyr Pro Ser Ala Glu Glu Leu Glu Glu Leu
        355                 360                 365

Gly Ile Tyr Lys His Leu Leu Pro Asn Tyr Ser Pro Met Leu Asn Gly
    370                 375                 380

Thr Ala Glu Ala Thr Asn Arg Pro Ile Val Gln Gly Ile Tyr Lys Val
385                 390                 395                 400

Val Leu Asn Phe Ser Cys Gln Val Leu Ile Leu Phe Pro Phe Ile Val
                405                 410                 415

Glu Tyr Ala Val His Ile Arg Asn His Thr Pro Ile Lys Glu Phe Asp
            420                 425                 430

Gly Ala Thr Pro Tyr Glu Arg Tyr Tyr Gly Leu Ser Lys Tyr Val Ile

435                 440                 445

Pro Phe Phe Gln Phe Gly Thr Asp Val Leu Ile Lys Cys Ala Ser Val
    450                 455                 460

Gln Glu Ala Ile Ser Leu Lys Leu Pro Ser Ser Arg Asp Lys Ala Phe
465                 470                 475                 480

Pro Thr Val Met Phe Gly Ala Phe Leu Gly Tyr Gly Ser Asp Ser Phe
                485                 490                 495

Thr Phe Arg Val Leu Val Ser Thr Lys Gly Tyr Pro Val Ile Thr Thr
            500                 505                 510

Ser Asn Ile Arg Pro Ile Ala Thr Met Gln Val Leu Asn Asp Tyr Leu
        515                 520                 525

Ala Tyr Ile Ser Glu Asn Ser Ser Ile Ser Tyr Asp Asp Thr Phe Leu
    530                 535                 540

Ser Pro Leu Asn His Pro Met Ile Arg Thr Asn Gln His Asp Arg Arg
545                 550                 555                 560

Gly Asp Asn Ile Asn Val Glu Tyr Glu Asn Arg Pro Asn Val Pro Phe
                565                 570                 575

Glu Tyr His Ala Glu Pro Pro Arg Thr Asn Ser Ser Thr Gly Ile Ile
            580                 585                 590

Asp Arg Pro Asp Ile Arg Pro Arg Ala Asp Pro Thr Trp Gln Arg Met
        595                 600                 605

Pro Asp Ala Asn Ile His Gln Glu Thr Thr Thr Val Gln Thr Pro Asp
    610                 615                 620

His Gly Glu Leu Asp Thr Met Ile Asn Asn Glu His Gln Leu Pro Arg
625                 630                 635                 640

Ser Gly Glu Gly Asn Tyr Pro Gly Gln Gln Val Arg Thr Asp Ile Ile
                645                 650                 655

Gly Gln Phe Arg Asp Arg Gly Pro Thr Thr Leu Asn Thr Pro Ile Asp
            660                 665                 670

Leu Gly Val Pro Asp Glu Thr Asp Asp Ile Ser Met Thr Ser Glu Asn
        675                 680                 685

Pro Ile Asp Ser Pro Asn Ser Glu Met Ile Ile Ser Pro Ser Leu Pro
    690                 695                 700

Thr Asn Glu Leu Glu His Gln Ile Asp Ile Ser Ser Gly Glu Met Ser
705                 710                 715                 720

Leu Leu Gln Thr Asn Met Glu Ala Asp Asn Glu Leu Lys Thr Asn Glu
                725                 730                 735

Met Val Leu Tyr Lys Ser Lys Asn Asp Gly Ile Ile Ile Gln Gln Gln
            740                 745                 750

Gln Phe Thr Glu Asn Leu Ser Asp Glu Asn Glu Glu Asp Ser Ser Thr
        755                 760                 765

Asp Glu Glu Thr Leu Glu Asp Lys Lys Gln Gln Arg Leu Glu Tyr Asn
    770                 775                 780

Ile Ser Pro Asn Asp Glu Trp Ile Asn Asn Asp Val Gln Asn Glu Asp
785                 790                 795                 800

Asp Thr Gln Val Pro His Val Lys Glu Pro Ile Asn Tyr Glu Thr Gln
                805                 810                 815

Ser Arg Asn Gly Thr Asn Met Pro Arg Ile Glu Met Gly Ile Ile Glu
            820                 825                 830

Asn Leu Ser Asp Asp Gly Lys Asn Thr Pro Arg Glu Leu Arg Met Val
        835                 840                 845

Thr Tyr Asp Asn Asn Lys Lys Ile Gln Lys Tyr Gln Asn Ser Asn Ile
    850                 855                 860

Glu Ile Ser Glu Pro Arg Asn Glu Asn Lys Asn His Thr Phe Ile Glu
865                 870                 875                 880

Ser Asn Leu Glu Leu Leu Asp Asn Gln Glu Met Phe Gln Glu Asp Pro
                885                 890                 895

Gln Val Glu Asp Ile Arg Leu Thr Thr Pro Lys Lys Asp Lys Ser Leu
            900                 905                 910

Ser Pro Asp Phe Asn Gln Thr His Asn Glu Ile Gln Leu Phe Met Ala
        915                 920                 925

Asp Ile Asn Glu Asp Met Leu Glu Glu Tyr Asp Glu Asn Ile Asn Met
    930                 935                 940

Asn Glu Val Leu Ala Asp Ser Thr Glu Thr Leu Asp Lys Glu Leu Asp
945                 950                 955                 960

Leu Asp Glu Glu Ser Gly Arg Ile Glu Tyr Ile Ala Asp Arg Val Arg
                965                 970                 975

Xaa Lys Thr Glu Val Ser Met Val Arg His Thr Gly Asn Xaa Leu Gln
            980                 985                 990

Lys Asn Gly
        995

<210>  12

<211>  1249

<212>  DNA

<213>  Candida albicans


<400>  12
tctctatgta ggctgacagg tgaaaattat gaattaattg cattggccaa tgacaaatga      60

atagacaaaa cagcaaataa ggttgcaaaa gtagcccaaa caaactagat ttcggttacg     120

aattttccat ctttcaaaac aatgaatttg tttagagctc tgtgccattt attgcaacta 180

aaatgaatat gcaattaaac aatcagagat gtattggatt atccccgtgg tatacttttg 240

agttcaccat ttgttttttt tttgggttta aattagtgct cctactaaaa atcgcattta 300

tcttacactc accattttga taagttatct ctggtcaatc gcaaatacta tgcttctaat 360

taagagttct atgtaaatcc catttaattt tgatcaatct attggtttga agtaagagtt 420

gattttctgt aaagatttat ttggccagtg tagttcggtg tcaaaaatat attatgatgt 480

acactaaaaa acactaaatt tcaagtcaat ggggaacaca aaactgaatt aattactata 540

tgttggtttg tgcactattt tgtgtcagaa actgatcaat gaaaatgatg gttattatga 600

gaatggaaaa tttttccatc acacatcagg tgatgacaga actaaactat attgtgtagt 660

ataaataagg gtatgaaata ccaacatccc agaatatcaa cgagatagaa gagaggagtt 720

tcaatatata tcttgtgaat aataacttcg ttctaattca ctatacacaa ctagacgtgt 780

acacgctcaa tctcaggtaa agaaagttta tattccatca ctatataaca acaatcaggc 840

tttgcaaaaa aacatttaaa actaatactg gtaatatgga aatataacgc ctcgtagttc 900

tacgcacgtg gcatccttta tctatttatt caatttaccc ctaatttatg aattagctta 960

ataagagcag tcaaattaac acggctcaat taatagtact taataatatg aagccgatca 1020

attaaccgat cctttgaata atttgaaaat aaaataaagt aatataaata ggtatgcatt 1080

ttccctacat ttatttcctc tttctatttt aatttgtttc ctaaacagca acaacaacaa 1140

ttgaaattca aaaatggttt ctgtttctaa attattgaac aatggattgt tattagctgg 1200

tcaaagtgtc ttccaagatg ttgctactcc acagcaagct tctgtgcaa 1249

<210> 13

<211> 5611

<212> DNA

<213> Candida albicans

<220>

<221> misc_feature

<223> "n" may be any one of a, g, c or t

<400> 13
tctctatgta ggctgacagg tgaaaattat gaattaattg cattggccaa tgacaaatga 60

atagacaaaa cagcaaataa ggttgcaaaa gtagcccaaa caaactagat ttcggttacg 120

aattttccat ctttcaaaac aatgaatttg tttagagctc tgtgccattt attgcaacta 180
aaatgaatat gcaattaaac aatcagagat gtattggatt atccccgtgg tatacttttg 240
agttcaccat ttgttttttt tttgggttta aattagtgct cctactaaaa atcgcattta 300
tcttacactc accattttga taagttatct ctggtcaatc gcaaatacta tgcttctaat 360
taagagttct atgtaaatcc catttaattt tgatcaatct attggtttga agtaagagtt 420
gattttctgt aaagatttat ttggccagtg tagttcggtg tcaaaaatat attatgatgt 480
acactaaaaa acactaaatt tcaagtcaat ggggaacaca aaactgaatt aattactata 540
tgttggtttg tgcactattt tgtgtcagaa actgatcaat gaaaatgatg gttattatga 600
gaatggaaaa tttttccatc acacatcagg tgatgacaga actaaactat attgtgtagt 660
ataaataagg gtatgaaata ccaacatccc agaatatcaa cgagatagaa gagaggagtt 720
tcaatatata tcttgtgaat aataacttcg ttctaattca ctatacacaa ctagacgtgt 780
acacgctcaa tctcaggtaa agaaagttta tattccatca ctatataaca acaatcaggc 840
tttgcaaaaa aacatttaaa actaatactg gtaatatgga aatataacgc ctcgtagttc 900
tacgcacgtg gcatccttta tctatttatt caatttaccc ctaatttatg aattagctta 960
ataagagcag tcaaattaac acggctcaat taatagtact taataatatg aagccgatca 1020
attaaccgat cctttgaata atttgaaaat aaaataaagt aatataaata ggtatgcatt 1080
ttccctacat ttatttcctc tttctatttt aatttgtttc ctaaacagca acaacaacaa 1140
ttgaaattca aaaatggttt ctgtttctaa attattgaac aatggattgt tattagctgg 1200
tcaaagtgtc ttccaagatg ttgctactcc acagcaagct tctgtgcaac aatataacat 1260
cgtcaattct cttggcggta gtgcccctta tattcaaaga aacggatatg ggatttctac 1320
tgatatccct gctggttgtg aaattgctca aattcaattg tattcaagac atggtgaaag 1380
atacccaagt aaaagtaatg gtaaaagttt agaagcaatt tatgctaaat ttgaaaacta 1440
caaaggtact tttaaaggtg atttggcttt cttaaatgat tatacttatt ttgttactga 1500
taaaaacaat tacgaaaagg aaactagccc aaaaaattct gaaggaacct atgccggtac 1560
aaccaatgcc ttgcgtcacg gtgctgcgtt tagagccaaa tatggatcct tatacaagga 1620
aaattcaaca ttaccagttt tctcttccaa ttcaggtaga tgttaccaaa cttcaagata 1680
ttttgctaga ggatttttag gtgatgactt taaagaaggt aaaactgtca agtttaacat 1740
catttctgaa gatgctgatg ttggtgccaa tagtttgact ccaagaagtg catgttccaa 1800
gaacaaagaa cggagcagta gtactgccaa aaaatataac acaacatatt taaatgctat 1860
tgctgaaaga ttagttaaac caaacccagg tttgaatttg actacaagtg atgtcaacaa 1920

tttattcagt tggtgtgctt atgaaatcaa cgtcagagga agttcaccat tctgtgattt 1980

attcaccaat gaagaattca ttaagaactc ttatggtaat gatctttcca aatattattc 2040

taatggtgct ggtaataatt acaccagaat cattggttca gtgattttga attcatcctt 2100

ggaactttta aaagacaccg agaactctaa tcaagtatgg ttatcatttg ctcatgatac 2160

tgatttagaa attttccatt ctgctttagg attattggaa ccagctgaag atttaccaac 2220

atcttacatc ccattcccta acccatacgt ccattcttct attgttccac aaggtgccag 2280

aatatacaca gaaaaacttc aatgtggaaa cgatgcttat gttagataca ttatcaacga 2340

tgctgtcgtg ccaattccaa aatgtgctac tggtccaggg ttctcttgta aacttgatga 2400

ttttgaaaat ttcgttaaag aaagaattgg agatgttgac tttattaaac aatgtggtgt 2460

caatagtacc tacccatctg agcttacttt ctactgggat tataaaaatg tcacttacaa 2520

tgctccttta gaattgtaag acatcattag atcaatttag atatccaaac atttattcgt 2580

tattctcttc gtatattatt tatattcttc cttttcttga aaaaaaaaat agacaattta 2640

tttagacttt ataactttta cttcgtgttg caacaaattg agcattttac acgaaacttt 2700

aaataattga atccttcgaa aaccaaagtt ttattggtcg acgggttggt taacatggaa 2760

tatatcactt tctaataact atgtcacacc aacaaatatc aatatgagtg tttcagacaa 2820

atacccagaa cttgttagac aatttttcct tcttgatgaa gtgaaggaaa ttttgccgaa 2880

ctatccaaaa tacaaaattt tactgcaaac tcctgaagtc gatcgtgaat actacaaaaa 2940

catcaccagt cctgaattca ttagacaatg gcagccagaa gtcctcaatc actaccgaaa 3000

taactggacc gaagtcactc ctctttgtgc tattgtacat gatagaacca ttgatgccgg 3060

tttgagaatc caaaagtttt tccatccatc catcttaccg aatgaacttc atggcgatgt 3120

ttggatactg gtaaaagaga acaaagaaga actcgatgcc tttatagaaa atgtgcaatg 3180

tcttcaaaat tatgttagag atagctccaa cagtaaatac acttattatc gttgtgagta 3240

ttgcaaaaag aataaaggtg ttaaaagtaa aaaaactgat tgcaagcata aaattgcagt 3300

acatgctctt gaaggtggaa aatacaaaat agtctggcac tttcagcata accatgcttt 3360

cgatccaaga aggattacaa aggcaaccag aaactggttg atggacttag cttcaacaaa 3420

tataccaagg gcaagttctg acagcaggag atcagtgact aaattcaaac tgagttcatt 3480

tttactttct gacaaattta aaatttccaa caaggtattt aattattata aaaacaaaaa 3540

taaagagagc caggcacatc ttgacaaaaa tgttatcaaa agtttaaaaa tatgggtttc 3600

atatataaat acccttaatg aatttgccgt gtttaaaaag agatcaacaa atactgaaaa 3660

tgntgaattc tgtgacgtgg aaggcgatgc tctgaatcct gagtctacgt ggtattttgg 3720

aattattctt ttgagcaatc tccaatatat gctgagccca caaactgttt tccttgatag 3780

tacacataaa ttaggccacg gccctcacaa cgaggacata ataacatata tctttatcac 3840

aaaaagctct ttatctggag gagggatacc aataggttac ttaataacaa atagagagtc 3900

tcatgagccg ttagcatcat ttttgagatt ttttgttgaa aagaaaatac aaatcaaaag 3960

attcgtgata gattgttcag ctactgaaat aaaagctatt gaagaaggat ataatgttgg 4020

tatcattgaa cccacagatg gatcatcaag tgctggtgat aaatttgaag ctatcataac 4080

gttttgcact tggcattgtt tgagagcttt taataagacc attaacaaac ttattacaat 4140

acaaaataga acaaataatg agcaaatatc cccaaatgaa attatcacag aagttgacgg 4200

agaaatgaca gatgaagaat tcataaatca gatagccact caaggggttg ttgcacaatc 4260

aaacttaact gcaggtagga ataaggaaga gataattgca aatcaaagaa ttgctctttc 4320

atatatggta gaattaaaac ggaaaaaagc cattgaagaa gctaatgatt ttttgcatgt 4380

aatcgaagcc acgtttcggg aatacccgga ctttgttgca tacgcccaga aaacattcaa 4440

aaccacaggg aaatactggt taaactgcca ttttggtaat tacagagaac ttacaaataa 4500

ttgtgtgaaa agttatcacc aagttttaaa aaccaaatat ttcgaaagac gcagaaaata 4560

ccgagttgac cgagtaattt ggatgtttat tgaacccatt gctaagtact atgagtatta 4620

ccattcagct gttattgtta catccctgtt aaggtacatt gataaagctg aagaagcttc 4680

caaactcaaa gcagaagcag tttcagatga ggacatgagg caaatgattg ttgaccttcc 4740

aggttatatt gcagttaaat cgttcaatgg atcaaattat tacaagatta gttttggtga 4800

acgtggaatc ttttcctgcg aatgtccgta caacgagtat tcaattgatt ggtgcaaaca 4860

cattttctta tataagcgtt ataaggtggc taaaggattg gacataccta ttgtcgagct 4920

tgaaagaaac cctttggctg acttaagtgg tttaaacggt actaatgaga tagttgaacg 4980

agaaacagat actattggaa atgaatcaga agacgaagaa ttagttgata gtgagtcagg 5040

atttaagaat gccacctata atgagagtga ttttggtgac gataattttg attctatgga 5100

aaatgatcca gatggtgacg aaccagattt tagtattgaa aacacagaac caactgaagt 5160

atcccaagaa gagaccgaag aagaaattgg tgccaggctt gcacgcgaca gagttgatcc 5220

tgggttctcc atagatgacg acaatattgg aaacgacttc gaactcgctg actcttctca 5280

agtttttaca gacggtggaa cagcttatta cacacaaaac acagaatcag acccatttat 5340

tgaatggcct ataagtgaaa caattgatct gcaagaaagt gctgatgtta ttttagaaat 5400

cgaaagcata gaaggggttt atgctaagaa agctgctaga aatattaagc aacgggaaga 5460

gaattatagt agtttagata cagaggttaa aagaattcaa gatgaggaga aatctcaaag 5520

ggagaaggtt aaaaagctaa gggcattaat taaaaaagaa gagatggaac ataaaaagaa 5580

aatggcggca gtgaatagga ttcaaaagaa a 5611

<210> 14

<211> 1308

<212> DNA

<213> Candida albicans

<220>

<221> misc_feature

<223> "n" may be any one of a, g, c or t

<400> 14
tggtgccatt tttagaattg atgtctgaaa tagaatatga ggtccagaga agttttattt 60

ttgttataca tcattttttt tttttgcttt gtctcaccga atattatttg attcctaaaa 120

aattgtaata ccctgtgttg gtttgtgcac tattttgtgt cagaaactga tctatgaaaa 180

tgatggttat tatgagaatg gaaaattttt ccatcacaca tcaggtgatg acagaactaa 240

attatattgt gtagtataat aaagggtatg aaataccaac atcccaggat atcaattata 300

tagaagggaa ggagtttcaa tatatatctt gtgaataata acttcgttct aattcactat 360

tcacaactag gcgtgtacac gctgaatctc aggtaaagaa agtttatatt ccatcactct 420

gaagtcatac attaatatta aataaacaat ctaacactag catgcattca taacctatag 480

atcattctaa acaagctgtt aacacaaatc caatcaattg aatttatcat ataatgaagt 540

aacttttttc aaggcaacat ctattctttt attaatctcg acgtctgttt gattaagttg 600

ctctaacatt ttatttagat ccttctctat attttctgca atatcaaaca ccgattgctt 660

tttgtctgaa gttgctggta tatcaccact tccgccaatt gtcgtatttc cactgtcctt 720

tgttactgac agattggcac tgacattacc tgaattgttc atgtttgctg ttgaaagagc 780

aggaactgta cttggataag cagccgattc aaaagaagat gtggacatga gtgtcaagaa 840

aatgtgtaga atcagtacaa gactggaaaa cagaaggaac aaagtgaact ggatattgta 900

gttttgttga tagtactcgc gagctttaat ttttttttgt aactggcgga atcagatctt 960

atgcaatact caaatccaaa gaaacagtca atccagatga aaggcatgta atcgctagtt 1020

ttcataaaca gaatcatgtt actagtcata ttttctataa aaattcaata cttcattctt 1080

tttgttcaat actaactata aatgcttaca aatagattca aatttcaacc agatccacca 1140

cttcattagg ctcaaccaat tcttcataaa tagaaacgtc ttcctcagcc aagcttaatt 1200

gatgggaaac cctagcttgc attgaaggaa aaatacataa tccaaataac aaactgtctt 1260

tccnaatatt ctcaaaattc gacttcaccg tcttccaacc aagcaggt 1308

<210> 15

<211> 1672

<212> DNA

<213> Candida albicans

<220>

<221> misc_feature

<223> "n" may be any one of a, g, c or t

<400> 15
cctatcaggt acttccccac ttggattggc ttctgcctct cttcttctcc caaccatcat 60

cccaatatca ttccacccat cgtcttcatc gttgtcgtct tttgttggtn tctcttcttg 120

tttttctagt ttaccactat aaaaatcaat caattcagtt tgttttatgg catcagattt 180

ataaattttt ttaattttat caacataatt atcaacaatc caatcaagat gtaatttatt 240

caatttttct tgtaaagaat caccaccacc atttcctatt ccttccattc ttgataatat 300

attccaatta gtttcatgac ataatttcgt taattcatct aaatcattca attgttgttt 360

atcattaata atttgattta tattgatgga aattttatca attaaatttt tagaaatttt 420

agaatttaaa taatttttga ttataggata ttgtaattca tttataaatc taattaaatt 480

agtaattgat ttaataaaat tgttgtcctc gttgtctgat acaatttcta atttaatagt 540

atcttccaat tcatcaacaa tcaaactaag ttgttttgaa ggggtggggg tggagtcccc 600

caatattgaa tccactaatt tatcccaatt ttccttatat ttatcgtatg cattcatatt 660

attatgtcca tttttcaata aaaaccgatt gaaatcttgt aaaattgcta tattagtaat 720

agtcaatgga tcaggaatta aaagaatagt taaatattca ttcaattgat taacaaaatt 780

ttcataaagt gaatcgactc gtttcttgat ttgtttatat ataatatatt gagaatttgt 840

atcaatgatg atttgtttaa ataaattatt taaatattgt aaatctaata tactttgtaa 900

tgttttcggt ttccccaaat acgtttcaat ttcttttaat ttagaattga tctcttgtaa 960

ttcattcaat tgttgtaaat tgtcagtaac gatttcaaat ttattattca attcagtaat 1020

tgttaaatca gttaaattgt tactttcagt ggtatttgaa tcttgaggaa tttcttcaaa 1080

ttgttttcgg aaatcattat cattttcaag ggttgttttg tttattttgg ataatgtttt 1140

atttatgttc tgttcaatat cttttaaata taattcttga tcttctaatt gttgttcaat 1200

cgatggcatt attggtgttg tataaaaatg gaattttgta aagttgaatg tgttggcaac 1260

acttgtgttt gtatgggcgt atattttttg aggagatcaa agcaaaaaat attttgagac 1320

ttatacacgc aacatacaga acagttgttg gtttgtgcac tattttgtgt cagaaactga 1380

tcaatgaaaa tgatggttat tatgagaatg gaaaattttt ccatcacaca tcaggtgatg 1440

acagaactaa actatattgt gtagtataaa taagggtatg aaataccaac atcccagaat 1500

atcaacgagg atagaanggg anggagtttc aattanaata atcctgtnga ataaataaac 1560

ttccggntcc taaattcnnc taataccnac caaaccttag naccgtngta acancgcctc 1620

caatcctcca ngggaaaaag aaaangtttt aataatttcc cnatcccgga tt 1672

<210> 16

<211> 690

<212> DNA

<213> Candida albicans

<220>

<221> misc_feature

<223> "n" may be any one of a, g, c or t

<400> 16
tgatacgatt gaatggtgga gacaaaatat ccgatgtgtt gaaagataaa attgtactcg 60

aatatcccac aatatatgtt gctgcaaatg acgagtgttt acaagataga attatagata 120

gccttcaatt ggccgaggag gaagaagatg acaccactga ctcaagtgag gatgattcta 180

gtgactcaga gagtgatgat gatgatagtg atagtggtag tgaaaccagt agtattggag 240

acggttcagg tgaagataac gattctgatt cggcaccgga agagacatct ctgaaactac 300

cacctttttc acagaaattc tttgaagcgt cagctgagcc aaaaccaata atagaagaga 360

taggatctaa caagactgta gaagaaccat aacgaatgaa tataaaatac ttgtattatg 420

tagtgccaat aaaagttgaa acggtcgcac tactttttag tcctgttggt ttgtgcacta 480

ttttgtgtca gaaactgatc tatgaaaatg atggttatta tgagaatgga aaacttttcc 540

atcacacatc aggtgatgac agaactaaac tatattgtat agtataaata agggtatgaa 600

ataccaacat cccagaatat taattatata gaanggaagg agtttaatat atatcctgtg 660

gaataacaac ttcggtctaa ttcactatac 690

<210> 17

<211> 1912

<212> DNA

<213> Candida albicans

<400> 17
ctaggtttta attcactatc ataaagatca atggttagcc caaaattaaa atatggaagc 60

caaaacttcc gtggtcaaaa aatgaactaa gaagctaaag tctttttgaa acagtatgcc 120

attatgtttt tcagatgttt ttacttggtt gttatattaa aatccaaagc tctggctctt 180

atcaagaatt tgtcagtcaa ctcatcatca aatgagtgga tatattactt tcaagaatca 240

tcattaccaa gttgtcaaac gattgctaag caaatgttga agaatactga ttatttcagt 300

tttgagaaac ctaaccccaa agataattta aggagaatca aaatttgaaa gaaaaggatg 360

aaaagttgga gaaagaaacc ctattgaaaa tttaagtact gattgtttca gaaaatcatt 420

gaatatgaaa caacagaaag gatattttac caactaatga acattttcct cccttataca 480

ccttaaaata cattaaatcc ttctggaata gttttttctc acaagacatt ttggtgtata 540

acattggtac tattgttgct gtcatgacaa ataaggaatg ctacaaaacg tcaaggtaga 600

agctatcgat gttttttcca gctaatgaca ggacaacgtt agaaacgaag tgtgcagacg 660

atttggttac aaagattgca agtgtatcaa ttatgctagc atatacctta tattttcgtt 720

gagagtattt ttatcatcgt tggtctgcaa aacttcaaag aaggggtgct atatgtgtta 780

aatgctgaga atcgaacact gtatctcatg gcgataaaat tcaaaatatt gtcgttagta 840

tgagaagatt ttgctgatat ttacttatat ttcacaatgt tcagtaaaga tccttatgac 900

ggtggtacaa tatgggacat gctatctgac acgttgacaa ccactaaaat cagctgttac 960

cgatagagac catacagatt gacgcaacac ataagtatac tcgaaaagct aacccaccat 1020

atcaggcatc aagccaaaaa tcaattttga ctgaaaatgg acgtcattaa ctctgagtcg 1080

ctaaaatcaa ggtatgaaat atttgccaaa gaggaaatcg atcagagtcg caatttctgt 1140

tcaatattca accaaataca attttccaac ctataaatct ccaccatctg tgttatgtgc 1200

tgtcattgag tttgcaactg atatttttgc tatatcttta cgttgcaaaa tatgcggggt 1260

gatgttaaac ttacccgaat tctccgtgta tcacatgtta ttatgccaaa tatgcatatc 1320

taggaaaaca gtctcaacca tctaacacac acattttctc accactgaag ctatgaagat 1380

agcccattcg ggaacggtaa acgacgtagc gggaaaaatg tgcttaaaag aatatgggaa 1440

aataaacggg tagacgtcat ttcccagtac catattctat tcagtcgaac gtcttcattc 1500

ttatcaacgg gggactggtc cagagacctt tcttatttta ttgtgattca gtagcgtcta 1560

ccatatacaa tgatattgta acttccgatc aagtggaaac accgggagct tccaaagtat 1620

ggtatccgaa tataaagcca cccaaaatcc aattcaccac gagctaacac ctggggaaaa 1680

cgaggtgtct aaacctcctc aacttgattt cgagacttcg gtagtaggga agtttaaagg 1740

gcctattaca accacaaaag tggcaccacc accctccatg ggaggtctat taagtacatg 1800

gaaacgcatg ctctggttga tacatcacct caatcaaaaa aattggtgtt ccacattcga 1860

aggaactaaa accgacgaga acctatcaca cggtgtcgac gatgataaga aa 1912

<210> 18

<211> 6140

<212> DNA

<213> Candida albicans

<220>

<221> misc_feature

<223> "n" may be any one of a, g, c or t

<400> 18
   agtaaaaaaa gaagaaaaaa aagctaaaat tgggacaata tgctaagtat atatagggga 60
   agacgtcgaa cagcaaccac ggaaaaataa tagtgattgt ctttatccgt tattggctgg 120
   atggcgacgc cacaacctga aatttggttc caactgttga ggatgattta tgtttgtgat 180
   tagaactaaa atcattcgag aaaaaaggaa taggagagaa ccaactttag tcgtgtaaaa 240
   agtaacatct gccaattata aactatacgt agtccaaata atttacggta tatttctgta 300
   ccccttcttg gcaatatcac aagaatatca taatgttcat gaaccctctt tgaacacgta 360
   gacaagtaaa cccaatgagg gggcagtgtt ctattcttgt aaactgcgca ccaaaaacgg 420
   ggcttaaaaa ataagttatg aaaactataa ataaccatga aaatcaccct actcccttcc 480
   tcccttcctt ccttccttcc ttccttttct cttttcctct acccacacta ctcacaatgt 540
   tcggtatttt tgaggaaaac tacgattctg tttacaaagg caaccacgaa gccaagttct 600
   ctcacgaagc agttgctggt gctgcttcat ttgctgctgt caagttgttt gaagatagac 660
   aaagaagaga agggaaacca gttagtcacg cctttgctaa agaagcttta gctgctattg 720
   ctggtggaga agtcgacaaa ttatttgaaa ccaaagggtt ggactatttg gatagagaga 780
   gacttagaga tcaagctatc aacaacgctc aaagaggtta cgacgaccat tacggtcaac 840
   acgaagaatg gtctccagaa cacagaccac cttttgacta ccaaagatat taagtagaaa 900
   ctgtgtagtg aatttacaat ttttttgaca agaattaact taaacctcgt ttttaggttt 960
   tgtgcggctt ttgtcaattg acgatcctgt atatttcgtc ataattcaca cattcttaaa 1020
   attatgcaca catccttgaa atgtgttaat attcccaaca ttatcaatta tatgtgttca 1080
   gaattggttg caaagttatc aactcaattc acgctatata aaccttacaa attctctaca 1140
   tttttatatt tttttatatt ggcttttctt ttagaatcaa tcaatacttt ttttatcatt 1200
   tagatacatc tttcatctat taatagatta tctttctata tatcaaaaca cgacacagtc 1260
   acgtgccaaa aaggatataa gaaggaactt cagaaaatta attttctgat tatactactt 1320
   actagatttc ataaagtcaa tatctgattg atacaacttg gttcattatt cataaaactt 1380
   tacaactaat tcnacaagna aacccnacaa aaaaatccna atnaaataat cnnnnnaata 1440
   ttataattaa ttaattacaa aaaaaaacaa aaaaatacac acacacatac acacacacaa 1500
   aatcttgttg caaaaaaaaa aaaataataa taatataata agaattaatt aacaatgtcg 1560
   tttccacgga cacattcacc aagaccatct ggttcacgag aacaggaaga tctcacactg 1620
   atgattaaag cttttagaga ttcaatggaa gctaagcttg acttgcattc gcagaagctt 1680
   actgctttgg tagcaaacat tcccagaacg gacgaagggt ttgaagattt atcacaaagg 1740
   atcactgttc ttaaaaatca tcaaaaagca tttttgccca aacaagaaaa agaaatcgga 1800
   agtcttctcc acagacaaag agaggaagaa ggtgatatta aggatttcaa aacagtcgtt 1860
   ggtgaagaaa aagaagaatt gcaccaggtt gaagatttcg ttttaaaaga tcaagaagaa 1920
   ttacgaaacg tcgaaaagaa agttttgaaa gaagaagaag aattgcaaaa agtggaagag 1980
   tcaatggaaa aggaaaaaca agagttatac caggttgaag actttatttt gcaaagagat 2040
   gagacggtaa agaaacttgg agaaagcaat caatctcaac aggaaccata tacacctgca 2100
   acttctggtt cggatcagag attcagatct caacaaccta acattggaaa taccttagcg 2160
   caggatctag cattaattcc aaaattagat ctggaaattt gcaaaattgc agtcaaatat 2220
   ccaaaattat ttgaaacaaa attaagacca ccaccaccca gagactttca atataaaatt 2280
   caactcacag accacactca aatttattca aaaccatata aatgcaatca agaagaacaa 2340
   gctctcatca aggatttcat caatgaaaaa ttagaagcag gcgttttggt accagctcca 2400
   attgatgctt ggttacaccc aatatttcca atcagaaaaa ccaatgccaa ccaatcctcc 2460
   accaaaatag cagttgattt aagacgtctc aataaggtca cagtacgaat gtacacttat 2520
   ccaacagaca caaaagacct cttatcctca ctaacagatt cccactattt tagcgcttta 2580
   gacttaaaga atgcgttcta tcaggtaagc atacacaagg atagtataaa atattttggg 2640
   atttcaacat ccgaggggaa ttattgcttt acaactttac cgtttggagc aatcaattcc 2700
   ccaaccatct ttactaactt tgtgagacag attttagagg ggatcccatg tatatttata 2760
   tacatggatg atatcctcat ccatactaaa accttacatg accacatgtc attactcagg 2820
   agaatcatgg agaaactaaa tgagcatcag tttcaaatga attataacaa gatgcaatta 2880
   ttaacaacaa aaatcaattt cttagggtac agcattcaag cgaacaaaat atcaccagat 2940
   atttccaaaa ttcaagcaat acaaaattgg gaattgccca cgaccactac tcaaatcaga 3000
   gcatttgtca atttcagcaa ccactttcgc atcttcatcc cagaaatagc aaaatttact 3060
   aatccattaa atgaattatt gaagaacaac aatggtaaaa acataaagat tgaacacacc 3120
   caagcatcca ttgatggtta caaggcatta aaagccgcca tcattggatt gccgacgctt 3180
   caactttaca atccaaaact accaaccatc attttcacag atgctagcca catggtagta 3240
   ggaggatatt tatgtcaacc aacattcaga aatgacaaag aagtccttgt cccaattgca 3300
   ttttcatcac ataaattaac agaaacacaa agcagatatg ctgctatgga aaaggaactt 3360
   ttggcaatta ttgtgatatt ggaaaaattt agatatcact gcagcaatac ggtagagatc 3420
   tatacagatt atcaaagttt ggcatcatat ttagataaga aaactactcc accaccgaga 3480
   attgctaggt ttttagatct aattggatca ttttccccaa aagtgtacta tttaagtgga 3540
   aagaaaaatt tcgttgctga tatcattaca agatatcaaa ctcaaaatat taaggaattg 3600
   gtagatgaag acaagatact aggacagact tttacagtca agagaaattt gaaacaacaa 3660
   ctattaccaa gattggaagc aattgaattg gaaaatctta atgaatcaca ggttcacaaa 3720
   atccaaactt cattagaaca acaacaacaa catgatttgg aagacaatga tgaagagtta 3780
   cctctccaac tgtttaaatt aatgaatgat gagttatttg taatcattaa caaccaactt 3840
   ttaaaatacc ttccaagact ggaatacaat gatatttgtc aaacaatcca tgacaaacac 3900
   catccatcaa ctagagtaac agactactta tgcacactcg catattggca tcctgaccat 3960
   ctattaattg ctacaaacat tacgagaaag tgtcactatt gtcaactaaa cacgtcaatt 4020
   cgtgaggcca ttagaccata ccgaccactt gaaccactca aggcatttag cagatgggga 4080
   atggactact ctggaccata ctttaacaca gtccaacaca ggtacatatt agtagccgtg 4140
   gaatatgtca ctggtttaac tattgcagta ccaacattgc acaaagacgc agataacgca 4200
   atcagtcttt tacaatcaat cattctgatc atgtcagcac ctacagaatt agttacagat 4260
   caaggtaaaa aaattttcat cacaagcttt ggctacccta tgtgaccaga ataacataca 4320
   acaccatatt acctccgccc accacccacg tgggaatggt cgggttgaga aggtgaacca 4380
   cctattgaag aaaatattga aagcattaac taacgatacg atgcaagact gggatttaaa 4440
   actatatgac gctttaagaa tctacaatgc tacacctaca atttttaact acactccact 4500
   ttatcttgca cttggaattg aaccacacca taatttaaat caattacaaa aagatttaat 4560
   tgaaaatttg caaaaagaat tgcccccaga ggtccaatcc acagaagaac acgaagaaaa 4620
   cccaaatgat gaacaacaag aagagggcag agaacaacaa atttcaagag aagaacaaca 4680
   ggacggcaga gatcttgtac acttaagaat ttacgaattg gaagcaatta agaaagctcg 4740
   caagttacac acaaatttga aaacacgaag aaacgcagtc caaaatatgt taaaggaacc 4800
   atatggcatt ccagcacttt ttacaaaggg acaatgggta tacagaatta gagctaaagc 4860
   acgaaaatat gaatcaaatt ttgatggtcc atatcaagtt caagaagtat taggtaaagg 4920
   tgcttataaa ttgagagaca tcactggaag agaaaaagga atctacaatc aggatcagtt 4980
   gaagttagca tattcagcag acaacgatcc aatacaggtt tttagttctt ttaataaaga 5040
   atatgatcga gtacaacaaa aattgttaga caaaattcaa tcagaaagag atcatcaatt 5100
   aaattgtttg tcagtccaac atttacacag acaaagaagg ttactcgata tatccagctg 5160
   tcttgagcaa attctgcaat aatttcgcta atcattggag gaaagggtag atgacgatcc 5220
   tgcatatttc gtcataattc acacattctt aaaattattc acacatcctt gaaatgtgtt 5280
   aatattccca acattatcaa ttatatgtgt tcagaattgg ttgcaaagtt atcaactcaa 5340
   ttcacgctat ataaacctta caatttctct acatttttat atttttttat attggctttt 5400
   cttttagaat caatcaatac tttttttatc atttagatac atctttcatc tattaataga 5460
   ttatctttct atatatcaaa acacgacaca gtcacgtgcc aaaaaggata taagaaggaa 5520
   cttcactgaa atgcaatcac ttcgcattat tcaagatctt tttctattgt ggctggtttt 5580
   tggtgattgc tatgtttggt tttttttttc tggaacacaa gcaaccaaat ttttcaactg 5640
   ttacgtcaca catttactgt cacactcact tactggcaca caaagaacaa agcaatcatc 5700
   cggcgtaaac ttttggtctt tgagatgcaa aagttgcaaa gcaattggca cttctactaa 5760
   gatggttcca gtaaaaattg tgttttatag tacatcaata atcaaacaat acttaatgat 5820
   gtaacaatac cttaaaaagc ccccactata tttctttttt ttttaagttt gctatataat 5880
   ttattatgtg ttattattat tgacttaatt gttagcattt tattgcttga gatcgtttgc 5940
   ttgtcactcc accctgaaga aaatttgaat aattgctatt aatttattta tttcttggac 6000
   acaccccgta ttgtcgtatg ggtataaatt ccgtttcatt tctcctccct atttcatatt 6060
   tcataacttc ttaaatcaat attcaaacca actccaaatt ataaactatc aaacaaagaa 6120
   acaaaaaaac acacaacaca 6140

<210>  19

<211>  916

<212>  PRT

<213>  Candida albicans

<400>  19
   Met Ser Phe Pro Arg Thr His Ser Pro Arg Pro Ser Gly Ser Arg Glu
   1               5                   10                  15
   Gln Glu Asp Leu Thr Ser Met Ile Lys Ala Phe Arg Asp Ser Met Glu
               20                  25                  30
   Ala Lys Leu Asp Leu His Ser Gln Lys Leu Thr Ala Leu Val Ala Asn
           35                  40                  45
   Ile Pro Arg Thr Asp Glu Gly Phe Glu Asp Leu Ser Gln Arg Ile Thr
       50                  55                  60
   Val Leu Lys Asn His Gln Lys Ala Phe Leu Pro Lys Gln Glu Lys Glu
   65                  70                  75                  80
   Ile Gly Ser Leu Leu His Arg Gln Arg Glu Glu Glu Gly Asp Ile Lys
                   85                  90                  95
   Asp Phe Lys Thr Val Val Gly Glu Glu Lys Glu Glu Leu His Gln Val
               100                 105                 110
   Glu Asp Phe Val Leu Lys Asp Gln Glu Glu Leu Arg Asn Val Glu Lys
           115                 120                 125
   Lys Val Leu Lys Glu Glu Glu Glu Leu Gln Lys Val Glu Glu Ser Met
       130                 135                 140
   Glu Lys Glu Lys Gln Glu Leu Tyr Gln Val Glu Asp Phe Ile Leu Gln
   145                 150                 155                 160
   Arg Asp Glu Thr Val Lys Lys Leu Gly Glu Ser Asn Gln Ser Gln Gln
                   165                 170                 175
   Glu Pro Tyr Thr Pro Ala Thr Ser Gly Ser Asp Gln Arg Phe Arg Ser
               180                 185                 190
   Gln Gln Pro Asn Ile Gly Asn Thr Leu Ala Gln Asp Leu Ala Leu Ile
           195                 200                 205
   Pro Lys Leu Asp Ser Glu Ile Cys Lys Ile Ala Val Lys Tyr Pro Lys
       210                 215                 220
   Leu Phe Glu Thr Lys Leu Arg Pro Pro Pro Pro Arg Asp Phe Gln Tyr
   225                 230                 235                 240
   Lys Ile Gln Leu Thr Asp His Thr Gln Ile Tyr Ser Lys Pro Tyr Lys
                   245                 250                 255
   Cys Asn Gln Glu Glu Gln Ala Leu Ile Lys Asp Phe Ile Asn Glu Lys
   260 265 270
   Leu Glu Ala Gly Val Leu Val Pro Ala Pro Ile Asp Ala Trp Leu His
   275 280 285
   Pro Ile Phe Pro Ile Arg Lys Thr Asn Ala Asn Gln Ser Ser Thr Lys
   290 295 300
   Ile Ala Val Asp Leu Arg Arg Leu Asn Lys Val Thr Val Arg Met Tyr
   305 310 315 320
   Thr Tyr Pro Thr Asp Thr Lys Asp Leu Leu Ser Ser Leu Thr Asp Ser
   325 330 335
   His Tyr Phe Ser Ala Leu Asp Leu Lys Asn Ala Phe Tyr Gln Val Ser
   340 345 350
   Ile His Lys Asp Ser Ile Lys Tyr Phe Gly Ile Ser Thr Ser Glu Gly
   355 360 365
   Asn Tyr Cys Phe Thr Thr Leu Pro Phe Gly Ala Ile Asn Ser Pro Thr
   370 375 380
   Ile Phe Thr Asn Phe Val Arg Gln Ile Leu Glu Gly Ile Pro Cys Ile
   385 390 395 400
   Phe Ile Tyr Met Asp Asp Ile Leu Ile His Thr Lys Thr Leu His Asp
   405 410 415
   His Met Ser Leu Leu Arg Arg Ile Met Glu Lys Leu Asn Glu His Gln
   420 425 430
   Phe Gln Met Asn Tyr Asn Lys Met Gln Leu Leu Thr Thr Lys Ile Asn
   435 440 445
   Phe Leu Gly Tyr Ser Ile Gln Ala Asn Lys Ile Ser Pro Asp Ile Ser
   450 455 460
   Lys Ile Gln Ala Ile Gln Asn Trp Glu Leu Pro Thr Thr Thr Thr Gln
   465 470 475 480
   Ile Arg Ala Phe Val Asn Phe Ser Asn His Phe Arg Ile Phe Ile Pro
   485 490 495
   Glu Ile Ala Lys Phe Thr Asn Pro Leu Asn Glu Leu Leu Lys Asn Asn
   500 505 510
   Asn Gly Lys Asn Ile Lys Ile Glu His Thr Gln Ala Ser Ile Asp Gly
   515 520 525
   Tyr Lys Ala Leu Lys Ala Ala Ile Ile Gly Leu Pro Thr Leu Gln Leu
   530 535 540
   Tyr Asn Pro Lys Leu Pro Thr Ile Ile Phe Thr Asp Ala Ser His Met
   545 550 555 560
   Val Val Gly Gly Tyr Leu Cys Gln Pro Thr Phe Arg Asn Asp Lys Glu
   565 570 575
   Val Leu Val Pro Ile Ala Phe Ser Ser His Lys Leu Thr Glu Thr Gln
   580 585 590
   Ser Arg Tyr Ala Ala Met Glu Lys Glu Leu Leu Ala Ile Ile Val Ile
   595 600 605
   Leu Glu Lys Phe Arg Tyr His Cys Ser Asn Thr Val Glu Ile Tyr Thr
   610 615 620
   Asp Tyr Gln Ser Leu Ala Ser Tyr Leu Asp Lys Lys Thr Thr Pro Pro
   625 630 635 640
   Pro Arg Ile Ala Arg Phe Leu Asp Leu Ile Gly Ser Phe Ser Pro Lys
   645 650 655
   Val Tyr Tyr Leu Ser Gly Lys Lys Asn Phe Val Ala Asp Ile Ile Thr
   660 665 670
   Arg Tyr Gln Thr Gln Asn Ile Lys Glu Leu Val Asp Glu Asp Lys Ile
   675 680 685
   Leu Gly Gln Thr Phe Thr Val Lys Arg Asn Leu Lys Gln Gln Leu Leu
   690 695 700
   Pro Arg Leu Glu Ala Ile Glu Leu Glu Asn Leu Asn Glu Ser Gln Val
   705 710 715 720
   His Lys Ile Gln Thr Ser Leu Glu Gln Gln Gln Gln His Asp Leu Glu
   725 730 735
   Asp Asn Asp Glu Glu Leu Pro Leu Gln Ser Phe Lys Leu Met Asn Asp
   740 745 750
   Glu Leu Phe Val Ile Ile Asn Asn Gln Leu Leu Lys Tyr Leu Pro Arg
   755 760 765
   Ser Glu Tyr Asn Asp Ile Cys Gln Thr Ile His Asp Lys His His Pro
   770 775 780
   Ser Thr Arg Val Thr Asp Tyr Leu Cys Thr Leu Ala Tyr Trp His Pro
   785 790 795 800
   Asp His Leu Leu Ile Ala Thr Asn Ile Thr Arg Lys Cys His Tyr Cys
   805 810 815
   Gln Leu Asn Thr Ser Ile Arg Glu Ala Ile Arg Pro Tyr Arg Pro Leu
   820 825 830
   Glu Pro Leu Lys Ala Phe Ser Arg Trp Gly Met Asp Tyr Ser Gly Pro
   835 840 845
   Tyr Phe Asn Thr Val Gln His Arg Tyr Ile Leu Val Ala Val Glu Tyr
   850 855 860
   Val Thr Gly Leu Thr Ile Ala Val Pro Thr Leu His Lys Asp Ala Asp
   865 870 875 880
   Asn Ala Ile Ser Leu Leu Gln Ser Ile Ile Ser Ile Met Ser Ala Pro
   885 890 895
   Thr Glu Leu Val Thr Asp Gln Gly Lys Lys Ile Phe Ile Thr Ser Phe
   900 905 910
   Gly Tyr Pro Met
           915

<210>  20

<211>  3470

<212>  DNA

<213>  Candida albicans

<400>  20
gtatatttca agacgttatt tcttgtgacc cttggatgac tactcaaaat acttgacagt      60

tcaacccact atgcaacaaa tctgatgcta ctgccgaaat tatcgaattc atcaatcatt     120

gggaaaagtt ctttctggga aatggcaatt accatacgaa aattctccgg tcggataatg     180

gaggggaatt cttaaacaaa acattgacta cctatcttga ttcaaaatat attactcacc     240

aaacctccaa tgcctatgaa catcatgaga atggcgctgc agaacgagct attagatcgg     300

ttaaagacat ggctcgagta atattgcttc aatccaaatt accagtgccg ttttggtccc     360

tagcaacccg atgtgctgcg tttgttatga atcgtcttcc tcataaaaca ataaatggta     420

agattcctta tgaagtatgg actaaacaac ttgtcaatct caaaatgatg aaaccgtttg     480

gctctcaagt atatgtgaaa attcctattg gagtcaaaag tttttctgca caagcacttt     540

ctggaatcat ggtgggatat gccactaata agaaaggcta ccttgtatat gatcccacac     600

aaaatcgaat attcacatcc tcacaaataa tatgtcatcc gagcatttat ccagcagcca     660

accttacgtt taacgaaccc ttaattatct catcgaaagt cacggctgct catcttcacc     720

cccttaccat ttccaattta gttattccac ctaccaatgc tgtatctgag acacctcttg     780

caaattgtgt gctctcctca aattcgtcag tatgtcccaa agtttgccaa ttacaaactg     840

tcttggaaca tggggaggat aaaatatatg cactgattat accaatatcg atcggcaata     900

tgaaacgcac aagaacaaat gaaaacaaaa tatgccagct agatgaatcg aacaatacca     960

ccataccaga tagtgtaatt ttatcggcta acaatgtgtt attaaactta gaatcgagat    1020

cttccattcc caaaagttat aaggaagcta taacatctaa tgaaaaatcc aaatgggctg    1080

atgctatgga tagcgagttt aattcattac aatccaacaa cacgtggtca cttgaaccac    1140

taccggaggg acgcaaagct attggtgtca aatgggttta tacaatcaag gacaccggtc    1200

gctacaaggc tcgccttgtg gcacttggtt atcgacaaca ggctggtgtg gactttctcg    1260

aaacgtatgc tcccgtgatt cgtggagaat caatcaaact aatctttgca ctcgcgtcaa    1320

aatccaaact aaagattcat tccatagatg ttaccacagc tttcctcaac ggggaaatac    1380

tggaactcat atttgtgaaa caacctccgg gatatgaaga taagaagcgt cctaatcatg 1440

tttgtaagct caatcgcagc ttatatgggc ttaagcagct gccactaatg tggaacatta 1500

aattaaatga tgtacttata aaggaaggtt tccgtcgact tggtggtgac ttagggatat 1560

acattagtaa ggacaaaaga acaataatgg gagtttatgt tgacgacatt ctcatttgtg 1620

gaccttctga cagtgaaatt gaacaagtaa agaacaacgt gagaaaatac ttctcaataa 1680

ctgataatgg attatgccga aaattccttg gaattaacgt ctatcaacaa gcaaatgaaa 1740

taagattaag tttgaatgat tatataagga gaatgattga ggagttaaaa ttatctgtct 1800

cagaaacaaa cccagtatct ataccatctg atgtcaatta tgaaatattt aaagttaacg 1860

aaaatgatga tgagaaacca tgtgatcaaa ccaaataccg aagtttgata ggcaagctct 1920

tgtttgccag taatactata aggtttgaca tcgcctattc tgtcaactcc ctatccaggt 1980

ttatcaacga tcccaaagaa aaacattgga ttgcagctgt caaggtggta aaatatctca 2040

gtggtactca acggtatggt atttgttata acggtaacgg tgacttgaat atttacgctg 2100

atagtgattg ggcttccact ccatctgatc gaaagtctat tacggggtac attgttacct 2160

atgctggagc gccgataagt tggcgttcca agaagcagaa cgtgatagcc ttgagtacga 2220

cagaagcgga gtttatggct ctcacagagt ccataaagga agccctttgg ctaatataca 2280

tttttcgaga tattaatgtg atattgaaat taccaattgt gatatatgaa gacaacctac 2340

tgtgtcagaa attacttgaa aatcctcgat tccataatag gacaaaacac attgacttga 2400

aatataaatt taccaaagac catatagaag ctggtacaat caaagtggaa tcaactaatt 2460

cagcagataa cttagccgac atgctaacta aacctttacc aaaaattaaa tttaaacatt 2520

taagatggct agcaggatta agacctttag attgattaga taatgataaa atgaaataaa 2580

gattaatttg gagatgcagg ttgatgggga ggatgttgga aaaatgaaat atgatcaatc 2640

ctgcatctag aacctgtggc agaatgaaac ctacgagatt atgaatgact tgtgaataca 2700

agttgaatgt tacagaatgt taccaagaag gttacacttg aatatatgaa tgactagaaa 2760

gtgaattgaa tgttacagaa cctgaataac aatgttacac gaatgtgtga atgatatgag 2820

tttatctata gtaatgtgac atatacacaa aggtgtgaat gaccgagaaa acagatgtta 2880

cattacgggc actggagagt gcaagtctaa agaatcttgg agtagaaata agtaatataa 2940

aaaggaccaa agattcttta gagaaaagta aatgaaacta tattagattt tatataacta 3000

actaacaaat aaataaaaaa tataatatgt ctacaatgcc accaacttcc aaacgtacta 3060

gaaagagaac tagaaccgat gataatgctg aaccaactat tcaagatcct tcaccgccac 3120

ttgctaatgt tgaacccaca attcaagaga ctccaccgct ggttgaagtt agtgatgaga 3180

ctaattcaac tgaaatcaat gagacaaata gtaatactca tgaagaaaca aatgtattaa 3240

ctaatgtgca ctcctctcca atcgagacag ttactgagag gaacttcaat tttcaacaat 3300

aataatattg gttggattta cacgtacgtt gttgttacaa agacgtgagc agagtgagag 3360

agatcaacct tcatattcaa tctcatctca atcaacgctc aatttttttt tcttctccct 3420

ctctttgttg tttaactaag tttgttccct tccatccaag caagttagaa 3470

<210> 21

<211> 748

<212> PRT

<213> Candida albicans

<400> 21

Met Ala Arg Val Ile Leu Leu Gln Ser Lys Leu Pro Val Pro Phe Trp
1               5                   10                  15

Ser Leu Ala Thr Arg Cys Ala Ala Phe Val Met Asn Arg Leu Pro His
            20                  25                  30

Lys Thr Ile Asn Gly Lys Ile Pro Tyr Glu Val Trp Thr Lys Gln Leu
        35                  40                  45

Val Asn Leu Lys Met Met Lys Pro Phe Gly Ser Gln Val Tyr Val Lys
    50                  55                  60

Ile Pro Ile Gly Val Lys Ser Phe Ser Ala Gln Ala Leu Ser Gly Ile
65                  70                  75                  80

Met Val Gly Tyr Ala Thr Asn Lys Lys Gly Tyr Leu Val Tyr Asp Pro
                85                  90                  95

Thr Gln Asn Arg Ile Phe Thr Ser Ser Gln Ile Ile Cys His Pro Ser
            100                 105                 110

Ile Tyr Pro Ala Ala Asn Leu Thr Phe Asn Glu Pro Leu Ile Ile Ser
        115                 120                 125

Ser Lys Val Thr Ala Ala His Leu His Pro Leu Thr Ile Ser Asn Leu
    130                 135                 140

Val Ile Pro Pro Thr Asn Ala Val Ser Glu Thr Pro Leu Ala Asn Cys
145                 150                 155                 160

Val Leu Ser Ser Asn Ser Ser Val Cys Pro Lys Val Cys Gln Leu Gln
                165                 170                 175

Thr Val Leu Glu His Gly Glu Asp Lys Ile Tyr Ala Ser Ile Ile Pro
            180                 185                 190

Ile Ser Ile Gly Asn Met Lys Arg Thr Arg Thr Asn Glu Asn Lys Ile
        195                 200                 205

Cys Gln Leu Asp Glu Ser Asn Asn Thr Thr Ile Pro Asp Ser Val Ile
    210                 215                 220

Leu Ser Ala Asn Asn Val Leu Leu Asn Leu Glu Ser Arg Ser Ser Ile
225                 230                 235                 240

Pro Lys Ser Tyr Lys Glu Ala Ile Thr Ser Asn Glu Lys Ser Lys Trp
                245                 250                 255

Ala Asp Ala Met Asp Ser Glu Phe Asn Ser Leu Gln Ser Asn Asn Thr
            260                 265                 270

Trp Ser Leu Glu Pro Leu Pro Glu Gly Arg Lys Ala Ile Gly Val Lys
        275                 280                 285

Trp Val Tyr Thr Ile Lys Asp Thr Gly Arg Tyr Lys Ala Arg Leu Val
    290                 295                 300

Ala Leu Gly Tyr Arg Gln Gln Ala Gly Val Asp Phe Leu Glu Thr Tyr
305                 310                 315                 320

Ala Pro Val Ile Arg Gly Glu Ser Ile Lys Leu Ile Phe Ala Leu Ala
                325                 330                 335

Ser Lys Ser Lys Leu Lys Ile His Ser Ile Asp Val Thr Thr Ala Phe
            340                 345                 350

Leu Asn Gly Glu Ile Ser Glu Leu Ile Phe Val Lys Gln Pro Pro Gly
        355                 360                 365

Tyr Glu Asp Lys Lys Arg Pro Asn His Val Cys Lys Leu Asn Arg Ser
    370                 375                 380

Leu Tyr Gly Leu Lys Gln Ser Pro Leu Met Trp Asn Ile Lys Leu Asn
385                 390                 395                 400

Asp Val Leu Ile Lys Glu Gly Phe Arg Arg Leu Gly Gly Asp Leu Gly
                405                 410                 415

Ile Tyr Ile Ser Lys Asp Lys Arg Thr Ile Met Gly Val Tyr Val Asp
            420                 425                 430

Asp Ile Leu Ile Cys Gly Pro Ser Asp Ser Glu Ile Glu Gln Val Lys
        435                 440                 445

Asn Asn Val Arg Lys Tyr Phe Ser Ile Thr Asp Asn Gly Leu Cys Arg
    450                 455                 460

Lys Phe Leu Gly Ile Asn Val Tyr Gln Gln Ala Asn Glu Ile Arg Leu
465                 470                 475                 480

Ser Leu Asn Asp Tyr Ile Arg Arg Met Ile Glu Glu Leu Lys Leu Ser
                485                 490                 495

Val Ser Glu Thr Asn Pro Val Ser Ile Pro Ser Asp Val Asn Tyr Glu
            500                 505                 510

Ile Phe Lys Val Asn Glu Asn Asp Asp Glu Lys Pro Cys Asp Gln Thr
        515                 520                 525

Lys Tyr Arg Ser Leu Ile Gly Lys Leu Leu Phe Ala Ser Asn Thr Ile

530                 535                 540

Arg Phe Asp Ile Ala Tyr Ser Val Asn Ser Leu Ser Arg Phe Ile Asn
545                 550                 555                 560

Asp Pro Lys Glu Lys His Trp Ile Ala Ala Val Lys Val Val Lys Tyr
                565                 570                 575

Leu Ser Gly Thr Gln Arg Tyr Gly Ile Cys Tyr Asn Gly Asn Gly Asp
            580                 585                 590

Leu Asn Ile Tyr Ala Asp Ser Asp Trp Ala Ser Thr Pro Ser Asp Arg
        595                 600                 605

Lys Ser Ile Thr Gly Tyr Ile Val Thr Tyr Ala Gly Ala Pro Ile Ser
    610                 615                 620

Trp Arg Ser Lys Lys Gln Asn Val Ile Ala Leu Ser Thr Thr Glu Ala
625                 630                 635                 640

Glu Phe Met Ala Leu Thr Glu Ser Ile Lys Glu Ala Leu Trp Leu Ile
                645                 650                 655

Tyr Ile Phe Arg Asp Ile Asn Val Ile Leu Lys Leu Pro Ile Val Ile
            660                 665                 670

Tyr Glu Asp Asn Leu Ser Cys Gln Lys Leu Leu Glu Asn Pro Arg Phe
        675                 680                 685

His Asn Arg Thr Lys His Ile Asp Leu Lys Tyr Lys Phe Thr Lys Asp
    690                 695                 700

His Ile Glu Ala Gly Thr Ile Lys Val Glu Ser Thr Asn Ser Ala Asp
705                 710                 715                 720

Asn Leu Ala Asp Met Leu Thr Lys Pro Leu Pro Lys Ile Lys Phe Lys
                725                 730                 735

His Leu Arg Trp Leu Ala Gly Leu Arg Pro Leu Asp
            740                 745

<210>  22

<211>  1550

<212>  DNA

<213>  Candida albicans



<220>

<221>  misc_feature

<223>  "n" may be any one of a, g, c or t



<400>  22
gtgttgtgtt gggtttgaat ttctgtataa ctcaatttgg agattttttt tttttttttt      60

ttgaaatttt tattagtcgt gtacattgtt acaattgttt ctcgttcccc tttttttttc 120

ctttctttgt tttgttttgt ttaccttgtg ataattttat acgtgttgag agggctctcg 180

tcgtgcccgt gtccgtttcc gtttccgtgt cctgttgggt cccctccgcc catgccgcac 240

cgcaccgtac ggtaatgata tctgattgtt gttggagcgt tcttcgctaa caggttcttt 300

ctttttgttc aggggtttcg aaagataatg tagaaacacc agggcttata actgagagtt 360

agagtagtgg agattagtag tagtagtaca atcctatagc ccaaacatta ttggagagat 420

cttaccaaat agcaatcatc atgatgtatt tactactaca taaatnattt aagacgacat 480

ttaccagcaa taaacaacat gaccaactaa ttaacaaaca tttgaaaaac ataaagtaat 540

tagaaagttt aaaaagtgta caaccagtgt ggaaaaagaa tggaattgga attgaacaaa 600

gttattaatt actgaaaaag gaaatttaat ttcttgaaag gcaaatcttt gtttgttttt 660

ttttttgggt cttttctttc atttaataag cgtggggtat taatagataa tgatattgtt 720

gttgttattg tgatattgtt gtgaaatttg acatatgata agataagttt ctttcttttc 780

tttcaactag tataattgaa ctaaagacca ccaccaccac caccacatag ttagcaacct 840

gatatgctgt tcatgtaaca gtaaattatc ttggtactat accacttgtt gtaatatagc 900

taatgctaat tcttgattag tgtggaaagc ctaataaggt tatattgtgc acaggttaac 960

taccttaata tagttattgt taatacagtt attgctgttg actactattg ttattgttaa 1020

attaaagtgt taggttgagt taattgatta gtgaaaacca actaactacc gtattaaatt 1080

attgtattaa gattgattcc tattaaggat aaaacagaga gtgtgttaga aagagaaagg 1140

gtggattata aatatgtgta aaatcccctt tagagactaa ccactagaaa tctattgatg 1200

gtttcatata tagagattaa cgattatatt tataatataa gttggtagtt gctagtatat 1260

ntgaaagcac tacagtatag tatgtcagaa tcagatcatt taaactctac taataataca 1320

ggaaacactt tcattagtct agatcaagcc agtacaataa tggcagatca aactcaagga 1380

gctaacccac aacaatgata attcatcttt tttgtcaaga cgatagttaa tgttacaagc 1440

actttnattg ggctcgaaat agtggtaaat agggtccata ggatatgacc tgttacaagt 1500

ttatttcgat gatcnagccg gcctctgtga ttacggcaat tatttttacc 1550

<210> 23

<211> 2132

<212> DNA

<213> Candida albicans

<220>

<221>  misc_feature

<223>  "n" may be any one of a, g, c or t

<400>  23
tttttaaaag aattaattaa atatgatgga tgatagaaat taaaggaaaa agaagaagaa      60

caaaacaaaa gtttaattga aaaaaaaggg agaaatgaat attgaattat tcagctttta     120

tattgctgat agatgttgaa aaaaaaacgg aagaatgggg atagcaaaac tgtgggtgag     180

attaactcat ctatggcgct aaaagtcttt tttttttctc ttttattagg gggcacataa     240

attattcttt tcattgataa tcccgagtcc gttttttgtt cattattcgg aatatattac     300

cgtattggga acgataatta ttattagttc tccccgatgg ttcgattttg ctggtgcaaa     360

aatataaatc cgatataact ttattggtgc tttgataaat ccgttttata agttggtaga     420

catatacagg atgataataa tttaacggat ttataagttg gaatcatttg gatgaatccg     480

cttggggagg cgttttccaa ttttagaagt ttaactatca attttatgtg acatccgagt     540

atacacattt tgtgaatttg atcttgtaaa ctcacttggt gtaccatggc atttataaca     600

acactttcta gaatcggctg agttacatgc atttcctcta tttgtagatt aatggaaatt     660

catgaaatcg ttcacatttt tttctataat gagtatcgtt cggtttccat aagtagggga     720

ctaaaaaata attgatatct ctaatcagtg acagctctag tcaacttgac cgtaatgttt     780

tgacgaccat tatatttctt gtttgaacta ttgatttatg agtgttgtcg taacaaaaga     840

tcaattcccg tcaaaacgca tttggcactt aatctttgat tgaaccgatt ttgatctcaa     900

aacatagtac caaggtcaat tatgttcgct aatgaaagaa agctgtgacg aaaacctcaa     960

attcatgaag aaagaattac tgttgtggaa aataaaaaag tctttcttct gatactttac    1020

aagtccctca accacaaata caaaaatgaa agttacccat cgatcttttt cattggttaa    1080

gaattaatac gagaatatca aattatctta gagagggtct cacagagcaa ctttctgagg    1140

cacacggtca ccaacatgat ttgttataaa aaattcaacc aaattttgga aaaaatgaaa    1200

acaaaacaaa acaaaatctg aaacatcccg aaagtcacaa atgcttgatt acttaaaatt    1260

acttatttgc ttcaagacgc tattattatt attatgacat aatactactt gaataacagt    1320

gaactgtaat tgtattaaga acaaatcata acaaaggaag atgatgacga tgatgatgac    1380

cccttgaaat atcccagggc acatgcattg tgatgattgt tgtaatatag ctaatgctaa    1440

ttcttgatta gtgtggaaag cctaataagg ttatattgtg cacaggttaa ctaccttaat    1500

atagttattg ttaatacagt tattgctgtt gactactatt gttattgtta aattaaagtg 1560

ttaggttgag ttaattgatt agtgaaaacc aactaactac cgtattaaat tattgtatta 1620

agattgattc ctattaagga taaaacagag agtgtgttag aaagagaaag ggtggattat 1680

aaatatgtgt aaaaatcccc tttagagact aatcactaga aatctattga tggtttcata 1740

tatagagatt aacgattata tttataatat aagttggtag ttgctagtat atttgaaagc 1800

actacagtat agtatgtcag aatcagatca tttaaactct actaataata caggaaacac 1860

tttcattagt ctagatcaag ccagtacaat aatggcagat caaactcaag gagctaaccc 1920

acaacaatta ccatattata tgaagaagac tataacaaaa ctgtagatag taggggattg 1980

ggtatttccg ggggagtaga agtattgggg ttatctaagt ccatctttaa ccacccaaca 2040

atccaacaac aacccaacna cgtttttccc caattctcng gagatnactt gattaacttn 2100

aaatttttcc ntggccaaaa aatttccttt tc 2132

<210> 24

<211> 1734

<212> DNA

<213> Candida albicans

<400> 24
   aataaccaac cagctgctca tttttagatg tatgtatttt ataggaaaat tgaataactt 60
   gttattacta tggcctgttt tctaaagcca agttgtttct tcttatattt tttttttcta 120
   aacaccgttt gttgaagatg gctttatccg tatactattg ggcgtcgatt ttcgcacaaa 180
   agcttttatc cacggaatat ttgcgataat atagtacaaa agtgtgttct agtcttgtaa 240
   atgtccaata tttttagtac aacgatggaa acccgtatag cgcagacaca gtttggatag 300
   atttacgtag gtgatgagga gttaaattga atattcttgt ataatttcaa gagctgtgac 360
   tactatttaa attttttcca cttcactttc tttctcttct ttgacattca agttagtctt 420
   tctgtatttg aataatacta catttatcat gtctcacgtc tcaattgtaa ctggtgcttc 480
   tagaggtacg ttttaatgaa caaaatctat gatgttgaga cttccaattt gaactttagt 540
   actaactcaa ataaaggcat tggtaaggct atcgccgaaa ttcttttaaa aactccatct 600
   tcaaaagttg tgattgttgc tagatctcaa gctccattgg aatctttcca aaagcaacac 660
   ggctcggaca gagtagcatt tgttgctggt gatattacag atccagcaac gtctaagact 720
   gctgttgaaa ctgccatctc caaatttggt caattaaatg ctgtcatgtt gtaatatagc 780
   taatgctaat tcttgattag tgtggaaagc ctaataaggt tatattgtgc acaggttaac 840
   taccttaata tagttattgt taatacagtt attgctgttg actactattg ttattgttaa 900
   attaaagtgt taggttgagt taattgatta gtgaaaacca actaactacc gtattaaatt 960
   attgtattaa gattgattcc tattaaggat aaaacagaga gtgtgttaga aagagaaagg 1020
   gtggattata aatatgtgta aaatcccctt tagagactaa tcactagaaa tctattgatg 1080
   gtttcatata tagagtttaa cgattatatt tataatataa gttggtagtt gctagtatat 1140
   ttgaaagcac tacagtatag tatgtcagaa tcagatcatt taaactctac taataataca 1200
   ggaaacactt tcattagtct agatcaagcc agtacaataa tggcagatca aactcaagga 1260
   gttaacccac aacattttgt agtcgtaaac ttgaaattca aagagaaggg ggggaattaa 1320
   attgggtgca acgtgtttgt caaaaatttg gtgtgaaaaa aattaattta acactctgca 1380
   ttgtaccata gggaatataa tacccagaaa taagagaaat tatcacgtga gactaaaact 1440
   aaatataata aattaatatc acaattgaga aagacactga aactaacttc ttggtgtatt 1500
   aattttcaac acttgatcac aagtgcgggg attaatcata attgcaaaga gtgtgttaga 1560
   aagagcgaag gtggattatg aatattggag aatcctcttt agagactatc cgctaacaaa 1620
   atagatgaac ttgctcaaca gaaacaacta atcgactaac tgactaaaat taatatacta 1680
   agtatagatt aagttatcac gttaatattc tatactatcc atctccatca cttt 1734

<210> 25

<211> 5734

<212> DNA

<213> Candida albicans

<220>

<221> misc_feature

<223> "n" may be any one of a, g, c or t

<400> 25
gagattgtag tgaagaattc agctcattat tactgttttg tcgttgctgg aaggaggagg 60

gataattcaa tgcgccacaa cagtgttact atgcatgtgg ttctgactga ctgatattgt 120

ttaaaaatta accagctctc aaataacaaa agtttaaatt ttcaaggttt gtaaacatgg 180

cagctagtag taggatggtt cataatatta attaattatt agtaataatg gctaagtttt 240

tgaagcattg ttttaaattt tcaaattgaa attcaatttc attacaaatg gattactaac 300

ggaattccta agctcaactg aataccgtga ttgaaacatt tgaatttgta tcttttagat 360

tagctatttt tacttttttt gtcattgtag ttggttatga taattacaag aaactaaagt 420

ttaatatttt aatattcatt ttcttttttg gccaacttgc aaataacaca caaacccaaa 480

attaaataat tagatttaat gcatgcataa ttacacagaa tgtttagcct taacaagtat 540

tctagaaaca agaaagaaaa aatgtcgtct tggcgtttat cttaattgta ttctgtaaac 600

tgggttaatt cttatttcca acttttcatt tttttggatc ttgtatggaa taaaaattaa 660

atatggtatg ttttagggtt gtattaacaa tacttacaat tatcaatcat acagctttac 720

tatttttatt tatcagcaaa taggggaatt caagttgcat gtgttattca gtggcagtga 780

atcataaaac agccaacttg cagcttattt cactccagga gcaatcatca cggaattccg 840

tttcccatct cattttcata ctctgtggat tatgtataga ggctatttac aatatcacca 900

agcagtaaaa cattctctcc tcaaaataac aataagatta gtcaagatga acgacttgaa 960

tctattcata tgcattacac atttagtttc tattacaaat agtgatgcaa tggtgcaaga 1020

ttacgtcttg tctgcactaa ctatttgtaa cgatgattat gtgatcaaga attggaattc 1080

ttattatatt cagtcgtgag tgtaagctat ttcgttaggg ttatcttaac tcgaagttaa 1140

agttccaaaa ctattccatt tggagtttct gttgttgaga aatacaaaat actcttcttg 1200

gtggggagga aatccattaa tgattataaa atgaaactct tggtaaccta attgaaacac 1260

cacattcagt acattttcaa ccgtcactat tattattgtg gcaaatggat taaacaatag 1320

acctaactta atctaatgga aattttaaat ccatgaaagg ggtgaaaatt tgaaatcaaa 1380

ataactatct gaactgaaat accccatgga tctgatatct tatacaatct atcaactaaa 1440

cagggaagag tacctggaat tccaaatgac aattcctatt ataattattt aaacagacta 1500

tgccgtattg tttgtgacat tcattgtttt ccacaactct aatgtcaaat ttttgttatt 1560

gtcatgtaat cccggtgttt cttttttctt ttcggtgttg cgttccatga tattttgtta 1620

tctcttgttt agattgagat aaagaattgg ttagcagtgt agccatttat gagtggtttg 1680

taaaaacaag aattacaagg tttgaatgaa ttccaggcag gcagtattat aaaacctcga 1740

aataactaat caaaccatca gaaaagaaag cttactatga tgtactgctt aatctcatat 1800

ctatcttaca aacttaattc actgattgtg gcttgtccgt gaataattcg gaaaccttgt 1860

ctttttcggt ccagtagggg gtgccatagt cttgggtggt gacaaaaaaa aaaaaaatta 1920

tagttggggt ggtggggtgt acgtctgagt aagtcagggg aatgaactca agacaaaaat 1980

agaagttcta aacatggtac gttctgctaa gtaatatcat cgatctatct attttgctct 2040

aaattttcat aagcaaatcc agaacttcct cgtcagtttc aatttcaagc atacgaaggg 2100

atagtgatta aattatattt tgaaccttct attactgatt aagtgttcct attagtctac 2160

ggattagacg gttagaatgg gatttncaaa agcacaaagg tcaagactta taggaaattc 2220

atagaaaaaa cactctgaag tactcgatgg ttggatatat aatagttttg ctaatttaaa 2280

ctcttgctgt tcggctaagc tattgtaccc aaatgcggta ctccgatagt cttataaata 2340

atacttggca aaagttcaat aaatatatgt caatggtatt gctttccaat taccattgac 2400

gaggttgtaa attaattcat acttaggtga catcgattaa tttaacaaat atgtctgttt 2460

caacgcttac atcatcagtc ttgcaggaaa aatgttattg ccacgacacc tcaaattagc 2520

ccaacccctt cgtctaccaa aacaatgtca aaaacccact taaaagaagt cggacaaacc 2580

tgaacccggt attttataaa gtagttttgt gaataatatc agtacaacga ttacactttc 2640

cgtctcaaga ctggaagttg caaagccatg acaattgctc aaccaaatgt gaatttttag 2700

gttccatagt cttgatcggg taatgtaaac actttaactt ttagtaaatg ataccaccaa 2760

gaagaaagca ctattttaag ctttatttaa cactatacat tggaaaataa aaaagtggct 2820

atgagaatta aacaagatga ccgagtaatt aaaatagtgc tgtcggtgtt aagcaatacc 2880

gctagggttc aatcaattaa gtgctgcttt tttttgtcgt tgtatttcca ttcctccact 2940

cctttcttta ctcttgcaat ctaacatatt ttttttaaaa agaaaacata ttgatactta 3000

catgtggtaa ctattgtctg attcatcaat tccgctcttc aatctcggtg ttcggataat 3060

ttcgatgaaa ttataattac ctgccgcaat tctagaaatt cctttttttt cttttctttt 3120

tctcggagtt ggttccaata caaagattga attgaattag gtgagaagaa gaagagtctt 3180

aacaccagat gtattacagc tttaaacttt gtttctaatt tgaccacaaa aagttgtctg 3240

gacgcctcag tttgaaatta gttttgggag atttctgttt tctcattggc cttactctat 3300

ggaagttttt atacaagagc ttccttctaa aattaactct ttgtgttgta atatagctaa 3360

tgctaattct tgattagtgt ggaaagccta ataaggttat attgtgcaca ggttaactac 3420

cttaatatag ttattgttaa tacagttatt gctgttgact actattgtta ttgttaaatt 3480

aaagtgttag gttgagttaa ttgaatagtg aaaaccaact aactaccgta ttaaattatt 3540

gtattaagat tgattcctat taaggataaa acagagagtg tgttagaaag agaaagggtg 3600

gattataaat atgtgtaaaa tcccctttag agactaacca ctagaaatct attgatggtt 3660

tcatatatag agattaacga ttatatttat aatataagtt ggtagttgct agtatatttg 3720

aaagcactac agtttagtat gtcagaatca gatcatttaa actctactaa taatacagga 3780

aacactttca ttagtctaga tcaagccagt acaataatgg cagatcaaac tcaaggagct 3840

aacccacaac acattcttct tgtaaaatta attctattat aattcaggtc ttagtcgacg 3900

caaaatacca tgttgcaatt gtccgtaaac aattatacaa caatttaacc aatgcaacat 3960

caattgaaat caagaattca acacttgaac atttttcttg ttttcagatc tcgtcaaaac 4020

accagtcaat aaagcttgga aagttttagc acaaccatca aagtagaaag cctaacttat 4080

aggttcgaat tacgtgaatt ttggtttcac taatcacgcc ccaaaaaaat tcanaaaagc 4140

ttagtatgta acatttattg caaatttttt attgttcgtc ataaatgata attagtaaat 4200

gaggttacag aatagttatg ttttacttca taaccaattc tactattttt ttttgtatta 4260

taacctcgga taacacaaac aaaaaaaaag tactactacc aattaatgtt tagtagattc 4320

tacacaaact tgataatgcg ggagttattt ttttttgaag ccactttatt ttcagccgac 4380

ttatctagct acgagacaga acaatactta gcactaattc ttaaaattcc atactatttc 4440

tatcattcaa aatgcatttt aacaatcaat tgtcaaatgt gaatgcaaca aagtcctgaa 4500

tttataaaaa aaagtagatc attgatgcaa aaagtgaatt ctttggaaag ctttactttg 4560

aaccgaaagg agaaggcaag tcgtgcaaca agttattatt tcgtgtacag tatccaattt 4620

tggtttttcg acactaggtc tagactccag aaacaaagtc ctaataagaa aggtgttcaa 4680

aaacaattta attttagtaa aaaaacacaa cctgcatttc gcaatttatg accaaattga 4740

gttagctaat tatagggcat caacaataat atccagcctc acacaaatca gaaacagtca 4800

tataacaact cgaatgcaaa tatcaagact atgttatgat aagagtagtt gggccaataa 4860

gataaaacag aaaaagaaaa ttttatattc tttaaatctt tgggtgacag atcagctcca 4920

attctcttga aattggcaca aatacttcgt cttttttcat tcatcagtat atcacgtgta 4980

gaattgatgc tgatattcaa aaattacccc taaagttgct tatcaacgca acttaagatt 5040

tcatacaagt cgataacgaa tctgaatttc agcttgctct tagattaaac aaaatggtag 5100

attcaatcaa ttagataacg ccaaataaca tttgatgttt tgcggcaata tttggatggt 5160

gtcaactagg agaaaattga ttccccgcca tatctcataa gcctctagct gtccactttt 5220

ctaaataatt gatatggatc accacattgg ggtctaaatg aaacaacgta acccgaaaac 5280

gtgtcaaatt cggaattcgt atgtataatt caaacaatac aagaaatatg gagaaagcag 5340

atacacacat acacactcaa agagcttggt agaataacaa taacttgata taatacgtac 5400

tattcataca caattactta attgatttgc aatcattcct aaaaaaattc tcttttattt 5460

tttttttaat tggtaatatc ggtggtatac aatgatttac ctagttaaac aattgaaaac 5520

aagaaagtat aaaatttctt catttatttt gcttaccctc taccttggta attacaccga 5580

tgtgagtttg gaaatctgat aatcccagaa attggatcta attggntcat atttagattt 5640

caacaaatca taaacagttc tagactccat gtatttcttt tggtgtgtgt atatttttgc 5700

caatgtctcc aaagcaaatg gaactcgtca cttg 5734

<210>  26

<211>  1875

<212>  DNA

<213>  Candida albicans

<220>

<221>  misc_feature

<223>  "n" may be any one of a, g, c or t

<400>  26
cctccggccg ctaattacaa ggctgcttta tattgttata ccttggggta aatgccctct      60

ggcattgagc tatttccaat tcccacttcg gtattttttt ttacagcctc gttagacgag     120

ttcttgatat tactaaatta gttgtttact gagtggcctg atggttcctc gtcactctag     180

tttttggtct atataagggt cagaaatttc ccttctcctt aggtccatca agtcaagata     240

tacattagtt ggtagcatcg tatggaattt tcgtatgaac ggcataccaa gtattaattt     300

ccgatcgaaa ttttttagga cgtcttgata atcaggacaa acatcatgaa aggtctatac     360

gacgaaagtt tactttacac aaggggagac catatgtctt ctttattaac aactagttat     420

atagcgaaca aataagttta tacagaaata tatgtacaca aacaaagtta ttgtttatta     480

attatttaat tagctcggaa gaataactct gtgatactgc atacattcaa acaaaatcaa     540

tctagtttcc aacatctttt tcacttggta atgtaattat tcttgttctg gcaccgacaa     600

tgggtattgt tttgtagctg gaggactaat atggggtacc acctcaattt ttggatccca     660

gctcccacgc aggggtggct tctgatctaa ctcactttcg aaaatatcct gatagtttcc     720

aattaattca gcaaaatagc tcttgtttgt acccttaacc aatgacatga tatccttttt     780

attatcaccg ataccacctg tgtcttcgtc ttgttgtaat atagctaatg ctaattcttg     840

attagtgtgg aaagcctaat aaggttatat tgtgcacagg ttaactacct taatatagtt     900

attgttaata cagttattgc tgttgactac tattgttatt gttaaattaa agtgttaggt     960

tgagttaatt gattagtgaa aaccaactaa ctaccgtatt aaattattgt attaagattg    1020

attcctatta aggataaaac agagagtgtg ttagaaagag aaagggtgga ttataaatac    1080

gtgtaaaatc ccctttagag actaaccact agaaatctat tgatggtttc atagatagag    1140

attaacgatt atatttataa tataagttgg tagttgctag tatatttgaa agcactacag    1200

tatagtatgt cagaatcaga tcatttaaat tctactaata atacaggaaa cactttcatt    1260

agtctagatc aagccagtac aataatggca gatcaaactc aaggagctaa cccacaacac 1320

gtcttcttca gtattaggga acaacatact aacttgacct tttctagctt caaccaaaaa 1380

ttcctctata tccattaatg gaatttcatc aaactgagca gccccaaaaa acgttttgct 1440

tccaaagtct aaatgagcat ggaatttcct tatgaaaggt ataccaagta ttaatttctt 1500

atggaagctg tccactacag caaaattctc ttggaatgta ataccattaa actggaactt 1560

gaggttaatt atttggttaa agtttctgtt gatttttggt ccaataaagt acccaaacta 1620

ctagagctcc aacaacattt tcagaaaatg gccaataata caataagtgg gtatatttta 1680

tcaaaagagt ttatattatg gttactcgac gggtattatt ctctgttgga ttaaggcatc 1740

tgggcgaccc agtgggacca aaattccaga gtagtggttt ggtttaggac tttaccaagg 1800

nccatgatta gggaatattn taaccaaaaa attaaaatta ccatttaatt cnaaaaccta 1860

acctaaattc cctaa 1875

<210> 27

<211> 1712

<212> DNA

<213> Candida albicans

<220>

<221> misc_feature

<223> "n" may be any one of a, g, c or t

<400> 27
taaccatgga attcctngaa ttantnataa ttaaccaaat tttttagggn ttattaggac 60

ctaggattga attccatgtt tatttaataa ttaancccca gtttggccaa ctatgaaata 120

gtataatggt taaatgcaaa ataaatatag tatgaacaat atgatagttt tagtgtgaat 180

tttgaataag aaaaagaagg gataaggata tttttactag gaaactcaat tataattact 240

aatgataaaa actccatcag ctactattat tactcaaatt ttaaatcatt tgtttatcac 300

ctacacaaac agggattgtc caatattgat tactaaaatt agaacaaata agagaatata 360

attgaagtta aataattctt ttactaaatc tattgaccaa gaactacatc aagggaaagt 420

gttgcatata catctaatgt ttattcttgg ttagagtatt gatacaaaat tatatcatca 480

ccaacgaatc acattaaggg aaagtgttgt gcatatacct gatgcttagt cttggttaaa 540

gtatttgtgt gaaaggttat cgtgaccaaa gattatagta agggaaagta ttatgaataa 600

atccaatgtc tacttttaca gaagtattga catgagagat tataactatc aagaattgca 660

ttaagggaaa gtgttgtaat atagctaatg ctaattcttg attagtgtgg aaagcctaat 720

aaggttatat tgtgcacagg ttaactacct taatatagtt attgttaata cagttattgc 780

tgttgactac tattgttatt gttaaattaa agtgttaggt tgagttaatt gattagtgaa 840

aaccaactaa ctaccgtatt aaattattgt attaagattg attcctatta aggataaaac 900

agagagtgtg ttagaaagag aaagggtgga ttataaatat gtgtaaaatc ccctttagag 960

actaaccact agaaatctat tgatggtttc atatatagag attaacgatt atatttataa 1020

tataagttgg tagttgctag tatatttgaa agcactacag tatagtatgt cagaatcaga 1080

tcaattaaac tctactaata atacaggaaa cactttcatt agtctagatc aagccagtac 1140

aataatggca gatcaaactc aaggaggtaa cccactacag gttatgagcc tcgcccgctt 1200

attgaattta gataatatag gggcaatgaa agcttttgaa agtgttgatt ttcctgaatc 1260

attaaaacta gaatccaaga ttaattttca agtgtggaga aatgaaatcc ttagatatgc 1320

acgtggtatt ggtgctgagt ttgaaaactt tgtattgaat gaaactccag ctcacctgta 1380

tgatcttaga ttgggaaata tgcttcatca attattgatt cgcactgtga aagaaaaagt 1440

tagaatgcct aggcaagaac ttggaaaatc aggaaaagaa ctttatcttg atcttattaa 1500

atcattcggt actcaatacc catacgataa atttgagata gttaaatact attgggatca 1560

gttaacaaac cctttaatta atgtgaagag acgttttgaa attgaagaag tatgggttca 1620

atacattaat gctcaaactg caacagagag agaagttctt aattcatttg tttggttaca 1680

tttgtcaaaa tctatattac cacaagagta cc 1712

<210> 28

<211> 1540

<212> DNA

<213> Candida albicans

<220>

<221> misc_feature

<223> "n" may be any one of a, g, c or t

<400> 28
tgtggaatta agatgacttt gtgattaaat tgttgacttc tttaagcctt ttaatgtgga 60

ggaaaaagaa aaatctataa ttaaaaaaaa aaaagataaa gcagataatt ctttgatctt 120

tatatacttg gtctatatgt agtaggggaa agtcggagtc ggaatttgaa aaaaaaagag 180

aaaaaagaac gaatatttag actgtaaaat tcaaacccct gctgattagt atataaaaaa 240

aatgagttca tttttccttt cttttttttt ttttcgcgcg gatagcaacg gtcattaagt 300

taacgagata aaaaagaaac aaccagataa ttatgaaaag ttgtgatggt gtcacgtgcg 360

aacatgagag tcatgaattt tgacgaaaac gtcaagcttc agtttacaaa agacctcttt 420

attaaaatcg aattgcttat agggtcgtcg atgatgagaa ggtgtatgtt gtaatatagc 480

taatgctaat tcttgattag tgtggaaagc ctaataaggt tatattgtgc acaggttaac 540

taccttaata tagttattgt taatacagtt attgctgttg actactattg ttattgttaa 600

attaaagtgt taggttgagt taattgatta gtgaaaacca actaactacc gtattaaatt 660

attgtattaa gattgattcc tattaaggat aaaacagaga gtgtgttaga aagagaaagg 720

gtggattata aatatgtgta aaatcccctt tagagactaa ccactagaaa tctattgatg 780

gtttcatata tagagattaa agattatatt cataatataa gttggtagtt gctagtatat 840

ttgaaagcac tacagtatag tatgtcagaa tcagatcaat taaactctac taataataca 900

ggaaacactt tcattagtct agatcaagcc agtacaataa tagcagatca aactcaagga 960

ggtaacccac aacatagaat acgttttcaa ctacttaagt atccactaac ctaaattttt 1020

tttttaataa aatttcattg tattagtctt tcttactgct tttaatcaac tataagtata 1080

ggtttccgtt ttttttgcag taaaatttat cgttcaggag aaataacaaa atgtacacga 1140

cttattcgca gcattttttt ttttgttttg ggtttttgta tcaaattgtt acaacaacaa 1200

caacaacctc aattcttaac caaatctacc cctcctattt tttttncnca tacacacaat 1260

acatcttaca ctatcttttg ataggcttta tngaagangt atttanggng tgtaatgaca 1320

atctgcttaa cncatatatn tatntanngn nngtngtcaa caatagcttt atctactttt 1380

tttttttggn nacnccngna acttcaggnc cacnnntttg ccnattttgg ggccccnatt 1440

nggaaaacat gggnattggg annacagctt tttttaggnn naaangggtn ttnccntttn 1500

tggtgggctt ggaaagnaac agcntntaaa nnaatgggct 1540

<210> 29

<211> 2025

<212> DNA

<213> Candida albicans

<220>

<221>  misc_feature

<223>  "n" may be  any one of a, g, c or t


<400>  29
tggggagcaa atgtgaaatt aaagagtgtg gtgatatgta attttttttc aaaaaagatt      60

ggattgacga agcattatat attcgtctaa aaaccatttt tgctggttcc gcaataaatc     120

tcggagatta tttctcgatt accaatttat gttgttttgt gacatttctt atattttgtt     180

ctattttaca cgactattta ttgttaataa atatgtcacc taaagaatat ttctatttag     240

ttttacatat gttttttgac gacaatcaac tattacaaat taacctacat tttttaattt     300

gaatatatac aatttatatt gaattaacat taccatttag tttttgataa gaatagattg     360

cgctatttca aacatttgtt aaattattta ttgtgaaaca actatgtaga ataaaagtat     420

gaacaaattc tacgttcatc atgtggggtg tgccttcata tatatctttg gatgagaatg     480

ccaagaaaaa tgatggcgtg acaattcaat acggcaaaac aaactaatcc cctctaagat     540

tttactagtg tgtttcccta tcgtctgagg aaaaggtaac aaaacatcgt ttaaccaatt     600

ggtgtttgtt acgatggtga cgttgagtac tgcatatagt tgcaacggca aattgcatcc     660

agcgagttaa cagcgaatgg caaagtgaag cctccgactt gtgttcattg actactggga     720

ttggactggg aataacgact taactaatta atgttctcgt ggactcgttt agctagaact     780

aacatttgtt ataatatagc taatgctaat tcttgattag tgtggaaagc ctaataaggt     840

tatattgcgc acaggttaac tcccttaata tagttattgt taanncagtt attgttgttg     900

actactattg ttattgttaa attaaagtgt tagggttagt taattgatta gtgaaaacca     960

actaactacc gtattaaatt attgtattaa gattgattcc tattaaggat aaaacagaga    1020

gtgtgttaga aagagaaagg gtggattata aatatgtgta aaatcccctt tagagactaa    1080

ccactagaaa tctattgatg gtttcatata tagagattaa cgattatatt tataatataa    1140

gttggtagtt gctagtatat ttgaaagcac tacagtatag tatgtcagaa tcagattatt    1200

taaactctac taataataca ggaaacactt tcattagtct agatcaagcc agtacaataa    1260

tggcagatca aactcaagga gctaacccac aacagcattg attatataat catctatgta    1320

gccaatatac actaccgtcc aaactcccac tacacacttg taacagtgtt ttacaaatct    1380

atgaacgaat aaccgattca aatgacacaa taaagaacat ttcaccgatt tgaattgcta    1440

atcggtacta taatattgat ggaaggttaa gagtttaatg ctaccctagg tttaccggag    1500

atcaacagtt gcatatacaa aacgtgttat ctgtctacga atggctttct atgtgtataa    1560

aatgtttcat caattgataa ttaattatta atctgcttac tgaggtaaac cccttttaat 1620

gcaatagcaa atatgaggta tttttttgct attgacatgc gtatatgaat ccatttgtat 1680

caaattgccg atataatgaa atggaaatta agggaaaaaa aaaagtttat atccaaattc 1740

atgcgattaa caggttcttg tgattataat tggtaacccc ctccccccta aaactcatat 1800

ctgccaaaag aggaggatat ttgaatatgc tattatgaac cccattgatt ttgactacaa 1860

ttggatttgt cgggtattga aacccaaaca tattataatt tgctatgcgt ttaaatcaac 1920

cgtttactgg tagatcctat actataaata cagccaacaa tccccaattg ttcagataaa 1980

gtaacactca atatcatttg atcaatcaat caagaggatt acaaa 2025

<210> 30

<211> 3583

<212> DNA

<213> Candida albicans

<220>

<221> misc_feature

<223> "n" may be any one of a, g, c or t

<400> 30
   aaaannttcc ccatngccta ttcctaggnc ccaaaaccag ttgtccgaaa ctccatggat 60
   gccagaagtg gtggtcctcc gccgttatgg ttggaaaaga aaaagaaact tgacgaattg 120
   aaagtcaaag aagagcggca agaaagaagg aagaaggggc aaagaaaaag gaagaagagg 180
   caaagaaaaa ggcagaggaa gcgaagaagt gttttatttt acttttctgt caaatttgca 240
   ctacttttaa tttgtgtgca aatattctat tttacttgat ttttatatac ttttatttta 300
   caatactttt ttataggact ttttatatct tttctttatc aactgttcgc tatagggtag 360
   gtcttccaag ctaattttac ccgacacaag atgaaatatt ttctgttgag cactcgttgt 420
   cgacagtgaa aaattttcac tcaagaaaat attttatcat cactttttct agaagggagg 480
   ttcaagtgtt ggagaataga cagcgaacac ctgatattcc caaggtcgaa ttagattgaa 540
   agataaataa tagtcatatt tattttgtat ttagtcaata aattatcttt ttatatttaa 600
   attcttagta ttgtcatacc acgtagattg atacggacat acttagcaca tttaacatat 660
   attaagcacc gattacctgt gacattccgg agtttactgt ttcgcgcacg ctggcagacg 720
   aacatcaact catcttttat acaatatatt cttacgatta taactttcaa ttaagaaata 780
   caacttctta ttagcattct cctacaagtt cttaagttcc taggaatttc ttcgaaacta 840
   taattaaaga cggaaaagtg taaaacaaac agaaagcaga ggaggccaag aagaaagcag 900
   aggaggccgc cccacaaaag tttgacaact ttgacgactt tattggcttt gacatcaacg 960
   acaataccaa cgacgaagac atgttgtcca acatggacta cgaggaccta aaattggacg 1020
   acaaagtacc tgccaccaca gacaacaact tggacatgaa caacatactt gaaaacgacg 1080
   agctgatact agacgggttg aacatgacat tgctcgacaa tggcgaccac gtaaacgaag 1140
   agtttgatgt agacagcttt ttaaaccagt ttggtaatta ggggctctgt tctacaagac 1200
   atatacagat agtgcaggaa taagaaaaga aatattttat atagctatat atttcaagtg 1260
   tttattctgt tcaacaagtt ctaaccgtag atacaccaaa tcaccaagtc agacattact 1320
   gagctagctt aacggtccaa ctactttaaa ttgcaatccg ttctttactt gagtcagtcg 1380
   actctacaac aactatcctg aggtgattat tttttggtgg aaattttgac caaattctta 1440
   agcaaaaatc tagtttctac tgataaataa atacacattg ctctacttct gtactccaca 1500
   ctctgctatt gcttgatagc catccttaaa tcaacagaat ccactaattc tgctacttcc 1560
   agaaccatga ctactctaca tttttaacca tctcaattaa ttaccatctt tttctctcat 1620
   tatttggcac tatggccgag ttggtctaag gcggtagact caagaattat tcttctcctg 1680
   cgatccaggg gtttctacta tcgtaagatg caggagttcg aatctccttg gtgtcattat 1740
   tttttttttt ccaagaacct ctcatttttt tttttcaaaa attatttcta caatttcctc 1800
   tattcttaaa aatctttggt attaaactaa aaatgtacct aactaaacta ctaggctgga 1860
   aaataataaa tctaacgtta acgaaataag caaaagtaat tttttttttt caagacaatt 1920
   ccatgtttgg ggatgaaaac tgcctgcaat tatatatcct gtaacaatcc ccttatatca 1980
   acaacaaccc gagaacaaca aaaagtccac tggcagaaac cttaccacca atattctcaa 2040
   tttgtgtcac tgattgggca gtttgtgtcg atatccatga tgtggtcaaa ctggcagcag 2100
   tggtagatgg ataaacactt tcagcagcaa cagtaaccga gttgacaact tccttagcag 2160
   cttgtgtatc acactcttca tcatcatccc agctatcatc ctcatcgtca cactctggtt 2220
   caggagtttg atcatcttca tcatcgtagc catcttcacc agggcaaaca taatcgttac 2280
   cagatccacc ccaccagctt ccagacgatc caccagtaac tgaagaagaa ccggaatcac 2340
   ctgaactaac accagaactg gatccagaag tagtaccacc acttgatcca gcaccagaac 2400
   cccaccaaga gcctgtgcca gatccagaac ttgatccacc tgttggcaca cattcgccat 2460
   catcttcttc ataccattcc cattcaccat catcagagga gccactggca gaaccaccgg 2520
   cattgtcttc cccttcatag ccatcatctt cccagtcatc tggatagaca gtgtgtgtgg 2580
   taataacagt cacagtcgtg gtatatagct gtccacctgg agcaacagtt gtcagtggac 2640
   atgtggttgt gattgtcaac gtaacagttt catcacagat ttcaccagat tgtgtgagat 2700
   aagtggtaaa tgtctgacca ccaccagtat atgtgataga aacaacttcc gtttcagtat 2760
   gttgattagt ggttggaggt aattttgtgg tgagtgtttg agttgttggc accccatcgg 2820
   aagtaaatgt tctagtggtt gacacagttg gatggatagt aggaatttca gtttcacaat 2880
   cagtctcgtc atcgtcgtca tcagaagtgg ttgactttgt tgggagaaca gtaatagatc 2940
   ctgacccagt tggaataata gttggaagaa cagacgttgt tggaagaact gacccacttg 3000
   gaatgatggt tggaacgtct gtctcacaat cagtctcaat tatcttctgt agtggctttt 3060
   tgaaacaact gacgagacac ttgtcttact ttgactggtg attggaaggg ttggaattgt 3120
   aggaccaaaa tttgggcctt ccattggatc tttacactct ccaccactgc acaactttaa 3180
   tttggaacca caactggaac tagtttctgt ttcaaggctt taccagttga cctgatcgta 3240
   ataagccacg gggttaccaa cttgttgcat cttcactgat cagccatcaa tctttgataa 3300
   gccctgattt ctctcatcta tgcaacaatc ttctattgtg aatcatttgt tttgctaaac 3360
   ttgtagttgg tgtccaaaaa aaaaagtgat gtaaaattta aatttttctg aacttgtcgt 3420
   gtaaaaaagt ctccagaaaa agggacaaca cacacaccaa tttttcacca taccacacaa 3480
   ttcaccaata agctctctca tatccatcna ataattacag tacagcctcc tattcncaat 3540
   ttttggnatt taaaccagtt cccttggcag gtcaccagtt cat 3583

<210> 31

<211> 770

<212> DNA

<213> Candida albicans

<400> 31
tgatttgaga aataccattg aagatctaga gttaaaaata aggaatttgc atgtacatga 60

ggataatcaa gcggtcatta caatcttaaa gaatgataat ttccacccac atagaccgat 120

tgatatatgt tacaaatttc tcagacaaaa attgaaagat ggattttttt caatatcata 180

tgttgaatct ggagataatt tagctgactc attcacgaaa gctttaggaa gaaataaatt 240

gattgaacat accaaaagga ttagagaaag aaaggattat gataataatg ctacactgat 300

agtggacgtt aggacgctcg aagagattaa gataaacaag aaattggtac atcattaatt 360

aatttagctg tttacctgaa tcaggggagt gttcgctata gggtaggtct tccaagctaa 420

ttttacccga cacaagatga aatattttct gttgagcact cgttgtcgac agtgaaaaat 480

tttcactcaa gaaaatattt tatcatcact ttttctagaa tggaggttca agtgttggag     540

aatagacagc gaacacctga tattcccaag gtcgaattag attgaaagat aaataatagt     600

catatttatt ttgtatttag tcaataaatt atctttttat atttaaattc ttagtattgt     660

cataccacgt agattgatac ggacatactt agcacattta acatatatta agcaccgatt     720

acctgtgaca ttccggagtt tactgtttcg cgcacgctgg cagacgaaca               770

<210>  32

<211>  106

<212>  PRT

<213>  Candida albicans

<400>  32

Asp Leu Arg Asn Thr Ile Glu Asp Leu Glu Leu Lys Ile Arg Asn Leu
1               5                   10                  15

His Val His Glu Asp Asn Gln Ala Val Ile Thr Ile Leu Lys Asn Asp
            20                  25                  30

Asn Phe His Pro His Arg Pro Ile Asp Ile Cys Tyr Lys Phe Leu Arg
        35                  40                  45

Gln Lys Leu Lys Asp Gly Phe Phe Ser Ile Ser Tyr Val Glu Ser Gly
    50                  55                  60

Asp Asn Leu Ala Asp Ser Phe Thr Lys Ala Leu Gly Arg Asn Lys Leu
65                  70                  75                  80

Ile Glu His Thr Lys Arg Ile Arg Glu Arg Lys Asp Tyr Asp Asn Asn
                85                  90                  95

Ala Thr Ser Ile Val Asp Val Arg Thr Leu
            100                 105

<210>  33

<211>  598

<212>  DNA

<213>  Candida albicans

<400>  33
cttcaatgct tcacttgtac tagtacccat gattgtatag tggtgtggtt gatcgacttc      60

aatataacaa gagagagatg agatgagatg cttttatcgc gtatatattt ttttttccat     120

tgacaattct gatttcacaa attgttcgct atagggtagg tcttccaagc taattttacc     180

cgacacaaga tgaaatattt tctgttgagc actcgttgtc gacagtgaaa aattttcact 240

caagaaaata ttttatcatc actttttcta gaatggaggt tcaagtgttg gagaatagac 300

agcgaacacc tgatattccc aaggtcgaat tagattgaaa gataaataat agtcatattt 360

attttgtatt tagtcaataa attatctttt tatatttaaa ttcttagtat tgtcatacca 420

cgtagattga tacggacata cttagcacat ttaacatata ttaagcaccg attacctgtg 480

acattccgga gtttactgtt tcgcgcacgc tggcagacga acagattaga agcttggtaa 540

atctttggtt attcatcacg tcttgagaat aatacaaagt ttaatatagt attttcaa 598

<210> 34

<211> 1082

<212> DNA

<213> Candida albicans

<400> 34
ataaccacaa taatcggcct cgtaaacgtc gtcagtggct caaacacatt gctgcacctt 60

gagctctaga acaaccccac actcactagc catcgccaca ccaacaacca aattgctgat 120

ccagaaaaaa taccaccccc gtagtccggc ttgtatggaa taattgcttg gccaggtacg 180

tccccacctc atcgtgtctt ttctggttga aatatgtcat ctcccgggct aacagtaccg 240

tatctctgtg gctggggcat ctatactctt tcattctcgg cttacaaatc tatcttgttc 300

acacatttca tatatctggg acttgtcgaa ctctctgcac tctatcataa actggaactc 360

gcttgcattc tgggacacac actggagctg gaatccatgg tcaggaaatg tgaaaatttt 420

cttctcggga aatatttgtg acaattagtc ctagtacacg atagtttcat tacgcccact 480

aaaagtgtct actgaaactc ggtctctata tcgtcaatat ctttcatttc tcttcctggc 540

ttttcactgc gacttattgt tcgctatagg gtaggtcttc caagctaatt ttacccgaca 600

caagatgaaa tattttctgt tgagcactcg ttgtcgacag tgaaaaattt tcactcaaga 660

aaatattttc atcatcactt tttctagaaa ggaggttcaa gtgttggaga atagacagcg 720

aacacctgat attcccaagg tcgaattaga ttgaaagata aataatagtc atatttattt 780

tgtatttagt caataaatta tctttttata tttaaattct tagtattgtc ataccacgta 840

gattgatacg gacatactta gcacatttaa catatattaa gcaccgatta cctgtgacat 900

tccgaagttt actgtttcgc gcacgctggc agacgaacac ttatcaaggt gctactcccg 960

cgcatcagtt tcctctgggt tctctttttg atcttggtga actacctttt tttcccactc 1020

gcgtgagaag ttcaacactt ttttttaccc atccaccaaa ctttattctt ttccccacca 1080

tg 1082

<210> 35

<211> 994

<212> DNA

<213> Candida albicans

<400> 35
tagatattta tatatgtata tgattagacc aacataaaac tagacgtcca aatatttatt 60

tatttattta ttgatatata ttcttattta ttactgttat gatcttttga ttcacacaga 120

gatttaatcc aaatcaatac cttttgtttt gtagaaatct tttgcttctt caatttgtat 180

tttcaattct ttgtatttat gttctttgtc tttgaatgta acaattcccc aacctaacgt 240

tgataaggca taagacccaa atgtgactaa tccccaccat ggcaagtatg gcaatatttc 300

atcgtgtatt ttagctggag ttggaatcac acctgtgata agagcaaaat aaatagctga 360

taaggcaaaa attgttaatc ctgtttcagt agctttagtc attcttatag ttagacttgt 420

taaagggtag ttgtgttaat tgaagatatg ctggaaaact atacttttcg ttgttttttt 480

ttttcaatct aggtcgggtg tgctgttatt ttttttctct cttcttggtt cttagtattg 540

gattatatgt tggtttatgc gacgtttgtg tcagggaaat aacaccttga tataagtcgt 600

gcgtattagg tcaacattgg tgaaaaattt gcactcatcg agagccagga attagtataa 660

aaagaagaga aaagaaagat atttaggata tttattatat agggaccgag tttcaggaga 720

cacttttagt gggcgtaaac ttcattcact ctgttttttg cttattacaa attatcacct 780

atcgtgtact aggactaatt ctcacgaata ttccgtgtat acaaacactt attgccaact 840

tatggtgcgg aactttattt gtctgaacca aaatcaaagt cacatcattt aaatgaacgt 900

tgacataaat agattcttta ttcaatagaa acaatttctt cctttttctt ttctttgtat 960

tattggttag atttccattc catatacaca caag 994

<210> 36

<211> 1348

<212> DNA

<213> Candida albicans

<400> 36
tgtatggtac atgtacgaca gcccaaaaaa tggtatcatt tagaactgta ttggagaaca 60

ttagttttgg tccaacattg cgtgatgatg gtatgttttt cgtattatag tacaatgatg 120

gctcaatgat ttattttagg tttatatgtg gatgatatct taatggacag aatctcagat 180

ggaatcgtta tcagatttgt tgaacaagag agagtttatt tcgcgtcaaa atcaatttag 240

gtctcatgac agaatatgtg agataaaatg tccacgtaag caaaactggg tgatactttg 300

aattaagaga tactcctaaa taagcaaacc aaggatttta aactacacaa ttcgtatggt 360

aaaacgtgct ttgagttcca aatgatagat gcgagatacc aacaaaatag aactgtcgca 420

aatgctgaag acaatttcac tgaggttcga aatgaaaaat tacttaattc aattaaaaaa 480

tttataccaa aaggtggtct ggaagtgctg atatgaacac gaaatttaat gcattctgtg 540

gaaaattcgt ttaagctcac aatcggaaaa tactaccatt ctacatttgc agaaaattaa 600

aattgtgttg tgaaatatct acatcctaca aagttcaaga catttattga tggtatattc 660

aaaggactcg atgttgagaa tgataataac ctgaaccaag acgctacaaa tgctaattga 720

gtaattcgta attgctaaac aacgccattt cgaatcaggg gagtgttggt ttatgcgacg 780

tttgtgtcag ggaaataaca ccttgatata agtcgtgcgt attaggtcaa cattggtgaa 840

aaatttgcac tcatcgagag ccaggaatta gtataaaaag aagagaaaag aaagatattt 900

aggatattta ttatataggg accgagtttc aggagacact tttagtgggc gtaaacttca 960

ttcactctgt tttttgctta ttacaaatta tcacctatcg tgtactagga ctaattctca 1020

cgaatattcc gtgtatacaa acattatacg tgtctgtaac tacgcgaaac tacttcgtct 1080

cagttttttg ttacaaacaa ctttccgtat agacctgaga ttttgtcagc ttgattgaat 1140

ggaagagttt actaaagtac cagaaaggtg ttttatagat aacatgtaga tatataaaaa 1200

tgttatatta caaatgactt ccaaaagaaa ctgtacgaat tttgctgttt attaaaaacc 1260

agttcctgaa aactagtatc ttagcttcag tacatttagc ccacctaaat tggacctatg 1320

acaagttcta ctttcccgac aatgctaa 1348

<210> 37

<211> 3034

<212> DNA

<213> Candida albicans

<400> 37
tggttggtct tatcagtaga ggagtgagta tcagttgctg tggttttttt tttttttttg 60

tcgtcttcaa attttgttgg tttatgcgac gtttgtgtca gggaaatatc accttgatat 120

aagtcgtgcg tattaggtca acattggtga aaaatttgca ctcatcgaga gccaggaatt 180

agtataaaaa gaagagaaaa gaaagatatt taggatattt attatataga gaccgagttt 240

caatagacac ttttagtggg cgtaaacttc atttactctg ttttttgctt attacaaatt 300

atcacctatc gtgtactagg actaattctc acgaatattc cgtgtataca aacaaaattt 360

tcgaaactag tcaatcacaa caaatttgtt tgagttcaac tgaaacgata acaaccatca 420

taattcgatt gaatactttg tgtcgtctct ttctttctat gcattctact acttgtcgac 480

tacatatatc cagccatgtc ttgcatatat cctagcaact cctccctccc ccctattgtt 540

gttgtttttt ttaataatat ttagtatatg tatcaatggt aaaaactatt ttttgtattt 600

tttttggttt tgtaaatttt gatagttttt ttattgaaaa cttcaaatct caaaaatttc 660

taataacaac aacgacaaca attattaaat gatactctac tcaaaaagaa aatttgatga 720

aatgccaaga acaatataat ttagtcagta cattaatact caattacaac aacaacaaca 780

acaacaacaa caacaactgt tcaatgcaat aataagagag aaaccaatag aactaattta 840

gtttttcaaa tagccaacct tcaaaaaaaa ataaattatg tgaatgcata aaatatgtat 900

tattagtagt agtttgtagt tgttgtaacc agaattctca atacatactt tttcatatcg 960

atcctttttc ttcttcctcc tcgatttttg gattatatta actaaatttt gcatttacgt 1020

ttataatgat tttcaataca aaaaaaaaag cattataaac tatatattat cttgaatagt 1080

aaaaataaat tagtattgat agaaagtttt ttacatctga cattatttac taatttaagg 1140

aagaatggga cttaaaaaaa tatctaaaaa cccatgtgtt ctagtttttc atttgttatt 1200

agcttattat actttacatt attatttttg ctataatcta gaaaaaaaaa agtagacttt 1260

agatctaatg tataattggt atattgatag ttttttaatg ttttttttat taaatcattt 1320

catttatttg gtcttctttg ttttggtatt gtctatgtgg ggtggcggag ttgggtgcaa 1380

cgcaaacaaa aatatttttt agcaattaag tttttgccgt actgtatgga aattagttcc 1440

attatgatag cattttgcat ctttgattaa tttttatcat tccatagcaa caattacttc 1500

tttctcctcc ggtgtcaatc aatcccatat aggtcttgca ttgttttgtc aaacgtttca 1560

aattgggaat tgtttagttt gaaaaactat agatttcctt atcttgattc agatctctct 1620

ctcagccatg cttatgtaac ttagctattg tttctgttat tgttattgtt gtttggtgat 1680

tatcgacatt tgggttcatt ttataaaagc aaacgagaga tcgatagcaa ttataaaaac 1740

cattacacac acccaaaaaa atcaaagtaa tatgttatct aataggacaa ctgatgtatc 1800

ctttaattta aatattttgg aataaaagta cacccctttc catcatattc atgtgcaatt 1860

taaaaggaat caattatcaa aaacccaact aaccaacaag tttctggtat atagcctttc 1920

tgtccaattt tttttttttt tttgaaatct aaactactgg cctctttaaa ctaaaatcaa 1980

agatcacttc ttaattagtt ttgtagatcc agaatcgtta ccaatactgt taataaatga 2040

ttgaatgatg taatttcaaa tagcaatcgt tgagtatatt ataatcaatg aatagctaga 2100

tttagagaca attataataa taacgaatca tcacaaaaaa aaaagtggtg tacagaaaac 2160

gtatgtatgt aaactagata caatggaaag ggctgggagc ggaggggggg ggggggggtt 2220

taattctgat taagaaaaaa aggggaagga catggaattt atccacatga gagaaagggt 2280

tcctaaaaga tgtcctttac ggtgggcccg gggaacccca attttcagaa atttcacctg 2340

tttggggcgc ataatgttca caacccaggg ttgccttaat gacgtattct ttacaatttc 2400

atcaaaccag ttgttgttgt ttaaataaaa gttgatagtt gtattgctca aattcaaggg 2460

gggagggggt ggtgaattca tatttctcat atatcacact catatttgcg aatacttgaa 2520

ttactctaca tttatgcttt tcacatggat caatttaata taagtacatc aatccaatat 2580

gaacatgaat gtaccaacta aaattaggtg ttagtctgaa ttcttgttca ccattgttta 2640

gttttgtttg tgatgaatct caagatacag attggtttta caataatacg tttgttgttg 2700

ctgtatgaac aggcagtcac ccttcctccc ccacaaaaac atattctgta taatctatgt 2760

aatattataa gatccaatca aaacatcacc accaaataat actgtagtaa tgcctaatct 2820

aattactaaa tagaaatata gaatggggta tggttgagat ttttgggtaa ggtccaattt 2880

gccaaaaaaa aaaaaatatg caaccttttt ccctcctcca cctccttcct atttcgtgaa 2940

attcggtaga atccgaaaga ctaatgaaga aaaaatcaag aaaaaaggtt aaggtcattg 3000

atcaattgat ggcaaatatg taagtaagtt cgat 3034

<210> 38

<211> 3504

<212> DNA

<213> Candida albicans

<400> 38
ttttctcttc tagcttgcaa tttttgttga cgtttactag tagcagaatt ggtttgttta 60

gtttctgctt gttgttcctc tggtgtagag ccatttgatt tattcttttt aatgaatggt 120

aaaataaaat tactcaattt gtaaatagca aatccaggaa ttatcaagta cccataccat 180

actttattac ttccaaaaat aatcatcaaa atatcgaacc cccaagtcaa atagataaca 240

tcaaaataat attcatataa actccccagt aatctaatgt cttcaccact tgaaactaaa 300

gagttaccat tggtatattt gggacgacca aatttttcca aagaatattg taaaaatata 360

cttgggatgg agaaaattat ccacggttta taggaagatg gacgatggaa aatggagata 420

attaaaaaca caataatgtt aattgatgcg gaaatgatta ataattgatt taatatgttg 480

gtattggcta ctgccaactt cttagctgat gcagatgcca ttgttaatat tgttaaattg 540

ggtaaatagt atgaaggaag ctttggcagg cgttgttatt tttttcacca attattatca 600

tcacctgcgg aggttagtca atttgagatt gtgcgaggga aaaaaaacga cctccataca 660

ctacctcaag tataagtcca gtccaattgt tcgctataga gagatttcct agccggaatg 720

cacgacaatc ctgagacgga agtcgatcgt cgatgcccat ggtgcgtggt gaaaaatttt 780

cttagaaaat ttgttctttc cttcaactgc tttgaagaga gggaggttca agtggtttaa 840

gtacgacggt cacaaagatt gcggcttatg aggcccgaac tgagttgaaa tacaaaatca 900

agatataatt atatacctta cttgtctata ttgttttata atacattctt cagatattta 960

aatttctgtg tatcatccta taaaacagag atacattcag tgcatttagt atactgagtg 1020

aactggtacc tgtgacattc aagataactg tttcacgcac gctggcagac gaacaccaat 1080

agtatgatga agaactgacc atggtgtaag aggtttgatg gagtttcttt tttttagaag 1140

aggttgataa gccaacagat gaggagtaac aagtaactcg caacattgta taacataagt 1200

ttacatcaaa tcagaattta ctaagaaaat caatccattc aaaaggcact caatcattga 1260

aaaaacgagc ttaatgagta gacggtctgt tcatatgaaa caattgaaag ggttgaatat 1320

tgtttggaaa attatataat tcatgtcaaa ctgggaggct taaattatgg tcactccaca 1380

gattatgaaa cgtagttaca caattcttgg acctggaaat cccacaagag agcgttagtt 1440

agtttgcact ctcctcacca gttaaactac ccatgattct ccaatgtggc ttatttaagt 1500

atcagacaac agatacatgg tttccaagtg gtctcatttt tggtttactg gagtctgcat 1560

tccccacaaa agtacctttc aaaactaatt aatgtagctt ctatttgata gcctctgtta 1620

tggaaataga tttgctctgc ccagtgggtg taattattcc cagctggaac tattccgata 1680

gatatgtttt aatgtcaatt taaatcttgt aataatagta aggatgcggt ttatccgcga 1740

tcttcttaat acctgtggag ttactccaga acagaggttc aattttttct tggttggtaa 1800

attatccgag taacacgggg tagcttggtt actccagttg agaatgtaaa ctatagatga 1860

agatttcaac acgcaattat taccccacct tggcgaatta ctaatcgact atttgttaat 1920

ccagaaaaaa ttatacacaa acactgcctt tttttaaaaa aagcgttatt ttgatggaac 1980

gataattaac gatggttctg cacaaaaatg tggtccaaag ccccagacta ttctgaagta 2040

tgatttgtta cttaatttag tgaataatta aacataaaat ctggagaaaa attttttttt 2100

tgctctcatg accagtggca aattcttggt aacgaggctt aacattaatc cgcaaattac 2160

ctggcaacag agaaaacacc cagaaagttc tgtcgtatga gaaaacctac agttgtttcc 2220

gatttctccg agcactaaac ataaagagac cagtaatgct aaaaaaattt ttatttctgc 2280

attactgttt ttagcaaata cacgtctaat ttattgtatt tgttaaacat tcttttcctg 2340

aaattttaag aaaatgtttt ggtttgttgg aattccattt aaacggtact ttggggtgca 2400

gacagcaatc catttggaga gtggcaagtc tacacgaatt tagctaaggt tcactatatc 2460

gtgtaacaag aaatttctat accaaataaa cagcacttga ttgaactaca atatgtaaaa 2520

acttgctttt attaccagtc ttcatacata ccccggtctt ctcttttcaa tattctgtat 2580

atgtctttac aactcttaac actccgtaaa tgtgcctttc gaatactttt gcagctggat 2640

atttttccgg tgcacctttt cagttatctt ttgcaacttt tcgcgagcaa tgacaaaagt 2700

ttggggcgtg aggcaacaaa atgcatggca ttaccagtac agtatcgcca caagtggttt 2760

tccttggcat ttcttgattg tttagtagaa caattcaata agactttttt gatcatgaat 2820

tttttttgcc atgaaggtgc tttcattgtt caaggttgaa ggggaattga aaaatttgta 2880

gagtcacaat caaatgactt gataatttga tagaaaaaaa aaagaaacct taaaaaatat 2940

tcataccaat gtatgcataa ccataaagaa cttactaatt atgcacctgc aatcagaaag 3000

tcatttctta cgatgatttg ccaaatgacc gtaaaacgac tagcaaaaac agtgacattt 3060

tttttgaaaa ggtggagatg aaaaccattc tggtttgttt cgtcatttac acaaatattc 3120

gacacaaaaa ctattaattc aatacaaaca aaaaaatgtg caggaagtct tggaaccgat 3180

acaaaaattt ttacaaacca cgtacactat tgttttgggg aagaattagt cggggaagaa 3240

ggcccagaaa cttgagtaaa gagtggattc aacactttat aatagtatca ttttgtaaca 3300

caaaaatgaa atacacccaa taaaaactgt tgaaacattt atccgtcaag cttattcgat 3360

ggagtacaac actttacatt tcttccgaaa caataactat ataaacccat gtaagtctcc 3420

cctcttttgt ttcaaacgtc ttatcaattt ttctcttcac tacttttcca acttaacaat 3480

cttcacttat aatctcaacg aatc 3504

<210> 39

<211> 3955

<212> DNA

<213> Candida albicans

<400> 39
tgttaattga tactaagtgt aattgattgg aatactagaa aaaaaagaaa gaagaagaaa 60

agaaagaaga aaaaactcaa ctttctttcg aaaatcaagg atcaatgttg gtatttatat 120

actttttttt ttagtcaaac tctacgaaat gaaattcaaa gagaataatc cacagaagag 180

gagagagggc aaaagtgggg ggaccaaagg gggttagaaa acaggaaaca gcaatagaga 240

gcaataattg aaaaatagtg ttgtcaacaa tagaacaaat tggtcaaact ttaaatgcaa 300

aacatgaaat tcccaatttc cagaataaat aatatcagca tacatggccc cgaaaactac 360

tttaccgtgt cgctttaacc ccccccttcc taaaacgaga caattagaca tacattccac 420

aattatcata atcccctttt ttttccttac aaaacacttt atttttgtcg ttttcgttat 480

ttgcttcgac gacattgtaa actctttgga tttgcagtag tagtgctcct ggtgtaaggt 540

gggtttggtt gtagagtaaa agaaacgaca attgattaca cctcgatatg catacgcatg 600

gcaaagagaa taccgagtta atagtgagtc tattagtgtt gcaggaaaag ttatacgaac 660

aacattttgt ttagtgtgga tattccagat caacaacaat atgactaaaa tcatagctct 720

aattttcagt ttacctttgt ttattacgat actgccacag tcgtgctgta ccagggtcag 780

ttttagaaaa actattctag aaatgatgag tagaaatgta ctattatgag caatatttca 840

aaaagtgaaa ttataattgc tgctgacaac accaacaata catacaaatt tggaaacgag 900

caaatcgaga aaatttcaat ccgtttagca agttgttcgt tgtcgtcatt gtcgattagt 960

ttcagtttct agaggtgaaa ttttctatgg caccaaaacc aaagcctcaa ttttaattta 1020

ctctgtgtgg tacaaaatac attagagagg atcctctcca aacaggattg caggaagttt 1080

tacacgagaa tgatttacta cacgacgttg aattaaaaag ctcaaccagt ttgtcagcaa 1140

ttttgttcta tctgttcaat ttcttgtata aaataaagca atatgagaga gcatctaaat 1200

caataatgtc aacacaatat taaactttga gaaggattgt tcaacaaaac aatccgatga 1260

atagaagaag aataatatca aattgttcct gattgattgt tgttatttat tttttatctc 1320

cgaattcctg cacaatggct caacaacagc caacacggat cacacattaa attttttttt 1380

cgtgcaggac cccgtggtgg tggctgtggc tgtgattgtg atcattgtag tttctgcctt 1440

gatgatgaca aaaaatgata gagttcagta tgaggaagaa attaagcgat atcggtttat 1500

gatgtgttta gttattaatt gctctcaatg gttttcaaca acgtatacaa aactggtggt 1560

gcttgaaacg aatgagtaat acagatctaa ttaagctgtg attttctaag tttgccttgt 1620

ctctacagtt caaaaaaaaa gaacagaaca cctcagaggc tgttgtgatg caatttttag 1680

gaacctcaac aacaaccact gactgatcta agccagcatc tgtttaatgg gttttcaaaa 1740

agaatggggc aaacggggaa ttgaaccccg ggcctcctcg aattttgtgt ttggtgaaca 1800

acccaaacga ggaatcatac cactagacca ttcgcccaat tcgatgactt ggaattattc 1860

tagttatttt tgacatacaa agctcagctt tattacagat agtcatgttt gcatggatga 1920

attagtacta ctaataatat aagaaaacta gttaattgga gtcaatgtct tatacatgtc 1980

ttctgatggg ttatgcattg attaattatg aatttctttt aaatacaatc tattgctatt 2040

atttgtatgt aaaactttac ccaaaaacca acaaaaaaga gtggtcttgg ataaagatta 2100

aagtaattcc aaaaagattt ggtaattagc tatattgttt tgacgtacat ctataactac 2160

aaatagccat tcagtttgat tatgtatatt gacatagttg gatttgtaat ttctgttaaa 2220

atggaaaacc ctaatcaaat gtatatgttg aataggtagt taaattgtac aacctactac 2280

ttgttgtcaa ttgaattcag agccaatact tatatctcct ggaaactgat acacaaacga 2340

attgttaaac tataacactc gacgttcaca tctaaggatt catcgtcgtt aagatttata 2400

ctcattagca aactcacttg ccatattaaa cacttctcaa tctatttccc acaatccaat 2460

taatcagcac gaaaactaag atactatata tatctgccta tacctgatat acacatggca 2520

catggcgtat cccacaaaaa accgtcaaga caacaccaat atgacaatgc caattataca 2580

attgcatata ccacgtgact tcattttatg gtcatgagaa attaacttat catggggtta 2640

ggcgagaata tcaactgttc gctatagaga gatttcctag ccggaatgca cgacaatcct 2700

gagacggaag tcgatcgacg atgcccatgg tgcgtggtga aaaattttct tagaaaattt 2760

gttctttcct tcaactgctt tgaagaaagg gaggttcaag tggtttaagt acgacggtca 2820

caaagattgc ggcttatgag gcccgaactg agttgaaata caaaatcaag atataattat 2880

ataccttact tgtctatatt gttttataat acattcttca gatatttaaa tttctgtgta 2940

tcattctata aaacagagat acattcagta catttagtat actgagtgaa ctggtacctg 3000

tgacattcaa gataactgtt tcgcgcacgc tggcagacga acatcaacac tgatcatttg 3060

tttttttttt atttctcctt tttctccttt ttctttcttt tttcttcttt cttcagacgt 3120

tgttgattta ttttatcgac agcatccttt tctttggcca catatccaag cgatatactg 3180

gccaaagcga agtcctttta taaagcaatg ctaccaaatg taacagttcg aggtcagaag 3240

attaagcggg tatgttcaca cggatatttt atggggtatc acttgtacca aacactttga 3300

tacgataaga atatttgtaa tactaacttc agtgtctttc ataatcagct cataacctgt 3360

tggaatttaa attcgtatgt tgttcattca aaattttgat aaatgggacg agaaatcatc 3420

gttgcctcct aattagatta tgacttagta ctaactaaac tgtttatcat tttttaaagc 3480

gttgggctcc atgttagaat agattattag ggcggtacgt atttcataat ttatatatag 3540

gtacttattt ttactaattt attgcacagg aaaagataaa aggtatcgat tatacctatc 3600

agcaaggttt aagcaaaatg aagtattttt accatatttt tccattttta tatagataca 3660

tcaagaggtt tattttaagt tcacctggat aaaccattca actaacccaa ttgaattgaa 3720

tgacaatttg atctccaaag agggattcat ttctattctg gagagataaa cgtcattgtt 3780

taggaaagag caagagataa gaaatctttt gtatattgta tatatattat taatgttata 3840

ttacactatt gtttgtttgt ttgttataat tatatgtgag atttcatatg taagatgttg 3900

ttatctcttt ccattattta gcttttttga aaaagctatc aatggctcca cgttt 3955

<210> 40

<211> 1434

<212> DNA

<213> Candida albicans

<400> 40
tagatgcaat aggtgtatga aatgtatcta gattatatca tgaagccctt gcaataaaat 60

ctagccaaaa atttgtgtac tgcaattgtt cgctatagag agatatccta gccggaatgc 120

acgacaatcc tgagacggaa gtcgatcgtc gatgcccatg gtgcgtggtg aaaaattttc 180

ttagaaaatt tgttctttcc ttcaactgct tttaagagaa gggaggttca agtggtttaa 240

gtacgacggt cacaaagatt gcggcttatg aggcccgaac tgagttgaaa tacaaaatca 300

agatataatt atatacctta cttgtctata ttgttttata atacattctt cagatattta 360

aatttctgtg tatcatccta taaaacagag atacattcag tacatttagt atactgagtg 420

aactggtacc tgtgacattc aagataactg tttcgcgcac gctggcagac gaacagcaat 480

tctgtaattg tcgtagagta gcaacaaatc ttcccgatga ttggtacttg tgttagtcta 540

cacgacatgt gttttggtac acttgaactg tatgtccaag aatggaaaca tatgcgggaa 600

ggacgcgaaa gatgagtttg gtatagaagg gataagaact gtaaaatata ttatgtagtt 660

atatatttta attatgggaa attgagtgtt tattctgttc aacaagtttc aaccgtagag 720

attacattta aagtctgtgg tcgaaatcca caagatacag caaattcatg aattcaccta 780

tttaaatcaa gtttaccaag caccattgcc tagaacttgc catatcatca attaagtcag 840

acattactaa tttgagcaaa gcttttagct taatgggcca actaatttaa gtcgaattgg 900

taatgcaatc tgttcttcat ttgagtcgct tgctacggct ccatgacaca tccatttgat 960

tgttttaatt cgagcaatta tccaccataa ctctcagtaa tatcattaac agttttacgc 1020

ttaataagca tagaaagttg tatgaagttg tctcctaggt atgctagaga gatttgtata 1080

tacgaccagt aaagagtgtg atgaggtgtt tactgtaggg taaattgcaa ttgacttgag 1140

ttgatagcgg ttattacaaa agtatagatt caacaaatta agacaagtac caaacgatag 1200

gccgaatgtg acttataccg ttgaagttca agcgttttta acaaatagaa atgtgagatt 1260

aatgagttcg acaaatgttt tactagatac tattaatttc gatgtactat ataagtttaa 1320

ccagctataa ccggcagagc agacttcctg aaactcaaat tggttgtgtt tggacttgag 1380

ttacaccaca aagtttgaca atcgtgagga catagcaacc tatcaagcca ctca 1434

<210> 41

<211> 1608

<212> DNA

<213> Candida albicans

<400> 41
tgctagtatg tattttggct ctttgatcct gaatgcgaca atgcaataca aatagtagaa 60

ataatgatgg tgatactact agtattaata ataatccgag aaacgatatc acaaaataaa 120

tcagtgccca atgaggttga tgcacaaata ttagtggtgt gtaaaactaa agagaatatc 180

tcgctatgat ttctattgat aagaaaagat gagagattaa ggaaatatct tctgtaaagt 240

tgtatcgcca cctttttttt ttgtagtagt agtatcggtt ttggttttgg ttttctcatt 300

agttaagatt cttgcgataa ggcacgacct tgatcatttg catgtttctc gtttaattgt 360

ttttatttct ttttttttta tggtgtgtgg tagtagttac agatatcgac ggttgcaagt 420

gcacgagtgc tgcgactgac cggatcgtca tgctaaaaga ttcaggggtg tgtaagagcg 480

tgccaagtcg aggaggaacc aacatttcac aactgcttca ggatagggca ttctttttct 540

tctttctatt tgatctagcc ttgcgtctat tcgtgttgtt ggttggtaca agcgaatatc 600

ccaataaggt ttttgttgcc tatgtgcatc gtgttgtagc atagtaacga gagatacgat 660

tcttcttctt ctccttcccc ttttctttgg attgctttat atttatatat atatattgtc 720

atcatcgtca cgaaattcac tatcattatc aattattttg ttttttctct atctttgtcc 780

tcctcgttta atccttatca cagttttggg ttgttgcaat ttcttttcat tctccagttg 840

aggcttacac tttctcttgg agtttccgtt tataattttt acacacacaa aagcacaaac 900

tacactttgt cttcacagtg tataacagat accacagtat tactaagggg gaaaactaac 960

ctaaccaaag ggactgacaa aataagtgga aagactacaa atgacgccct taatatacga 1020

gagagaattg aaaagacata cacataatgt tcgctataga gagatttcct agccggaatg 1080

cacgacaatc ctgagacgga agtcgatcgt cgatgcccat ggtgcgtggt gaaaaatttt 1140

cttagaaaat ttgttctttc cttcaactgc ttttaagaaa gggaggttca agtggtttaa 1200

gtacgacggt cacaaagatt gcggcttatg aggcccgaac tgagttgaaa tacaaaatca 1260

agatataatt atatacctta cttgtccata ttgttttata atacattctt cagatattta 1320

aatttctgtg tatcaaccta taaaacagag atacattcag tgcatttagt atactgagtg 1380

aactggtacc tgtgacattc aagataactg tttcgcgcac gctggcagac gaacaattgc 1440

ggcgaaaaaa aaaagaggtc gccaaaacta aactgttggg acgatttgct gccaatcaca 1500

atgaaaaaaa aaaagaacag ttggtttgaa acttcttcct ctaatacaga attaactgat 1560

ctttctatca ctgtttaaac tattcattac tctcaagaac ttaccatg 1608

<210> 42

<211> 1385

<212> DNA

<213> Candida albicans

<400> 42
aataagtgga tttatcatta ctattatcgt aatgctcaat caggggagtg ttggtttgtg 60

cactattttg tgtcagaaac tgatcaatga aaatgatggt tattatgaga atggaaaatt 120

tttccatcac acatcaggtg atgacagaac taaactatat tgtgtagtat aaataagggt 180

atgaaatacc aacatcccag aatatcaacg agatagaagg gaggagtttc aatatatatc 240

ttgtgaataa taacttcgtt ctaattcact atacacaact agacgtgtac acgctcaatc 300

tcaggtaaag aaagtttata ttccatcaac agtactagta ttagtattag tagttgcttt 360

gtcatataca aatagattaa ttaaactaac taacaaccta tatcaaatca aatcatcagt 420

tatatcatca tcaacatatt catcatcttt attcattcta taaattgtca ttgccatact 480

tgcaaaattc aataaactca taatccaatc cggcaaagca attccatata attcaatgag 540

attaaatgtt aaatctaaga aattcccaat taattcaata ataagcatca ttttatcaaa 600

tcgtaaatct tttaatactt ttttgtattt tttatttaaa tcttcattta taaaatttat 660

tccagtcttg tttttagtgg tggtagtaga atttaataaa tcaacttcaa tattaacttt 720

tctaatttta cgtattacat ttagtaattg agatatggtt ttcctgatta aaaaaaccaa 780

tattaatacc caaattttat tggtttgttt taaaaatcga tttaaaaatt gtgggaacat 840

tggtaaattt gataataaat gtaaattatc taataaattg gcaagatttt ctaaaatatt 900

aacaaacata aattctattt ttttcaaact aaatgtattt ggtctatagt attttatagg 960

tttattatta ttattaggtt tactccctga cttgggtttc ttcactggag attgacctcg 1020

ttcttgtcga ttgttgtgag atgatttatt aatatcaaat ttattaaata ctgaagggta 1080

ttttggtttt ggaggtaatt tagccttagt aggggttgat aatggttgtg atcgactttg 1140

taacttttgt tgttgttgtt gttgtgctag taaaatggtt aatttatcaa gtttatctga 1200

tgtgattgaa gtattaccct gttgttgttc tttttgagct agaagaagta aattattgat 1260

aatttattgt tgacgtgagt caggattagg atcaattgaa gtatgtttta agtttaattt 1320

ttgaattaaa tcaatattct cctgtattgt tgtagtgaac attacggata ttaataataa 1380

ataaa 1385

<210> 43

<211> 1483

<212> DNA

<213> Candida albicans

<400> 43
tgaataatca ggggatgcaa gttattgatt ttgccagtat ccaattttac ttgtggtttc 60

gagaaagttc tttctctcat tggtagttta aagttaactg aaattcaaat tataggagtt 120

tttgaacata aaaagcatat acaacttgag tagcatgtat atattgcata taaagattct 180

ttttttttgt aattgagttt gccaaacatt ttagtcactc ccaatatatc gtcaactcgt 240

aaatgtgata attcaggtca agtgcctacc tctaacgatt agccaacatt ttttgaaaca 300

aaaatatatt tcaaaggaac acagtgaaaa cctctctatg taggctgaca ggtgaaaatt 360

atgaattaat tgcattggcc aatgacaaat gaatagacaa aacagcaaat aaggttgcaa 420

aagtagccca aacaaactag atttcggtta cgaattttcc atctttcaaa acaatgaatt 480

tgtttagagc tctgtgccat ttattgcaac taaaatgaat atgcaattaa acaatcagag 540

atgtattgga ttatccccgt ggtatacttt tgagttcacc atttgttttt tttttggggt 600

taaattagtg ctcctactaa aaatcgcatt tatcttacac tcaccatttt gataagttat 660

ctctggtcaa tcgcaaatac tatgcttcta attaagagtt ctatgtaaat cccatttatt 720

ttgatcaatc tattggtttg aagtaagagt tgattttctg taaagattta tttgacagtg 780

tagttcggtg tcaaaaatat attatgatgt acactaaaaa acactaaatt tcaagtcaat 840

ggggaacaca aaactgaatt aattactata tgttggtttg tgcactattt tgtgtcagaa 900

actgatcaat gaaaatgatg gttattatga gaatggaaaa tttttccatc acacatcagg 960

tgatgacaga actaaactat attgtgtagt ataaataagg gtatgaaata ccaacatccc 1020

agaatatcaa cgagatagaa gagaggagtt tcaatatata tcttgtgaat aataacttcg 1080

ttctaattca ctatacacaa ctagacgtgt acacgctcaa tctcaggtaa agaaagttta 1140

tattccatca ctatataaca acaatcaggc tttgcaaaaa aacatttaaa actaatactg 1200

gtaatatgga aatataacgc ctcgtagttc tacgcacgtg gcatccttta tctatttatt 1260

caatttaccc ctaatttatg aattagctta ataagagcag tcaaattaac acggctcaat 1320

taatagtact taataatatg aagccgatca attaaccgat cctttgaata atttgaaaat 1380

aaaataaagt aatataaata ggtatgcatt ttccctacat ttatttcctc tttctatttt 1440

aatttgtttc ctaaacagca acaacaacaa ttgaaattca aaa 1483

<210> 44

<211> 879

<212> DNA

<213> Candida albicans

<400> 44
ggctcgtaga ttcggtatac ttgtctagaa taaaaatgaa aatgaatgtt agttgaaatg 60

tcaggtggtg gtggtggttt ttttttagat ttcaaaaact atacatactc ctatgagatc 120

aattttcttg attgaatatc ttggtaaaat ggttatgagt tcattttctg ccaaaaaggt 180

aatttctgat ggcataagat tcccttgaag gttttttggg agtaccatga cgggttaagg 240

attatttgtt aatggttaaa actagatagt agtagtctat atttaattta tttttttttt 300

tttgacacct tgtgcgaaag atctctgttg gtttgtacac tattttgtgt cagaaactga 360

tcaatgaaaa tgatggttat tatgagaatg gaaaattttt ccatcacaca tcaggtgatg 420

acagaactaa actatattgt gtagtataaa taagggtatg aaataccaac atcccagaat 480

atcaactata tagaagggag gagtttcaat atatatcttg tgaataataa cttcgttcta 540

attcactata cacaactaga cgtgtacacg ctcaatctca ggtaaagaaa gtttatattc 600

catcaatctc tctcgatgtt gtaaagagac gcgtcaatta acaataaact ctaattttgt 660

ttttcttcta caaaactacc aaacataatc atgtcaaggt aaattacaat gatatttaat 720

tacgtaaata cttctatacc cttattgata ttcaatcatt ttcttcttat acgtggaagt 780

tcttccagat gtcatggcct tggcccttct agcaggtttt ggaccgtcac tatctctact 840

atacgggtca aatccacgtc tctgtctacc attagtcta 879

<210> 45

<211> 974

<212> DNA

<213>  Candida albicans

<400>  45
acccgtctag tatcagctcg tcgttttcaa gtatgttgtt catgtccagg ttgttgtctg      60

tggtggcagg tactttgtcg tccaatttta ggtcctcgta gtccatgttg gacaacatgt     120

cttcgtcggt attgccgttg atgtcaaagc caataaagtc gtcaaagttg tcaaactttt     180

gtggggcggt ctctgctttc tttctggcct ctgctttctg tttgttttac acttttcgtc     240

tttaattata gtttcgaaga atttcctagg aacttaagaa tttgtaggag aatgctaata     300

agaagttgta tttcttaatt gaaagttata attgtaagaa tatattgtat aaaagatgag     360

ttgataaaga aaagatataa aaagtcctat aaaaaagtat tgtaaaataa aagtatataa     420

aaatcaagta aaatagaata tttgcacaca aattaaaagt agtgcaaatt tgacagaaaa     480

gttgttggtt tgtgcactat tttgtgtcag aaactgatct atgaaaatga tggttattat     540

gagaatgaaa aatttttctt tcacacatca ggtgatgaca gaactaaact atattgtgta     600

gtataaataa gggatgaaat accaacatcc cagaatatca actatataga aggcaggagt     660

ttcaatatat atcttgtgaa taataacttc gttctaattc actatacaca actaggcgtg     720

tacacgctca atctcaggta aagaaagttt atattccatc aaaagtaaaa taaaacactt     780

cttcgcttcc tctgctttct tggcttgctc tgccttcttg gcctcttctt ccttctttct     840

tgccgcttct tctttgactt tcaattcgtc aagtttcttt ttcttttcaa ccataacgcc     900

gagacaccac tctgcatcat tgagtttcga cactgtttgg tctagaatag catggaagtt     960

ttggatttcg ccgt                                                       974

<210>  46

<211>  3868

<212>  DNA

<213>  Candida albicans

<220>

<221>  misc_feature

<223>  "n" is any one of a, g, c or t

<400>  46
aatgaagtaa cttttttcaa ggcaacatct attcttttat taatctcgac gtctgtttga      60

ttaagttgct ctaacatttt atttagatcc ttctctatat tttctgcaat atcaaacacc 120
gattgctttt tgtctgaagt tgctggtata tcaccacttc cgccaattgt cgtatttcca 180
ctgtcctttg ttactgacag attggcactg acattacctg aattgttcat gtttgctgtt 240
gaaagagcag gaactgtact tggataagca gccgattcaa aagaagatgt ggacatgagt 300
gtcaagaaaa tgtgtagaat cagtacaaga ctggaaaaca gaaggaacaa agtgaactgg 360
atattgtagt tttgttgata gtactcgcga gctttaattt ttttttgtaa ctggcggaat 420
cagatcttat gcaatactca aatccaaaga aacagtcaat ccagatgaaa ggcatgtaat 480
cgctagtttt cataaacaga atcatgttac tagtcatatt ttctataaaa attcaatact 540
tcattctttt tgttcaatac taactataaa tgcttacaaa tagattcaaa tttcaaccag 600
atccaccact tcattaggct caaccaattc ttcataaata gaaacgtctt cctcagccaa 660
gcttaattga tgggaaaccc tagcttgcat tgaaggaaaa atacataatc caaataanca 720
actgtctttc caaatattct caaaattcaa cttcaccgtc tttcaccaag caggatctcg 780
tgattggacc aattctaatt cagaagttct tctcacacaa gtccgaacga ctcgatccat 840
cataatggat acatcgttca cgttgccacc aaatcgaatg actctgtttg cacctgtaca 900
aagtagaaca tatgcatgga aaagtaaaac tagtaaaacc gcataatgaa accaataatt 960
catcatatgt tgattgagtc tgaaccccat caaatataaa acaaaagtga gtttaaccat 1020
agttataaga agcagtcttc cgttggtgta taatctatcc ataagatcgt caatttcagc 1080
atcttcaaca tcaatgttat tagcgtcacc tggaacggct tgttcattag attctgattc 1140
caggtcacta ccaatatcat acatcattac tagtactttt tgaatcaatg gctcaccaga 1200
agccagttta aacaccttgt gaacttttgc tgcacccata ggaccgagta gtagataagg 1260
atcgtgcaag ccgttatcca caacaatgca ttgtgctgta cccaagctta ctttcttcac 1320
aatattgtct actttcaaag taagttcata ctcaacatta gacaagtcat cctgtttcac 1380
tagaattttt ttccctgaat gctgttcaac catagtatcg tacgatgttc cctccatttc 1440
ccatgtggat ccaccacgta cctgaatact ggcaggttta atggggtcta tgttaggagt 1500
tgaagactct gatggattat tgacaaatgg aatagagtct tgttgacttg gcaccagcgt 1560
ttcataattt gaaggtgaag gtactgggtt agccgaggtt ggtgatgttg aaatatcact 1620
atcaattcct tgttctgagg atgagctagt agcagttgga tttgttgtgc ttcttgcagc 1680
agacaaatct gatgttgatt ctaatggcac tgaattcgac agcgccaaat tgggttgctg 1740
taaagagtca ttggtggcag ggagaaatct aaatctatca tttgactgaa agtccttcca 1800
aaattctctg ctcaacaacc caccagttcc atttacatgt tcatgctttg taagtttcaa 1860
ttttatgaca ctgttattct gttccaaaag ctcttgattc aatcccaaca attcataaac 1920

actagcttcc tcttcttgaa atgaggttgg tattatattc ccttcgtatg atagttttat 1980

ttgttctata aatgtacgtg tgacagaacc ttcgtcattc ttagctatta ttaattgctt 2040

gagttgctta accgtagttc ggtcatttat ttcaatcatt gacttttcat tctgtaaatt 2100

aggaagattt gactccaaca aaacccggaa tcttttgaaa ttactattca tttctaaagg 2160

tttgggttgt gtgattgaag ctaatggtgt gtgtactaag tggtttttca attataaata 2220

ttgatgaact acactatata tacactgaga aaaacacgac caaaattgac accgcactaa 2280

aaacacggaa ttaccgtatt ctttttgtta acgattttgt ttcattacac gactgtcgtt 2340

atacacacat ttagagcaaa ttattttaga ttgatcagtg ttagcaactg gctatcgata 2400

atagagtacc ttcccgagtt agaatgtctt attagaacaa caattgtttc atataaattt 2460

gtcgcaaagc acacgtaata tactatatgg aaggggctaa gtaaaaatgt cccgtttctt 2520

cttaatatga gaactcgtgt acgacacaat ttgctgtgtt gttaatcgag tatgctacaa 2580

cctgaaaatg gaccatagac ccaaactact tctctctttc tagcaccaca aaccccacaa 2640

ttagcacaac aatgaattgg acttcacttg tatatctatg gttcattttc aaaagcatat 2700

ttgctgactt aacatcacac caactcaaga gcaaagtggt attcctagat actactatcc 2760

tggatgaagt ggcccgaagc tatttgggat cagaggacgg aaatgttaca catggtaatt 2820

atgaaatatt gtcaattgca aatgggcgcc aatgacggaa acatcacatc atatttatgc 2880

cagttgccaa gaaccaaaaa aatggcacca acaaaaccca agcccaccat gtcagttcat 2940

gaattgaaat cgcgagctat tgacttgata tcggaatcct ttgtcgaagg taccagttgc 3000

gtattttctt tcaacttgca tgcaaattat tggactatag gctattgcca tggaatcaac 3060

gttattcaat tccatgagaa tttggatgat tttataagcg gaattcataa accccattct 3120

ccaaatcatg tatatacatt aggcaatttc ctgaagcaaa cactgccatt agaattcgag 3180

tttgatacta aagaacgcac aataagtcaa agattgttag gagaagtttg tgatttgaca 3240

ggagaaccac gtaccattga caccatttat agatgtgacc atatacttga aattgttgaa 3300

ttaacagaga taagaacatg tcaatatgag ttacacataa acgttcctaa gttgtgcctg 3360

ttgccggaat ttaaaaggac taaccttgaa gaaggtgtct cagaaatact ctgtacaaga 3420

attgaataag cattaaattt aataaaaaac atcaaaaagt gtatgtcaaa gtatttttac 3480

ctttgtaatt agtagtttgt cagtttctat ataaacatag ggtagttcgt atatacgata 3540

tcggagcgat tctaaataag tcgtggaaat tggccgacaa tgggatttga attttacttg 3600

tgtgtgtgtg tgtgatctga ataatagtag tgctaaacaa cttaaattaa agaaaaaaag 3660

acaaaacaaa aaaaattaaa tctgcttatt gaaaattttt cgaaataggc taacccgtgt 3720

ttattagata ttagatagta cgatttgttc aagtgtcaaa gatagcaaat ttttattgtt 3780

tcttcttttt tatatacagc ttgttttaat ttcaggatca ttttacacta acctactcat 3840

cagcctattt taatttatcc ttttggct 3868

<210> 47

<211> 469

<212> DNA

<213> Candida albicans

<400> 47
taacgaatga atataaaata cttgtattat gtagtgccaa taaaagttga aacggtcgca 60

ctacttttta gtcctgttgg tttgtgcact attttgtgtc agaaactgat ctatgaaaat 120

gatggttatt atgagaatgg aaaacttttc catcacacat caggtgatga cagaactaaa 180

ctatattgta tagtataaat aagggtatga aataccaaca tcccagaata ttaattatat 240

agaagggaag gagttttaat atatatcttg tgaataacaa cttcggtcta attcactata 300

cacaactagg cgtgtacacg ctcaatctca agtaaagaaa gtttatattc catcaagtcc 360

catctgttaa atatttttgt atctttttat ttttattttt ttttctttta atttcattta 420

catacattaa cacatctact aaccatatat cacgagatac aaaggcaag 469

<210> 48

<211> 4545

<212> DNA

<213> Candida albicans

<400> 48
tgacgatcct gtatatttcg tcataattca cacattctta aaattatgca cacatccttg 60

aaatgtgtta atattcccaa cattatcaat tatatgtgtt cagaattggt tgcaaagtta 120

tcaactcaat tcacgctata taaaccttac aaattctcta catttttata tttttttata 180

ttggcttttc ttttagaatc aatcaatact ttttttatca tttagataca tctttcatct 240

attaatagat tatctttcta tatatcaaaa cacgacacag tcacgtgcca aaaaggatat 300

aagaaggaac ttcagaaaat taattttctg attatactac ttactagatt gcataaagtc 360

aatatctgat tgatacaact tggttcatta ttcataaaac ttaacaacta attcaacaag 420

gaaacccaac aaaaaaatcc aaataaaata atcaggaaaa tattataatt aattaattac 480

aaaaaaaaac aaaaaaatac acacacacat acacacacac aaaatcttgt tgcaaaaaaa 540

aaaaaataat aataatataa taagaattaa ttaacaatgt cgtttccacg gacacattca 600

ccaagaccat ctggttcacg agaacaggaa gatctcacac tgatgattaa agcttttaga 660

gattcaatgg aagctaagct tgacttgcat tcgcagaagc ttactgcttt ggtagcaaac 720

attcccagaa cggacgaagg gtttgaagat ttatcacaaa ggatcactgt tcttaaaaat 780

catcaaaaag catttttgcc caaacaagaa aaagaaatcg gaagtcttct ccacagacaa 840

agagaggaag aaggtgatat taaggatttc aaaacagtcg ttggtgaaga aaaagaagaa 900

ttgcaccagg ttgaagattt cgttttaaaa gatcaagaag aattacgaaa cgtcgaaaag 960

aaagttttga aagaagaaga agaattgcaa aaagtggaag agtcaatgga aaaggaaaaa 1020

caagagttat accaggttga agactttatt ttgcaaagag atgagacggt aaagaaactt 1080

ggagaaagca atcaatctca acaggaacca tatacacctg caacttctgg ttcggatcag 1140

agattcagat ctcaacaacc taacattgga aataccttag cgcaggatct agcattaatt 1200

ccaaaattag atctggaaat ttgcaaaatt gcagtcaaat atccaaaatt atttgaaaca 1260

aaattaagac caccaccacc cagagacttt caatataaaa ttcaactcac agaccacact 1320

caaatttatt caaaaccata taaatgcaat caagaagaac aagctctcat taaggatttc 1380

atcaatgaaa aattagaagc aggcgttttg gtaccagctc caattgatgc ttggttacac 1440

ccaatatttc caatcagaaa aaccaatgcc aaccaatcct ccaccaaaat agcagttgat 1500

ttaagacgtc tcaataaggt cacagtacga atgtacactt atccaacaga cacaaaagac 1560

ctcttatcct cactaacaga ttcccactat tttagcgctt tagacttaaa gaatgcgttc 1620

tatcaggtaa gcatacacaa ggatagtata aaatattttg ggatttcaac atccgagggg 1680

aattattgct ttacaacttt accgtttgga gcaatcaatt ccccaaccat ctttactaac 1740

tttgtgagac agattttaga ggggatccca tgtatattta tatacatgga tgatatcctc 1800

atccatacta aaaccttaca tgaccacatg tcattactca ggagaatcat ggagaaacta 1860

aatgagcatc agtttcaaat gaattataac aagatgcaat tattaacaac aaaaatcaat 1920

ttcttagggt acagcattca agcgaacaaa atatcaccag atatttccaa aattcaagca 1980

atacaaaatt gggaattgcc cacgaccact actcaaatca gagcatttgt caatttcagc 2040

aaccactttc gcatcttcat cccagaaata gcaaaattta ctaatccatt aaatgaatta 2100

ttgaagaaca acaatggtaa aaacataaag attgaacaca cccaagcatc cattgatggt 2160

tacaaggcat taaaagccgc catcattgga ttgccgacgc ttcaacttta caatccaaaa 2220

ctaccaacca tcattttcac agatgctagc cacatggtag taggaggata tttatgtcaa 2280

ccaacattca gaaatgacaa agaagtcctt gtcccaattg cattttcatc acataaatta 2340

acagaaacac aaagcagata tgctgctatg gaaaaggaac ttttggcaat tattgtgata 2400

ttggaaaaat ttagatatca ctgcagcaat acggtagaga tctatacaga ttatcaaagt 2460

ttggcatcat atttagataa gaaaactact ccaccaccga gaattgctag gtttttagat 2520

ctaattggat cattttcccc aaaagtgtac tatttaagtg gaaagaaaaa tttcgttgct 2580

gatatcatta caagatatca aactcaaaat attaaggaat tggtagatga agacaagata 2640

ctaggacaga cttttacagt caagagaaat ttgaaacaac aactattacc aagattggaa 2700

gcaattgaat tggaaaatct taatgaatca caggttcaca aaatccaaac ttcattagaa 2760

caacaacaac aacatgattt ggaagacaat gatgaagagt tacctctcca actgtttaaa 2820

ttaatgaatg atgagttatt tgtaatcatt aacaaccaac ttttaaaata ccttccaaga 2880

ctggaataca atgatatttg tcaaacaatc catgacaaac accatccatc aactagagta 2940

acagactact tatgcacact cgcatattgg catcctgacc atctattaat tgctacaaac 3000

attacgagaa agtgtcacta ttgtcaacta aacacgtcaa ttcgtgaggc cattagacca 3060

taccgaccac ttgaaccact caaggcattt agcagatggg gaatggacta ctctggacca 3120

tactttaaca cagtccaaca caggtacata ttagtagccg tggaatatgt cactggttta 3180

actattgcag taccaacatt gcacaaagac gcagataacg caatcagtct tttacaatca 3240

atcattctga tcatgtcagc acctacagaa ttagttacag atcaaggtaa aaaaattttc 3300

atcacaagct ttggctaccc tatgtgacca gaataacata caacaccata ttacctccgc 3360

ccaccaccca cgtgggaatg gtcgggttga gaaggtgaac cacctattga agaaaatatt 3420

gaaagcatta actaacgata cgatgcaaga ctgggattta aaactatatg acgctttaag 3480

aatctacaat gctacaccta caatttttaa ctacactcca ctttatcttg cacttggaat 3540

tgaaccacac cataatttaa atcaattaca aaaagattta attgaaaatt tgcaaaaaga 3600

attgccccca gaggtccaat ccacagaaga acacgaagaa aacccaaatg atgaacaaca 3660

agaagagggc agagaacaac aaatttcaag agaagaacaa caggacggca gagatcttgt 3720

acacttaaga atttacgaat tggaagcaat taagaaagct cgcaagttac acacaaattt 3780

gaaaacacga agaaacgcag tccaaaatat gttaaaggaa ccatatggca ttccagcacc 3840

ttttacaaaa ggacaatggg tatacagaat tagagctaaa gcacgaaaat atgaaccaaa 3900

tttcgatggt ccatatcaag ttcaagaagt attaggtaaa ggtgcttata aattgagaga 3960

catcactgga agagaaaaag gaatctacaa tcaggatcaa ttgaagttag catattcagc 4020

agacaacgac ccaatacagg tttttagttc tttcaataaa gaatatgatc gagtacaaca 4080

aaaattgtta gacaaaattc aatcggaaag agatcatcaa ttaaattgtt tgtcagtcca 4140

acatttacac agacaaagaa ggttactcga tatatccagc tgtcttgagc aaattctgca 4200

ataatttcgc taatcattgg aggaaagggt agatgacgat cctgcatatt tcgtcataat 4260

tcacacattc ttaaaattat gcacacatcc ttgaaatgtg ttaatattcc caacattatc 4320

aattatatgt gttcagaatt ggttgcaaag ttatcaactc aattcacgct atataaacct 4380

tacaatttct ctacatttta tattttttta tattggcttt tcttttagaa tcaatcaata 4440

cttttttatc atttagatac atctttcatc tattaataga ttatctttct atatatcaaa 4500

acacgacaca gtcacgtgcc aaaaaggata taagaaggaa cttca 4545

<210> 49

<211> 916

<212> PRT

<213> Candida albicans

<400> 49
   Met Ser Phe Pro Arg Thr His Ser Pro Arg Pro Ser Gly Ser Arg Glu
   1 5 10 15
   Gln Glu Asp Leu Thr Ser Met Ile Lys Ala Phe Arg Asp Ser Met Glu
   20 25 30
   Ala Lys Leu Asp Leu His Ser Gln Lys Leu Thr Ala Leu Val Ala Asn
   35 40 45
   Ile Pro Arg Thr Asp Glu Gly Phe Glu Asp Leu Ser Gln Arg Ile Thr
   50 55 60
   Val Leu Lys Asn His Gln Lys Ala Phe Leu Pro Lys Gln Glu Lys Glu
   65 70 75 80
   Ile Gly Ser Leu Leu His Arg Gln Arg Glu Glu Glu Gly Asp Ile Lys
   85 90 95
   Asp Phe Lys Thr Val Val Gly Glu Glu Lys Glu Glu Leu His Gln Val
   100 105 110
   Glu Asp Phe Val Leu Lys Asp Gln Glu Glu Leu Arg Asn Val Glu Lys
   115 120 125
   Lys Val Leu Lys Glu Glu Glu Glu Leu Gln Lys Val Glu Glu Ser Met
   130 135 140
   Glu Lys Glu Lys Gln Glu Leu Tyr Gln Val Glu Asp Phe Ile Leu Gln
   145 150 155 160
   Arg Asp Glu Thr Val Lys Lys Leu Gly Glu Ser Asn Gln Ser Gln Gln
   165 170 175
   Glu Pro Tyr Thr Pro Ala Thr Ser Gly Ser Asp Gln Arg Phe Arg Ser
   180 185 190
   Gln Gln Pro Asn Ile Gly Asn Thr Leu Ala Gln Asp Leu Ala Leu Ile
   195 200 205
   Pro Lys Leu Asp Ser Glu Ile Cys Lys Ile Ala Val Lys Tyr Pro Lys
   210 215 220
   Leu Phe Glu Thr Lys Leu Arg Pro Pro Pro Pro Arg Asp Phe Gln Tyr
   225 230 235 240
   Lys Ile Gln Leu Thr Asp His Thr Gln Ile Tyr Ser Lys Pro Tyr Lys
   245 250 255
   Cys Asn Gln Glu Glu Gln Ala Leu Ile Lys Asp Phe Ile Asn Glu Lys
   260 265 270
   Leu Glu Ala Gly Val Leu Val Pro Ala Pro Ile Asp Ala Trp Leu His
   275 280 285
   Pro Ile Phe Pro Ile Arg Lys Thr Asn Ala Asn Gln Ser Ser Thr Lys
   290 295 300
   Ile Ala Val Asp Leu Arg Arg Leu Asn Lys Val Thr Val Arg Met Tyr
   305 310 315 320
   Thr Tyr Pro Thr Asp Thr Lys Asp Leu Leu Ser Ser Leu Thr Asp Ser
   325 330 335
   His Tyr Phe Ser Ala Leu Asp Leu Lys Asn Ala Phe Tyr Gln Val Ser
   340 345 350
   Ile His Lys Asp Ser Ile Lys Tyr Phe Gly Ile Ser Thr Ser Glu Gly
   355 360 365
   Asn Tyr Cys Phe Thr Thr Leu Pro Phe Gly Ala Ile Asn Ser Pro Thr
   370 375 380
   Ile Phe Thr Asn Phe Val Arg Gln Ile Leu Glu Gly Ile Pro Cys Ile
   385 390 395 400
   Phe Ile Tyr Met Asp Asp Ile Leu Ile His Thr Lys Thr Leu His Asp
   405 410 415
   His Met Ser Leu Leu Arg Arg Ile Met Glu Lys Leu Asn Glu His Gln
   420 425 430
   Phe Gln Met Asn Tyr Asn Lys Met Gln Leu Leu Thr Thr Lys Ile Asn
   435 440 445
   Phe Leu Gly Tyr Ser Ile Gln Ala Asn Lys Ile Ser Pro Asp Ile Ser
   450 455 460
   Lys Ile Gln Ala Ile Gln Asn Trp Glu Leu Pro Thr Thr Thr Thr Gln
   465 470 475 480
   Ile Arg Ala Phe Val Asn Phe Ser Asn His Phe Arg Ile Phe Ile Pro
   485 490 495
   Glu Ile Ala Lys Phe Thr Asn Pro Leu Asn Glu Leu Leu Lys Asn Asn
   500 505 510
   Asn Gly Lys Asn Ile Lys Ile Glu His Thr Gln Ala Ser Ile Asp Gly
   515 520 525
   Tyr Lys Ala Leu Lys Ala Ala Ile Ile Gly Leu Pro Thr Leu Gln Leu
   530 535 540
   Tyr Asn Pro Lys Leu Pro Thr Ile Ile Phe Thr Asp Ala Ser His Met
   545 550 555 560
   Val Val Gly Gly Tyr Leu Cys Gln Pro Thr Phe Arg Asn Asp Lys Glu
   565 570 575
   Val Leu Val Pro Ile Ala Phe Ser Ser His Lys Leu Thr Glu Thr Gln
   580 585 590
   Ser Arg Tyr Ala Ala Met Glu Lys Glu Leu Leu Ala Ile Ile Val Ile
   595 600 605
   Leu Glu Lys Phe Arg Tyr His Cys Ser Asn Thr Val Glu Ile Tyr Thr
   610 615 620
   Asp Tyr Gln Ser Leu Ala Ser Tyr Leu Asp Lys Lys Thr Thr Pro Pro
   625 630 635 640
   Pro Arg Ile Ala Arg Phe Leu Asp Leu Ile Gly Ser Phe Ser Pro Lys
   645 650 655
   Val Tyr Tyr Leu Ser Gly Lys Lys Asn Phe Val Ala Asp Ile Ile Thr
   660 665 670
   Arg Tyr Gln Thr Gln Asn Ile Lys Glu Leu Val Asp Glu Asp Lys Ile
   675 680 685
   Leu Gly Gln Thr Phe Thr Val Lys Arg Asn Leu Lys Gln Gln Leu Leu
   690 695 700
   Pro Arg Leu Glu Ala Ile Glu Leu Glu Asn Leu Asn Glu Ser Gln Val
   705 710 715 720
   His Lys Ile Gln Thr Ser Leu Glu Gln Gln Gln Gln His Asp Leu Glu
   725 730 735
   Asp Asn Asp Glu Glu Leu Pro Leu Gln Ser Phe Lys Leu Met Asn Asp
   740 745 750
   Glu Leu Phe Val Ile Ile Asn Asn Gln Leu Leu Lys Tyr Leu Pro Arg
   755 760 765
   Ser Glu Tyr Asn Asp Ile Cys Gln Thr Ile His Asp Lys His His Pro
   770 775 780
   Ser Thr Arg Val Thr Asp Tyr Leu Cys Thr Leu Ala Tyr Trp His Pro
   785 790 795 800
   Asp His Leu Leu Ile Ala Thr Asn Ile Thr Arg Lys Cys His Tyr Cys
   805 810 815
   Gln Leu Asn Thr Ser Ile Arg Glu Ala Ile Arg Pro Tyr Arg Pro Leu
   820 825 830
   Glu Pro Leu Lys Ala Phe Ser Arg Trp Gly Met Asp Tyr Ser Gly Pro
           835                 840                 845
   Tyr Phe Asn Thr Val Gln His Arg Tyr Ile Leu Val Ala Val Glu Tyr
       850                 855                 860
   Val Thr Gly Leu Thr Ile Ala Val Pro Thr Leu His Lys Asp Ala Asp
   865                 870                 875                 880
   Asn Ala Ile Ser Leu Leu Gln Ser Ile Ile Ser Ile Met Ser Ala Pro
                   885                 890                 895
   Thr Glu Leu Val Thr Asp Gln Gly Lys Lys Ile Phe Ile Thr Ser Phe
               900                 905                 910
   Gly Tyr Pro Met
           915

<210>  50

<211>  253

<212>  PRT

<213>  Candida albicans

<400>  50

Met Gln Asp Trp Asp Leu Lys Leu Tyr Asp Ala Leu Arg Ile Tyr Asn
1               5                   10                  15

Ala Thr Pro Thr Ile Phe Asn Tyr Thr Pro Leu Tyr Leu Ala Leu Gly
            20                  25                  30

Ile Glu Pro His His Asn Leu Asn Gln Leu Gln Lys Asp Leu Ile Glu
        35                  40                  45

Asn Leu Gln Lys Glu Leu Pro Pro Glu Val Gln Ser Thr Glu Glu His
    50                  55                  60

Glu Glu Asn Pro Asn Asp Glu Gln Gln Glu Glu Gly Arg Glu Gln Gln
65                  70                  75                  80

Ile Ser Arg Glu Glu Gln Gln Asp Gly Arg Asp Leu Val His Leu Arg
                85                  90                  95

Ile Tyr Glu Leu Glu Ala Ile Lys Lys Ala Arg Lys Leu His Thr Asn
            100                 105                 110

Leu Lys Thr Arg Arg Asn Ala Val Gln Asn Met Leu Lys Glu Pro Tyr
        115                 120                 125

Gly Ile Pro Ala Pro Phe Thr Lys Gly Gln Trp Val Tyr Arg Ile Arg
    130                 135                 140

Ala Lys Ala Arg Lys Tyr Glu Pro Asn Phe Asp Gly Pro Tyr Gln Val
145                 150                 155                 160

Gln Glu Val Leu Gly Lys Gly Ala Tyr Lys Leu Arg Asp Ile Thr Gly
                165                 170                 175

Arg Glu Lys Gly Ile Tyr Asn Gln Asp Gln Leu Lys Leu Ala Tyr Ser
            180                 185                 190

Ala Asp Asn Asp Pro Ile Gln Val Phe Ser Ser Phe Asn Lys Glu Tyr
        195                 200                 205

Asp Arg Val Gln Gln Lys Leu Leu Asp Lys Ile Gln Ser Glu Arg Asp
    210                 215                 220

His Gln Leu Asn Cys Leu Ser Val Gln His Leu His Arg Gln Arg Arg
225                 230                 235                 240

Leu Leu Asp Ile Ser Ser Cys Leu Glu Gln Ile Ser Gln
                245                 250

<210>  51

<211>  2093

<212>  DNA

<213>  Candida albicans


<400>  51
ttttcccaca aataatatca acaatatttc atattttcca tcatgctaga gaagatcaag      60

ttataactac attaattggt tatgtttata aattgactca aatttgttta aaatttgaat     120

tacattctga aattagaaaa atcattgata aattaattaa atttactact ttaactcaca     180

cacctaaaaa ccttaatgaa attttaatta ctgaagtcaa attagataat aaaaccgaaa     240

tttatgttag tgattatgct tgttcatttg gtcgtgattt taaagctcaa ttatcaacgg     300

tggttttatt taaaataatc aagaaaaata atcttaaatt gaaaaattgg gataaaattg     360

tggaaattat tgaaaaatta tatcaatatt cattgattat tgatgagaag gatactacta     420

ctactactac taccaatgat aataaggaag gtgatgatga aaaggataat aaggaagcca     480

ctgttgagac tgacaactca atattgaaat tattgccttc aaaagatatt aaaaaattcc     540

ctattaaaag aataactaat gatctgtttc tttcaatatt gaaaaattta attgataatc     600

aacctactga agaagaaatt caatcaactt tagcagctat ggattgtatt aaatcattag     660

atatcttgaa tgtattaaga attgttgctg aatccaagaa acaagctaac taaatctaaa     720

caatctaaac atctaaacat ctaaatatat atatatatct attgtattat tatatttgta     780

aaattttgta gtttgcagtg gttggaataa atgataggag gatgttccat ttgtgataca     840

ctatttctac aaactgtcaa attcaataat caaacttgtt gccaagaaaa gataacaaag     900

aaggctattt ggtttacaag gtacaacaag aacatgggta tatcaccacg atagtttagt     960

aattttgtaa atcttctttc tctgttttac ttagcctcat ttagtccttt ctttcagttc 1020

caaagtagga tgtgcaacat ggccaattat caacaataag ctagcattgc ataatggtag 1080

tgattgtact gaagagaaca atacactaat ctattccatt gacgacggaa taagtggact 1140

gataattcac atggataatt cagtccactc tgagaggaat ttcctcttta tataatagaa 1200

aattcctcaa ggtattagat tgtatatttt ctatagataa ctaaccttga acacaagaat 1260

actatcgcct ttcgttgcag attatcgctc aaaacttttc aataactttt gggtcttttt 1320

ttaacaataa ccaataaatc attacaaaga attacaaaaa gggctataat gacaaatttc 1380

acatagataa gaaatatagg ttttattact ttttgcataa ttgctgactt ctatttttgg 1440

tttggagata tttagaacgt ttgattgtgg gggtattact tccaaaaaaa acaaaaattt 1500

gtaaaccctg acgatcctgt atatttcgtc ataattcaca cattcttaaa attatgcaca 1560

catccttgaa atgtgttaat attcccaaca ttatcaatta tatgtgttca gaattggttg 1620

caaagttatc aactcaattc acgctatata aaccttacaa attctctaca tttttatatt 1680

tttttatatt ggcttttctt ttagaatcaa tcaatacttt ttttatcatt tagatacatc 1740

tttcatctat taatagatta tctttctata tatcaaaaca cgacacagtc acgtgccaaa 1800

aaggatataa gaaggaactt cacccccttg ctcttcttat tattgtgtgt ggtgtaagtt 1860

cagcgggtag tcctacctga tttgaggtca aagtttgaag atatacgtgg tggacgttac 1920

cgccgcaagc aatgtttttg gttagaccta agccattgtc aaagcgatcc cgccttacca 1980

ctaccgtctt tcaagcaaac ccaagtcgta ttgctcaaca ccaaacccag cggtttgagg 2040

gagaaacgac gctcaaacag gcatgccctc cggaatacca gagggcgcaa tgt 2093

<210> 52

<211> 2099

<212> DNA

<213> Candida albicans

<400> 52
acatttttca atattgaaag ataaatatag cattccaaaa aaaaaagtga cttctgtgtt 60

cacatttaat caacaaattc ccacaacagc ttgcacaaac tgctatctac taggcttacg 120

agacacaagt gttaccaaat agtgatacac ttatacttta actcatagaa gagaattaga 180

tactcggaat attactcaac atattcccaa aataatcgta aagataaatc tttgagagtt 240

aatactagag agctcaattc taggcacaaa taccacactt tttacgagta gtgggtaaga 300

gttcgtacac atgatgcaac aactttctag tacctacttg cacaaagtgt agtttgcaaa 360

aaactttgct cctccatagc atgtatctca atactccaga aaatccgata aagcaactct 420

ccgatggtca tgcaagtatt cgcctttctc ttttgtagat ttatgtagtt tcaagatgac 480

actgaactcc tgagtattaa agtagattaa taatagaagg tattgcctaa tgccgagaaa 540

gtaaacacca gatcaaatat atgctttact atgaaacttg tttgtgttgt gtggattggc 600

caaacaaaga tcatgctgat atctgtaaat ctctggaacg ggggatagga ataaacttga 660

aacaatataa acgaggtgtt ttccttttct ggtgcttgat ttgaaacgtg tacattccct 720

ctttttctct tagttaacaa tattgcataa tagtgaggat gtgagcgtaa gacagaaagc 780

agcagcatgg gaatagttca gcctattatt gtcgcaaagc tgcatattgc ttcttctatt 840

aaacttttga atcttctctt ttaagtaaat taattaataa cttgattgtt ccatttacat 900

ccattttcta tttctgtgta atcttcgttt attttgcggt ttgaatactt ccaaatttaa 960

ttaaatttgt tcctaaaata gaagctgtta tacttgcgcc gccaaaccca ttttaatagt 1020

gatccttatt tcaatttaat ttgttcacgt tatatctctg aatttgatta atacttgcta 1080

cagatatttg gaaatcataa tttatgattt ctccggaatg taactgagtg gccagaagat 1140

atatagtaac acataaatac gtacacaaca ccagaacaac cgcaacattc aagtggaact 1200

agtatgtgtt gaaaaaacag acaaattaat cgggatagga agagatggga aaggggggtg 1260

agagaaaagc aaagaaaaaa aaaaaagaaa aaaaagaaca aaaatcaaat ggtacaaaaa 1320

aaaagacaca tcttctacac aattaacaaa aactgccttc tgatggcaag aaatctacct 1380

cacatacata cttaaatgga ataaagaaag taatctataa aaataattta acatgactaa 1440

cgtatttcaa gtaaaaaggt caaaattaga gaacccacca caatcaacta ttttctactc 1500

tcaattgttt tttcttttta gttcttataa ttatcaacat tttccttact caaatctttc 1560

accttgacga tcctgcatat ttcgtcataa ttcacacatt cttaaaatta ttcacacatc 1620

cttgaaatgt gttaatattc ccaacattat caattatatg tgttcagaat tggttgcaaa 1680

gttatcaact caattcacgc tatataaacc ttacaatttc tctacatttt tatatttttt 1740

tatattggct tttcttttag aatcaatcaa tacttttttt atcatttaga tacatctttc 1800

atctattaat agattatctt tctatatatc aaaacacgac acagtcacgt gccaaaaagg 1860

atataagaag gaacttcaac ctgttctttt cttttttatt tttaaatttg attattatta 1920

attttttttt cctttctttc cttaccaatt tttctttgct tgacttattc aaaaggtgaa 1980

acagggattt tccaattcac atagccaaaa gtatttttgg tttccacatt ccttcaaaac 2040

aatatttgtg ctacctcccc cttcccacca aaagtatccg attccaacca taaagcagc 2099

<210>  53

<211>  3284

<212>  DNA

<213>  Candida albicans

<400>  53
taataagtac caactaaatc aaaacaagcg accaaattga ataataggaa gacaaaaaaa      60

aaagagagaa aacagtacca aaatagatat agtatgtagt tacatttact caacatagtt     120

attaggtaca aatccaattc tgtagctctc atcatcaatt cttgagactc caatcaacca     180

atttaactca tctgaatgat acaatgtatc aatattctga aaatctaata aaatttcaat     240

attatcgccc tgtttaaatg acaaatcacc tggttcataa ccactaaaat cgtattttgc     300

agttttcaaa actttattat cggtgttaat gttcaacttt tcaaaaaagc tttgtatcaa     360

attcaacttg taagtcaaac tcataggctt ttcaaacgta aaaggttcat actggattgg     420

cttggttgtg attgggcttt ccttaatctc attcttactg ccattgtata tccttcttaa     480

tttagcttcg gatgaatcat ggtttgagta cgaaacactt gacatggagc taattgatga     540

agcttctgac ataatagttg cgctctcgtc ttcaaaatct gatagcagta tagaatccat     600

agaatctgta gaaatagaat ataaccgtga ggcacctgca gaagacattg gcgagacaag     660

aacagaatgc ctcataatag cagtgtttga cctaggtggc aattcaggac catctttctt     720

cggcactgct ggtaccttta tatcttcctc atcgactaat ttccgtggat gatatgtttc     780

cgatgggttc atcgatggat cttggtactg tttgtatgcc accaagggat cgatttctaa     840

agtatcattg aatatgccat ttaccttgtc ttttgtattc acaacatgtt tcttttcaac     900

aaatttatta ctcatattac gccaaaatct gtaatagttc agcagcgaat cttcatcatt     960

gatctcctta tcaagcaaat ccgggtgttt ctcgtgcaca attgttagaa gagactctat    1020

ctgcaacctt gtagctgtac tgttcagttc ccaatcgtct attatttcag tatacgattt    1080

tggtgaattt tctttaatca atccataaaa ctctgtaaaa tattgaaaag tatcagttag    1140

ctttttaaac gtctccaatt gttgacataa tatcatcttg gtaatatttt caacaaactc    1200

atcaagaaat gaaactatgt taggcaataa ttcaatacac tttttattca agctgttgaa    1260

cgcagcatca actgtctgat atgttgtttc taatttctca agtttgtcat tatctttctc    1320

gtccaatgga atcgctttct ggttcaattt ctcaattttg cgatgcaaat gatcctgttc    1380

tgttcgtttc atattacgct ttttaatcaa tttcaaagtt ttcttcaagt atttcttcat    1440

ttcgtcaatt ctatatttga gagattcgtc atatgcttcc caattatttt ccaaatcaaa    1500

ttttaagttc tccaccgtga tcaaataatt attcaactct tcatttatag attcattcaa 1560

aaattgcatc tcctttgggt gtacatgtgg gatttcttgt gttgcttgcc atgaatcaaa 1620

ttcttggtaa tactcgttga ttttatcaaa acgcaaagag tcttgaccaa tcaagttgat 1680

aaatccttta ataattttaa tattcaggcc gagcacatgt ggcaagaaac tcttggacaa 1740

atggtgattc tgcgatgtga tgtacttcaa accagaaact gattgtttga tatcgtgata 1800

ataaatctca acaagttcat catccttatc gtaatctctg gtgtggaatg taactgtgtc 1860

ttcaatgttg taggatatat ttttgaattc tgattcagtg tacttgtacc cgtccttaat 1920

atgagttcca atattagacg atatcagaac aatattattt ttcaattgat ccacaaccat 1980

cgttgtcttt tatctatcag tagtaaattg aaaggtgggg ggatagaaaa tgaactagaa 2040

aaagaaagtg atgattctaa aaaaaaaatt tctcaaatac aaatactaag ataagtgttg 2100

attatatgac aacagggttg gaaagtcaat tattaattaa ggaccattgt agttaagctg 2160

cgcatagaag cagaaatgtg tgcaagaaca ggaacggacg ggaaaaataa taagctattt 2220

gaattaacac gaaataacgt gacctaaatt aaaataagaa taaggaaaaa aaaaaaagat 2280

aggctttgaa ttaatggttt agtcactttt gaactgataa ttgttgatct tgaactagta 2340

atgattagtt taaaaaccca acaggaacac ttagtttgga aaatatgagt ctccatagat 2400

cttctcttta acttatgcac ggagcttaaa agtacagtta gactcaaaaa cgaatatttt 2460

agtgcaatct ctacagtatt ggggtctgct cacaatcaag aagaataacc atttaaaggc 2520

gctctgttgt agaaattgtt tgtctctaca aacgaccacg attagtaaga gaggggagga 2580

aagacaagaa aaaagggggt aatcatgata attgctaaaa agttgaattt ttgtaaagtc 2640

cacccgagag ttggtagctt tttagattct agatctaaca gcagttctct gtaccgtgtc 2700

aaaatatcaa ttgtggatcc aatacagcta ttgtagtggt acttactgat gacgatcctg 2760

catatttcgt cataattcac acattcttaa aattattcac acatccttga aatgtgttaa 2820

tattcccaac attatcaatt atatgtgttc agaattggtt gcaaagttat caactcaatt 2880

cacgctatat aaaccttaca atttctctac atttttatat ttttttatat tggcttttct 2940

tttagaatca atcaatactt tttttatcat ttagatacat ctttcatcta ttaatagatt 3000

atctttctat atatcaaaac acgacacagt cacgtgccaa aaaggatata agaaggaact 3060

tcaacctgtt cttttctttt ttatttttaa atttgattat tattaatttt tttttccttt 3120

ctttccttac caatttttct ttgcttgact tattcaaaag gtgaaacagg gattttccaa 3180

ttcacatagc caaaagtatt tttggtttcc acattccttc aaaacaatat ttgtgctacc 3240

tcccccttcc caccaaaagt atccgattcc aaccataaag cagc 3284

<210> 54

<211> 791

<212> DNA

<213> Candida albicans

<400> 54
aataatgtca atttattacc aagtttccaa agttgtcttg ttggtagatt atattgttta 60

cagattatgg tacgttataa aggtactaat aatgatcaaa atgaatttgc tgataatata 120

gttaaactag atgtaccaat attagtagga taaataaaga atcaataacc atggcacgtg 180

aatatgaaaa ggtaggggct aatataagtg taagtgtagt gtataaatta caaaacaaaa 240

aaggctgttg ttattaagat gagtcaactg tgtaagtgac gatcctgcat atttcgtcat 300

aattcacaca ttcttaaaat tattcacaca tccttgaaat gtgttaatat tcccaacatt 360

atcaattata tgtgttcaga attggttgca aagttatcaa ctcaattcac gctatataaa 420

ccttacaatt tctctacatt tttatatttt tttatattgg cttttctttt agaatcaatc 480

aatacttttt ttatcattta gatacatctt tcatctatta atagattatc tttctatata 540

tcaaaacacg acacagtcac gtgccaaaaa ggatataaga aggaacttca tcttgattgc 600

gccgcaagca acaaacaata agccaaggaa agtatatact ccagatctac tatgagtatg 660

acacagctta ttaatgatca agtctacaac ttctactact aaacacgttc ttaacaaatc 720

aaacagtatt caattgtttt aaaaaacact atacaaaatt aatcaataaa aaacaactaa 780

agctaattct a 791

<210> 55

<211> 4581

<212> DNA

<213> Candida albicans

<400> 55
tgggaattat tagaggattc tttttcagtg gatatataaa taacgaataa attccttgtt 60

taattatttt aagggaagaa aaaaaaaata atcaaacaac caaccctctt tataattaac 120

aagactacaa cttaataaaa atgggatatc caccaaattt caaaattgtt actaaatcat 180

taacagaaaa cattttatta gcatcaacgg ctttttcaag agttgataaa ttcaattttg 240

gtgctcgtat ggcggtattt aaatttcctc aatcaaataa aatcatttta tggtcaccat 300

taccttatac accacaagta attgatgttt tgacaaaatt taccaataat accaatgaat 360

caaatttaaa tattgcttat gtgataattc ctgatcgtga acataattta gctgctaaat 420

catataaaga aaaatttccc gggtgtaaat taattggaat ggaaggatta gatgaaaatt 480

cattgaaatt ggattataaa tttataaaac tgatgggtaa taaagtttta aaaaatgatg 540

aattaaaaca aatctttaat gacagtgaca gtggcttgat tgttgataat tttgaatttg 600

tttatttacc aaatcatgca aatcaagaat tggttgtatt tgataaatca tcatcaacat 660

tatttgaagc cgatttatta ttcaatttag gtgtaccggg gtcaactctg ggtgaaacca 720

ttttagaaca atattcacca gagttggggt tccctaaagg gtttaatcct cattctggtt 780

ggtcatttat aactagatat ttacaaccat attctaaagt tggtcgtttc ttatttagaa 840

aaattgttga tataaatcat agtaaacctg gattagaagc tatttataat tcatgggatt 900

ttaaaactat tgttatgtgt catggaaata ttataactaa agatgctaaa gaagcattta 960

aacatgtttt tgtataaaag taaaagaatt gaagaagata gtcaaatagt aataatcaga 1020

atatatgtat gttttttttt gaagaaaatt aaagaatata ttcacgaaat aataataata 1080

aaaataaaaa gactaactat tttgaataga aaaaaaaggt ggcactattt caatgagata 1140

aaccaattgt gaatatacgt agatgccttg cagcagacaa tataaccaaa tgttgaacaa 1200

tatgtgggat aaatagcatt ttcatctgtg ccattgatat tgcatttata tcctattgtt 1260

gaacagtgac agcacctgtg gcggtggcta ttacataaca gaacaagtgg aacagcagtt 1320

accagtcaga acagatctaa cagcattgtt tttagcagca gcatctttat ctttggtttg 1380

accagatcca gtttttttag attgttgttg agcagccatt ttttatttga atttgttgat 1440

tgagttaata tagtttataa gaattgagag ttacttgttt gagttgttga ttaagaatag 1500

attaaacaaa aatatacaag agaatctgta gacatattta tactcatgaa tttatatata 1560

tatctatgct tatattcatt tgatgtataa attgacatga ttatgaactg caagaggttt 1620

gattttgatt tgtctgcaaa aaaaatatgc tctatttttc gcaattaccc cccaaccccc 1680

ccctcacaaa gttccgagtt tagttggaaa aatgtttcga tagagtaaaa tttcaggaac 1740

aaaattgact aattgggaga tgacaatgag aaacagtttt gagacttgat catacttccc 1800

catacgctca cctctttacg ttaaatatag ctctttacgt tctctacaat aatttttttg 1860

acttattgat atttcttaaa atggttacat gaaataaaac aaagagattc ataggaatat 1920

tactttttca ggtagacaca atgcagctaa ggttggattt ctcaggaaat atcattcaag 1980

ctttatctgt tagttagtgc tgttatttat tactggtgaa ctacaccaaa gcatactgaa 2040

ggcattttac gaggtttttg aaagctctta ctatgtagca actcatctag tacttagtag 2100

aggaagtgca tcaagtatgg atcaaccaag tgttacctta tatcattggt ttaaacattg 2160

taagactcag ttcgaaaaaa aaattaaggt ttctacttac cactttcatg tggcttaaag 2220

ttgtggatgt gatattgaat atgtttcaga tttgtcatga aacaataaga acaataataa 2280

agaagaaatc aaatcaatct tcaatgtatg tatgtttctg tatggcgcat gtgggttctt 2340

tgttttaaaa aaaaaacttt aaattgagtt tgttttttct ttctttgtta gtcaatcaaa 2400

ctttaaaaaa gaagaacaag tagaaatagt atagtaaatt gatatagata cttttattac 2460

taataacaaa tctttaatgg aatttatctg aaattaattg tcaagtttta attcagtaat 2520

gattgatatt actctaaaac aaatgctgtg tggggttgtt ttgtttgacc tgaagtgtcc 2580

aagctttcct gcttcatgat ctaactcttt gtactgctac acctacattg ggaaatattg 2640

accttatagt aacacttact ttcttttatt aattgtctaa actatgcttt tgatcaattc 2700

acacgtactt catttcttct cccctgacga tcctgcatat ttcgtcataa ttcacacatt 2760

cttaaaatta tgcacacatc cttgaaatgt gttaatattc ccaacattat caattatatg 2820

tgttcagaat tggttgcaaa gttatcaact caattcacgc tatataaacc ttacaatttc 2880

tctacatttt tatatttttt tatattggct ttcttttaga atcaatcaat acttttttta 2940

tcatttagat acatctttca tctattaata gattatcttt ctatatatca aaacacgaca 3000

cagtcacgtg ccaaaaagga tataagaagg aacttcaccc ccttgctctt cttattattg 3060

tgtgtggtgt aatagtttat ggtgtggtgt atgattgcgt gtgtgggtgc aaaaaaaagg 3120

tgaagaaaaa aatacctcaa aataaaaaca acttcaaaca ttcccctcat tttctttcac 3180

agtcatttgg tttcaatctc tattggtctt ctttaatcat cactatttat tccagtttat 3240

aagtcgaaaa aagttagttc attgttcaat tgggtttatt tatatttaat actatgcact 3300

tgttcttcct tgactaactc acatgagaaa gagagagtga ggagagggtg aatctattct 3360

ttctattgat tatgcataat tttcaatcag gtgataaata acattatcga ttgttctgtg 3420

tatacgtttg catatctttc ttatctatct tcatagtaag agagagatta gatatcatga 3480

tattgaatag agcgtgtaat tatcaattca ctatcattgt agaaccaccc tcagttgatc 3540

ttgtaattga aagttacaga tgagttgatt atgcgtatag gaaagtattg aagtaaataa 3600

agtccgtgtg tattatctct ttttctccgc attttattgc tttatcattc atcatctctt 3660

ttcttttctt tttattcttc ctttaataca atagtggtca agggggggag gaggaagaaa 3720

ttgcaatcta tagtaacatt gatgttcccc tctttctgat tagtaatccc cctttcacta 3780

ttagcaacaa taaactatat atatatgtat atcaaaccta ccttccttcc ggtcttcatt 3840

tttgttctct tttcgttgac tagaactttc ttaacaaact tcaaaactat catgcccgat 3900

ttatttgata atatttttaa taaaattggt acaaaattca ctggtggcaa aaccactcat 3960

cattatggtg gtgcatctca agtaaatacc gggaaatggt atagttatac cagtagtgcc 4020

agtaataata attattggtt acctcgagaa agtcaaacaa agacaccagg tactcaagca 4080

gaagaaccag aaacagttca atttaaagtg gatcgatcaa tgagtgttgg atcaattact 4140

gaagattctg gtgctgctgg tgctggcggt gatcgatcaa gaatgaatag tattactgaa 4200

taattgtata tacaacgtat ataaataggc tggtcttatt attattgctt ttaatttagt 4260

atcttttgaa agataaattg gttagtgacg tttttttttt taataaattt gtttctatat 4320

taatataaaa ttcagttatt attattaata gtaatccaat tgtaattatt tataatgata 4380

tatataaata tatttaatat acagtttgtt attattattc tttagttttg ctttaaaatt 4440

tattttactt tactttactt tatatgatat tatatctgta ttaatgacga actgaaattg 4500

gtgaaatcgg cattagatta tggactgagg ataaaacagt tgaataaggg ggaggaggtt 4560

tgatgtggtg gtgtcatatc a 4581

<210> 56

<211> 5325

<212> DNA

<213> Candida albicans

<400> 56
aatgggttta tacaatcaag gacaccggtc gctacaaggc tcgccttgtg gcacttggtt 60

atcgacaaca ggctggtgtg gactttctcg aaacgtatgc tcccgtgatt cgtggagaat 120

caatcaaact aatctttgca ctcgcgtcaa aatccaaact aaagattcat tccatagatg 180

ttaccacagc tttcctcaac ggggaaatac tggaactcat atttgtgaaa caacctccgg 240

gatatgaaga taagaagcgt cctaatcatg tttgtaagct caatcgcagc ttatatgggc 300

ttaagcagct gccactaatg tggaacatta aattaaatga tgtacttata aaggaaggtt 360

tccgtcgact tggtggtgac ttagggatat acattagtaa ggacaaaaga acaataatgg 420

gagtttatgt tgacgacatt ctcatttgtg gaccttctga cagtgaaatt gaacaagtaa 480

agaacaacgt gagaaaatac ttctcaataa ctgataatgg attatgccga aaattccttg 540

gaattaacgt ctatcaacaa gcaaatgaaa taagattaag tttgaatgat tatataagga 600

gaatgattga ggagttaaaa ttatctgtct cagaaacaaa cccagtatct ataccatctg 660

atgtcaatta tgaaatattt aaagttaacg aaaatgatga tgagaaacca tgtgatcaaa 720

ccaaataccg aagtttgata ggcaagctct tgtttgccag taatactata aggtttgaca 780

tcgcctattc tgtcaactcc ctatccaggt ttatcaacga tcccaaagaa aaacattgga 840

ttgcagctgt caaggtggta aaatatctca gtggtactca acggtatggt atttgttata 900
acggtaacgg tgacttgaat atttacgctg atagtgattg ggcttccact ccatctgatc 960
gaaagtctat tacggggtac attgttacct atgctggagc gccgataagt tggcgttcca 1020
agaagcagaa cgtgatagcc ttgagtacga cagaagcgga gtttatggct ctcacagagt 1080
ccataaagga agccctttgg ctaatataca tttttcgaga tattaatgtg atattgaaat 1140
taccaattgt gatatatgaa gacaacctac tgtgtcagaa attacttgaa aatcctcgat 1200
tccataatag gacaaaacac attgacttga aatataaatt taccaaagac catatagaag 1260
ctggtacaat caaagtggaa tcaactaatt cagcagataa cttagccgac atgctaacta 1320
aacctttacc aaaaattaaa tttaaacatt taagatggct agcaggatta agacctttag 1380
attgattaga taatgataaa atgaaataaa gattaatttg gagatgcagg ttgatgggga 1440
ggatgttgga aaaatgaaat atgatcaatc ctgcatctag aacctgtggc agaatgaaac 1500
ctacgagatt atgaatgact tgtgaataca agttgaatgt tacagaatgt taccaagaag 1560
gttacacttg aatatatgaa tgactagaaa gtgaattgaa tgttacagaa cctgaataac 1620
aatgttacac gaatgtgtga atgatatgag tttatctata gtaatgtgac atatacacaa 1680
aggtgtgaat gaccgagaaa acagatgtta cattacgggc actggagagt gcaagtctaa 1740
agaatcttgg agtagaaata agtaatataa aaaggaccaa agattcttta gagaaaagta 1800
aatgaaacta tattagattt tatataacta actaacaaat aaataaaaaa tataatatgt 1860
ctacaatgcc accaacttcc aaacgtacta gaaagagaac tagaaccgat gataatgctg 1920
aaccaactat tcaagatcct tcaccgccac ttgctaatgt tgaacccaca attcaagaga 1980
ctccaccgct ggttgaagtt agtgatgaga ctaattcaac tgaaatcaat gagacaaata 2040
gtaatactca tgaagaaaca aatgtattaa ctaatgtgca ctcctctcca atcgagacag 2100
ttactgagag gaacttcaat tttcaacagg ttattgcctc tatctccact gtggacaatc 2160
aaagtctctt gaaggataaa atttcttatg atcattggtt cagtaccttg aaagaaaatg 2220
caatcatgat tagtccagat tttcttgact ttattaacaa agacaccatg gatctccaac 2280
agtacccaac tgtctaccaa acattcttag atcgtcttat ttgtgccaca attgacccac 2340
atatcaaaca atctttaaaa tatcggaagt tatcaggaaa gaaaatgctt agtgaaatta 2400
tctctcaatt tggttctatg actattaaag acaaggttaa ctactccata attatggcta 2460
ccaaaattca ttctgatgtc accactcatt tagacaaaat gaatttactg gctcaatttt 2520
acgcatttct tatgcgtcaa cctcaggacc ttaaacctgc ccttttactt attgcgggta 2580
tcaatgactc acgtttcaat gaaacatact ttcacgataa caaagaatta acgatctcta 2640

agttggaacg gtatatcatt aatcaaaact ccaaaattac tccgtcggta ccaacacctt 2700

ctccacgtga cgctgttacg ggtttactgg ttacccagcc tacgtccgct ctgggacaaa 2760

gtgaagtgtt taatacacaa tgttttaatt gctttgggtt gggccacact gcacgtcgct 2820

gtgcctctcc gaaacgtctt ggccaaataa acaaccttag atctaaatta cttgcgtttg 2880

aaactcgatc caaatccaga aagcgttttc cacctcaacc tcctcctacg aatcggtcgg 2940

caaactcaac aataataact aatccctcac ctactgacga taccatctcg tccaccactg 3000

aagattcttt tccacgggac gtctttggat gggcggcatc atctgaccaa atcaaatcaa 3060

aggacaacct ttctttattt tttgacacag gtgcctcggc acatcttatc aataatctca 3120

atctacttca tgattacaaa ccctctaaag aaaacaaaca tgtgatcact gcgaacggtg 3180

ataaaattcc tatcttagga actggaactg tgaaactcca acatggtcaa cacaagatat 3240

cacttcgcaa ttgccaatat tctccacatc tacacatcaa tcttatctca cccagactct 3300

tacttgatga ttccactagc atgactatca cccaatccgg gatttatcac tccaaaattg 3360

gacaaattgg gtattattcg actgaagatg gtaatctaat caagtgtatg ttccgtccca 3420

ttaccattcc tcatctttcg ttatattctc aatatgtcga aatgggtctt caatctaaca 3480

atgtactacg taacattcca gctttcacgg tccatattcc tcaactacat gactcccttg 3540

gacacacatc tactcaacaa gtttcaaatg tcatgaaacg tttcaatgtc actactgaca 3600

acattggtac ggactgcgaa acttgtcggc ttggaaaagc cattactcag attcccaaga 3660

tctcaaccca taccatctct agtcattgct tagaactact tcacgttgat gttcatggac 3720

caatatccgt tcctagtata tttcaagaac gttattttct tgtgatcctt gatgactact 3780

caaaatactt gacagttcaa ccactatgca acaaatctga tgctactgcc gaaattatcg 3840

aattcatcaa tcattgggaa aagttctttc tgggaaatgg caattaccat acgaaaattc 3900

tccggtcgga taatggaggg gaattcttaa acaaaacatt gactacctat cttgattcaa 3960

aatatattac tcaccaaacc tccaatgcct atgaacatca tgagaatggc gctgcagaac 4020

gagctattag atcggttaaa gacatggctc gagtaatatt gcttcaatcc aaattaccag 4080

tgccgttttg gtccctagca acccgatgtg ctgcgtttgt tatgaatcgt cttcctcata 4140

aaacaataaa tggtaagatt ccttatgaag tatggactaa acaacttgtc aatctcaaaa 4200

tgatgaaacc gtttggctct caagtatatg tgaaaattcc tattggagtc aaaagttttt 4260

ctgcacaagc actttctgga atcatggtgg gatatgccac taataagaaa ggctaccttg 4320

tatatgatcc cacacaaaat cgaatattca catcctcaca aataatatgt catccgagca 4380

tttatccagc agccaacctt acgtttaacg aacccttaat tatctcatcg aaagtcacgg 4440

ctgctcatct tcaccccctt accatttcca atttagttat tccacctacc aatgctgtat 4500

ctgagacacc tctgcaaatt gtgtgctctc ctcaaattcg tcagtatgtc ccaaagtttg 4560

ccaattacaa actgtcttgg aacatgggga ggataaaata tatgcactga ttataccaat 4620

atcgatcggc aatatgaaac gcacaagaac aaatgaaaac aaaatatgcc agctagatga 4680

atcgaacaat accaccatac cagatagtgt aattttatcg gctaacaatg tgttattaaa 4740

cttagaatcg agatcttcca ttcccaaaag ttataaggaa gctataacat ctaatgaaaa 4800

atccaaatgg gctgatgcta tggatagcga gtttaattca ttacaatcca acaacacgtg 4860

gtcacttgaa ccactaccgg agggacgcaa agctattggt gtcaaatggg tttatacaat 4920

caaggacacc ggtcgctaca aggctcgcct tgtggcactt ggttatcgac aacaggctgg 4980

tgtggacttt ctcgaaacgt atgctcccgt gattcgtgga gaatcaatca aactaatctt 5040

tgcactcgcg tcaaaatcca aactaaagat tcattccata gatgttacca cagctttcct 5100

caacggggaa atactggaac tcatatttgt gacacaaccc tccgggatat gaagataaga 5160

agcgtcctaa tcatgtttgt aagctcaatc gcagcttata tgggcttaag cagctgccac 5220

taatgtggaa cattaaatta aatgatgtac ttataaagga aggttccgtc gacttggtgg 5280

tgacttaggg atatacatta gtaaggacaa aagaacaata atggg 5325

<210> 57

<211> 2027

<212> DNA

<213> Candida albicans

<400> 57
tttgtttgat aaagaaaata aaaaaaagaa acaagggtag taaatgagta cagtagccct 60

gttgaacaaa gtctgcgata acttaattat gggtgaactc aaggggacag tgtctttgtc 120

tatcatccga tccttaatca agtctattac tgaatatcaa ttatttggac acctgtttat 180

aaattactat ccaatctatg ttctttcaat tctttccttc aatattttgc cagccaataa 240

gaccaaacat aatccaaata tacataccag tgaattctaa attgtttggt gaaacatcca 300

tttttgatct atttcaaatt gtattttctt ttagtagtag tagtagtagc agtaattgat 360

taattattat caatatccga aatgatgata agaataataa ttatatatat aagaaagaga 420

aaaagagaaa agaagaagaa gaagtataaa agaagttgtt atgggtttaa ttaaaaaaga 480

aaaaattcaa tgaaatttgt gttgtgttgt gttgggtttg aatttctgta taactcaatt 540

tggagatttt tttttttttt tttttttgaa atttttatta gtcgtgtaca ttgttacaat 600

tgtttctcgt tccccttttt tttttccttt ctttgttttg ttttgtttac cttgtgataa 660

ttttatacgt gttgagaggg ctctcgtcgt gcccgtgtcc gtttccgtgt cctgttgggt 720

cccctccgcc catgccgcac cgcaccgtac ggtaatgata tctgattgtt ggagcgttct 780

tcgctaacag gttctttatt tttgttcggg ggtttcgaaa gataatgtag aaacaccagg 840

gcttataact gagagttaga gtagtggaga ttagtagtag tagtacaatc ctatagccca 900

aacattattg gagagatctt accaaatagc aatcatcatg atgtatttac tactacataa 960

agaatttaag acgatattta ccagcaataa acaacatgac caactaatta acaaacattt 1020

gaaaaacata aagtaattag aaagtttaaa aagtgtacaa ccagtgtgga aaaagaatgg 1080

aattggaatt gaacaaagtt attaattact gaaaaaggaa atttaatttc ttgaaaggca 1140

aatctttgtt tgtttttttt tttgggtctt ttctttcatt taataagcgt ggggtattaa 1200

tagataatga tattgttgtt gttattgtga tattgttgtg aaatttgaca tatgataaga 1260

taagtttctt tcttttcttt caactagtat aattgaacta aagaccacca ccaccaccac 1320

cacatagtta gcaacctgat atgctgttca tgtaacagta aattatcttg gtactatacc 1380

acttgttgta atatagctaa tgctaattct tgattagtgt ggaaagccta ataaggttat 1440

attgtgcaca ggttaactac cttaatatag ttattgttaa tacagttatt gctgttgact 1500

actattgtta ttgttaaatt aaagtgttag gttgagttaa ttgattagtg aaaaccaact 1560

aactaccgta ttaaattatt gtattaagat tgattcctat taaggataaa acagagagtg 1620

tgttagaaag agaaagggtg gattataaat atgtgtaaaa tcccctttag agactaacca 1680

ctagaaatct attgatggtt tcatatatag agattaacga ttatatttat aatataagtt 1740

ggtagttgct agtatatttg aaagcactac agtatagtat gtcagaatca gatcatttaa 1800

actctactaa taatacagga aacactttca ttagtctaga tcaagccagt acaataatgg 1860

cagatcaaac tcaaggagct aacccacaac aatgataatt catctttttt gtcaagacga 1920

taggttaatg ttacaagcac tttattgggc tcgaaatagt ggtaaataag tccatagata 1980

tgacctgtta caagttattt cgatgatcaa gccggctctg tgattac 2027

<210> 58

<211> 2118

<212> DNA

<213> Candida albicans

<400> 58
tttttttaaa gaattaatta aatatgatgg atgatagaaa ttaaaggaaa aagaagaaga 60

acaaaacaaa agtttaattg aaaaaaaagg gagaaatgaa tattgaatta ttcagctttt 120

atattgctga tagatgttga aaaaaaaaac ggaagaatgg ggatagcaaa actgtgggtg 180

agattaactc atctatggcg ctaaaagtct tttttttttc tcttttatta gggggcacat 240

aaattattct tttcattgat aatctcgagt ccgtttttag ttcattattc ggaatatatt 300

accgtattgg gaacgataat tattattagt tctccccgat ggttcgattt tgctggtgca 360

aaaatataaa tccgatatta ctttattggt gttttaataa atccgtttta aaagttcgta 420

gacatataca ggatgataat aatttaaccg atttataagt tggaatcatt tggatgaatc 480

cgcttgggga gacgttttcc aattttagaa gtttaactat caattttatg tgacatccga 540

gtgtacacat tttgtgaatt tgatcttatc aactcacttg gtgtaccatg gcatttataa 600

caacactttt tagaatcggc tgagttacat gcatttcctc tatttgtaga ttaatggaaa 660

ttcataaaat cgttcacatt tttttctata atgagtacca ttctgtttcc ataagtaggg 720

gactaaaaaa taattgatat ctctaatcag tgacagctct agtcaacttg accgtaatgt 780

tttgacgacc attatatttc ttgtttgaac tattgattta tgagtgttgt cgtaacaaaa 840

gatcaattcc cgtcaaaacg catttggcac ttaatctttg attgaaccga ttttgatctc 900

aaaacatagt accaaggtca attatgttcg ctaatgaaag aaagctgtga cgaaaacctc 960

aaattcatga agaaagaatt actgttgtgg aaaataaaaa agtctttctt ctgatacttt 1020

acaagtccct caaccacaaa tacaaaaatg aaagttaccc atcgatcttt ttcattggtt 1080

aagaattaat acgagaatat caaattatct tagagagggt ctcacagagc aactttctga 1140

ggcacacggt caccaacatg atttgttata aaaaattcaa ccaaattttg gaaaaaatga 1200

aaacaaaaca aaacaaaatc tgaaacatcc cgaaagtcac aaatgcttga ttacttaaaa 1260

ttacttattt gcttcaagac gctattatta ttattatgac ataatactac ttgaataaca 1320

gtgaactgta attgtattaa gaacaaatca taacaaagga agatgatgac gatgatgatg 1380

accccttgaa atatccaggg cacatgcatt gtgatgattg ttgtaatata gctaatgcta 1440

attcttgatt agtgtggaaa gcctaataag gttatattgt gcacaggtta actaccttaa 1500

tatagttatt gttaatacag ttattgctgt tgactactat tgttattgtt aaattaaagt 1560

gttaggttga gttaattgat tagtgaaaac caactaacta ccgtattaaa ttattgtatt 1620

aagattgatt cctattaagg ataaaacaga gagtgtgtta gaaagagaaa gggtggatta 1680

taaatatgtg taaaaatccc ctttagagac taatcactag aaatctattg atggtttcat 1740

atatagagat taacgattat atttataata taagttggta gttgctagta tatttgaaag 1800

cactacagta tagtatgtca gaatcagatc atttaaactc tactaataat acaggaaaca 1860

ctttcattag tctagatcaa gccagtacaa taatggcaga tcaaactcaa ggagctaacc 1920

cacaacaatt accatattat atgaagaaga ctataacaaa actgtagata gtaggggatt 1980

ggttatttcc ggggagtaga agtattgggt tatctaagtc aatctttaac aaccaacaat 2040

caacaacaac caacaacgtt tttcctattc tcggagataa cttgattaac ttaaaaattt 2100

tcttgtcaaa aaatttct 2118

<210> 59

<211> 4929

<212> DNA

<213> Candida albicans

<400> 59
taattcgcgt atgaatgaga ttgatgccac tgttggtgct gaagttttaa aaagaaaaca 60

aatggaagat atgcaaaaca ataatagtaa taatggaggg aaaagattta aatcagatcc 120

agtttctgat caagaaatat tagatgcttg ggaaaataat caattggata ggttttcagt 180

ggatcaattg aaggcattta gaagaaaata tcctgatgtc aaatcagcta ataagaaagc 240

tgacttgatt gaaaatatca gtgagtttat aaggactcat agaaaatgag ttaatatgta 300

atagtgatat gtttatagct ctgtaaatac atgtaaattt tttggttgcc aatgaattga 360

ttgagactga aaatcgtttg tggtttgcca atgaacatta aacttattac ttgatctaga 420

aggcagttac ttgtttaaag aagtgatgag tcgtgattaa gtaaagtttg cagcactaaa 480

tattgtatgg tatttgactt aattttttct gcaaaaaaaa ttacaaattt ttaatgaaaa 540

aacaaaacac aagataataa cattatagaa taaagattat aggatcctac caacatagtt 600

ccattgctga tcaggacgtt taataaaaga gcttcccaac agagacatat cttaataata 660

acaggctatt ttctgccttt aaaaagccat ctaggctcaa aaacctcaaa ataattcatc 720

tcccaccttg gcagcagagt agccataaca cagccaaatc aatttctata gtttacataa 780

tatataaaag gtttctaata gccagtaagc ttatagaaat tacccttttc aagtgatttg 840

atgaacaaat tatattcttg tacaaaatag tatatttaaa attaagaatt tggcttgcaa 900

aagaaactct cggtagctta gttggtaaag cattagactg taactgagtt attgtttgca 960

aacaaacaat tggaatgcga tctaaggatc gggtgttcga ctcactcccg ggagattttc 1020

ttttttacca ccaccatagt taacacgcta ccatatgaga cagaaatcta gcatgaatgg 1080

cttatataca agtggaccat ttagaagcat gagctgtgtc ctagtttttt atcatttaca 1140

attgaatttc cctctgaaat taaaattcta aggtattcat ttatctcaac tttcttagat 1200

gctgttagtg ggttaaaact tggtaatgaa ccactgacgg aagttatttt tgtgagaatt 1260

aactataaat atatcagctt ggtttttttt aacaacttag acagcaataa ccaacaccca 1320

actaattaat caacattgtt ataaagttgt tttcatctgt caaaccaggc acatggtagc 1380

acatcaaaat cactctcgat agcttagttg gtaaagcatt agactgtaac tgttcattct 1440

ggatattgat atctaaggat cgggtgttcg actcaccctc gggagaaata tttttttttt 1500

gcttataatt ccttcaaata tttacctcca gtatcggtat tgaattaaat acagagagca 1560

attggaaagg ttattttttt tgttatttat tccaaaaatt tcaggactca aagtttaata 1620

agccaaagcc tattttgtac tgcgcttccc tttaaagccc ctgctagccc ctgggcttgt 1680

tgttgttgtt gtgtatggaa caagtttatt aaatcccatg acgacgatga tgtaattgat 1740

tttgagaaaa aaaaggatga acaatggaaa aaggtacaat gggttatata ctttgccatg 1800

tggttgaaaa tatgtttaac ggctgtagaa ctttttttta ttttgtgtta gtgagtgaat 1860

ttcgctacaa ttgttattat actccacaat tcagatttgt tgataacgtt taattactta 1920

aattttagta tgcatattga tatatttttt ctatgagatt gacgattaat tatcggtttg 1980

taaaattcta ttgaaacaca ttcaccagtg caacaattag acattttctc aaaaccatga 2040

atagcttgca actaaaacaa acaataaggc tgtacacttt gctggcaata aatcagtgtc 2100

aagtcaatat aaacagtctt aagaacaatg agaaactcaa aagttagggt agttagttga 2160

ttacaaaaga aagagaccac ttagagacaa aataacaaga aatgacatca ccattgtaat 2220

agatacattt tccagttatt caagcaattg attgaatgta ttcatagcaa aatacattta 2280

agacatacaa gcttaaacat gggttattct ctagtggtgt tgttgttgcg attctaagac 2340

tccaatctat gattaataat cggatcacca tttgcacatg aactacatta agtactaaaa 2400

aatatgcaat tcgcctgttt tcttattgat taaatttaac aataaacttg tctttagctt 2460

tggcaaaagc ctccttgaaa atcctaacta agcacgttgg aagagcaatg gaattgtggt 2520

tagttataga aagcaaaaca atctgaaatt gtaaagtatt agatgatgtg caatgatatc 2580

agaataaaat agttgctgtt gaaaattttg ttcaagactc ttcacacagc atagcaaata 2640

gttatacata aagagaaaag ttcaacgtgc tttgttgccc gtgtctattt gtttttttaa 2700

agccgaattc accactagag ggagtatata tgattcagag tatcaccatc atcatcatcg 2760

agcccccgta aaaacttacc aactttcgtc gacatttccg atgagaaact tgattttttt 2820

ttccttccgt tgaaataatg tcagatagct cgcaaatatc ggaacgagca aattcttggt 2880

ccagcaccaa taattcggaa aatcacactc agttaatatt tacttacaaa ataaatttat 2940

ttgtaattta atggctataa aatgggaacg tagtaagaaa atcaacagct gttgtaatat 3000

agctaatgct aattcttgat tagtgtggaa agcctaataa ggttatattg tgcacaggtt 3060
aactacctta atatagttat tgttaataca gttattgctg ttgactacta ttgttattgt 3120
taaattaaag tgttaggttg agttaattga ttagtgaaaa ccaactaact accgtattaa 3180
attagtgtat taagattgat tcctattaag gataaaacag agagtgtgtt agaaagagaa 3240
agggtggatt ataaatatgt gtaaaatccc ctttagagac taatcactag aaatctattg 3300
atggtttcat atatagagat taacgattat atttataata taagttggta gttgctagta 3360
tatttgaaag cactacagta tagtatgtca gaatcagatc atttaaactc tactaataat 3420
acaggaaaca ctttcattag tctagatcaa gccagtacaa taatggcaga tcaaactcaa 3480
ggagctaacc cacaacaaca gcctagtctt cttgacacta aaaaaaaaga gataaaaaac 3540
aatttcagcc aatcacatgt actacatttg taatagattt tattacttca gctgcttatt 3600
acacaaacaa ggttgaattg atattgtgta gagtaaattt tcggaaatag tttgaattgg 3660
gtgatcattt tctttatttt ttttatgtct tgtttctgtg aagatcggaa tgccagagtg 3720
gagctcgtga attgcaccac taattgcagc agcaccatat ttcaaataaa gtttctcatg 3780
ttgtagtaag gattgcttgt ctccatgaaa ccaatcactt aactaagccc caggctaatt 3840
agtgtgtctt caaacagttt tgtactagag aaactcagac cttcccaggg caagtaacaa 3900
cctaaaaaaa tgccacaaaa ctaaatgcaa tttcagtttg atatgatagg caatgacatc 3960
aacacctgga aaaaaaaaaa actttcaggt gatgaaacga ttaaggatta aagtttgcaa 4020
cgaaaaacaa gtggaactaa actttgcctt attgttttgt tccgcttacc taatgatgtt 4080
tactccttag aacaaacaac atcaactact tttaatcctg acgacgaaga agaagaccaa 4140
aaagaataat tagccgcagc tacggtggtg gcactagtag tagtgctagt gcttgttgtg 4200
tctcatccaa gagaaatgga aaaactgcaa aaatgccgca actttgaaca ttttggaaca 4260
caatacaact ttttttttcc ttttggattt acgattagcg cgatagacgt gaccataaaa 4320
ataccacacg atgtgtagat cctctaaaaa taatgtacac atttccaggc ttttgtttac 4380
tgcttaataa tttgtcatca tcggtaacaa tgatagtctc cccaccctaa ctacagtaga 4440
cggaattaga caccaaagat cttataaatc aaccccaaat tttcccattt tgatttttga 4500
ttttttcgta ttccttgttg tttccataat tttttagtta ctcctcctca actaaactag 4560
ataactcgtc acagttaaca acagaaaggt atgttaaata tttatttcgt tctaaattca 4620
agtttggtat agaatattgc aaacaacaac aatctgaaaa atggacttta atttgctcta 4680
caaaatgcaa acacatctag aattaatatt tggtctggaa accgtatacg gaagttatgg 4740
ataatcacgt tatcctgata tctattatta acaccaccac aatatctatt atttcatgta 4800
tggattgcgg tgccaagatc aaagaatcat tttaacccga tatcttacat ttcacctcga 4860

tctaaatgtg attcagtatc accggctcat tgtttcacca ctcaacctcc ccatactggg 4920

agtacatat 4929

<210> 60

<211> 4954

<212> DNA

<213> Candida albicans

<400> 60
tgttataaaa aattcaacca aattttggaa aaaatgagaa caaaacaaaa caaaatctga 60

aacatcccga aagtcacaaa tgcttgatta cttaaaatta cttatttgct tcaagacgct 120

attattatta ttatgacata atactacttg aataacagtg aactgtaatt gtattaagaa 180

caaatcataa caaaggaaga tgatgacgat gatgatgacc ccttgaaata tccagggcac 240

atgcattgtg atgattgttg taatatagct aatgctaatt cttgattagt gtggaaagcc 300

taataaggtt atattgtgca caggttaact accttaatat agttattgtt aatacagtta 360

ttgctgttga ctactattgt tattgttaaa ttaaagtgtt aggttgagtt aattgattag 420

tgaaaaccaa ctaactaccg tattaaatta ttgtattaag attgattcct attaaggata 480

aaacagagag tgtgttagaa agagaaaggg tggattataa atatgtgtaa aatccccttt 540

agagactaac cactagaaat ctattgatgg tttcatatat agagattaac gattatattt 600

ataatataag ttggtagttg ctagtatatt tgaaagcact acagtatagt atgtcagaat 660

cagatcattt aaactctact aataatacag gaaacacttt cattagtcta gatcaagcca 720

gtacaataat ggcagatcaa actcaaggag ctaacccaca acaatgataa ttcatctttt 780

ttgtcaagac gatagttaat gttacaagca ctttattggg ctcgaaatag tggtaaatag 840

gtccatagat atgacctgtt acaagtttat ttcgatgatc aagccgcctc tgtgattacg 900

gcaattattt tactattgat aatgagtaaa agttcacaac caatagaaga tatccaccca 960

agcaatttct ctcgacgaac atctttagaa tagttggtat aataacctta cgaaacatta 1020

ataaagaaat tgtacccgat cttgttttcg agtcaaaaac aaagaaatca aacctagaat 1080

caacaatgtt ctagccatca tctcccgcca cccaagtgat gtacccctat ttcttgattc 1140

tattattttc tgaccctgtg agggaacaaa gatactatct ttaataaaga aacaaaacct 1200

caacaacaac aacaacacac taacacacta agaaactaaa acttgacgac aatatgatat 1260

tgtgatatat taatactgcc caacattcat cgtcgtcaaa tcagaattca gagcaaaaaa 1320

gagacgttta cgttacattc cccgatgttt ttgtgacgta acaagccgaa gagagggaaa 1380

aaaaaagtat ggttattgaa aatctagtta ggatctactt tcctttttgt ctcatctatt 1440

tatcaaacac tatcaacgcg ttttgaattg acgaccagat ctatatcatc tagtttataa 1500

tattctttgt cagatctgaa ttgatcaatg tgtggttgtt gtttgtagtt ttttgttgga 1560

tttaaactac tcacaaacat caagcttttg agtaagaatt gaatcaaatt caatattgtc 1620

ttgtcacttt ttttctgcgt ggtacactac tacgaaacaa aatttaaatt gtcgtgttct 1680

ttttgataat ttgtttgtta taattttttt gcttgtgtga aaaaaaaaga gaaatgataa 1740

ttcgtttttt ttataggggt ttttctaatt caactcttat aataaattaa cttatcaaca 1800

ccgtaaatat aattaaacca actgtgttgc gccataaata aataagttgt ttcgggatca 1860

acacatctcc aacaaattga atcgtaggtg aaaatttttt ttttactagt aattggtagt 1920

aatggtgttc acgagtattt ttttttgggg agtatttgtg tcccttacaa gaaataaagc 1980

cagggccatg aaaaaaaaat taatacaaaa caaaatattc gtatcagcac agcagcactt 2040

cccccccttt ccccttcggc acgccctaaa aagaatttac tcatgtagtc gttatcactt 2100

caacaccaca caagaatacc tcgagtgaaa gaaaattgct tggggaatgt gtgtaattgg 2160

ctatgtagaa tttggtatta ataacatttc tactgttttt cttgtgccat aacatacttt 2220

tatcgcgata tattgcaaag cccccccttc tagctcctaa taaaaaaaac ccacattact 2280

attatattta aagtgtgaat tggaggggac aaaaacagaa caatgagcaa tttataatag 2340

tgaataacct ttagcaaaaa aaaaacattg taaattcaat atttgacgat ggatttaaca 2400

aacaatcaat caaattctta gtgttgaact gaactgaagt gatatttttt gccatatgca 2460

caaaatctta aatattcaag tctacacgag aaaacccaaa aaaaatgtta ttgtttcaaa 2520

aattaatgct tatgtaacac aacgccaaat ttaaaccatt ttttttgtgg ttactaaaaa 2580

aaaaaacaaa caaaacaaat aaaaaaaaag gattacaaat ttcaggcaca ttgtttaaat 2640

ttactgacgc caattattgt ttgattcaag tataagttga gaatgatttt cccaatttat 2700

taaaactaca tacaaaagaa tattaacctt tctattttct ttattttttc aatttaaaag 2760

atataaaatc gtttcacctt ttctttaaaa ttataatttt caagacttac cttatttgcg 2820

ttttctaatc gcgtccactc ctttattact actattagct taagtctttc gttcaaaaaa 2880

caactacaat gcgtgccaac tatttgttat tattagctgc cacagctgtt caagctgctc 2940

cattcattaa gagatatgaa aacactactg ctccagccag tcaattgtcc acttcattgg 3000

ctgatggttc cactaccatt cttggttctt catcatccag tgttgaagaa gatgaaacca 3060

tcacttccac tatcgttcaa tatgttactg tcacttcttc tgacaccact tacgtttctg 3120

ccaccaacac tttgactact actttaacta ctaaaccaac cccagttatc accactgaag 3180

ctgaagatga cgaagaagac aatgaaacca ttacttccac catcctccaa tacgttactg 3240

ttacttcttc tgacaccact tacgtttctg ctactaacac tttgactact actttaacta 3300

ccaaagcagc cgaagctact gaatccgaag aagaagaaaa cgaaactatc acttccacca 3360

ttcttcaata cgtcaccgtc acttcttctg acaccaccta cgtttctgcc accaacacta 3420

taaccagtgt tttgactacc aaagcagcag tatctaccaa cgacgtcagt gaaaatgcca 3480

aggctgctac tactgaagat gatggtgaaa ccactacttc aaccattact agtatcgtta 3540

ctattactga tgccaatggt aacaccgaag tgttgaccga agttgcagct gagaccagtg 3600

gtgcagaaga tgcttcctac tgtgttcctt ctactgtcac tgttactgtc actgctgaac 3660

aaacttccga agttgtttca actattgttc acactaccca agttccactt actgctgaat 3720

ttacccttga tgataccact actaccctta catcttgggt cgacttgact tctacagatc 3780

tcgttactat aacttctact tcaagtgtct atgattcata ctcaactggc gtttctcaat 3840

cccatccaat tcctcatact ccaactacac aatttcggac tatgccccac caatcagttc 3900

ttactactct ttgtaaagag cttgatatga aagtttgtga tagtgatact actaccgccg 3960

ccaccaccac acctttagag taaagatttg tttttaaaaa aatcattctc atcatttttt 4020

ttttattggt tttccatttt atgtcgtttt tgacgttact catttgtttt tattgtattt 4080

tgataactgg gtttatttga atttttgctt ttttttattt ttatttttaa cattgttatt 4140

cctttttcct ttgattattc ctttagtggt tggtgttatt ttgatttttg cttacatttt 4200

tgcttacatt gttatatttg ttattccttt gttagagttt ttttttattt ttgccctttt 4260

cccttttgga tttttttatc attgtctgtc ttattcaatg gttttctagt ctaaaaattt 4320

tggtctagtt gctatttcat atctctgttc attatctcta tccttttctt agaaacatca 4380

ttctctctct ttctctctaa cattcctctc tctcatattc tctacaattg tctagataga 4440

ttttttatag tccttattgt tttttatttc tctaactata tgtatcattt tttattcttt 4500

tacatatatc tttactcttc tttctctttt tatttttttt ggatataata aataaatata 4560

catttgccgt gttatattca aagatggatt gatattggaa ttggaattga aattggtgtt 4620

gcaaaaaaaa tagcaaccaa aaaaaatgac aacatcaaca acaaccacga ataggagaaa 4680

aaataaaaaa agaaagggaa agaaagaaag gaaaacaata gaggtggttt gattacataa 4740

gcaaccaaaa tttctcgcgt ctttcgctct gtttgttttt ctgcctttga aagggatgac 4800

agcagcagaa aagcaagaag aaaaaaaaca acacctacaa ttcttcattt gttttgagtt 4860

ggccctacat tcaaagatcc aatttagcag tcatcaagaa taatttacaa tcgatcgacc 4920

tcagtcatca ccaaatagtc aaaccaatta ttaa 4954

<210> 61

<211> 1047

<212> DNA

<213> Candida albicans

<400> 61
taataattga ttgggttttt gggaaatcac caattgtcta caaatctatc catatataac 60

ttaacactaa ggttaacctt gatcaagaag aagggagtgg gggggggggt gcatttatcc 120

tttatcttgg ctattgtggc gatgcataat tcgtaatata acgtaattaa tgagcaatta 180

aataaataaa ttgatctgat acaacaaaat aaaaagaaga aatttaatta atactgtggc 240

acgtgacagt tgattctaga tcaattcata gtccgcgtcc ccgaaccgaa caaaaacagg 300

gcaaaatgat tactgttgta atatagctaa tgctaattct tgattagtgt ggaaagccta 360

ataaggttat attgtgcaca ggttaactac cttaatatag ttattgttaa tacagttatt 420

gctgttgact actattgtta ttgttaaatt aaagtgttag gttgagttaa ttgattagtg 480

aaaaccaact aactaccgta ttaaattatt gtattaagat tgattcctat taaggataaa 540

acagagagtg tgttagaaag agaaagggtg gattataaat atgtgtaaaa atccccttta 600

gagactaatc actagaaatc tattgatggt ttcatatata gagattaacg attatattta 660

taatataagt tggtagttgc tagtatattt gaaagcacta cagtatagta tgtcagaatc 720

agatcattta aactctacta ataatacagg aaacactttc attagtctag atcaagccag 780

tacaataatg gcagatcaaa ctcaaggagc taacccacaa caccactcag atttagcccc 840

tctaaaatgc atatggcaca atgatctcac ctcggttggt taaacctttt tcttcttatt 900

aaatctatct tagttgtagg ttggtctccc ccccctaact agttttacaa ttcaattatt 960

aaaccaattg tcaattcttg gtattttgta aacaagactc attaataatc aatcgtcaat 1020

gcatatgatc aaaacaaata gaaactt 1047

<210> 62

<211> 7929

<212> DNA

<213> Candida albicans

<400> 62
aagagattgt agtgaagaat tcagctcatt attactgttt tgtcgttgct ggaaggagga 60

gggataattc aatgcgccac aacagtgtta ctatgcatgt ggttctgact gactgatatt 120
gtttaaaaat taaccagctc tcaaataaca aaagtttaaa ttttcaaggt ttgtaaacat 180
ggcagctagt agtaggatgg ttcataatat taattaatta ttagtaataa tggctaagtt 240
tttgaagcat tgttttaaat tttcaaattg aaattcaatt tcattacaaa tggattacta 300
acggaattcc taagctcaac tgaataccgt gattgaaaca tttgaatttg tatcttttag 360
attagctatt tttacttttt ttgtcattgt agttggttat gataattaca agaaactaaa 420
gtttaatatt ttcatattca ttttcttttt tggccaactt gcaaataaca cacaaaccca 480
aaattaaata attagattta atgcatgcat aattacacag aatgtttagc cttaacaagt 540
attctagaaa caagaaagaa aaaatgtcgt cttggcgttt atcttaattg tattctgtaa 600
actgggttaa ttcttatttc caacttttca tttttttgga tcttgtatgg attaaaaatt 660
aaatatggta tgttttaggg ttgtattaac aatacttaca attatcaatc atacagcttt 720
actattttta tttatcagca aataggggaa ttcaagttgc atgtgttatt cagtggcagt 780
gaatcataaa acagccaact tgcagcttat ttcactccag gagcaatcat cacggaattc 840
cgtttcccat ctcattttca tactctgtgg attatgtata gaggctattt acaatatcac 900
caagcagtaa aacattctct cctcaaaata acaataagat tagtcaagat gaacgacttg 960
aatctattca tatgcattac acatttagtt tctattacaa atagtgatgc aatggtgcaa 1020
gattacgtct tgtctgcact aactatttgt aacgatgatt atgtgatcaa gaattggaat 1080
tcttattata ttcagtcgtg agtgtaagct atttcgttag ggttatctta actcgaagtt 1140
aaagttccaa aactattcca tttggagttt ctgttgttga gaaatacaaa atactcttct 1200
tggtggggag gaaatccatt aatgattata aaatgaaact cttggtaacc taattgaaac 1260
accacattca gtacattttc aaccgtcact attattattg tggcaaatgg attaaacaat 1320
agacctaact taatctaatg gaaattttaa atccatgaaa ggggtgaaaa tttgaaatca 1380
aaataactat ctgaactgaa ataccccatg gatctgatat cttatacaat ctatcaacta 1440
aacagggaag agtacctgga attccaaatg acaattccta ttataattat ttaaacagac 1500
tatgccgtat tgtttgtgac attcattgtt ttccacaact ctaatgtcaa atttttgtta 1560
ttgtcatgta atcccggtgt ttcttttttc ttttcggtgt tgcgttccat gatattttgt 1620
tatctcttgt ttagattgag ataaagaatt ggttagcagt gtagccattt atgagtggtt 1680
tgtaaaaaca agaattacaa ggtttgaatg aattccaggc aggcagtatt ataaaacctc 1740
gaaataacta atcaaaccat cagaaaagaa agcttactat gatgtactgc ttaatctcat 1800
atctatctta caaacttaat tcactgattg tggcttgtcc gtgaataatt cggaaacctt 1860

gtctttttcg gtccagtagg gggtgccata gtcttgggtg gtgacaaaaa aaaaaaaaat 1920

tatagttggg gtggtggggt gtacgtctga gtaagtcagg ggaatgaact caagacaaaa 1980

atagaagttc taaacatggt acgttctgct aagtaatatc atcgatctat ctattttgct 2040

ctaaattttc ataagcaaat ccagaacttc ctcgtcagtt tcaatttcaa gcatacgaag 2100

ggatagtgat taaattatat tttgaacctt ctattactga ttaagtgttc ctattagtct 2160

acggattaga cggttagaat gggattttca aaagcacaaa ggtcaagact tataggaaat 2220

tcatagaaaa aacactctga agtactcgat ggttggatat ataatagttt tgctaattta 2280

aactcttgct gttcggctaa gctattgtac ccaaatgcgg tactccgata gtcttataaa 2340

taatacttgg caaaagttca ataaatatat gtcaatggta ttgctttcca attaccattg 2400

acgaggttgt aaattaattc atacttaggt gacatcgatt aatttaacaa atatgtctgt 2460

ttcaacgctt acatcatcag tcttgcagga aaaatgttat tgccacgaca cctcaaatta 2520

gcccaacccc ttcgtctacc aaaacaatgt caaaaaccca cttaaaagaa gtcggacaaa 2580

cctgaacccg gtattttata aagtagtttt gtgaataata tcagtacatc gattacactt 2640

tccgtctcaa gactggaagt tgcaaagcca tgacaattgc tcaaccaaat gtgaattttt 2700

aggttccata gtcttgatcg ggtaatgtaa acactttaac ttttagtaaa tgataccacc 2760

aagaagaaag cactatttta agctttattt aacactatac attggaaaat aaaaaagtgg 2820

ctatgagaat taaacaagat gaccgagtaa ttaaaatagt gctgtcggtg ttaagcaata 2880

ccgctagggt tcaatcaatt aagtgctgct tttttttgtc gttgtatttc cattcctcca 2940

ctcctttctt tactcttgca atctaacata ttttttttaa aaagaaaaca tattgatact 3000

tacatgtggt aactattgtc tgattcatca attccgctct tcaatctcgg tgttcggata 3060

atttcgatga aattataatt acctgccgca attctagaaa ttcctttttt ttcttttctt 3120

tttctcggag ttggttccaa tacaaagatt gaattgaatt aggtgagaag aagaagagtc 3180

ttaacaccag atgtattaca gctttaaact ttgtttctaa tttgaccaca aaaagttgtc 3240

tggacgcctc agtttgaaat tagttttggg agatttctgt tttctcattg gccttactct 3300

atggaagttt ttatacaaga gcttccttct aaaattaact ctttgtgttg taatatagct 3360

aatgctaatt cttgattagt gtggaaagcc taataaggtt atattgtgca caggttaact 3420

accttaatat agttattgtt aatacagtta ttgctgttga ctactattgt tattgttaaa 3480

ttaaagtgtt aggttgagtt aattgaatag tgaaaaccaa ctaactaccg tattaaatta 3540

ttgtattaag attgattcct attaaggata aaacagagag tgtgttagaa agagaaaggg 3600

tggattataa atatgtgtaa aatccccttt agagactaac cactagaaat ctattgatgg 3660

tttcatatat agagattaac gattatattt ataatataag ttggtagttg ctagtatatt 3720

tgaaagcact acagtatagt atgtcagaat cagatcattt aaactctact aataatacag 3780

gaaacacttt cattagtcta gatcaagcca gtacaataat ggcagatcaa actcaaggag 3840

ctaacccaca acaacagcct agtcttcttg acactaaaaa aaaaagagat aaaaaacaat 3900

ttcagccaat cacatgtact acatttgtaa tagattttat tacttcagct gcttattaca 3960

caaacaaggt tgaattgata ttgtgtagag taaattttcg gaaatagttt gaattgggtg 4020

atcattttct ttattttttt ttatgtcttg tttctgtgaa gatcggaatg ccagggtgga 4080

gctcgtgaat tgcaccacta attgcagcag caccatattt caaataaagt ttctcatgtt 4140

gtaataggat tgcttgtctc catgaaacca atcacttaac taagccccag gctgattagt 4200

gtgttttcaa acagttttgt actagagaaa ctcagacctt ctcagggcaa gtaataacct 4260

aaaaaaatgc cacaaaacta aatgcaattt cagtttgata tgataggcaa tgacatcaac 4320

acctggaaaa aaaaaaaact ttcaggtgat gaaacgatta aggattaaag tttgcaacga 4380

aaaacaagtg gaactaaact ttgccttatt gttttgttcc gcttacctaa tgatgtttac 4440

tccttagaac aaacaacatc aactactttt aatcctgacg acgaagaaga agaccaaaaa 4500

gaataattag ccgcagctac ggtggtggca ctagtagtag tgctagtgct tgttgtgtct 4560

catccaagag aaatggaaaa actgcaaaaa tgccgcaact ttgaacattt tggaacacaa 4620

tacaactttt tttttccttt tggatttacg attagcgcga tagacgtgac cataaaaata 4680

ccacacgatg tgtagatcct ctaaaaataa tgtacacatt tccaggcttt tgtttactgc 4740

ttaataattt gtcatcatcg gtaacaatga tagtctcccc accctaacta cagtagacgg 4800

aattagacac caaagatctt ataaatcaac cccaaatttt cccattttga tttttgattt 4860

tttcgtattc cttgttgttt ccataatttt ttagttactc ctcctcaact aaactagata 4920

actcgtcaca gttaacaaca gaaaggtatg ttaaatattt atttcgttct aaattcaagt 4980

ttggtataga atattgcaaa caacaacaat ttgaaaaatg gactttaatt tgttctacaa 5040

aatgcaaaca catctagaat taatatttgc tctggaaacc gtatacggaa gttatggata 5100

atcacgttat cctgatatct attattaaca ccaccacaat atctattatt tcttgtatgg 5160

attgcggtgc caagatcaaa gaatcatttt aacccgatat cttacatttc acctcgatct 5220

aaatgtgatt cagtatcacc gcctcattgt ttcaccactc aacctcccca tactggcagt 5280

acatattttt tttttcattt tagagagttt taacataact tatcggcatt ttcaataatg 5340

tttatttgga aatttagtat ataccgataa atcctgaatt ctcgtattgg cgatggattt 5400

accaaaaaaa tggggaatga gtgtacacca agaaaaaaaa gaaaaattca agaaaaagcg 5460

agtgactaaa aatgtcgtgg gaatttaatt tatcctggaa agatgccccg attcagaagt 5520

aatgtcgagt actttcaccc acatacaatg aacgactttt atttattcct tcaccccaca 5580

cagcaacaac tacatttaaa tttcagtatt taagcgacca tgaatttaaa ttacaatact 5640

ccacagatta aagcattttg tttataactt ttctattctt atcaattttt tttggtatag 5700

ttgtggtttg cgtcacggtt gttttctttt tttcattttc cttagtttac tccacataca 5760

catacacgta catttctata tataccccat gattcccccc ccatttgatt tttgttgttg 5820

ttgttcagca atatctactt tatttattgg tttttatgtt tatatgatac taacttgtct 5880

ttgtttgctt tagtcatgaa ctccgatata ccacctccac caccacctcc agaatatacc 5940

cagtcccatg aagatttacc agcatacact tcgtcgttga actattatgg attatcattg 6000

attaaaacag aattcataac cccatatcaa tacaatagcg gtaaccgttc ctggaaacca 6060

gtattgcttg aattgaactc tactcaattg aaaatataca acttgaacat tgataagaaa 6120

ctacaagatt tgctaatatg tttatatttt gaattaaatt gtttagatca attaactaaa 6180

gacatcaatt ctcattataa aaagagtaaa ggttttgact ttagtgaatt atcgtctaat 6240

gatgccgacg atgtcggcga tttgttttcc ggtgatgcat atggtggtac tgatagctcc 6300

aagttatctt taaatgattc caagtttggc aaattgaaaa acaaattgag aaatcaaaaa 6360

tctaataaaa ccttgcaatc aataaaagct cattacgatg aattaaaaga taacaaattt 6420

ttctttgaac caacatcctc aacaaaggaa tataaccaat tcgctaaaaa gtatagagga 6480

aatttgttgc actgttattc tttggcaaac ttgcagattg gggaagcacc atctttgaac 6540

caaataattt cagcaatcta caaggaagag cataatggca acaccaacaa ttcatcactc 6600

gtcaaataca aaaacacatt gcgtcttcga attgaatata aacaaatctt acttcaattt 6660

tggtctttct acggtatgat cagttggttt aggaatttca ccattggaag agatttgagt 6720

gtacccgtcg aagcaagaca tgtatcgaaa ctcaaatcta taccctcaag aaacactagt 6780

caaaacaatg cattattggc cgctactgcc gcagctgcaa actatggaag aaacagagcc 6840

aatactccag tggacggtgt cgaagaagac atatccatgt ttcgctccaa ctatttgact 6900

attaaagatg aagataatac tcattctgac accagtagtg agaattcatc tgtgttcgac 6960

aatgagagaa gagggtccat agtttcaaca actacgtcaa tcgaaccagt cgactatgtt 7020

actattaaca attacaagtt ttattcccaa gagtacacct ttaccactgt tgagaaacaa 7080

tacatttcca attgcatacc agatttgaac tcttttgata aatggaatgg caagttaatc 7140

accgtcagta acgtggatca ttttattaga gataagagat cttttgaaga caaagatgac 7200

gttttcatta gttatgctgc attggggaac ttggtacaat catatgataa aaaatcacat 7260

aacgactcat ccatgcttac cacccaaact tttatcattc atcaaaaagg gttagttggt 7320

ttaggaacac aagtttgatt cttaaaacat atatagattg atagatacca tttaatattt 7380

ctaaacatat ctttacgaat taataaatac gacttttaat gatataaggt attttggttg 7440

taattgtaga tttggcaaaa aaaaaaaaaa taaacaacca tcgtagtagt tgttgttaca 7500

gtggttcaag ttcacgccct aaattcttgt ggctgtctcg cctttaactt tctttcttcc 7560

tcccttaact taacatgtac gtgtacttaa tattattttg aaaaattttt tttttctgtc 7620

tgtttctctc tctcctttgt tcccaacacc agttggtact tttaattcta ttttattttt 7680

acgttgatct gatatttatt tatatattta tatatttcca tcaattctaa aacttaatta 7740

cttcaaagac caagttcttg aatcttcttt tgtttttgct tgtttgtata ccaaaacact 7800

ctttttcaat tatttccctg ctgtttttct ttagaaaagc attgtccatt tgtctattag 7860

tctgtaactg gaaatttgtc ccgtccttaa attatttttt ttttgaagaa tcttttcatt 7920

tgaatcatt 7929

<210> 63

<211> 2292

<212> DNA

<213> Candida albicans

<400> 63
gatattaagt cgtctaatgc tattttttat ttgaaaaaaa aaaaacaaga aaacaaatgt 60

ataaaggtgg aaggaaaata aaaattaaaa aaaaaaaaaa ctcgaatatt aaaatgaaag 120

tggacaatta attgattgat taataaattg gttttattag tattatgtaa gggatttcaa 180

agaagtcatc taaaaattgt taatgtagat gtagatgtag atgtggttgt tgttctatgt 240

gtttacagaa attgatcatc aaagtccaag attttacatt gcctcgccag ttctattttt 300

ataaatattg gctgtgtgtt ttgggtgtgc ttgggccggg cagagggtgg gagagaggca 360

tgaatgcgga agaggaagga ggtcattcca ttccattcca tcgcctcatt cttctccatc 420

gttcattcat ttaattacga cagcagcaga agaaaaaaaa aaagaattca gatgtagatc 480

acgtgccaat attatgaaat attccatttt gggaaagtca gcttcaatgg cttacatggt 540

agcgcatact catagatttt aaaaaatctg aataatttgt tagttctcta tgaatgaata 600

aacagattac tgataagaac cagattaatt acttagaggt tttcttattt tttctttttt 660

gatagcaaaa gtattcatga attattcgta ttcgtaaaaa atttaagaag gagggagaac 720

aacaactgtt aacccaaatg gtgtttttgt taaaactcta tctactaaat tcaacatttg 780

tgaagataaa agtggttcaa attttttgta tgaaaaaaca acatagattt atatagcaac 840

atcactacag taatatatcg aatacaataa atatatatat ataataaatt aaaataaaaa 900

taaaaatata catctacaat atgaaaaaaa tcatttaact atatagtatg tctaaattat 960

cgaatgaaag ttagtaatac aaactcccat gtttagtggg gagcttggta gagccttcaa 1020

ggcaattcat agtaggttgg aggaggccct aatcagaggg tctgagttga acaaaagcgc 1080

ccaaagcttt gtttgattca ttggaatata ctctcggtta tgtcgaaagt attggagctg 1140

aaaatagaaa agaaaaaagt gaataattat gataattatt ggtgtgattt tgtcaccttt 1200

ttatacccaa ttttttttta tcaagagaga ttcttagatt tgccattttg agtgtttcaa 1260

atttcccatg tggattgaat tttcaaaatt ggttacatat atccttgaaa gtgttcataa 1320

tttttgtgtt gtaatatagc taatgctaat tcttgattag tgtggaaagc ctaataaggt 1380

tatattgtgc acaggttaac taccttaata tagttattgt taatacagtt attgctgttg 1440

actactattg ttattgttaa attaaagtgt taggttgagt taattgatta gtgaaaacca 1500

actaactacc gtattaaatt attgtattaa gattgattcc tattaaggat aaaacagaga 1560

gtgtgttaga aagagaaagg gtggattata aatatgtgta aaatcccctt tagagactaa 1620

tcactagaaa tctattgatg gtttcatata tagagtttaa cgattatatt tataatataa 1680

gttggtagtt gctagtatat ttgaaagcac tacagtatag tatgtcagaa tcagatcatt 1740

taaactctac taataataca ggaaacactt tcattagtct agatcaagcc agtacaataa 1800

tggcagatca aactcaagga gttaacccac aacattttgt agtcgtaaac ttgaaattca 1860

aagagaaggg ggggaattaa attgggtgca acgtgtttgt caaaaatttg gtgtgaaaaa 1920

aattaattta acactctgca ttgtaccata gggaatataa tacccagaaa taagagaaat 1980

tatcacgtga gactaaaact aaatataata aattaatatc acaattgaga aagacactga 2040

aactaacttc ttggtgtatt aattttcaac acttgatcac aagtgcgggg attaatcata 2100

attgcaaaga gtgtgttaga aagagcgaag gtggattatg aatattggag aatcctcttt 2160

agagactatc cgctaacaaa atagatgaac ttgctcaaca gaaacaacta atcgactaac 2220

tgactaaaat taatatacta agtatagatt aagttatcac gttaatattc tatactatcc 2280

atctccatca ct 2292

<210> 64

<211> 2025

<212> DNA

<213> Candida albicans

<400>  64
tggggagcaa atgtgaaatt aaagagtgtg gtgatatgta attttttttc aaaaaagatt      60

ggattgacga agcattatat attcgtctaa aaaccatttt tgctggttcc gcaataaatc     120

tcggagatta tttctcgatt accaatttat gttgttttgt gacatttctt atattttgtt     180

ctattttaca cgactattta ttgttaataa atatgtcacc taaagaatat ttctatttag     240

ttttacatat gttttttgac gacaatcaac tattacaaat taacctacat tttttaattt     300

gaatatatac aatttatatt gaattaacat taccatttag tttttgataa gaatagattg     360

cgctatttca aacatttgtt aaattattta ttgtgaaaca actatgtaga ataaaagtat     420

gaacaaattc tacgttcatc atgtggggtg tgccttcata tatatctttg gatgagaatg     480

ccaagaaaaa tgatggcgtg acaattcaat acggcaaaac aaactaatcc cctctaagat     540

tttactagtg tgtttcccta tcgtctgagg aaaaggtaac aaaacatcgt ttaaccaatt     600

ggtgtttgtt acgatggtga cgttgagtac tgcatatagt tgcaacggca aattgcatcc     660

agcgagttaa cagcgaatgg caaagtgaag cctccgactt gtgttcattg actactggga     720

ttggactggg aataacgact taactaatta atgttctcgt ggactcgttt agctagaact     780

aacatttgtt ataatatagc taatgctaat tcttgattag tgtggaaagc ctaataaggt     840

tatattgcgc acaggttaac taccttaata tagttattgt taatacagtt attgctgttg     900

actactattg ttattgttaa attaaagtgt taggttgagt taattgatta gtgaaaacca     960

actaactacc gtattaaatt attgtattaa gattgattcc tattaaggat aaaacagaga    1020

gtgtgttaga aagagaaagg gtggattata aatatgtgta aaatcccctt tagagactaa    1080

ccactagaaa tctattgatg gtttcatata tagagattaa cgattatatt tataatataa    1140

gttggtagtt gctagtatat ttgaaagcac tacagtatag tatgtcagaa tcagattatt    1200

taaactctac taataataca ggaaacactt tcattagtct agatcaagcc agtacaataa    1260

tggcagatca aactcaagga gctaacccac aacagcattg attatataat catctatgta    1320

gccaatatac actaccgtcc aaactcccac tacacacttg taacagtgtt ttacaaatct    1380

atgaacgaat aaccgattca aatgacacaa taaagaacat ttcaccgatt tgaattgcta    1440

atcggtacta taatattgat ggaaggttaa gagtttaatg ctaccctagg tttaccggag    1500

atcaacagtt gcatatacaa aacgtgttat ctgtctacga atggctttct atgtgtataa    1560

aatgtttcat caattgataa ttaattatta atctgcttac tgaggtaaac cccttttaat    1620

gcaatagcaa atatgaggta tttttttgct attgacatgc gtatatgaat ccatttgtat    1680

caaattgccg atataatgaa atggaaatta agggaaaaaa aaaagtttat atccaaattc    1740

atgcgattaa caggttcttg tgattataat tggtaacccc ctccccccta aaactcatat    1800

ctgccaaaag aggaggatat ttgaatatgc tattatgaac cccattgatt ttgactacaa 1860

ttggatttgt cgggtattga aacccaaaca tattataatt tgctatgcgt ttaaatcaac 1920

cgtttactgg tagatcctat actataaata cagccaacaa tccccaattg ttcagataaa 1980

gtaacactca atatcatttg atcaatcaat caagaggatt acaaa 2025

<210> 65

<211> 2731

<212> DNA

<213> Candida albicans

<400> 65
acatattttt ttttaaaaag aaaacatatt gatacttaca tgtggtacta ttgtctgatt 60

catcaattcc gctcttcaat ctcggtgttc ggataatttc gatgaaatta taattacctg 120

ccgcaattct agaaattcct ttttttcttt tctttttctc ggagttggtt acaatacaaa 180

gattgaattg aattaggtga gaagaagaag agtcttaaca ccagatgtat tacagcttta 240

aactttgttt ctaatttgac cacaaaaagt tgtctgcacg cctcagtttg aaattagttt 300

tgggagattt ctgttttctc attggcctta ctctatggaa gtttttatac aagagcttcc 360

ttctaaaatt aactctttgt gttgtaatat agctaatgct aattcttgat tagtgtggaa 420

agcctaataa ggttatattg tgcacaggtt aactacctta atatagttat tgttaataca 480

gttattgctg ttgactacta ttgttattgt taaattaaag tgttaggttg agttaattga 540

atagtgaaaa ccaactaact accgtattaa attattgtat taagattgat tcctattaag 600

gataaaacag agagtgtgtt agaaagagaa agggtggatt ataaatatgt gtaaaatccc 660

ctttagagac taaccactag aaatctattg atggtttcat atatagagat taacgattat 720

atttataata taagttggta gttgctagta tatttgaaag cactacagta tagtatgtca 780

gaatcagatc aattaaactc tactaataat acaggaaaca ctttcattag tctagatcaa 840

gccagtacaa taatggcaga tcaaactcaa ggaggtaacc cacaacaggt tatgagcctc 900

gcccgcttat tgaatttaga taatataggg gcaatgaaag cttttgaaag tgttgatttt 960

cctgaatcat taaaactaga atccaagatt aattttcaag tgtggagaaa tgaaatcctt 1020

agatatgcac gtggtattgg tgctgagttt gaaaactttg tattgaatga aactccagct 1080

cacctgtatg atcttagatt gggaaatatg cttcatcaat tattgattcg cactgtgaaa 1140

gaaaaagtta gaatgcctag gcaagaactt ggaaaatcag gaaaagaact ttatcttgat 1200

cttattaaat cattcggtac tcaataccca tacgataaat ttgagatagt taaatactat 1260

tgggatcagt taacaaaccc tttaattaat gtgaagagac gttttgaaat tgaagaagta 1320

tgggttcaat acattaatgc tcaaactgca acagagagag aagttcttaa ttcatttgtt 1380

tggttacatt tgtcaaaatc tatattacca caagagtacc ttagaagtgc ccatccagtt 1440

cttgataaaa atgtgattaa aatatttctt gatacccatc caaaatgtga tattgatcaa 1500

attatgtcat ttgtaaataa tgaactgatt aattatgtag ggaaaaatga tacaagggaa 1560

aatgatatgg gacagaattt aagagagagt gatttaagag agagtgactt aagtgaaaat 1620

gatatacaac aaaatgagtt aagcgaaagc gattcaagtg aaaatgattt aagagaaata 1680

gcaacaaaag aaactgttag tgaacttttt gaaaatcaat gtcagaattg ttttggactt 1740

ggtcatgatt catatgaatg ttcactggca tttagaaaca atcagtatat tccagattta 1800

ttttctagac ttcagagttt tcgtggaaat agaattcaaa ataataatag aaatgtctgg 1860

tctagattct cagaacaaga tgagtcaatt gcaaatacag aaaaaggtaa ctagatctaa 1920

tgataaaaat gaaaatcagt ggcagtcaaa acaatttaca tattaaacaa gtttgaatgt 1980

aagttgttgt tgtttagata aactatgtca tggtatccaa agttttattt tatatttatt 2040

atttaagtgg tcatgtttat ttacttataa ttgttattta gtttttcaag tgtgaatttt 2100

acttacttat aattgtattt agttttcaag tgtgaatttt acttacttat aattgtcatt 2160

tattgttcaa gtgttatttt tacttactta taattgttat ttagttttca agtgtgaatt 2220

ttacttactt ataattgtta tttagttttc aagtgttatc tttacttact tataattgtc 2280

atttattgtt caagtgttat tttttactta cttataattg ttatttatgt gtccaagttt 2340

taatattatt tacttataat tgttatttat tgtatatgtg ttaatttaat tcaattgtta 2400

attgttattt attgttcaag ttttaatttt atttacttat aattgttatt tattgtttat 2460

gtgttaattt aatttaattt aattgttatt tttactattt aaatgttgat tttatttatt 2520

taatgttaac ttgtcatttt taattttact tattatattt tacgtgtgac tattatctat 2580

gataaaacac taatagtgga tattgagtgt ttatttgttt catcgcagag gatatttatt 2640

ggaggaggga gaaaatgtct atttggtata aggaagacca taaaagttgg ttccaaatag 2700

tcaaccaacc aataaacatt ccctcatgct t 2731

<210> 66

<211> 2858

<212> DNA

<213> Candida albicans

<400> 66
   cctccgggcg tctatttaca agctgcttta ttatttgtta ttacctgggt gtaaaagccc 60
   tcttgcattt gagctatttc tattcccact tcggtatttt ttttacagcc tcgttagacg 120
   agttcttgat attactaaat tagttgttta ctgagtggcc tgatggttcc tcgtcactct 180
   agtttttggt ctatataagg gtcagaaatt tcccttctcc ttaggtccat caagtcaaga 240
   tatacattag ttggtagcat cgtatggaat tttcgtatga acggcatacc aagtattaat 300
   ttccgatcga aattttttag gacgtcttga taatcaggac aaacatcatg aaaggtctat 360
   acgacgaaag tttactttac acaaggggag accatatgtc ttctttatta acaactagtt 420
   atatagcgaa caaataagtt tatacagaaa tatatgtaca caaacaaagt tattgtttat 480
   taattattta attagctcgg aagaataact ctgtgatact gcatacattc aaacaaaatc 540
   aatctagttt ccaacatctt tttcacttgg taatgtaatt attcttgttc tggcaccgac 600
   aatgggtatt gttttgtagc tggaggacta atatggggta ccacctcaat ttttggatcc 660
   cagctcccac gcaggggtgg cttctgatct aactcacttt cgaaaatatc ctgatagttt 720
   ccaattaatt cagcaaaata gctcttgttt gtacccttaa ccaatgacat gatatccttt 780
   ttattatcac cgataccacc tgtgtcttcg tcttgttgta atatagctaa tgctaattct 840
   tgattagtgt ggaaagccta ataaggttat attgtgcaca ggttaactac cttaatatag 900
   ttattgttaa tacagttatt gctgttgact actattgtta ttgttaaatt aaagtgttag 960
   gttgagttaa ttgattagtg aaaaccaact aactaccgta ttaaattatt gtattaagat 1020
   tgattcctat taaggataaa acagagagtg tgttagaaag agaaagggtg gattataaat 1080
   acgtgtaaaa tcccctttag agactaacca ctagaaatct attgatggtt tcatagatag 1140
   agattaacga ttatatttat aatataagtt ggtagttgct agtatatttg aaagcactac 1200
   agtatagtat gtcagaatca gatcatttaa attctactaa taatacagga aacactttca 1260
   ttagtctaga tcaagccagt acaataatgg cagatcaaac tcaaggagct aacccacaac 1320
   acgtcttctt cagtattagg gaacaacata ctaacttgac cttttctagc ttcaaccaaa 1380
   aattcctcta tatccattaa tggaatttca tcaaactgag cagccccaaa aaacgttttg 1440
   cttccaaagt ctaaatgagc atggaatttc cttatgaaag gtataccaag tattaatttc 1500
   ttatggaagc tgtccactac agcaaaattc tcttggaatg taataccatt aaactggaac 1560
   ttgaggttaa ttatttggtt aaagtttctg ttgatttttg gtccaataaa gtacccaaac 1620
   tactagagct ccaacaacat tttcagaaaa tggccaataa tacaataagt gggtatattt 1680
   tatcaaaaga gtttatatta tggttactcg acggtattat tctctgttga tttaaggcat 1740
   tctggtcgac cagtggacaa aattcaagag tagtgtttgt ttagacttta caggacatga 1800
   tagtatatat aacaaaaatg aaatacatta atcaaaacta actaaatcct aaattaatgc 1860
   caatttctat tgaattggtt tgctactttg taaaatttgt gagtaatctt aagtacttat 1920
   atggaaatca acaatggcaa aaatacaaga gaatgacccc atgacacatt cagtgcacaa 1980
   ttcatagtaa ctgcttggtc acttgcacat gactctgcta gtatactcaa ccactcttgt 2040
   gacttccata tagatactct cgatgaaatg tctcaaatta gaggacaaac aatctgctat 2100
   aatcttggct aatcacccat gtaacatgga ggaaccaaac acatagatat acggtaccat 2160
   ttcatacaga atttatcact aaagaaatta agaaaaactt gtgttatcaa agtggtttgc 2220
   gaactttgta gtaagggaga gtgttgagaa ttagagattc taagttccag aaaaatatct 2280
   atatttatat atatataggt agtgcaacac tacataaaag ggactgattt gaatgtatgt 2340
   atgtcaaatg acacccttat aatgttgagt gacatcatat caaaatggaa atctactgta 2400
   tcaattaaga gattactaaa agcaatatac ttaatatgag gtcgtacttt aagattgtga 2460
   atagtatcag tagcgagtgg ctatgtgttg tgatggagca tcactggtag tttcttagat 2520
   gtaaatctca gtgactataa gcatactaaa ttagttatga agatatgttc cattaaagta 2580
   tttaaaaaat aatagacagg ctatcaattt ctaatagatt taccgtccag attataaaaa 2640
   aattatcgag atacatatta caccgattga attaataata tgtctactac aaacccatca 2700
   cggaacttga tgcaattgat tgaataagtg tctctctaac gatgacatgt ccaattctaa 2760
   tcaaaataat tattattcta attgtaatat ctggtattta attatttata attcacgaaa 2820
   cagtttgatt ggtttctgat tcttctgaca aaaataag 2858

<210> 67

<211> 1636

<212> DNA

<213> Candida albicans

<400> 67
atgtttattt aataattaaa ccccagttga ccaactatga aatagtataa tgataaatgc 60

aaaataaata tagtatgaac aatatgatag ttttagtgtg aattttgaat aagaaaaaga 120

agggataagg atatttttac taggaaactc aattataatt actaatgata aaaactccat 180

cagctactat tattactcaa attttaaatc atttgtttat cacctacaca aacagggatt 240

gtccaatatt gattactaaa attagaacaa ataagagaat ataattgaag ttaaataatt 300

cttttactaa atctattgac caagaactac atcaaggaaa agtgttgcat atacatctaa 360

tgtttattct tggttagagt attgatacaa aattatatca tcaccaacga atcacattaa    420

gggaaagtgt tgtgcatata cctgatgctt agtcttggtt aaagtatttg tgtgaaaggt    480

tatcgtgacc aaagattata gtaagggaaa gtattatgaa taaatccaat gtctactttt    540

acagaagtat tgacatgaga gattataact atcaagaatt gcattaaggg aaagtgttgt    600

aatatagcta atgctaattc ttgattagtg tggaaagcct aataaggtta tattgtgcac    660

aggttaacta ccttaatata gttattgtta atacagttat tgctgttgac tactattgtt    720

attgttaaat taaagtgtta ggttgagtta attgattagt gaaaaccaac taactaccgt    780

attaaattat tgtattaaga ttgattccta ttaaggataa aacagagagt gtgttagaaa    840

gagaaagggt ggattataaa tatgtgtaaa atccccttta gagactaacc actagaaatc    900

tattgatggt ttcatatata gagattaacg attatattta taatataagt tggtagttgc    960

tagtatattt gaaagcacta cagtatagta tgtcagaatc agatcaatta aactctacta   1020

ataatacagg aaacactttc attagtctag atcaagccag tacaataatg gcagatcaaa   1080

ctcaaggagg taacccacta caggttatga gcctcgcccg cttattgaat ttagataata   1140

taggggcaat gaaagctttt gaaagtgttg attttcctga atcattaaaa ctagaatcca   1200

agattaattt tcaagtgtgg agaaatgaaa tccttagata tgcacgtggt attggtgctg   1260

agtttgaaaa ctttgtattg aatgaaactc cagctcacct gtatgatctt agattgggaa   1320

atatgcttca tcaattattg attcgcactg tgaaagaaaa agttagaatg cctaggcaag   1380

aacttggaaa atcaggaaaa gaactttatc ttgatcttat taaatcattc ggtactcaat   1440

acccatacga taaatttgag atagttaaat actattggga tcagttaaca aaccctttaa   1500

ttaatgtgaa gagacgtttt gaaattgaag aagtatgggt tcaatacatt aatgctcaaa   1560

ctgcaacaga gagagaagtt cttaattcat ttgtttggtt acatttgtca aaatctatat   1620

taccacaaga gtacct                                                   1636

<210>  68

<211>  2125

<212>  DNA

<213>  Candida albicans

<400>  68
tgagtagcct tttcttgggc gactttatta gcttcatcaa caagacgttt atcttcagct     60

tccttttcca taataattct cttccattct ggaattggtt ttggtttctt tttatttatc    120

tcctcttctt tcatagccaa caaaagagta cccaataata atataatggt gataccttgt 180

gcgtacattc ttgcttgaac agctttttgt gcggtatcca taattttgtc tctgttaacc 240

aatacccaag aaccatataa ggaaccagcc caagcactta tgataatttt atatttattg 300

tcattcaata cggtgaaaca tttgtcacta agcgataatc tgttccattc acggtattct 360

tccaaatatt tagcttcctg atactccgat tgatgcatct ttctatcgaa ttcaacagaa 420

ccttgatcag cgaaaaaggc agccacagaa attgttggca tagcaattat ggctgctttg 480

atacttggat tgaatgttgc aaatcttgct ggatgtctat gctttaaata ttggtacaaa 540

ccgactgaaa gtgcaccacc ataaaacaac cctttggcac cttctgaaat aatatgtgaa 600

atgtgagcgt ctttttcttc tttggataag atcttcattg tggaattaag atgactttgt 660

gattaaattg ttgacttctt taagcctttt aatgtggagg aaaaagaaaa atctataatt 720

aaaaaaaaaa aagataaagc agataattct ttgatcttta tatacttggt ctatatgtag 780

taggggaaag tcggagtcgg aatttgaaaa aaaaagagaa aaaagaacga atatttagac 840

tgtaaaattc aaacccctgc tgattagtat ataaaaaaaa tgagttcatt tttcctttct 900

tttttttttt ttcgcgcgga tagcaacggt cattaagtta acgagataaa aaagaaacaa 960

ccagataatt atgaaaagtt gtgatggtgt cacgtgcgaa catgagagtc atgaattttg 1020

acgaaaacgt caagcttcag tttacaaaag acctctttat taaaatcgaa ttgcttatag 1080

ggtcgtcgat gatgagaagg tgtatgttgt aatatagcta atgctaattc ttgattagtg 1140

tggaaagcct aataaggtta tattgtgcac aggttaacta ccttaatata gttattgtta 1200

atacagttat tgctgttgac tactattgtt attgttaaat taaagtgtta ggttgagtta 1260

attgattagt gaaaaccaac taactaccgt attaaattat tgtattaaga ttgattccta 1320

ttaaggataa aacagagagt gtgttagaaa gagaaagggt ggattataaa tatgtgtaaa 1380

atccccttta gagactaacc actagaaatc tattgatggt ttcatatata gagattaaag 1440

attatattca taatataagt tggtagttgc tagtatattt gaaagcacta cagtatagta 1500

tgtcagaatc agatcaatta aactctacta ataatacagg aaacactttc attagtctag 1560

atcaagccag tacaataata gcagatcaaa ctcaaggagg taacccacaa catagaatac 1620

gttttcaact acttaagtat ccactaacct aaattttttt tttaataaaa tttcattgta 1680

ttagtctttc ttactgcttt taatcaacta taagtatagg tttccgtttt ttttgcagta 1740

aaatttatcg ttcaggagaa ataacaaaat gtacacgact tattcgcagc attttttttt 1800

ttgttttggg tttttgtatc aaattgttac aacaacaaca acaacctcaa ttcttaacca 1860

aatctacccc tcctattttt ttttctcata cacacaatac atcttacact atcttttgat 1920

aggctttatt gaagaagtat ttaaggagtg taatgacaat ctgcttaact catatatata 1980

tatatagata gtagtcaaca atagctttat ctactttttt tttttggcga cccctgcaac 2040

ttcaggccca ccagtttgcc cattttggtg cccccattga gtaaacatgg ggatttggag 2100

cacacttttt tttaggtaaa aatgg 2125

<210> 69

<211> 1292

<212> DNA

<213> Candida albicans

<400> 69
ctaatccaaa aatccataac ccaactgctc aacggcgaaa tccaaaactt ccatgctatt 60

ctagaccaaa cagtgtcgaa actcaatgat gcagagtggt gtctcggcgt tatggttgaa 120

aagaaaaaga aacttgacga attgaaagtc aaagaagaag cggcaagaaa gaaggaagaa 180

ggggcaaaga aaaaggaaga agaggcaaag aaaaaggcag aggaagcgaa gaagtgtttt 240

attttacttt tctgtcaaat ttgcactact tttaatttgt gtgcaaatat tctattttac 300

ttgattttta tatactttta ttttacaata cttttttata ggacttttta tatcttttct 360

ttatcaactg ttcgctatag ggtaggtctt ccaagctaat tttacccgac acaagatgaa 420

atattttctg ttgagcactc gttgtcgaca gtgaaaaatt ttcactcaag aaaatatttt 480

atcatcactt tttctagaag ggaggttcaa gtgttggaga atagacagcg aacacctgat 540

attcccaagg tcgaattaga ttgaaagata aataatagtc atatttattt tgtatttagt 600

caataaatta tctttttata tttaaattct tagtattgtc ataccacgta gattgatacg 660

gacatactta gcacatttaa catatattaa gcaccgatta cctgtgacat tccggagttt 720

actgtttcgc gcacgctggc agacgaacat caactcatct tttatacaat atattcttac 780

gattataact ttcaattaag aaatacaact tcttattagc attctcctac aagttcttaa 840

gttcctagga atttcttcga aactataatt aaagacgaaa agtgtaaaac aaacagaaag 900

cagaggaggc ccagaagaag gcagaggagg ccgtcccaca aaagtttgac aactttgacg 960

actttattgg ctttgacatc aacgacatgc agaacgacga taccatcgac gataccatcg 1020

acgataccat cgacgaaacc atcgatgaaa ccatcgacga taccaacgac gaagacatgt 1080

tgtccaacat ggactacgaa aatctagatc cggacgagac catcgacgaa gtacctgcca 1140

ccacagacag cgacttggac atgaacaaca tacttgaaaa caacgagctg atattagacg 1200

ggttgaacat gacattcctc gacaatggca acaacaccaa ccacgtaaac gaagagtttg 1260

atgtagacgg ctttttaaac cagtttggta at                                    1292

<210>  70

<211>  568

<212>  DNA

<213>  Candida albicans

<400>  70
gattgtatag tggtgtggtt gatcgacttc aatataacaa gagagagatg agatgagatg      60

cttttatcgc gtatatattt ttttttccat tgacaattct gatttcacaa attgttcgct     120

atagggtagg tcttccaagc taattttacc cgacacaaga tgaaatattt tctgttgagc     180

actcgttgtc gacagtgaaa aattttcact caagaaaata ttttatcatc actttttcta     240

gaatggaggt tcaagtgttg gagaatagac agcgaacacc tgatattccc aaggtcgaat     300

tagattgaaa gataaataat agtcatattt attttgtatt tagtcaataa attatctttt     360

tatatttaaa ttcttagtat tgtcatacca cgtagattga tacggacata cttagcacat     420

ttaacatata ttaagcaccg attacctgtg acattccgga gtttactgtt tcgcgcacgc     480

tggcagacga acagattaga agcttggtaa atctttggtt attcatcacg tcttgagaat     540

aatacaaagt ttaatatagt attttcaa                                        568

<210>  71

<211>  946

<212>  DNA

<213>  Candida albicans

<400>  71
gattgtatag tggtgtggtt gatcgacttc aatataacaa gagagagatg agatgagatg      60

cttttatcgc gtatatattt ttttttccat tgacaattct gatttcacaa attgttcgct     120

atagggtagg tcttccaagc taattttacc cgacacaaga tgaaatattt tctgttgagc     180

actcgttgtc gacagtgaaa aattttcact caagaaaata ttttatcatc actttttcta     240

gaatggaggt tcaagtgttg gagaatagac agcgaacacc tgatattccc aaggtcgaat     300

tagattgaaa gataaataat agtcatattt attttgtatt tagtcaataa attatctttt     360

tatatttaaa ttcttagtat tgtcatacca cgtagattga tacggacata cttagcacat     420

ttaacatata ttaagcaccg attacctgtg acattccgga gtttactgtt tcgcgcacgc     480

tggcagacga acatcaactc atcttttata caatatattc ttacgattat aactttcaat 540

taagaaatac aacttcttat tagcattctc ctacaagttc ttaagttcct aggaaattct 600

tcgaaactat aattaaagac gaaaagtgta aaacaaacag aaagcagagg aggccaagaa 660

gaaagcagag gaggccgccc cacaaaagtt tgacaacttt gacgacttta ttggctttga 720

catcaacgac aataccaacg acgaagacat gttgtccaac atggactacg aggacctaaa 780

attggacgac aaagtacatg ccaccacaga caacaacttg gacatgaaca acatacttga 840

aaacgacgag ctgatactag acgggttgaa catgacattg ctcgacaatg gcgaccacgc 900

aaacgaagag tttgatgtag acagcttttt aaaccagttt ggcaat 946

<210> 72

<211> 951

<212> DNA

<213> Candida albicans

<400> 72
gatttgagaa ataccattga agatctagag ttaaaaataa ggaatttgca tgtacatgag 60

gataatcaag cggtcattac aatcttaaag aatgataatt tccacccaca tagaccgatt 120

gatatatgtt acaaatttct cagacaaaaa ttgaaagatg gatttttttc aatatcatat 180

gttgaatctg gagataattt agctgactca ttcacgaaag ctttaggaag aaataaattg 240

attgaacata ccaaaaggat tagagaaaga aaggattatg ataataatgc tacactgata 300

gtggacgtta ggacgctcga agagattaag ataaacaaga aattggtaca tcattaatta 360

atttagctgt ttacctgaat caggggagtg ttcgctatag ggtaggtctt ccaagctaat 420

tttacccgac acaagatgaa atattttctg ttgagcactc gttgtcgaca gtgaaaaatt 480

ttcactcaag aaaatatttt atcatcactt tttctagaat ggaggttcaa gtgttggaga 540

atagacagcg aacacctgat attcccaagg tcgaattaga ttgaaagata aataatagtc 600

atatttattt tgtatttagt caataaatta tctttttata tttaaattct tagtattgtc 660

ataccacgta gattgatacg gacatactta gcacatttaa catatattaa gcaccgatta 720

cctgtgacat tccggagttt actgtttcgc gcacgctggc agacgaacac aaatgcttga 780

actatctgcc gacttttttt tatttatggc gtgagacatt gttctcgcac acggttgtga 840

tttatctacc aggctctcat atttagagcg acaactactt tgagcaagca aaacgcatat 900

ctcaccacac accaattgta ggctattctc aaccggaaag tacaactagc a 951

<210>  73

<211>  107

<212>  PRT

<213>  Candida albicans

<400>  73

Asp Leu Arg Asn Thr Ile Glu Asp Leu Glu Leu Lys Ile Arg Asn Leu
1               5                   10                  15

His Val His Glu Asp Asn Gln Ala Val Ile Thr Ile Leu Lys Asn Asp
            20                  25                  30

Asn Phe His Pro His Arg Pro Ile Asp Ile Cys Tyr Lys Phe Leu Arg
        35                  40                  45

Gln Lys Leu Lys Asp Gly Phe Phe Ser Ile Ser Tyr Val Glu Ser Gly
    50                  55                  60

Asp Asn Leu Ala Asp Ser Phe Thr Lys Ala Leu Gly Arg Asn Lys Leu
65                  70                  75                  80

Ile Glu His Thr Lys Arg Ile Arg Glu Arg Lys Asp Tyr Asp Asn Asn
                85                  90                  95

Ala Thr Ser Ile Val Asp Val Arg Thr Leu Glu
            100                 105

<210>  74

<211>  9850

<212>  DNA

<213>  Candida albicans

<220>

<221>  misc_feature

<223>  "n" is any one of a, g, c or t

<400>  74
ctaatccaaa aatccataac ccaactgctc aacggcgaaa tccaaaactt ccatgctatt      60

ctagaccaaa cagtgtcgaa actcaatgat gcagagtggt gtctcggcgt tatggttgaa     120

aagaaaaaga aacttgacga attgaaagtc aaagaagaag cggcaagaaa gaaggaagaa     180

ggggcaaaga aaaaggaaga agaggcaaag aaaaaggcag aggaagcgaa gaagtgtttt     240

attttacttt tctgtcaaat ttgcactact tttaatttgt gtgcaaatat tctattttac 300
ttgattttta tatactttta ttttacaata cttttttata ggacttttta tatcttttct 360
ttatcaactg ttcgctatag ggtaggtctt ccaagctaat tttacccgac acaagatgaa 420
atattttctg ttgagcactc gttgtcgaca gtgaaaaatt ttcactcaag aaaatatttt 480
atcatcactt tttctagaag ggaggttcaa gtgttggaga atagacagcg aacacctgat 540
attcccaagg tcgaattaga ttgaaagata aataatagtc atatttattt tgtatttagt 600
caataaatta tctttttata tttaaattct tagtattgtc ataccacgta gattgatacg 660
gacatactta gcacatttaa catatattaa gccccgatta cctgtgacat tccggagttt 720
cttgtttcgc gcacgctggc agacgaacag attagaagct tggtaaatct ttggttattc 780
atcacgtctt gagaataata caaagtttaa tatagtattt tcaaattttg gaatacaaaa 840
gttgctaatt ggtaaataag ttattgattt atttcataaa tcttttttgg tatcatattt 900
caaagagttg caattgaaag ctaaagacat ccttataaat ggctgaattt agcgatgctg 960
agctcagaaa gatgatgggt acactttcac tcttggtaca agattccagg agagaaatta 1020
accacttgca tgataagttg gagaacaata gtgactcaaa atatcaatct ttagaaacgt 1080
acatcaactc aaagtatgca gatactataa aatcatttga aaaattaaaa tatttggaca 1140
ttgataattc agagttggtt aatacctgga tcatgtgttt taatcaggtt aaaaggtttc 1200
accctcaggt ttttgatgct ttcatggagg cagagaacga ggacgaaatt ggaatcgaaa 1260
agatccaata tacgccatac acaggtaaac acttgaatga tatgatcaga atcttctaca 1320
tgaagatatc cgaattaata gaaagaaaag ttagtccaaa tgtttctaga gagatgaatg 1380
atggacagcc acaatttgtt ccgaatttgt ttaaaaaagt ttacgagatg attatttcaa 1440
aaccagatgt ttctgctgct gaaagaattg gaaaagctct tttcaagtta caatctaaac 1500
tgagagaact tgaaagagaa tcagcatttt tgttatgtca acatttaatg accaatgacc 1560
accagcacga tgatattatt cttaaatttc tcgttagcgg tgtctcacca tggtacttac 1620
atctgcaaat ttacatgctg tcatataaac ttggattctc aaatttgttt ttagagattt 1680
atgctcaaca ttatgaattg tataaagcag atcccattta caaattgcca gatagtatga 1740
cattgttgaa tgaaataaga tcaaatagag attatcctaa agtggtaaat gctgcaaaaa 1800
atacagtaca agtcaataat gtttcatcca agaacaataa aaagaaggat gaatgacaac 1860
aattagccaa taaaattgag gaagtaggac gttatagcga aataaacgca acatctacat 1920
atcatgaaat tggcgatacc aacaaaaaca aagaacaatt aatattgaat ttgaaaaatc 1980
atacaaaatt aagtgaacaa aagaagaaaa caaacctatt ggtatatgat ctgggagcca 2040
cagtatccgt ggtgaatgat aagactttac ttaacgacat taaagaatca aatatcgaaa 2100

ttgcaactgc tgaaggggag acatctacgg cttatgcttt aggtactcta accatatctg 2160

tgaatggatt gaatgcgaaa ttagatggtg ttctatactt gccatctatt caattaaact 2220

taatatctat aaaacaattt gaagatttat gctacgcaat tttgatttcc gaaaatctaa 2280

tgtgtctagt tcacagtgac cacggaccta cggtcattgc gaaatattca cctaaagatg 2340

acttatactc aggcccaaga tcgggaacct ttttttaaaa gaattcataa tgaccaaacc 2400

cattttttgc ttgccnctgc taaaaaactt ttagaatcag agaccatatt tctggagaat 2460

ccctgaaaaa tccaatggat tgatcaagaa aaattagatc cgttgaaaat gaccaataaa 2520

gtagaaagag ttacctatgt cagcatacgc aacatcaaac aagaagtggc agacaaatat 2580

atgataaaag atctttacta ctatcattta ttaattaatc acctttcaca tgaaaaacta 2640

caattattag taaaaagggg agtgattaaa ccagtcaaat ctacttcggc tgagtcggcc 2700

attttaaatt gtcagatatg tgttgcagcc catgcaaaat tagctagcca taatcacact 2760

caacaacggg aattggagcg accattacaa cgcctccatt tggataccgc cggaccattt 2820

acctcaaata aaactaagag ctatcttaca accgtgattg atcaattttc cagatatact 2880

gaagttattg tatctgacac caaagcagtc aaacaaagca tattgcatag acttagggtc 2940

tggaacaata gatttcagtt taagatcgcg gagataagat atgataatgc attggagtat 3000

ccatcggctg aggagttaga ggagttagga atttataaac accttctccc aaactactct 3060

cctatgctta acggtacagc tgaagcaacc aaccgcccca ttgtccaagg tatttataag 3120

gtagtgttaa attttagttg tcaagtatta atacttttcc catttatagt ggagtatgcg 3180

gttcatatcc ggaatcatac acctataaaa gaatttgatg gtgctactcc ttatgaacgt 3240

tactatggtt tatctaaata cgtcatacca ttttttcagt ttggaaccga cgttttgata 3300

aaatgtgcta gtgtacaaga agctatttca ttaaaactac catcttcaag agataaagct 3360

tttcctacag tgatgtttgg tgcttttctc ggttacggct cagattcctt taccttcaga 3420

gttttagttt ccacgaaagg atatccagtt attacaacat caaacatccg tccaatagcg 3480

acgatgcaag tactcaatga ctatttggca tacatatcgg agaatagctc aataagctat 3540

gacgatacat tcttatcacc tttgaatcac ccaatgattc gcacaaacca acatgataga 3600

cgtggagaca atataaatgt cgaatatgaa aaccgtccaa atgtaccatt tgaatatcat 3660

gctgaacctc ctcgtacaaa ttcatcgacg ggaattatcg atcgaccaga tattagacct 3720

agagctgatc ccacctggca acgtatgcct gatgccaaca tacatcagga aacaacaact 3780

gtacagactc ctgatcatgg ggagttagat accatgatca acaacgaaca ccaactacca 3840

cgatctgggg agggtaatta ccccgggcaa caggtgcgca ccgatattat tgggcaattt 3900

cgagatcgcg ggcctaccac tctaaacact ccgatcgatc taggtgtacc cgatgaaaca 3960

gacgatatta gtatgacatc agagaatcca attgattccc caaattccga gatgatcata 4020

tccccatctt tacccacaaa tgaattggaa catcaaatcg atatcagttc aggggagatg 4080

tcgttattgc aaacgaatat ggaagcagat aacgaattga aaacaaatga aatggtatta 4140

tacaaatcaa aaaatgatgg tattatcatt caacaacaac aattcactga aaatttgtca 4200

gatgaaaatg aagaagattc atcaacagat gaggaaacat tggaagacaa aaaacaacag 4260

cgattggaat ataatatttc accaaacgat gagtggataa ataatgacgt tcagaacgaa 4320

gatgacacac aagtgccaca tgttaaggaa ccaatcaatt atgaaactca aagtagaaat 4380

gaaacaaaca tgccacgaat tgaaatgggc ataatagaaa acttaagtga tgatggaaag 4440

aatacaccac gtgaattacg tatcgtcacc tacgataata ataaagaaat tgaaaagtac 4500

caagacagta atatcgagat cctggaaccc agaaacgaaa atgaaaacca gacattcatt 4560

gaaagcaact tagaattact tgacaatcaa gaaatgtttc aagaagatcc tcaagttgaa 4620

gatattcgat tgacaactcc aaaaaaggac aaatcgttat cacctgattt caatcaaacc 4680

cataatgaaa tacaactatt catggcagat atcaatgaag atatgctaga agaatatgat 4740

gaaaatataa atatgaatga agtgttagct gactccacgg agacgttgga caaagaatta 4800

gatttagatg aagaaagtgg aaggatcgaa tatattgctg atagagttag aaaaaagaca 4860

gaggtactga tggtgcgcca cacgggaaat atttaaagaa aaatgataaa gattttggtt 4920

caataaaaag tcagaaaaaa tctgacgcac aaatggatga tgaagttgga attgctattt 4980

cgaagatcag aaactttcca tttagattga aggatggacg agcaagtttc ttccctccat 5040

ataaaacaaa atttggaaga tcagtgcatc cacctaaaag atatttaaat gccattgtta 5100

agaaaataga ttacaatcaa aaagaatggc gtcaaagtat ggaagaagaa atcgaaaaat 5160

ttaaggctaa ccaagtttac accgttgaaa aaacaccaaa gaacgttgtc ccattgaaaa 5220

ccatgtgggt acatacttac aaaaccaatg acctcaaaaa tcataattac aaaagccgtt 5280

gcgtggtaat gggaaactat atggtcgaaa atcgtgattt tgatccccat gccatctcct 5340

ccccggtagt agatctcaca agtatacgac tattatctgc catagctgtt gaaaataact 5400

tggttatgca ccaattggac atcgcctcag cttatttgaa cgccagtttg gaggatggaa 5460

gagtaatctt tgtgagacca ccgcgtggtt ttgaggttaa acctggctat agttggcgtt 5520

tacacaagtc tgtgtacggt cttaggcaga gtgcccataa ttggtactca cattttaaga 5580

atgtgttgga ggcaaatggt ttaaaacaaa cactacacaa tgatggcatt ttttggaaaa 5640

attatgaaaa tggagatgta ttatatgtga gtgtatatgt ggatgatgtt tttatcaaag 5700

cgaattcaat gagtttgtgc aactaaattt agagttgctt ttagtttact aaacaaattt 5760

tatccttgct aatcaatact atctattatg cacgatctag caaccttaaa acaaccaatg 5820

gaaaaattaa aaaaattccc tcatcaatct ggcatgttcg aattgaaaaa aaaaaaagaa 5880

aacaatagaa attcaataca atagagcata gaactggcca gaatgtgaga caataagtca 5940

gaacaagtga ttgccagtat aggtagggag aagcaacaaa gagagtttac acagctgaaa 6000

acaatcatat cgacggttat tgcaacttgg ttgctatttc aactattcgt aatggtccca 6060

tttttagcca acacaatttc agagaagacg cgaaaaagga cttggaaact tcatagttta 6120

gagccacaaa ctataagaaa taatagtacg atctaaattg gttccctagg ataatgccca 6180

acaaagaaat cccccaaata attgtaaatt gttcaacctt agtaactcta tctagcattg 6240

cggagttcct tgaaaatgaa ttggtttggt gttcctacct gttcagtact taatcactaa 6300

ctagacaaat tctttggcga aagctcaact tttgtgaagg tctttctcta ctatgaacat 6360

gactcccagc aagtctaggt ttggctgcac tatgagttta atttagtttt atcgggctaa 6420

tactacttat ttccgttatc ggtgtgaccc ccgaagaaag ggtattacgg ggctcataat 6480

tttttttttt ttggcaagta gagtgagatt caaaaaagaa aagtgaacca gagcaataat 6540

tgctattaat tttagttttt tactcactag ctatacttgg ctcccaaact gattttgtaa 6600

ccctttgagc aaggttgttg gtcaactgca agatcaacta agcaagatca cgccttatac 6660

gcaagccctg ccaaaaaata attcactctt gaaacaagga attagcagct attaggtaga 6720

cttttttttg tacctgtatt tcgttaccaa cactaaccga ggcactaccc aaactcatat 6780

aaacatgact aagagaaaac aatagagaag gggtttagtt gattttccaa tacattttag 6840

tgctgaatta catttatcta tttagtttag ttccataatc tttctaatat tgttgaacca 6900

ttagcaaact ttttagatta aaagctcttt tgtaactgtt ttttttctgt agttatcgcg 6960

taacctttcc ccctcagaat ttctaaaccc tcccccccct ttcttcaaaa cattaaagac 7020

tttgaacttt atcatcacca caaaaactta ttaagctcca gcaaatttca ggtgacacca 7080

aggaaaacaa caattaacat tcttggagtt aagagtatat gctggtgcat ggattaaata 7140

tgcctgttct taaccccagc gaaaagaata tgttattttt gaacaaaaaa atagaatatc 7200

tcaaataaat ttgttctccc cttttgtcta tctatccctt tagctttttg ccaaattcca 7260

acacaaaatg ctttagtctg cagaaatgat gactaaaata ttccttttct tcaaaattca 7320

tattttcaaa atttagcaaa tggttgtact agatatcaga attttatctg gtgagtttac 7380

tcaaccatag tagtcttttt ttagatcaaa aattagactt atgaacccta tattgaataa 7440

agttagtgtt ccccacagct attcataata aaaaagctta acaaaaagtt gagattatca 7500

gcgacgatcg atcatgtcgt tccagagatt gtgttatagc gcctccttat gaacaggtaa 7560

actattagtt gcatgtagat ctattgtgtt caaatttaaa ttttaagaat tgttagctca 7620

aaacaaagac gacctgaaat tccaaaaatc ataaagttta cccccaaaaa agtaacgaca 7680

ataaaggtgc accaagaaat aatggttgta gtttttcctt tatctgtttt agattgcttt 7740

attagggggt atcactaatt agcaattgta gcccttgctc gttattgttg cttgattttt 7800

tctaaaaaca tttgcttagc attattgttg taagacatat ttatctattg tttctcaccc 7860

ttttagacaa atgattagcg ccccttgaca cgatcacagc ctattgtttg gtgcactatt 7920

tgagctttaa agtactaact tgttttcaga ctatcaatct atgtgtttgt tcaaagccag 7980

gcactcgagt cattagtcaa caataggctg tatgttgcta tccatgtagt gccttgtcta 8040

cagaaatttg cttttttaat tcacaagcat gagatttttt gtttgtgtgg tatttgacgt 8100

aaatgtaaca tgattacttg aaattcgata cgatcttttt cgtcgtctat acaaaattta 8160

tcaagtgcta ctctgtgata ttttgcaaaa ccaatctcat tgttccttgc atgagaatga 8220

tttcgttgtc atcaaagaaa tataagcttt cattaccaca acaaatagca catggtacta 8280

ccttcccaat taaagtatga tgtaaccgtc gttgtcccct tatgtcaaat gcaaagtgaa 8340

cattcaaact taaatgcgag caagagcaat tataatatta cttcttctag ctttacaaaa 8400

taatattttc atcatttctg agtttattag tagaaacgtt aatattattt cagaaaagac 8460

tacaataaat tattggggta attcttagcg gtaggttctc ctgcccacga gtgctttgca 8520

ctgtaggtta aatttatttc ttcaggatat tcctacccct ctaggttgta ctaaccattg 8580

ataattactt gcaaatattt ttttcaaaaa aagaaaaccc tttacataaa taagctttat 8640

ataattatac gttgaaaaat gaccctaatt agtgtgcagt tttcaaatct taaatgtttc 8700

tctacccaat gattacagag atcatcaaca cttgtgaatg gacatcatat ctgtacgctt 8760

ttctaggctg cgaaattatg taacttcttg gtgtacaaaa aattgcaacc cctaagaaaa 8820

tcataagttt atatccaaga aaaaaatggt ttataagcgt ataatgaaaa taataatatt 8880

attaaccacg atggccaaaa gaaatctaaa gttggcaata attcgctagt tggggggaag 8940

ttgccaataa taaatgagca ggcgttttga tatttataat aataggtcac ctgttttgag 9000

tatttcctac agggactttt attttcataa ggtggatatg ctatcacttg gtgaaacaac 9060

ttcaaattcg tgtactttgc ttatgccaga tacttagcac tgggaaattg ttacaacccc 9120

atttctggaa atgtaacgtc acctgaaacc atcttatggt cctgccattg gtgtttcatc 9180

gtgttacaat gctaggtttt ttaaatgtct acaagtcaat attatattca agataaactt 9240

ttcaaaacat ctgatttatt atgacattat tcttgttgac atttttttgg ggtagacaag 9300

aaataattgc agataatata gaacacttat gccacgtggg tggatttaat agaatccttg 9360

taaaatatta tctctagaga attataaggg gaggagagaa gatctatggc aatgcaagaa 9420

aatgcaagat catcgtaaaa aaagtataag aatgactcca taagatatat aaacccactt    9480

gtttgaagag cgcttactac acggggttgt cttaatacaa aggcggcagg gttgcagtac    9540

ttctgtagtt tctaaccttt gtattcctta ggccctggaa tataatactt cctgtagtaa    9600

atgtcggagt ttaaattgct gacattgcaa gaaaataaaa ccaatataat attttttatg    9660

tcacgaaaga aatggaacaa caatgtagca ccaaaagggg tagagactag gcagtactat    9720

atttggaggt aaaagtatat tagaaaaaga acctatacat gaaccagtaa ccataacaaa    9780

aaaaaactaa acccaagcaa ttaaccatcc aaatttaacc cgttttataa tacaattttg    9840

accacatcta                                                          9850

<210>  75

<211>  305

<212>  PRT

<213>  Candida albicans

<400>  75

Met Ala Glu Phe Ser Asp Ala Glu Leu Arg Lys Met Met Gly Thr Leu
1               5                   10                  15

Ser Leu Leu Val Gln Asp Ser Arg Arg Glu Ile Asn His Leu His Asp
            20                  25                  30

Lys Leu Glu Asn Asn Ser Asp Ser Lys Tyr Gln Ser Leu Glu Thr Tyr
        35                  40                  45

Ile Asn Ser Lys Tyr Ala Asp Thr Ile Lys Ser Phe Glu Lys Leu Lys
    50                  55                  60

Tyr Leu Asp Ile Asp Asn Ser Glu Leu Val Asn Thr Trp Ile Met Cys
65                  70                  75                  80

Phe Asn Gln Val Lys Arg Phe His Pro Gln Val Phe Asp Ala Phe Met
                85                  90                  95

Glu Ala Glu Asn Glu Asp Glu Ile Gly Ile Glu Lys Ile Gln Tyr Thr
            100                 105                 110

Pro Tyr Thr Gly Lys His Leu Asn Asp Met Ile Arg Ile Phe Tyr Met
        115                 120                 125

Lys Ile Ser Glu Leu Ile Glu Arg Lys Val Ser Pro Asn Val Ser Arg
    130                 135                 140

Glu Met Asn Asp Gly Gln Pro Gln Phe Val Pro Asn Leu Phe Lys Lys
145                 150                 155                 160

Val Tyr Glu Met Ile Ile Ser Lys Pro Asp Val Ser Ala Ala Glu Arg

165                 170                 175

Ile Gly Lys Ala Leu Phe Lys Leu Gln Ser Lys Ser Arg Glu Leu Glu
            180                 185                 190

Arg Glu Ser Ala Phe Leu Leu Cys Gln His Leu Met Thr Asn Asp His
        195                 200                 205

Gln His Asp Asp Ile Ile Leu Lys Phe Leu Val Ser Gly Val Ser Pro
    210                 215                 220

Trp Tyr Leu His Ser Gln Ile Tyr Met Ser Ser Tyr Lys Leu Gly Phe
225                 230                 235                 240

Ser Asn Leu Phe Leu Glu Ile Tyr Ala Gln His Tyr Glu Leu Tyr Lys
                245                 250                 255

Ala Asp Pro Ile Tyr Lys Leu Pro Asp Ser Met Thr Leu Leu Asn Glu
            260                 265                 270

Ile Arg Ser Asn Arg Asp Tyr Pro Lys Val Val Asn Ala Ala Lys Asn
        275                 280                 285

Thr Val Gln Val Asn Asn Val Ser Ser Lys Asn Asn Lys Lys Lys Asp
    290                 295                 300

Glu
305

<210>  76

<211>  155

<212>  PRT

<213>  Candida albicans

<400>  76

Ser Glu Ile Asn Ala Thr Ser Thr Tyr His Glu Ile Gly Asp Thr Asn
1               5                   10                  15

Lys Asn Lys Glu Gln Leu Ile Leu Asn Leu Lys Asn His Thr Lys Leu
            20                  25                  30

Ser Glu Gln Lys Lys Lys Thr Asn Leu Leu Val Tyr Asp Ser Gly Ala
        35                  40                  45

Thr Val Ser Val Val Asn Asp Lys Thr Leu Leu Asn Asp Ile Lys Glu
    50                  55                  60

Ser Asn Ile Glu Ile Ala Thr Ala Glu Gly Glu Thr Ser Thr Ala Tyr
65                  70                  75                  80

Ala Leu Gly Thr Leu Thr Ile Ser Val Asn Gly Leu Asn Ala Lys Leu
                85                  90                  95

Asp Gly Val Leu Tyr Leu Pro Ser Ile Gln Leu Asn Leu Ile Ser Ile
            100                 105                 110

Lys Gln Phe Glu Asp Leu Cys Tyr Ala Ile Leu Ile Ser Glu Asn Leu
        115                 120                 125

Met Cys Leu Val His Ser Asp His Gly Pro Thr Val Ile Ala Lys Tyr
    130                 135                 140

Ser Pro Lys Asp Asp Leu Tyr Ser Gly Pro Arg
145                 150                 155

<210>  77

<211>  795

<212>  PRT

<213>  Candida albicans

<400>  77

Met Thr Asn Lys Val Glu Arg Val Thr Tyr Val Ser Ile Arg Asn Ile
1               5                   10                  15

Lys Gln Glu Val Ala Asp Lys Tyr Met Ile Lys Asp Leu Tyr Tyr Tyr
            20                  25                  30

His Leu Leu Ile Asn His Leu Ser His Glu Lys Leu Gln Leu Leu Val
        35                  40                  45

Lys Arg Gly Val Ile Lys Pro Val Lys Ser Thr Ser Ala Glu Ser Ala
    50                  55                  60

Ile Leu Asn Cys Gln Ile Cys Val Ala Ala His Ala Lys Leu Ala Ser
65                  70                  75                  80

His Asn His Thr Gln Gln Arg Glu Leu Glu Arg Pro Leu Gln Arg Leu
                85                  90                  95

His Leu Asp Thr Ala Gly Pro Phe Thr Ser Asn Lys Thr Lys Ser Tyr
            100                 105                 110

Leu Thr Thr Val Ile Asp Gln Phe Ser Arg Tyr Thr Glu Val Ile Val
        115                 120                 125

Ser Asp Thr Lys Ala Val Lys Gln Ser Ile Leu His Arg Leu Arg Val
    130                 135                 140

Trp Asn Asn Arg Phe Gln Phe Lys Ile Ala Glu Ile Arg Tyr Asp Asn
145                 150                 155                 160

Ala Leu Glu Tyr Pro Ser Ala Glu Glu Leu Glu Glu Leu Gly Ile Tyr
                165                 170                 175

Lys His Leu Leu Pro Asn Tyr Ser Pro Met Leu Asn Gly Thr Ala Glu
            180                 185                 190

Ala Thr Asn Arg Pro Ile Val Gln Gly Ile Tyr Lys Val Val Leu Asn
        195                 200                 205

Phe Ser Cys Gln Val Leu Ile Leu Phe Pro Phe Ile Val Glu Tyr Ala
    210                 215                 220

Val His Ile Arg Asn His Thr Pro Ile Lys Glu Phe Asp Gly Ala Thr
225                 230                 235                 240

Pro Tyr Glu Arg Tyr Tyr Gly Leu Ser Lys Tyr Val Ile Pro Phe Phe
                245                 250                 255

Gln Phe Gly Thr Asp Val Leu Ile Lys Cys Ala Ser Val Gln Glu Ala
            260                 265                 270

Ile Ser Leu Lys Leu Pro Ser Ser Arg Asp Lys Ala Phe Pro Thr Val
        275                 280                 285

Met Phe Gly Ala Phe Leu Gly Tyr Gly Ser Asp Ser Phe Thr Phe Arg
    290                 295                 300

Val Leu Val Ser Thr Lys Gly Tyr Pro Val Ile Thr Thr Ser Asn Ile
305                 310                 315                 320

Arg Pro Ile Ala Thr Met Gln Val Leu Asn Asp Tyr Leu Ala Tyr Ile
                325                 330                 335

Ser Glu Asn Ser Ser Ile Ser Tyr Asp Asp Thr Phe Leu Ser Pro Leu
            340                 345                 350

Asn His Pro Met Ile Arg Thr Asn Gln His Asp Arg Arg Gly Asp Asn
        355                 360                 365

Ile Asn Val Glu Tyr Glu Asn Arg Pro Asn Val Pro Phe Glu Tyr His
    370                 375                 380

Ala Glu Pro Pro Arg Thr Asn Ser Ser Thr Gly Ile Ile Asp Arg Pro
385                 390                 395                 400

Asp Ile Arg Pro Arg Ala Asp Pro Thr Trp Gln Arg Met Pro Asp Ala
                405                 410                 415

Asn Ile His Gln Glu Thr Thr Thr Val Gln Thr Pro Asp His Gly Glu
            420                 425                 430

Leu Asp Thr Met Ile Asn Asn Glu His Gln Leu Pro Arg Ser Gly Glu
        435                 440                 445

Gly Asn Tyr Pro Gly Gln Gln Val Arg Thr Asp Ile Ile Gly Gln Phe
    450                 455                 460

Arg Asp Arg Gly Pro Thr Thr Leu Asn Thr Pro Ile Asp Leu Gly Val
465                 470                 475                 480

Pro Asp Glu Thr Asp Asp Ile Ser Met Thr Ser Glu Asn Pro Ile Asp
                485                 490                 495

Ser Pro Asn Ser Glu Met Ile Ile Ser Pro Ser Leu Pro Thr Asn Glu
            500                 505                 510

Leu Glu His Gln Ile Asp Ile Ser Ser Gly Glu Met Ser Leu Leu Gln
        515                 520                 525

Thr Asn Met Glu Ala Asp Asn Glu Leu Lys Thr Asn Glu Met Val Leu

530                 535                 540

Tyr Lys Ser Lys Asn Asp Gly Ile Ile Ile Gln Gln Gln Gln Phe Thr
545                 550                 555                 560

Glu Asn Leu Ser Asp Glu Asn Glu Glu Asp Ser Ser Thr Asp Glu Glu
                565                 570                 575

Thr Leu Glu Asp Lys Lys Gln Gln Arg Leu Glu Tyr Asn Ile Ser Pro
            580                 585                 590

Asn Asp Glu Trp Ile Asn Asn Asp Val Gln Asn Glu Asp Asp Thr Gln
        595                 600                 605

Val Pro His Val Lys Glu Pro Ile Asn Tyr Glu Thr Gln Ser Arg Asn
    610                 615                 620

Glu Thr Asn Met Pro Arg Ile Glu Met Gly Ile Ile Glu Asn Leu Ser
625                 630                 635                 640

Asp Asp Gly Lys Asn Thr Pro Arg Glu Leu Arg Ile Val Thr Tyr Asp
                645                 650                 655

Asn Asn Lys Glu Ile Glu Lys Tyr Gln Asp Ser Asn Ile Glu Ile Ser
            660                 665                 670

Glu Pro Arg Asn Glu Asn Glu Asn Gln Thr Phe Ile Glu Ser Asn Leu
        675                 680                 685

Glu Leu Leu Asp Asn Gln Glu Met Phe Gln Glu Asp Pro Gln Val Glu
    690                 695                 700

Asp Ile Arg Leu Thr Thr Pro Lys Lys Asp Lys Ser Leu Ser Pro Asp
705                 710                 715                 720

Phe Asn Gln Thr His Asn Glu Ile Gln Leu Phe Met Ala Asp Ile Asn
                725                 730                 735

Glu Asp Met Leu Glu Glu Tyr Asp Glu Asn Ile Asn Met Asn Glu Val
            740                 745                 750

Leu Ala Asp Ser Thr Glu Thr Leu Asp Lys Glu Leu Asp Leu Asp Glu
        755                 760                 765

Glu Ser Gly Arg Ile Glu Tyr Ile Ala Asp Arg Val Arg Lys Lys Thr
    770                 775                 780

Glu Val Ser Met Val Arg His Thr Gly Asn Ile
785                 790                 795

<210>  78

<211>  257

<212>  PRT

<213>  Candida albicans

<400>  78

Met Asp Asp Glu Val Gly Ile Ala Ile Ser Lys Ile Arg Asn Phe Pro
1               5                   10                  15

Phe Arg Leu Lys Asp Gly Arg Ala Ser Phe Phe Pro Pro Tyr Lys Thr
            20                  25                  30

Lys Phe Gly Arg Ser Val His Pro Pro Lys Arg Tyr Leu Asn Ala Ile
        35                  40                  45

Val Lys Lys Ile Asp Tyr Asn Gln Lys Glu Trp Arg Gln Ser Met Glu
    50                  55                  60

Glu Glu Ile Glu Lys Phe Lys Ala Asn Gln Val Tyr Thr Val Glu Lys
65                  70                  75                  80

Thr Pro Lys Asn Val Val Pro Leu Lys Thr Met Trp Val His Thr Tyr
                85                  90                  95

Lys Thr Asn Asp Leu Lys Asn His Asn Tyr Lys Ser Arg Cys Val Val
            100                 105                 110

Met Gly Asn Tyr Met Val Glu Asn Arg Asp Phe Asp Pro His Ala Ile
        115                 120                 125

Ser Ser Pro Val Val Asp Leu Thr Ser Ile Arg Leu Leu Ser Ala Ile
    130                 135                 140

Ala Val Glu Asn Asn Leu Val Met His Gln Leu Asp Ile Ala Ser Ala
145                 150                 155                 160

Tyr Leu Asn Ala Ser Leu Glu Asp Gly Arg Val Ile Phe Val Arg Pro
                165                 170                 175

Pro Arg Gly Phe Glu Val Lys Pro Gly Tyr Ser Trp Arg Leu His Lys
            180                 185                 190

Ser Val Tyr Gly Leu Arg Gln Ser Ala His Asn Trp Tyr Ser His Phe
        195                 200                 205

Lys Asn Val Leu Glu Ala Asn Gly Leu Lys Gln Thr Leu His Asn Asp
    210                 215                 220

Gly Ile Phe Trp Lys Asn Tyr Glu Asn Gly Asp Val Leu Tyr Val Ser
225                 230                 235                 240

Val Tyr Val Asp Asp Val Phe Ile Lys Ala Asn Ser Met Ser Leu Cys
                245                 250                 255

Asn

<210>  79

<211>  3159

<212>  DNA

<213>  Candida albicans

<400>  79
aatctgtcca cctcgttttg agaggttctc aaaattcttt gtaattttca aacttcacct      60

ttggctttgt aaagttggtt ttttaaggaa tagctttgat tatttgacat tgcaaacagt     120

atagtcaaga tgcacacaga ttggacctga aattattcct tcgcaaaaac ttaaaataac     180

ccaaatatta aacatccact cggattcaaa tacctcagca ctcttttata ggcacttgta     240

taatttgtta tatgaatcat ttccagcttc cttgtagaac cgccaaatat ttgaatcaca     300

tgggaaacag atttgaccat ctaactttca tggttcttat gaaaaagatc tggaaatggt     360

gatatagctt gattgtctag catattcagc gattacccta ttttgtggtt gcctgggata     420

acccctggct gttgttggaa aagactcgtg acaagtattt ttgcccacga gtttctaatt     480

actgcgatat tatccagtta cattttcgca actcgttcta cttgagctcc ttctatgaat     540

caactagctg gctatttccc tggatagaaa accttcattc ttcttctcct ggttgagtat     600

caccgacttg tggccgtacc gttcaacccc ctacaataca ccatcaactt tatacttgta     660

atactcggct ttgccactcc ccaaactaac cactataagt tcatactcct tggcttgctt     720

gactttccta tttcttaacc cactactctt ctgtaccact ccgatcatca gattgacaga     780

ggttacttca tacccaacaa cattttcata ccagtcgacc ttctcctctg caccaccaaa     840

cccaacacat cggatttccc tgggatctct ctcaactctc aaacatattg ctttcttatc     900

taccctgaac gtgtgcacca ctaccacccc ttctatctca tataccacac tgaacgatga     960

gatcgcagca ctcccacaaa accgacaatg cagcggctca ggatacgaca ccctcaacga    1020

gttcaccttc atattcccga ccccaaacag tttgatgacc acccccgtgt tcacatctat    1080

aagctgacac tctaacccgt caacacgtat aaagaacccc acaaactcaa ccggaaatat    1140

cccacacagt ttcaggggcg ccacctctag ctttctgctc ttcatgctgt tgttgacgat    1200

gttcaccaca ataatatcca actccttcgt ctgcacaaca attctatcca tcacccttgg    1260

tgttcttatc tttattgcac agaccaactg ctgcttcaca tcataactct gtactttccc    1320

atcattacac gacacaacaa gtatctcccc actatccatg accatcacaa actcttccct    1380

actagtcctc tcacgctgtt tctgtccaaa cgatttcatc tgtattggtg gcggaaagtt    1440

cgcattgatc agcgaattta ccgacgacat tgacgcatca ctgcccctcc tctttctaat    1500

cattttacgt gctaaaaacc ccggcacagt tctccgcctg aaaaacgact ccaacacttt    1560

acctcgaaag tgcaccgaca gtgtccactt caactcccgc ttgtcataac cctgtatgac    1620

accctgtcta gtactcacca acacaaccat actcccatca tcattgagcc ccacatggct    1680

gaccggccac atctgacagg gtatggctag tggttcaggg tcgtaacagt actcgacatc    1740

ttggggttgg tagtgatata tctgaactcg tatccatcat ataactcttc tcctcagcaa 1800

actcaatggc ctgggttttt gccggaacca ctagtgcaac caccaacaag aggtactcca 1860

catagtaaat gtacgtgtta gactgggaaa caaccacact ggtttggtcg actcagcacg 1920

ctattcatca acaatacccc caacagaatc accaagttat ttgtcagcct cagtttgtac 1980

ttccaccact gaccccacca ccgcatagtt caccaaaagg gtcttgcata atccacgtcc 2040

caccatatca cttcaactcc catattcctc gatgcaagaa taaccacaat aatcggcttt 2100

cgtaaacgtc gtcagtggct caaacacatt gctgcacctt gagctctaga acaaccccac 2160

actcactagc catcgccaca ccaacaacca aattgctgat ccagaaaaaa taccaccccc 2220

gtagtccggc ttgtatggaa taattgcttg gccaggtacg tccccacctc atcgtgtctt 2280

ttctggttga aatatgtcat ctcccgggct aacagtaccg tatctctgtg gctggggcat 2340

ctatactctt tcattctcgg cttacaaatc tatcttgttc acacatttca tatatctggg 2400

acttgtcgaa ctctctgcac tctatcataa actggaactc gcttgcattc tgggacacac 2460

actggagctg gaatccatgg tcaggaaatg tgaaaatttt cttctcggga aatatttgtg 2520

acaattagtc ctagtacacg atagtttcat tacgcccact aaaagtgtct actgaaactc 2580

ggtctctata tcgtcaatat ctttcatttc tcttcctggc ttttcactgc gacttattgt 2640

tcgctatagg gtaggtcttc caagctaatt ttacccgaca caagatgaaa tattttctgt 2700

tgagcactcg ttgtcgacag tgaaaaattt tcactcaaga aaatattttc atcatcactt 2760

tttctagaaa ggaggttcaa gtgttggaga atagacagcg aacacctgat attcccaagg 2820

tcgaattaga ttgaaagata aataatagtc atatttattt tgtatttagt caataaatta 2880

tctttttata tttaaattct tagtattgtc ataccacgta gattgatacg gacatactta 2940

gcacatttaa catatattaa gcaccgatta cctgtgacat tccgaagttt actgtttcgc 3000

gcacgctggc agacgaacac ttatcaaggt gctactcccg cgcatcagtt tcctctgggt 3060

tctctttttg atcttggtga actacctttt tttcccactc gcgtgagaag ttcaacactt 3120

ttttttaccc atccaccaaa ctttattctt ttccccacc 3159

## Description

### RELATED APPLICATIONS

Reference is made to U.S. application Serial No. 60/106,342, filed October 30, 1998. U.S. application Serial No. 60/106,342 and all documents cited therein ("USSN 60/106,342 cited documents") and all documents referenced or cited in USSN 60/106,342 cited documents are hereby incorporated herein by reference. In addition all documents cited herein ("herein cited documents") and all documents cited or referenced in herein cited documents are likewise incorporated herein by reference.

### FIELD OF INVENTION

The invention relates to a novel retrotransposon. The novel retrotransposon is from the yeast *Candida albicans.* In particular, the invention relates to a retrotransposon pCal which belongs to the Tyl/*copia* group.

### INTRODUCTION

*Candida albicans* is an asexual yeast species which is the major fungal pathogen of humans. Although it is commonly found as a harmless commensal organism, inhabiting mucosal membranes and the digestive tract, it can cause superficial infections, such as oral thrush, in otherwise healthy people and can cause severe, often fatal, systemic infections in immuno-compromised patients. The recent increased use of immunosuppressive treatments and the increased incidence of immunosuppressive diseases, such as HIV infections, have meant that *C. albicans* infections are of increasing medical significance (Odds 1988). There is significant strain variation within this species, potentially affecting virulence, and mobile retroelements have been suggested as one source of this.

Retroelements are a widespread family of sequences that can replicate via the reverse transcription of' single-stranded RNA into double-stranded DNA, or are assumed to have arisen in this way. Two major types of retroelement are the retroviruses, such as HIV1 and Moloney murine leukaemia virus, and the retrotransposons such as Ty1 and Ty3 from *Saccharomyces cerevisiae* (Boeke and Sandmeyer 1991). The structures and lifecycles of retrotransposons and retroviruses are very similar. The major difference between the two groups is that the retroviruses can form infectious virus particles which can be transmitted between cells and between individuals. Retrotransposons can form intracellular virus-like particles (VLPs) but they lack the genes coding for the viral envelope so the VLPs are usually confined to the one cell.

Similarly to retroviruses, retrotransposons consist of an internal domain flanked by long terminal direct repeats (LTRs). In Ty1, for example, the LTRs are about 335bp in length and the internal domain is about 5.3kb long. The internal region has two long open reading frames (ORFs) homologous to the *gag* and *pol* ORFs of retroviruses. The *gag* gene encodes the structural proteins which make up the VLP while, downstream, the *pol* gene encodes the enzymes required for reverse transcription and integration - protease, integrase, reverse transcriptase and RNase H. The LTRs contain the promoter and the transcription termination signals and are functionally divided into three regions - U₃, R and U₅. Transcription proceeds from the U₃/R boundary in the left LTR to the R/U₅ boundary in the right LTR to produce an RNA molecule which has the R region repeated at each end. Translation of this terminally redundant mRNA is usually regulated to ensure that the structural proteins of the VLP (*gag*) are produced in much higher quantities than the enzymes (*pol*). This is because large quantities of the *gag* proteins are required for the assembly of the VLP but only catalytic quantities of the *pol* enzymes are required.

The most common method of down-regulating the translation of the *pol* ORF is to have it out of frame relative to the upstream *gag* ORF. A rare, programmed ribosomal frameshift is thus required for translation of the *pol* ORF. A number of retrotransposons employ a + 1 frameshift. Ty1 achieves this by tRNA slippage while the Ty3 mechanism involves the 'skipping' of a base. The Ty1-slippage mechanism involves a seven base sequence, CUU AGG *C*. It is thought that a tRNA^{LeuUAG}, which can recognise all six leucine codons, slips forward one base from CUU-Leu to UUA-Leu, during a translational pause caused by a rare tRNA^{ArgCCU} (2). The Ty3 + 1 frameshift also involves a seven base sequence, GCG AGU U. An alanine-valine sequence (encoded by GCG-GUU) is produced but tRNA slippage is not involved. It is thought that out-of-frame aminoacyl-tRNA binding or four-base decoding is responsible. Frameshifting is stimulated by the low availability of the tRNA decoding the AGU-Ser codon and also by the 12 nucleotides downstream of the AGU codon. Retrotransposons have also been found to use a -1 frameshift; an example is CfT-I of *Cladosporium fulvum.* Here the ribosome is thought to slip back one base on the sequence AAAA slightly upstream of the *gag* termination codon.

An alternative method of down-regulation has been found in the *copia* retrotransposon. Here the *gag* and *pol* ORFs are fused into one long continuous ORF, but a splicing reaction usually occurs prior to translation to excise most of the *pol* region from the mRNA. Only occasionally is a full-length RNA translated with the concomitant production of the *pol* enzymes.

Following translation the retrotransposon proteins and RNA can form into a VLP. This consists of a shell of *gag* proteins with the *pol* enzymes and genomic RNA packaged inside. The VLP is the site of reverse transcription. In general, the process of reverse transcription in retrotransposons is very similar to the well-characterised process of retroviral reverse transcription. Two important steps in the reverse transcription process are the priming of minus- and of plus-strand DNA synthesis. Minus-strand synthesis is most commonly primed by a cytoplasmic tRNA (often initiator methionine tRNA) which is packaged within the VLP along with the mRNA of the retrotransposon. The retrotransposon has a region adjacent to the left LTR, known as the minus-strand primer binding site [(-)PBS], which is complementary to the 3' end of this tRNA. The tRNA binds to the retrotransposon RNA at the (-)PBS and can then be used by reverse transcriptase as a primer for the synthesis of minus-strand DNA. Plus-strand synthesis is primed by a short purine-rich sequence, known as a polypurine tract (PPT), located just upstream of the right LTR. After minus-strand DNA synthesis has passed this sequence the RNA is nicked between the PPT and the LTR. The PPT RNA can then be used as a primer for the synthesis of the plus-strand. Reverse transcription is generally very inefficient; greater than 10% of cellular mRNA can be retrotransposon RNA yet the dsDNA form is not usually detectable by Southern blotting.

Following the synthesis of the dsDNA form of the retrotransposon it may integrate at a new site within the host genome. This process is likely to involve a complex of the integrase enzyme associated with the two ends of the retrotransposon DNA. In a process which is not well understood the integrase complex must be released from the VLP, move into the nucleus and then insert the DNA into a new genomic site. Studies with Ty1 and Ty3 have shown that the integration site-selection mechanisms of these retrotransposons are non-random and appear to be specifically adapted to avoid causing disruption to the host genome.

Retrotransposons can be divided into three major groups based on their reverse transcriptase sequences and the order of the genes within their *pol* ORFs. Members of the *Ty3*/*gypsy* group are the most closely related to the retroviruses and share a similar po/ gene order - protease, reverse transcriptase, RNase H and integrase. Examples of these elements are Ty3 of *S. cerevisiae, gypsy* of *Drosophila melanogaster,* Tf1 of *Schizosaccharomyces pombe* and *del* of *Lilium henryi.* Members of the *Pao* group, for example *Pao* of *Bombyx mori* and Tas of *Ascaris lumbricoides,* have a similar *pol* gene order to Ty3/*gypsy* retrotransposons but can be distinguished from them by their reverse transcriptase sequence. Ty1/*copia* elements are most easily distinguished from Ty3/*gypsy* and *Pao* retrotransposons and retroviruses by the gene order of the po/ protein - protease, integrase, reverse transcriptase, RNase H. This group includes Ty1 and Ty2 of S. *cerevisiae, copia* and 1731 of *D. melanogaster,* Tst1 of *Solanum tuberosum* and Tnt1 of *Nicotiana tabacum.*

The first *Candida* retroelement, TCa1, was identified through the discovery of multiple-copy isolated LTRs dispersed around the genome (1). These LTRs were discovered in an analysis of moderate repeat elements. Subsequently, composite elements, named TCa1, consisting of two LTRs flanking a 5.5kb internal domain were also found. In the *C. albicans* strains tested, one to two TCa1 loci were found, indicating between one and four copies of TCa1 depending on whether the loci were homozygous or not. TCa1 has many features of a typical retrotransposon including 388bp LTRs, beginning TG and ending CA, with six nucleotide inverted repeats, TGTTCG....CGAACA, at either end. The element is flanked by 5bp duplications of the host DNA and is transcribed to give an approximately unit length mRNA. Within the 5.5kb internal domain a (-)PBS and a plus-strand priming site are evident. The (-)PBS was not immediately obvious: no complementarily to tRNA^{iMet} (as used by Ty1 and Ty3) could be found. Bases 31 to 39 of tRNA^{Arg3} of *S. cerevisiae,* however, perfectly complemented the nine bases immediately adjacent to the left LTR (GATTAGAAG). There is, for some tRNA, a high degree of conservation between *S. cerevisiae* and *C. albicans* leading to the suggestion that a cleavage product of a *C. albicans* tRNA^{Arg} might serve as the primer. This suggestion is supported by the knowledge that the primer used by the *copia* retrotransposon is a cleavage product of tRNA^{iMet} containing only the first 39 nucleotides.

TCa1 has been shown to be transcriptionally active, but an analysis of 1200bp of its internal sequence has indicated that it is defective, there being multiple stop codons in all three reading frames. It is remarkable, given the clearly non-functional nature of this element, that the LTRs remain identical and that the plus-and minus-strand priming sites remain in apparently functional form. It is possible that the defective TCa1 retrotransposon has been maintained via the passive reverse transcription of its RNA by the products of a functional *C. albicans* retrotransposon. This passive replication would require that the element has identical LTRs and functional plus- and minus-strand priming sites but would be independent of the element's internal sequence.

The object of the invention is to provide a novel retrotransposon, in particular the isolation and sequencing of pcal, an unusual, novel Ty1/*copia* retrotransposon from *C. albicans.* The free, linear, double-stranded DNA form of this element is so highly expressed that it can be seen as a distinct band when uncut genomic *C. albicans* DNA is simply analysed on an agarose gel. It contains features conserved in TCa1 and other retrotransposons and has additional features previously unreported in the retrotransposon family.

The sequence of another *C. albicans* element, potentially retrotransposon-like in nature, has recently been submitted to the databases by a group in the U.K. (accession no. Y08494). This element has been named beta and is defined as an LTR. It consists of a repeated sequence about 400bp in length, flanked by 5bp direct repeats of the host DNA, and associated with tRNA genes. The borders of the element consist of short, imperfect, inverted repeats: 5'-TAATGTATA....TATACAACA-3'. Such an element is reminiscent of the isolated LTRs of other retrotransposons which are the result of homologous recombination between the ends of a retrotransposon with the concomitant deletion of the internal region. No significant similarity is detectable between the beta sequence and the LTRs of TCa1 or pCal of the present invention.

### SUMMARY OF THE INVENTION

The invention provides an isolated and purified retrotransposon having a copy number of between 40-150 (preferably 50-100) copies of free DNA of itself per genome (preferably 10-25 megabases, more preferably substantially 15 megabases). The DNA is preferably linear and is more preferably double stranded.

The retrotransposon may be isolated from fungi or yeast, preferably *Candida* and more preferably from *Candida albicans.*

The invention also provides a novel retrotransposon comprising at least one polypeptide positioned between at least two long terminal repeats, and wherein the retrotransposon is capable of integrating into the DNA in a genome providing a copy number of between 40-150 copies per genome. The copy number is preferably 50-100 copies.

The retrotransposon does not necessarily integrate into the DNA.

The retrotransposon preferably belongs to the Tyl/*copia* group.

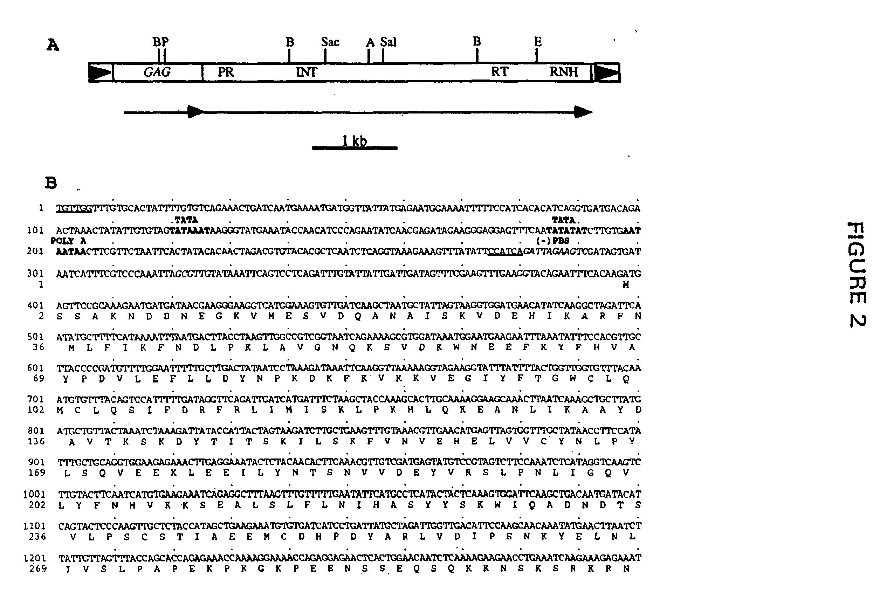

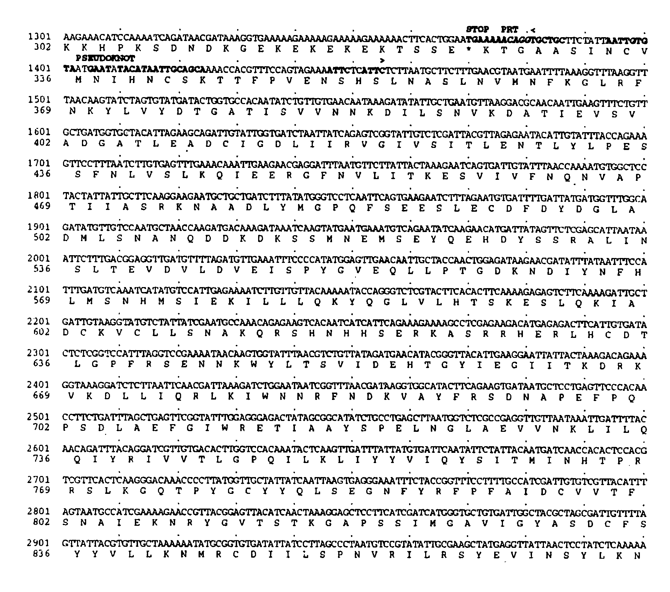

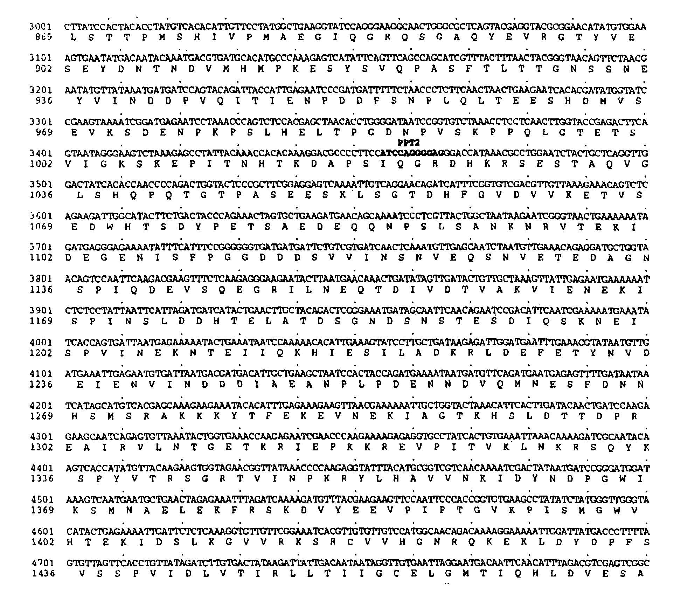

The retrotransposon is preferably isolated from fungi or yeast, preferably *Candida* and more preferably from *Candida albicans.*
The retrotransposon designated pCAL includes two long terminal repeats (LTR's) flanking an internal domain comprising at least two open reading frames. Advantageously, the LTR regions as identified in the sequence illustrated in Figure 2B may be used to introduce DNA into the genome of a cell.

Accordingly, there is also provided by the present invention a method of introducing DNA into the genome of a cell which method comprises introducing a transposable element comprising a nucleotide sequence encoding a desired protein located between two long terminal repeats sequences having the sequences illustrated in Figure 2B, which element is such that it can insert into the genome of said cell in the presence of an appropriate integration factor. Preferably, said integration factor comprises an integrase which preferably is itself included in said transposable element and which integrase is derived from the POL region of said pCAL retrotransposon.

The transposable element for introducing a desired DNA sequence into the genome of the cell also forms part of the present invention. This transposable element comprises an internal domain for receiving a nucleotide sequence encoding a desired protein flanked by two long terminal repeat regions having the sequences identified in Figure 2B. The transposable element may advantageously also be included in a DNA transfer system comprising said transposable element, which is capable of integrating into the genome of said cell in the presence of an appropriate integration factor and, said integration factor. In a preferred embodiment, the transposable element comprises an open reading frame encoding said integration factor which is an integrase protein, which preferably is encoded by nucleotide sequence within the *POL* region of the retrotransposon of Figure 2B.

The invention provides an isolated and purified retrotransposon comprising a nucleotide sequence selected from the group comprising:
(a) The sequence illustrated in Figure 2B;
(b) A nucleotide sequence with at least 65% similarity with the LTR and POL region of Figure 2B;
(c) A nucleotide sequence that hybridizes under conditions of standard stringency to the nucleotide sequence shown in Figure 2B; and
(d) A functional fragment of (a), (b) or (c).

The retrotransposon is preferably pCal.

The invention also provides the integrated form of the retrotransposon of the retrotransposon pCal, which has been designated TCa2 or sequences capable of hybridising thereto under standard hybridisation conditions.

The invention also provides an expression vector including any of the aforementioned retrotransposons or fragments thereof. The expression vector may be used to transform the cell into which the DNA is to be introduced. The expression vector may be introduced by any suitable means such as micro injection or electroporation or the like. The discovered promoter of RNA transcription is temperature regulated such that comparatively high levels of transcription occur at up to 37°C. Thus, levels of transcription may be regulated as required by altering the temperature.

The invention also provides the use of any of the aforementioned retrotransposons in a gene disruption system and in a gene discovery system. Upon active retrotransposition the retrotransposon can integrate into new sites in the fungi/yeast (preferably Candida) genome causing gene disruption which is preferably non-revertible. The retrotransposon can be 'tagged' with a selectable marker gene carrying its own promoter. This disruption system permits discovery (isolation and characterisation) of the disrupted gene.

The invention also provides a retroviral-like carrier system comprising any of the aforementioned retrotransposons, preferably pCal. The invention gives rise to virus-like particles in the yeast which can be modified to contain novel proteins such as enzymes.

The invention also provides a transformation and expression system for fungi/yeast (preferably *Candida*) comprising any of the aforementioned retrotransposons. The discovered promoter functions in a variety of yeasts including *Saccharomyces cerevisiae* and *Candida maltosa* and *Candida albicans.*

The invention also provides nucleic acid encoding a retrotransposon having a copy number of between 40-150 (preferably 50-100) copies per cell. The invention also provides the nucleic acid vector. The vector may be a gene expression vector. The vector may be a plasmid.

The invention also provides cells containing the nucleic acid including transposable elements and retrotransposons according to the invention. The cells may be contacted with a desired compound to identify its effect on the phenotype of the cell conferred by expression of the protein encoded by the nucleotide sequence provided in the transposable element.

The invention also provides the linear or circular, double stranded DNA copy of the retrotransposon.

Also provided by the present invention is a method of assigning a function to a nucleotide sequence which method comprise providing said sequence between the long terminal repeat sequences of the transposable element according to claim 1, 5 or 12 and introducing it into said cell and monitoring for the presence of an altered phenotype of said cell compared to a cell which has not had said nucleotide sequence introduced therein.

The invention also provides a nucleic acid fragment selected from the group comprising:
a) a nucleic acid sequence positioned between at least two long terminal repeats of the sequence of pCal as described in GenBank accession number AF007776;
b) a nucleic acid sequence with at least 65% similarity with the LTR and POL region of the sequence of (a);
c) a nucleic acid sequence that hybridizes under conditions of standard stringency to the nucleotide sequence of (a); and
d) a functional fragment of (a), (b) or (c).

The nucleic acid sequence preferably comprises a functional POL gene.

More preferably the nucleic acid sequence comprises two long terminal direct repeats flanking a series of genes in the order gag (group antigen), pol (polyprotein) where the pol sequence comprises an aspartic protease, an integrase and a reverse transcriptase/RNAseH, particularly as seen in Figure 2B.

The invention also comprises a functional (preferably temperature) inducible promoter isolated from a retrotransposon according to the invention. The promoter is preferably temperature inducible.

The invention also provides novel retrotransposons isolated from fungi/yeast, preferably *Candida.* In particular the invention provides retrotransposons 1-28 and more particularly retrotransposon 15.

The invention provides the use of the sequences 1-28 as probes and also provides use of the sequences 1-28 in any of the gene disruption systems, gene discovery systems, retroviral-like carrier systems, transformation and expression systems above.

The invention also provides the use of the sequences 1-28 in an expression vector as above.

The invention provides amino acid sequence equivalents to the nucleic acid sequences herein described.

Furthermore, the invention comprehends uses of the retrotransposons, the nucleic acid, e.g., DNA, RNA and amino acids of the invention, such as methods employing and/or compositions containing and/or comprising one or more a retrotransposon, nucleic acid, e.g., DNA, RNA and/or amino acid of the invention, including, for instance, wherein the retrotransposon is a vector containing and/or expressing an exogenous nucleic acid molecule.

### BRIEF DESCRIPTION OF THE DRAWINGS

Embodiments of the invention are now described, by way of example only, with reference to the drawings, in which:

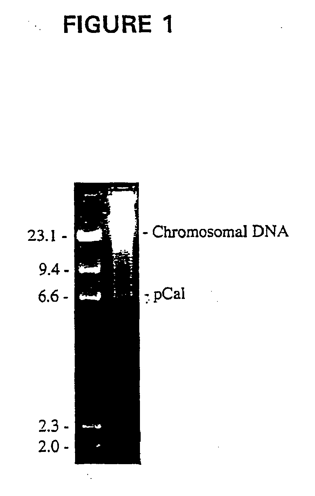

Figure 1 shows the presence of a high copy number, extrachromosomal element in *C. albicans* strain hOG1042. An uncut sample of hOG1042 DNA was electrophoresed on a 1% agarose gel alongside some marker DNA (sizes in kb indicated at left). A distinct band of about 6.5kb running ahead of the bulk of the chromosomal DNA (>20kb) indicates the presence of an extrachromosomal element in this strain. The relative intensity of the band suggests that the element exists at about 50-100 copies per cell (see text). The gel photo was scanned using a BIO-RAD GS-670 Imaging Densitometer and annotated using Adobe Photoshop™ 4.0.
Figure 2(A) shows the general structure of pCal. The boxed triangles represent the LTRs. The long boxes represent the internal region. The arrows below the boxes indicate the extent of the two long open reading frames. The positions of the encoded products are indicated: *GAG,* structural protein of the virus-like particle; PR, protease; INT, integrase; RT, reverse transcriptase; RNH, RNaseH. The termination codon at the end of each ORF is indicated by a vertical line. Selected restriction sites are shown above the diagram: B, *Bg*/II; P, *Pst*I; Sac, *Sac*I; A, *Asp*718; Sal, *Sal*I; E, *Eco*RI.
Figure 2(B) shows the complete nucleotide sequence of pCal and deduced amino acid sequence of the two long ORFs (translated using the non-standard *C. albicans* genetic code). Every tenth nucleotide is indicated by a dot above the sequence. The terminal inverted repeats of the LTRs are underlined. The putative poly-A signal and TATA boxes are highlighted in bold and labelled above the sequence. The minus-strand primer-binding site [(-)PBS] and the additional region complementary to the tRNA^{Arg} fragment are in italics. The stop codon at the end of the *gag* ORF, the adjacent purine-rich tract (PRT) and the stems of the pseudoknot are highlighted in bold, The PRT is also in italics. The 5' and 3' limits of the pseudoknot are indicated by < and >, respectively. The 3' polypurine tract (PPT1) and internal polypurine tract (PPT2) are highlighted in bold.

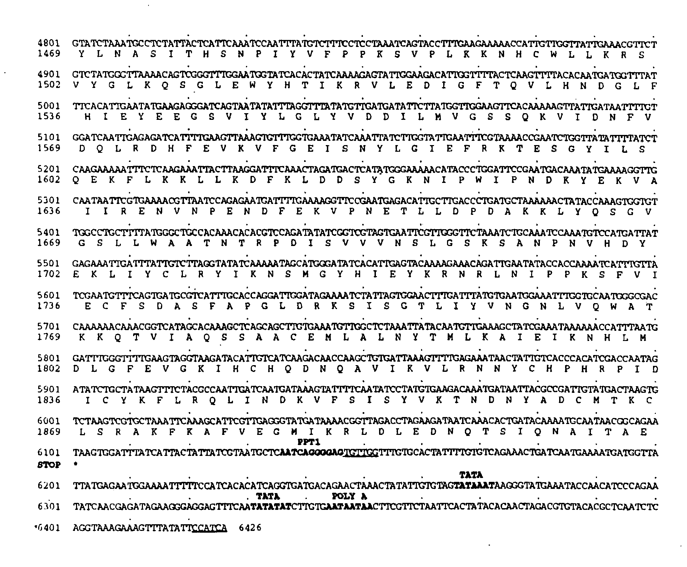

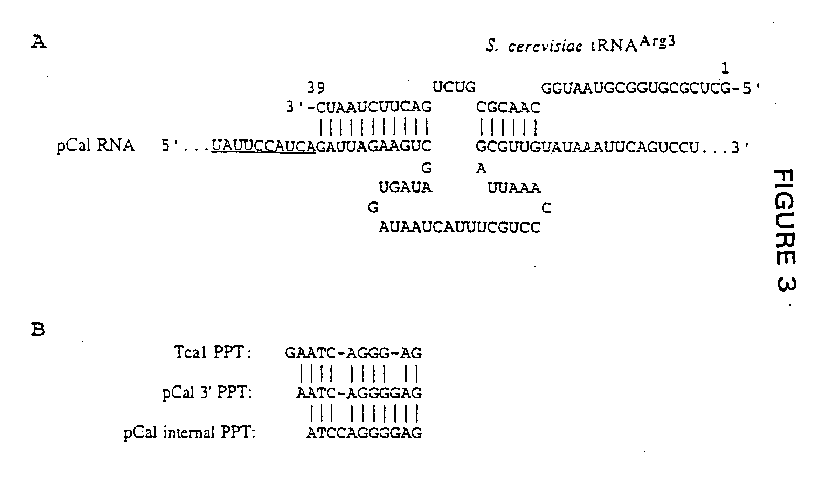

Figure 3 shows the plus- and minus-strand priming sites of pCal. (A) Minus-strand primer-binding site. The region of pCal around the (-)PBS (bottom) is shown compared to the first 39 bases of tRNA^{Arg3} of *S. cerevislae* (top). The region of pCal shown here extends from base 271 to 341. The bases of pCal within the LTR are underlined. For clarity, the bases of the tRNA molecule are shown in their unmodified form. (B) A comparison of the polypurine tracts of pCal and TCa1. The TCa1 and pCal 3' PPTs are adjacent to the right LTRs. The pCal internal PPT (bases 3455-3465) is from within the presumed integrase coding region.
Figure 4 shows the conserved motifs in the *pol* ORF of pCal compared to those of other Ty1/*copia* retrotransposons. Absolutely conserved amino acids are indicated by an asterisk (*). Positions containing 4 or 5 identical amino acids or in which there are only two types of amino acids present are indicated by a caret (^). The numbers in brackets indicate the positions of the motifs from the start of the *gaglpol* fusion proteins.

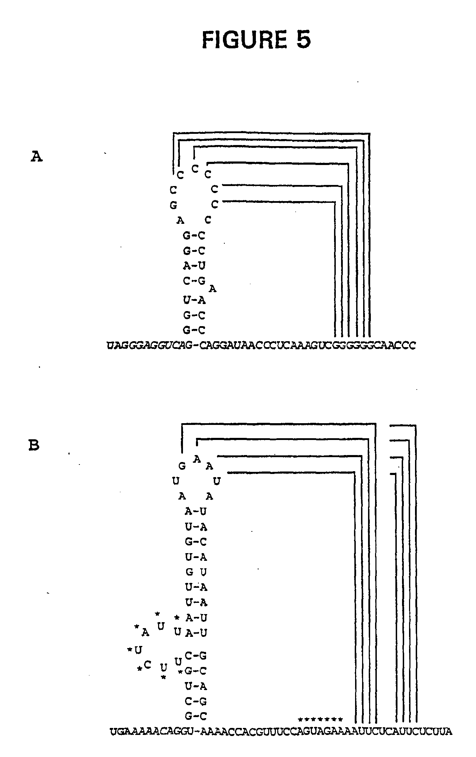

Figure 5 shows the comparison of the putative pseudoknot structures of Moloney murine leukemia virus (A) and pCal (B) at the boundary of their *gag* and *pol* ORFs. The stop codons are shown in bold and the 8bp purine-rich tract in italics. The long lines represent the base pairings in the second stems. Note that in pcal there are two downstream regions to which the first loop of the pseudoknot can anneal. The nucleotides in the bulge of the first stem of pCal also have a downstream region to which they can potentially anneal (bases marked *). Base pairing between these sequences could lead to the formation of an alternative pseudoknot.

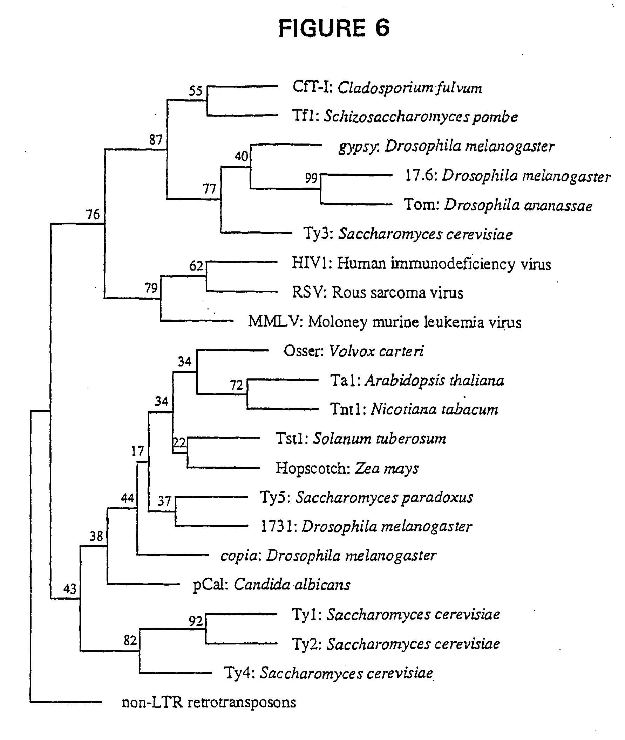

Figure 6 shows the phylogenetic tree of some LTR retroelements. The data used in the tree construction were the predicted amino acids of the seven conserved domains of reverse transcriptase identified by Xiong and Eickbush (1990). The tree was constructed using the UPGMA method available within the PHYLIP package (Felsenstein (1989). The percentages of trees, from 500 bootstrap replications, supporting each branch are indicated. Non-LTR retrotransposons were used as an outgroup to root the tree. The accession numbers for the sequences of the elements can be found in the Materials and Methods section of the Detailed Description.

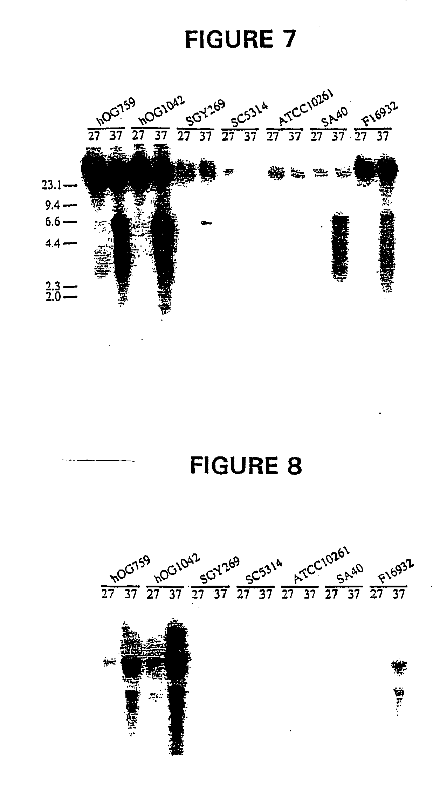

Figure 7 shows that the expression of pCal DNA occurs in a temperature- and strain-dependent manner. Cultures of the seven indicated *C. albicans* strains were grown at 27°C and 37°C to late log/early stationary phase following which total DNA was isolated. Approximately equal amounts of undigested DNA samples from each culture were then electrophoresed on an agarose gel and transferred to a nylon membrane. The membrane was then probed with an internal fragment of pCal. In the gel-blot shown above, the extrachromosomal pCal forms appear as a band running at about 6.5 kb and a smear of shorter forms running between 3 and 6.5 kb. The integrated chromosomal copies of TCa2 appear as a band at > 20 kb.
Figure 8 shows that TCa2 RNA expression occurs in a similar pattern to the expression of pCal DNA. Total RNA was isolated from cultures of the seven *C. albicans* strains, grown at 27°C or 37°C, as for the DNA in Fig. 1. Approximately equal amounts of RNA from each culture were then separated on agarose gels, transferred to nylon membranes and probed with the pCal internal probe. With longer exposures, TCa2 RNA could be detected in all of the strains.

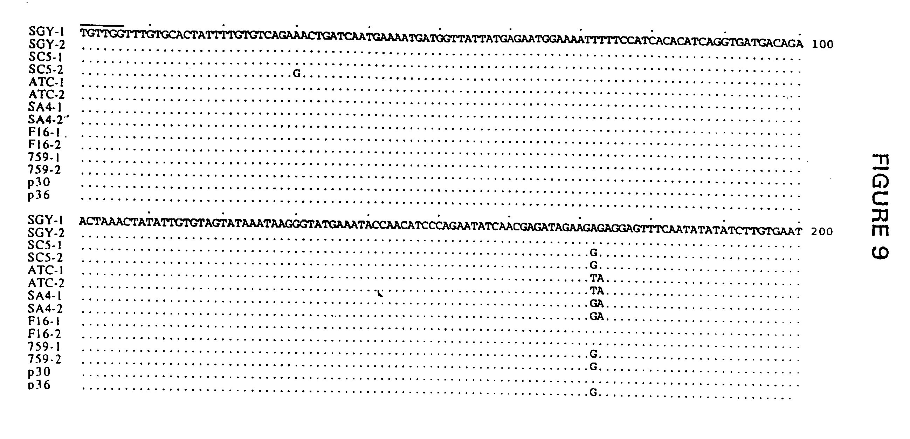

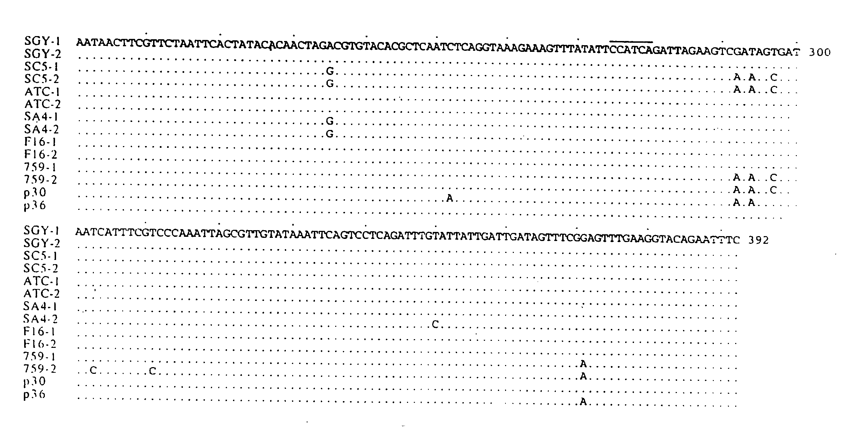

Figure 9 shows the comparison of the 5' regions of TCa2 retrotransposons from the various strains. The first ^{~}400 bp of TCa2 retrotransposons from each of the seven strains, except hOG1042, were amplified by PCR and cloned into a plasmid vector. The inserts of two clones from each strain were then sequenced and the sequences are compared above. The clones are labelled according to the strain they were derived from, for example, the first clone from ATCC10261 is ATC-1, the second clone from SC5314 is SC5-2 etc. Also shown are the sequences of p30 and p36, two of the original clones of pCal from hOG1042. The 5' half of the published pCal sequence was derived from p36. The sequences of the clones are listed in order corresponding to the amount of TCa2 RNA produced by the host strain, i.e. SGY269 produces the least and hOG1042 the most. The 6 bp inverted repeats at the ends of the LTRs are overlined.
Figure 10 shows the possible secondary structure of the minus-strand priming complex. The sequence of clone p759-2 is shown as it might appear bound to the *C. albicans* tRNA^{Arg(UCU)} fragment. The PBS of this clone is a perfect 32 bp match to the tRNA fragment. The remainder of the 5' untranslated region has the potential to form a stem-loop structure. The nucleotides of the retrotransposon from within the LTR are underlined. The AUG codon at the start of the *gag* ORF is shown in boldface.

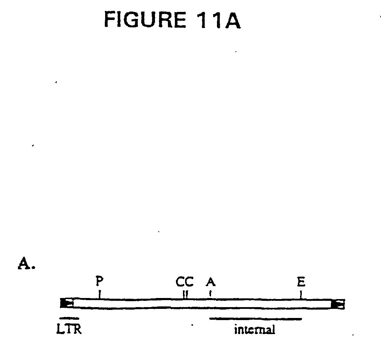

Figure 11(A) shows the location of the TCa2 probes and some important restriction sites. The structure of TCa2 is represented as the long box and the LTRs are the boxed triangles. The locations of the LTR and internal probes and certain restriction sites are indicated. P, *Pst*I; C, *Cla*I; A, *Asp*718; E, *Eco*Rl. (B) Copy number of TCa2. DNA was isolated from cells grown at 27°C then digested with *Eco*RI*.* The resulting fragments were separated on an agarose gel then transferred to a nylon membrane. The DNA immobilized on the membrane was then hybridized to the TCa2 internal probe. Lane 1, hOG1042; lane 2, SGY269; lane 3, SC5314; lane 4, ATCC10261; lane 5, SA40; lane 6, F16932; lane 7, *C. maltosa;* lane 8, *C. parapsilosis;* lane 9, *C*. *tropicalis;* lane 10, *C*. *pseudotropicalis.* Sizes in kb are indicated at the left of the picture. (C) Copy number of the TCa2 LTR. The membrane used in panel B was stripped and then reprobed with the TCa2 LTR.
Figure 12 shows the determination of TCa2 copy number in hOG759 and hOG1042. High molecular weight chromosomal DNA from each of the strains was purified away from the extrachromosomal copies of pCal as described in Materials and methods and then subjected to Southern analysis using the pCal internal probe. The DNA was digested with *Pst*l (lanes 1 and 2), *Eco*RI (lanes 3 and 4) or *Cla*l (lanes 5 and 6). Lanes 1, 3, and 5, hOG759; lanes 2, 4, 6, hOG1042. Sizes in kb are indicated to the left.

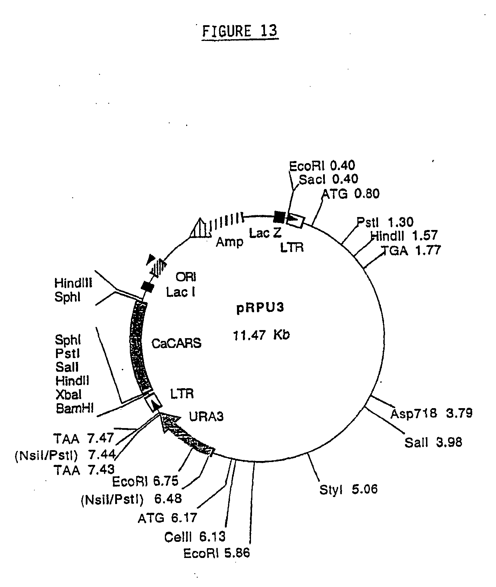

Figure 13 shows the plasmid pRPU3. The CaARS from pCARS (originally the Sphl fragment from pRC2312) was ligated in as a *Hind*III/*Bam*HI fragment into pRPU2.

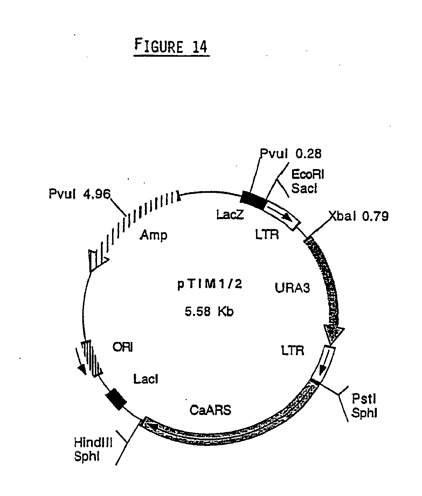

Figure 14 shows the plasmid pTlM1/2. Using CAL1 and CAL2 primers on p36 template the Sacl/Xbal products were cloned into p36K (creating p36Kf1) and then into pUXLC (creating pTIM1/p36fIUXLC) and pUXILC (creating pTIM2/p36fIUX1LC).
Figure 15 shows a Southern analysis of the TCa2 probe;

| | |
|---|---|
| Lane 1 hOG759 | Pst1 cut TCa2 probe |
| Lane 2 hOG1042 | Pst1 cut TCa2 probe |
| Lane 3 hOG759 | EcoR1 cut TCa2 probe |
| Lane 4 hOG1042 | EcoR1 cut TCa2 probe |
| Lane 5 hOG759 | Cla1 cut TCa2 probe |
| Lane 6 hOG1042 | Cla1 cut TCa2 probe. |

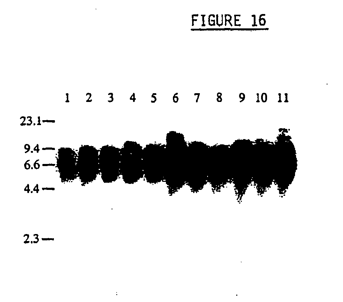

Figure 16 shows the generation of additional bands hybridising to TCa2 after culture, hOG1042 was grown for approximately 30 days in rich medium at 37°C by continually transferring cells between flasks. Nine independent colonies were isolated from the final passage. Genomic DNA was isolated from each of these colonies, and also from hOG1042 and hOG762 (a precursor of hOG1042). *Eco*RI-digested samples of DNA from each strain were then subjected to Southern blotting using as a probe a 2 kb fragment of TCa2 corresponding to the reverse transcriptase coding sequence. The results are shown in the figure. Lanes: 1, hOG762; 2, hOG1042, 3 to 11, 9 independent strains derived from hOG1042 after growth for approximately 30 days at 37°C. in several of the strains which had been subjected to passage at 37° TCa2 hybridised to more bands than in the parent hOG1042, for instance additional high molecular weight bands can be seen in lanes 4, 6 and 11. Additional bands of various sizes were also visible in these and other strains when the DNA was digested with other enzymes (not shown). Gain of bands was never found to be associated with the loss of any of the original bands, suggesting that the new bands represent additional copies of TCa2.
Figure 17 shows the nucleic acid sequence of retrotransposon 1 of 1309 base pairs.
Figure 18 shows the nucleic acid sequence of retrotransposon 2.
Figure 19 shows the nucleic acid sequence of retrotransposon 3.
Figure 20 shows the nucleic acid sequence of retrotransposon 4.
Figure 21 shows the nucleic acid sequence of retrotransposon 5.
Figure 22 shows the nucleic acid sequence of retrotransposon 6.
Figure 23 shows the nucleic acid sequence of retrotransposon 7.
Figure 24 shows the nucleic acid sequence of retrotransposon 8.
Figure 25 shows the amino acid sequence of the pol protein of retrotransposon 8.
Figure 26 shows the nucleic acid sequence of retrotransposon 9. This has a TCa2-like LTR.
Figure 27 shows the nucleic acid sequence of retrotransposon 10. This has a TCa2-like LTR.
Figure 28 shows the nucleic acid sequence of retrotransposon 11. This also has a TCa2-like LTR.
Figure 29 shows the DNA sequence of retrotransposon 12. This also has a TCa2-like LTR.
Figure 30 is the nucleic acid sequence of retrotransposon 13. This also has a TCa2-like LTR.
Figure 31 shows the nucleic acid sequence of retrotransposon 14. The pol protein is from nucleic acids 1169-1839.
Figure 32 shows the nucleic acid sequence of retrotransposon 15. The pol protein is from 1555-4302 base pairs. The LTR regions are from 979-1292 and 5212-5525 base pairs.
Figure 33 shows the amino acid sequence of retrotransposon 15. The pol protein is from 916 amino acids.
Figure 34 shows the nucleic acid sequence of retrotransposon 16. The pol protein is from 309-2332 base pairs.
Figure 35 shows the amino acid sequence of retrotransposon 16. The pol protein is 748 amino acids.
Figure 36 shows the DNA sequence of retrotransposon 17. The LTR zeta is from 887-1394 base pairs.
Figure 37 shows the nucleic acid sequence of retrotransposon 18. The LTR zeta is from 1418-1926 base pairs.
Figure 38 shows the nucleic acid sequence of retrotransposon 19. The LTR zeta is from 767-1274 base pairs.
Figure 39 shows the nucleic acid sequence of retrotransposon 20. The LTR zeta is from 3344-3851 base pairs.
Figure 40 shows the nucleic acid sequence of retrotransposon 21. The LTR zeta is from 812-1319 base pairs.
Figure 41 shows the nucleic acid sequence of retrotransposon 22. The LTR zeta is from 672-1179 base pairs.
Figure 42 shows the nucleic acid sequence of retrotransposon 23. The LTR zeta is from 467-974 base pairs.
Figure 43 shows the nucleic acid sequence of retrotransposon 24. The LTR zeta is from 787-1294 base pairs.
Figure 44 shows the nucleic acid sequence of retrotransposon 25.
Figure 45 shows the nucleic acid sequence of retrotransposon 26. The pol protein is from 2-322 base pairs. The LTR san is from 390-377 base pairs.
Figure 46 shows the amino acid sequence of retrotransposon 26. The pol protein of 106 amino acids.
Figure 47 shows the nucleic acid sequence of retrotransposon 27. The LTR san is from 143-523 base pairs.
Figure 48 shows the nucleic acid sequence of retrotransposon 28. The LTR san is from 558-939 base pairs.
Figure 49 shows the outline of the construction of the plasmid pRPU3. Plasmids from which DNA was derived from in this work are accompanied by a circle. The rectangular boxes indicate PCR products.
Figure 50 shows the construction of pTIM2 and p36f4UX1 LC. These plasmids contain a yeast autonomously replicating sequence (CARS) and the *C. albicans URA3* gene. In both plasmids the *URA3* gene uses the promoter in the left LTR and relies on the transcription termination signals in the right LTR. P36f4UX1LC also contains the gag ORF of pCAL as a fusion product with the *URA3* gene. The rectangular boxes represent PCR products and the circles the original plasmids from which DNA was obtained.
Figure 51 shows the outline of the construction of the plasmid pNRE5 used in an *in vivo* construction in the *C. maltosa* strain CHAU1.
Figure 52 shows the results of transformed colonies per µg DNA.

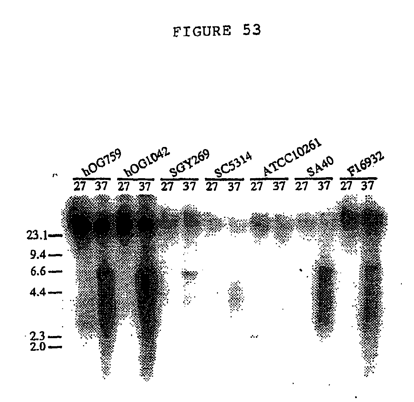

Figure 53 shows the expression of pCal DNA occurs in a temperature- and strain-dependent manner. Cultures of the seven indicated *C. albicans* strains were grown at 27°C and 37°C to late log/early stationary phase following which total DNA was isolated. Approximately equal amounts of undigested DNA samples from each culture were then electrophoresed on an agarose gel and transferred to a nylon membrane. The membrane was then probed with an internal fragment of pcal. In the gel-blot shown above, the extrachromosomal pCal forms appear as a band running at about 6.5 kb and a smear of shorter forms running between 3 and 6.5 kb. The integrated chromosomal copies of TCa2 appear as a band at >20 kb.

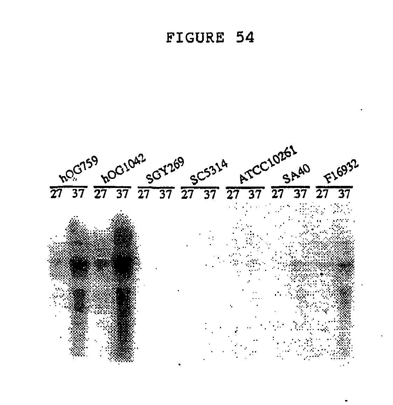

Figure 54 shows TCa2 RNA expression occurs in a similar pattern to the expression of pCal DNA. Total RNA was isolated from cultures of the seven *C. albicans* strains, grown at 27°C or 37 °C, as for the DNA in Fig. 1. Approximately equal amounts of RNA from each culture were then separated on agarose gels, transferred to nylon membranes and probed with the pCal internal probe. With longer exposures, TCa2 RNA could be detected in all of the strains.
Figure 55 is a Southern analysis of *URA3** colonies derived from two *Candida* strains, hOG1051 and hOG963. Genomic DNA from *URA3** colonies and their parental strains was digested with *Eco* RV and probed with the *URA3** gene (shown in the schematic diagram).

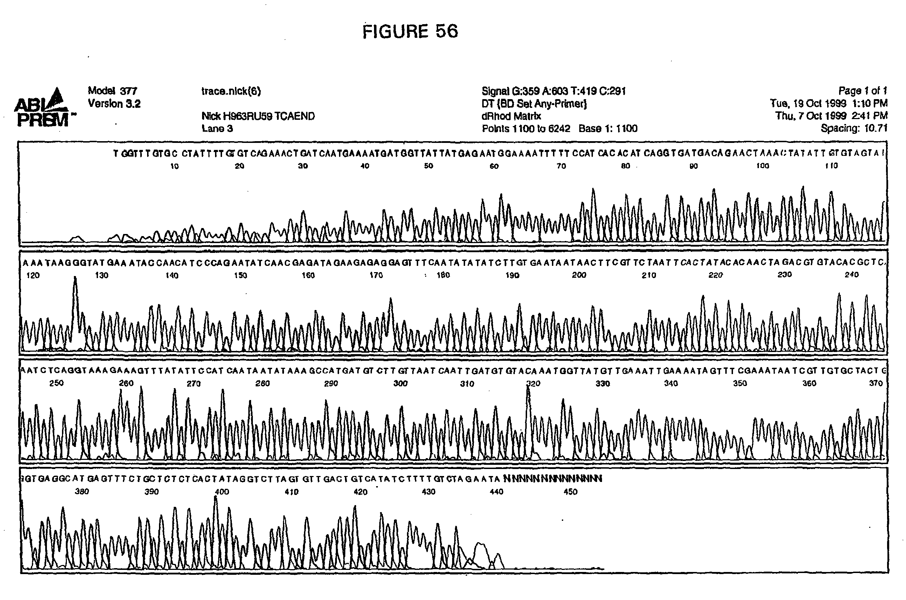

Figure 56 shows ABI PRISM chromatogram H963RU59; that is, sequence surrounding a TCa2/URA3 element integrated into a new position in the *Candida* genome. Position 291 shows the start codon of an ORF corresponding to a probable membrane protein. Position 276 represents the insertion site of TCa2/URA3, within the ORF.

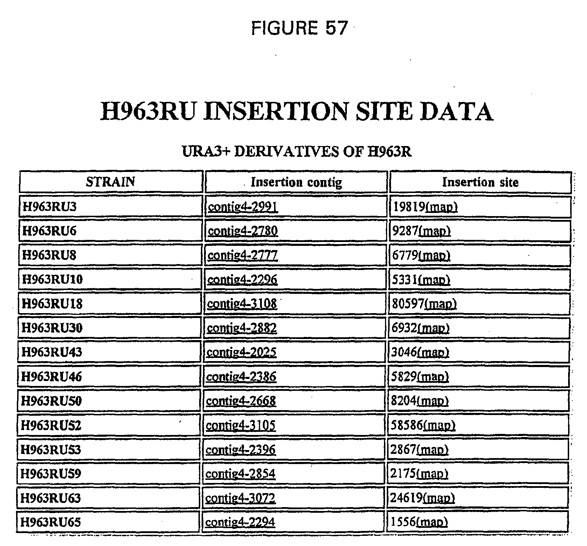

Figure 57 is a summary of the integration sites of TCa2/URA3 and the sequences around the integration sites.
Figure 58 is an ORF map of contig 4-2824 and shows the integration site in H963RU59 (URA*).
Figure 59 shows an analysis of intron processing from the *ura3* gene. The *URA3* gene was placed into TCa2 in all possible combinations. The vector was then transformed into *C. albicans* CAI-4 and *URA3⁺* transformants were selected. Constructs, which gave rise to *URA3⁺* colonies, are indicated.
Figure 60 shows the Integration of pRUIA. Integration of pRUIA results in the formation of a functional *ADE2* gene.
Figure 61 shows a Southern analysis of pRUIA integrated into hOG1051 and hOG963. Southern analysis was performed using a *URA3* probe, shown in the schematic diagram. Genomic DNA has been digested with *Eco* RI (E), *Hind* III (H) or *Xba* 1(X). H1051R appears to contain two copies of pRUIA.
Figure 62 shows a Northern analysis of tagged TCa2. RNA was isolated from cultures grown at 27°C and 37°C. A *URA3* gene probe was used in this analysis. The arrow indicates the transcript containing the tagged TCa2 (approximately 7kb).
Figure 63 shows a tagged retrotransposition.
Figure 64 shows the production of *URA3*⁺ colonies. Approximately 10⁷ cells were plated on each of the four plates. Only strains containing pRUIA give rise to *URA3*⁺ colonies.
Figure 65 shows a Southern analysis of *URA3*⁺ colonies. Genomic DNA from *URA3*⁺ colonies and their parental strains was digested with *Eco* RV and probed with the *URA3*⁺ gene (shown in the schematic diagram).
Figure 66 shows the general principle of inverse PCR as applied in this analysis. The agarose gel shows the result of inverse PCR on 10 independent tagged retrotransposition events.
Figure 67 shows ORF maps of tagged retrotransposition events. The arrow at the integration site indicates the direction of the TCa2 element. Tentative annotations of ORF have been made. Only the ORFs closest to the insertion site are shown.
Figure 68 shows the distribution of TCa2 insertions in relation to the nearest ORF.
Figure 69 is an analysis of the sequence around the insertion site. All sequences are shown in the same orientation with respect to the integrated TCa2.
Figure 70 shows the removal of an integrated retrotransposon. Recombination between LTR sequences results in the loss of the *URA3* gene. The result of this recombination is a solo LTR.

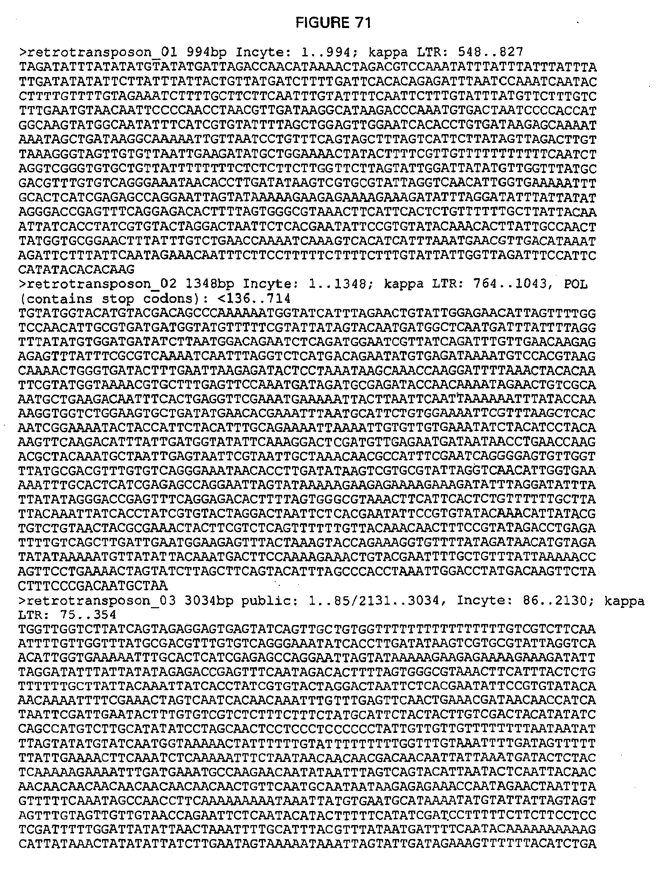

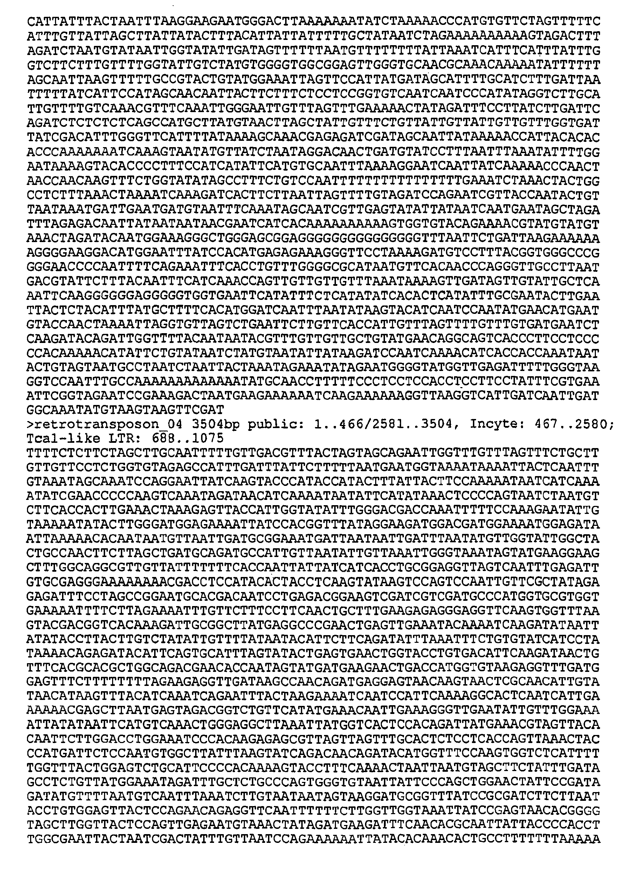

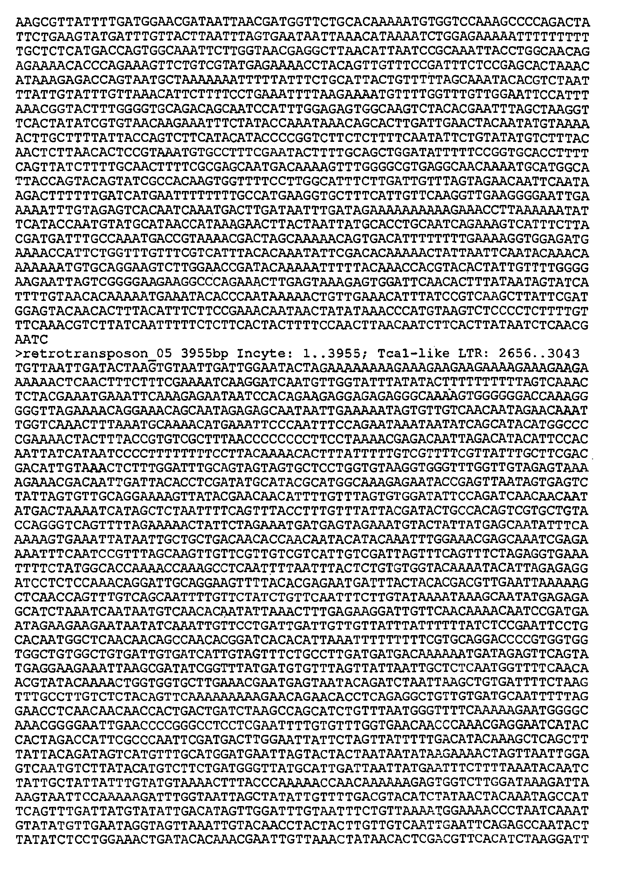

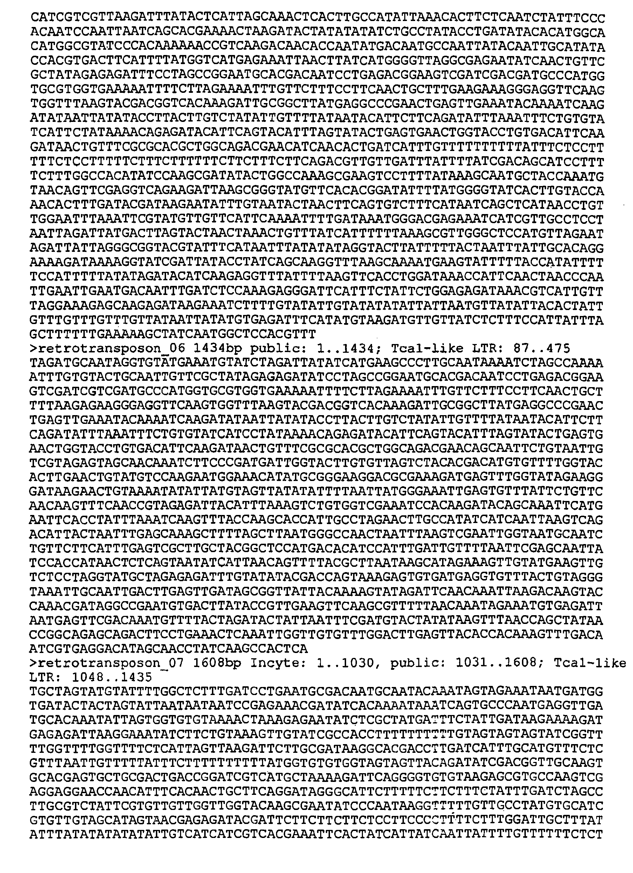

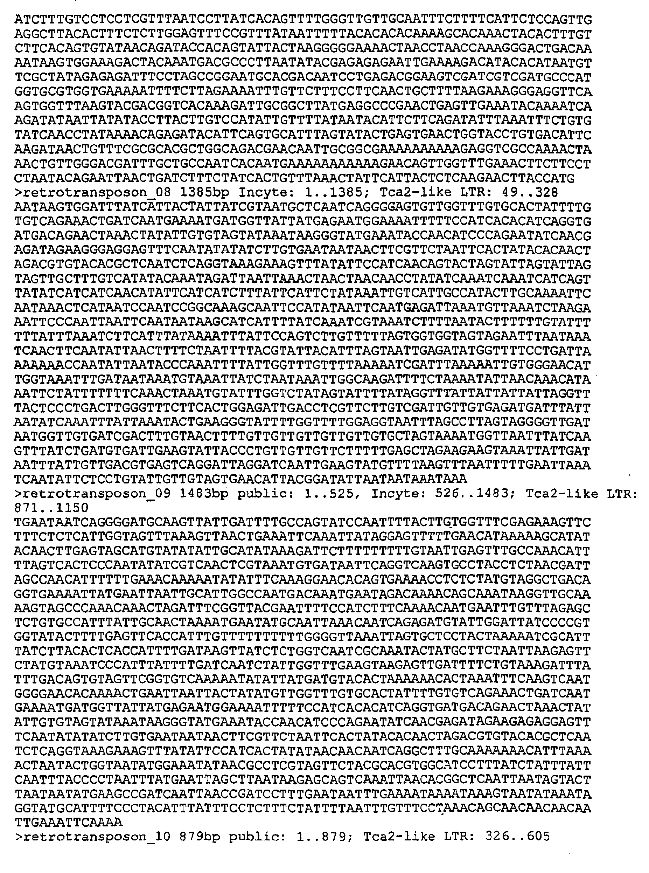

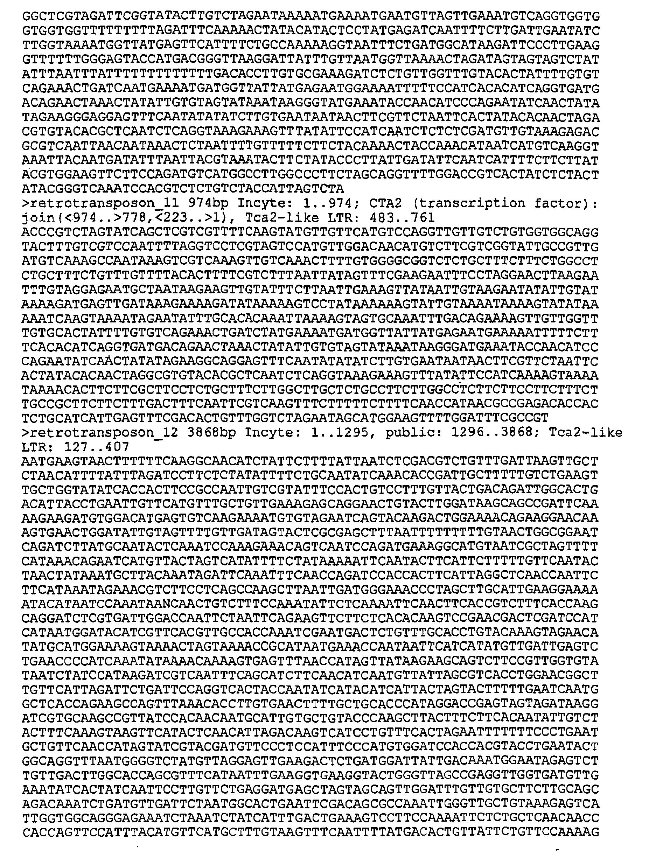

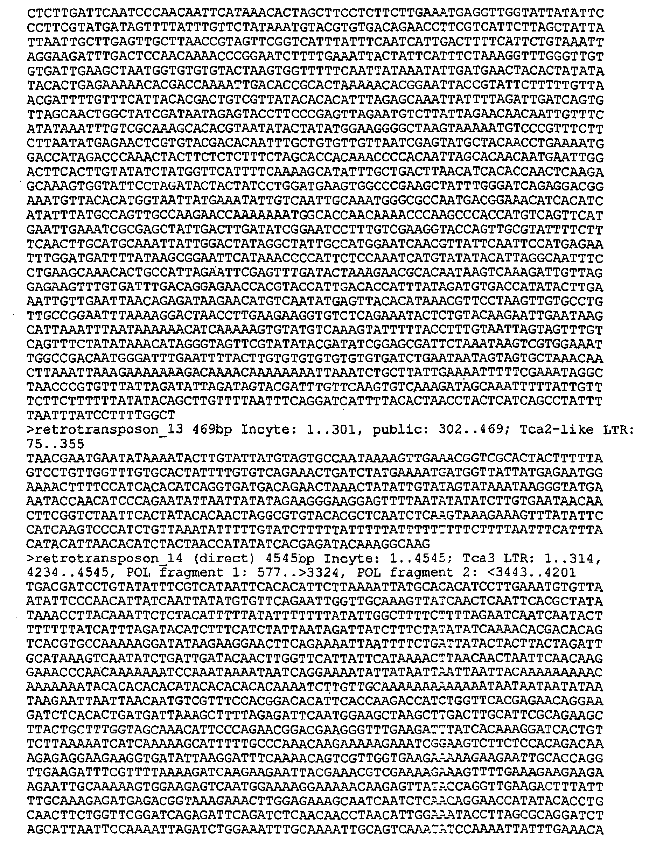

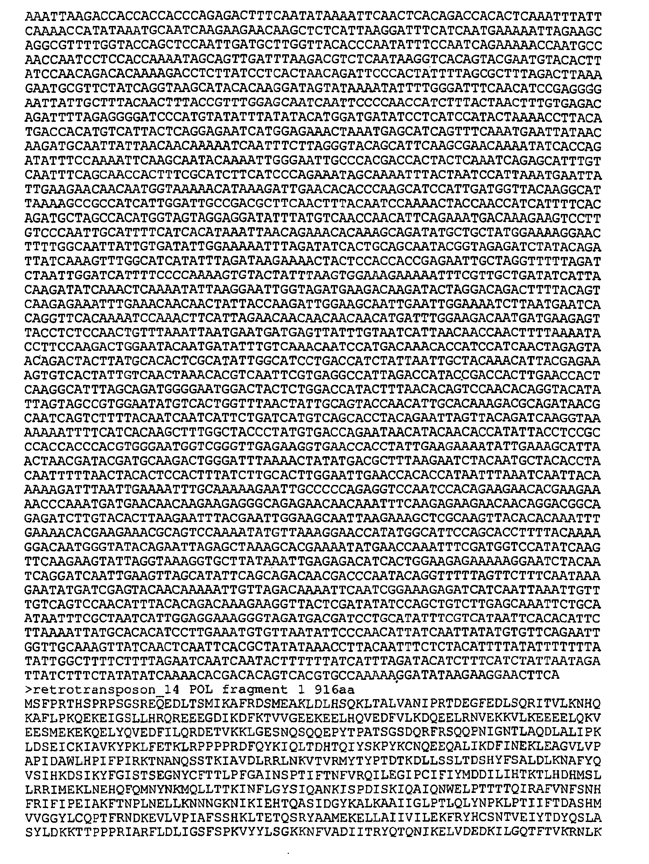

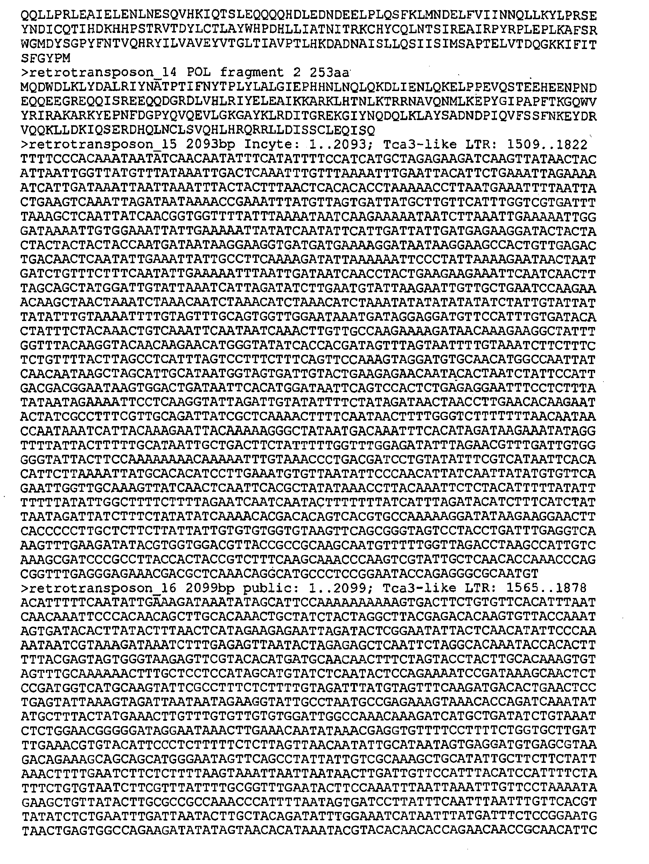

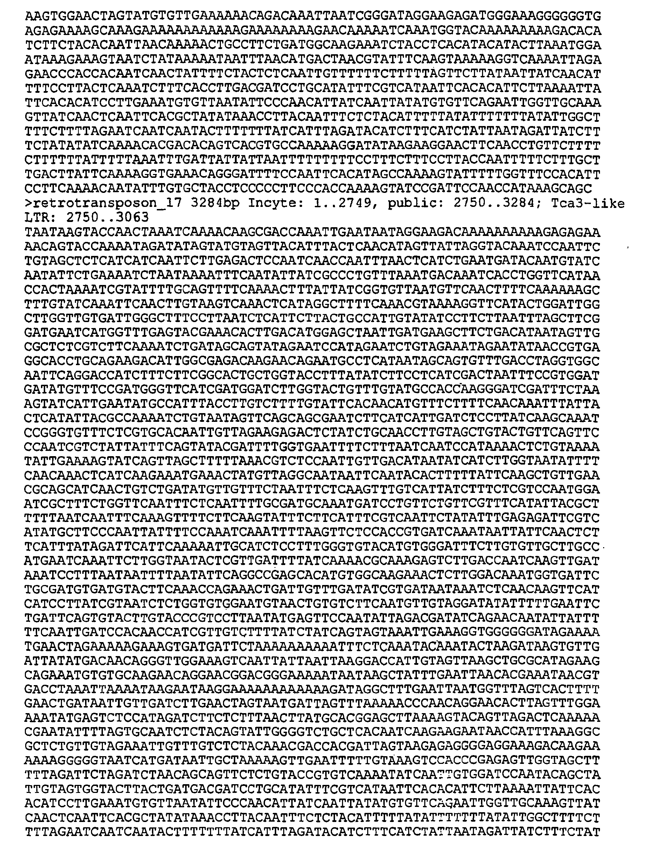

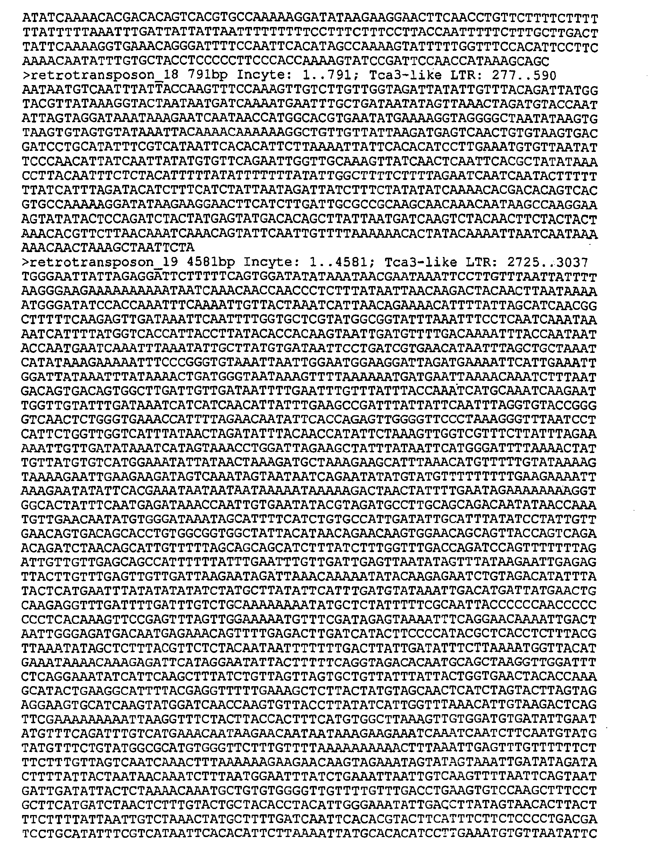

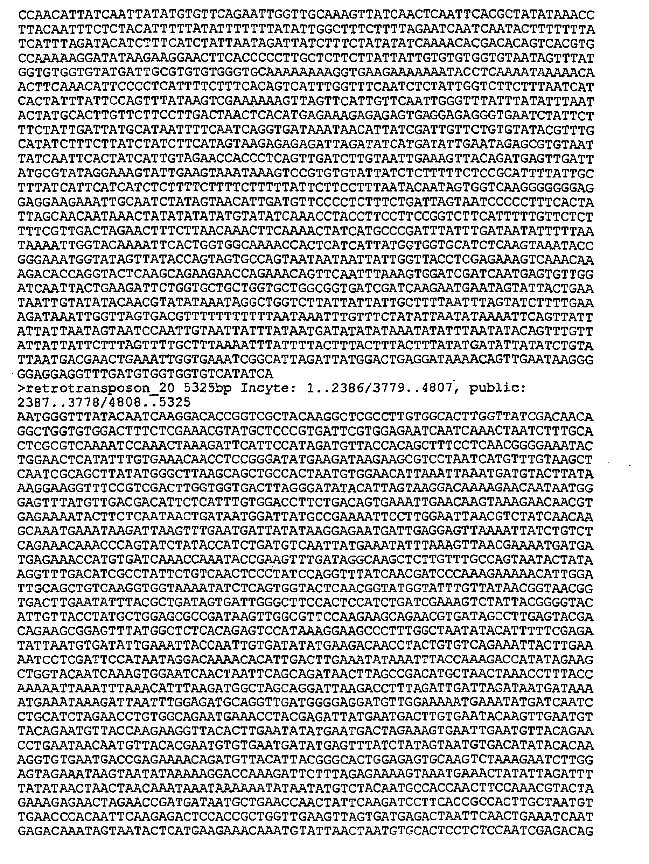

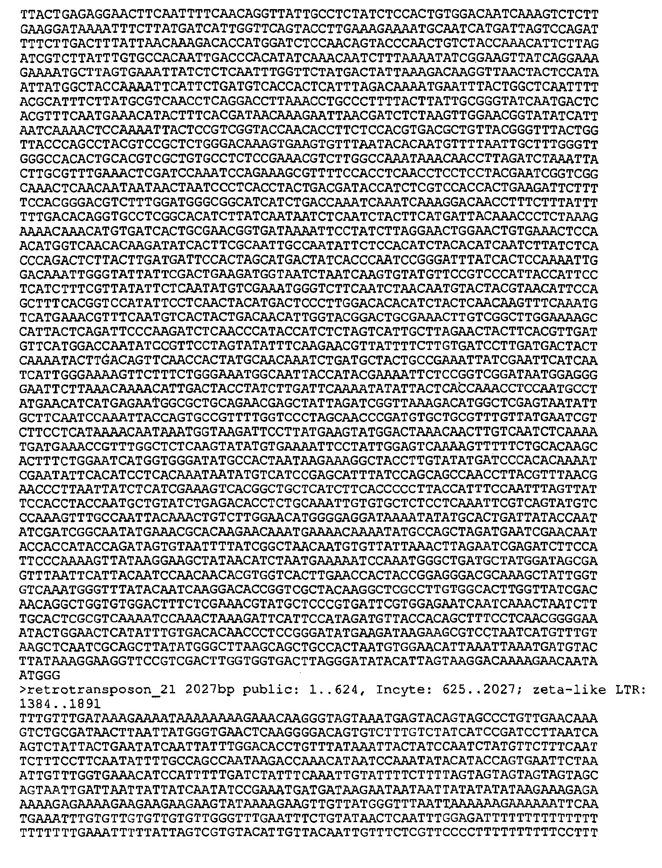

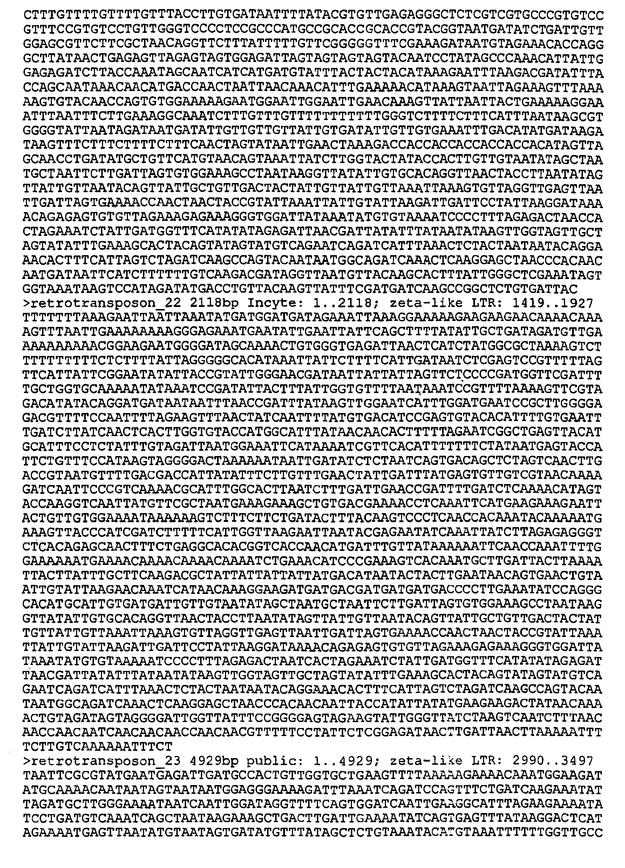

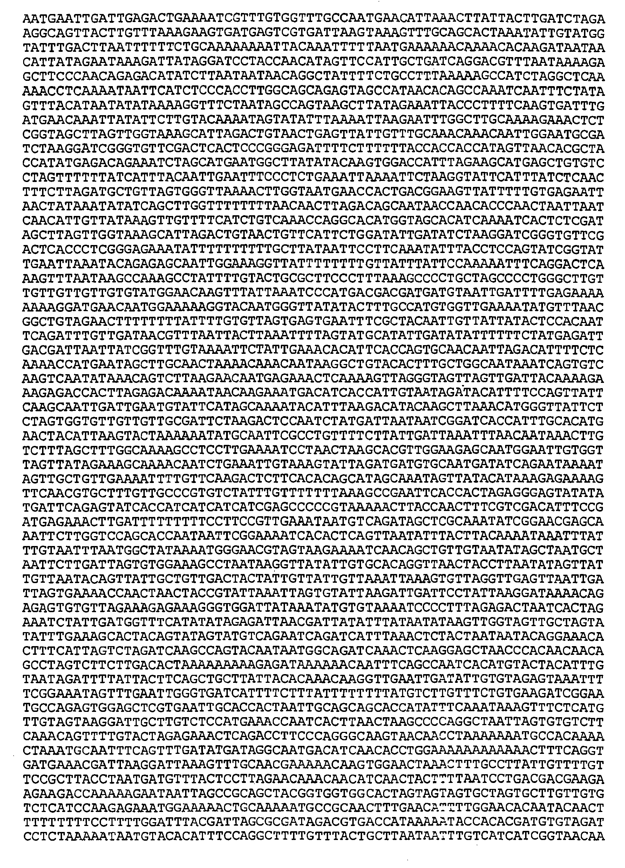

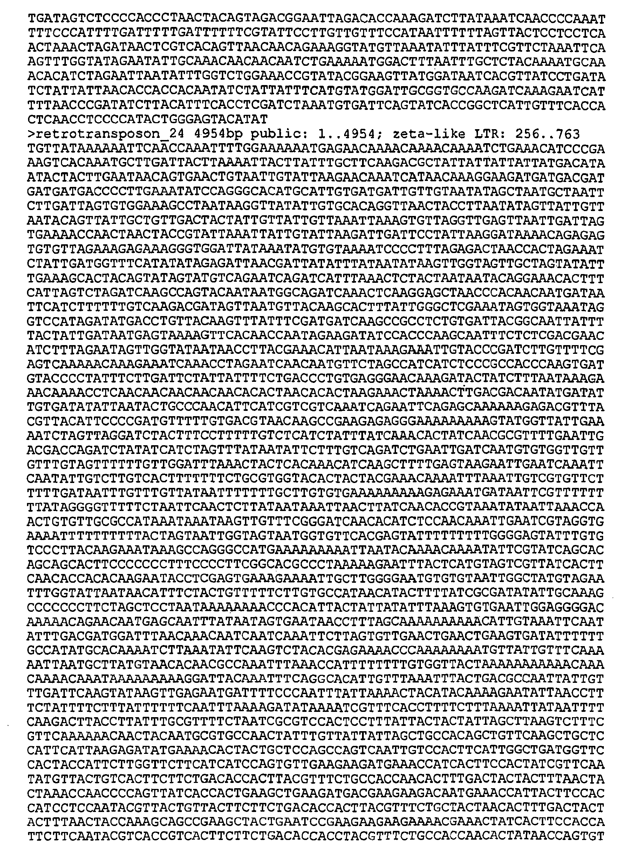

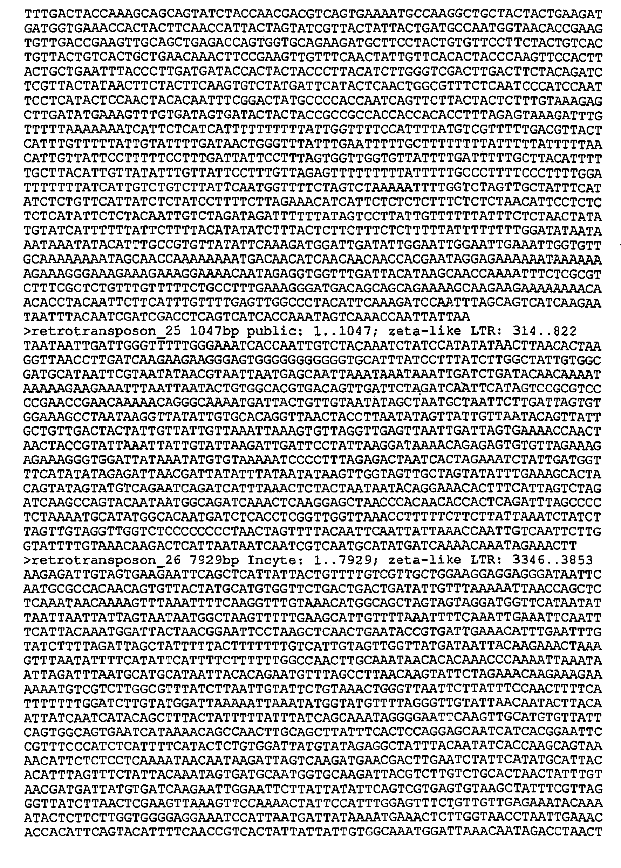

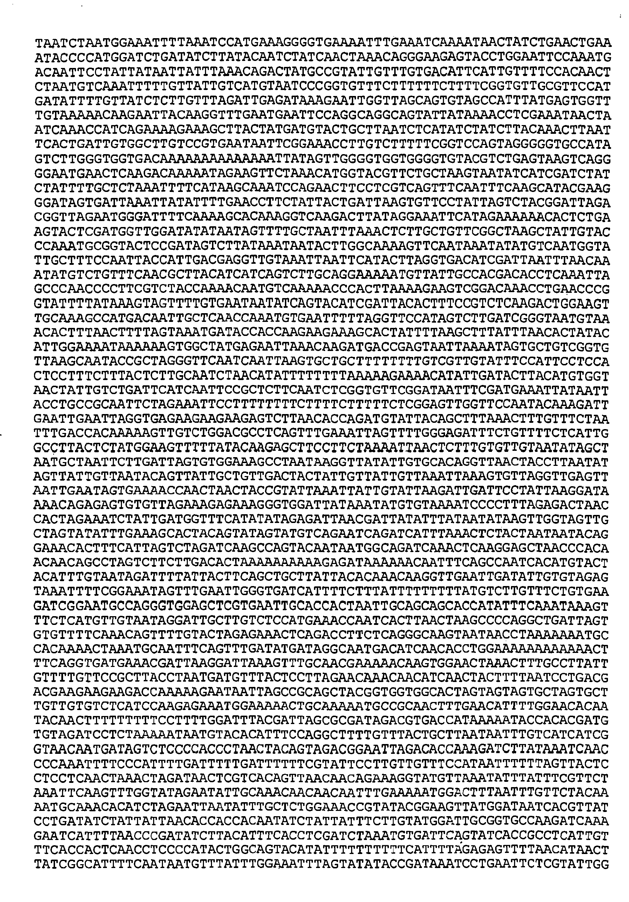

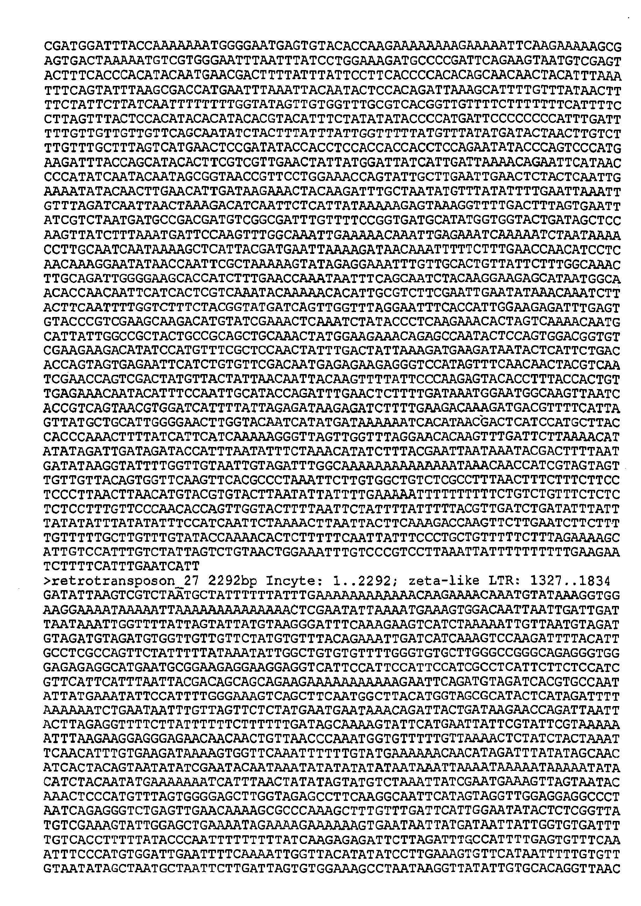

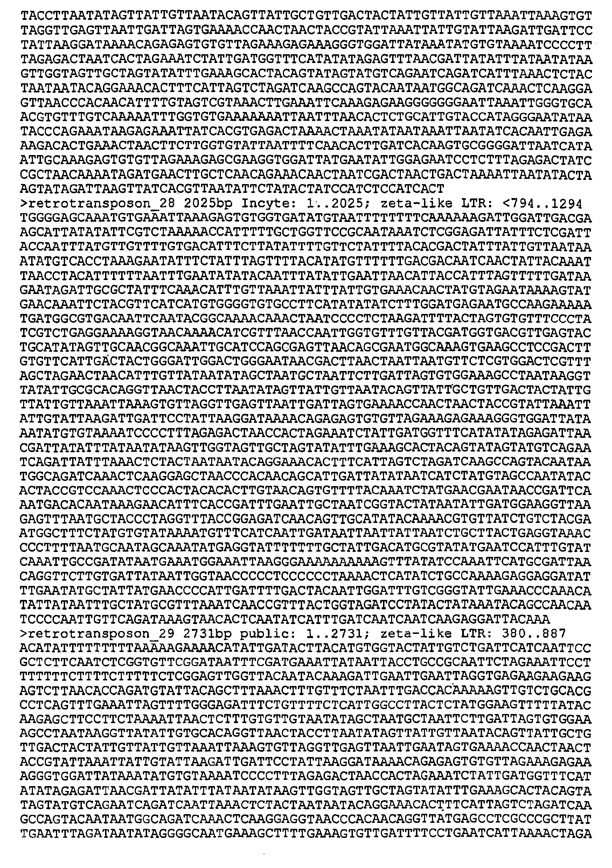

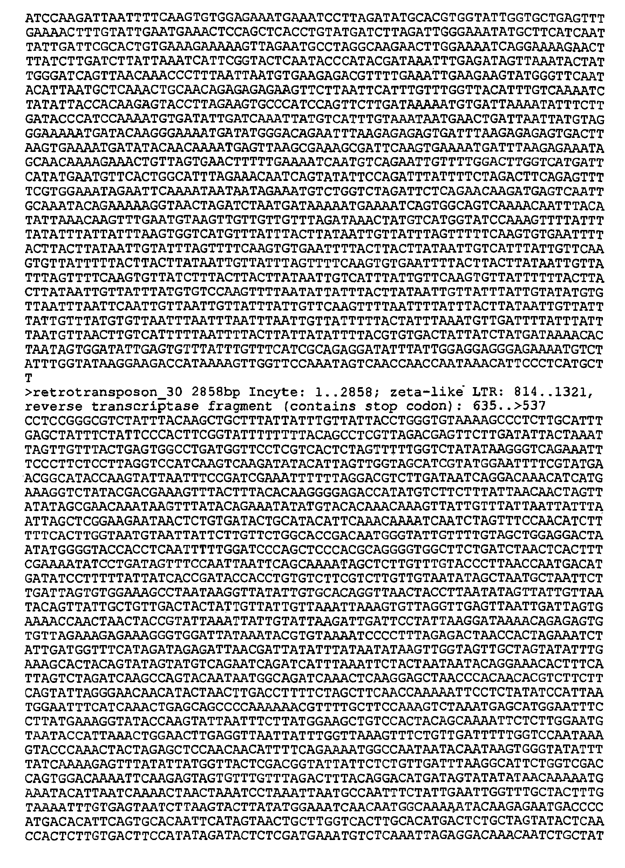

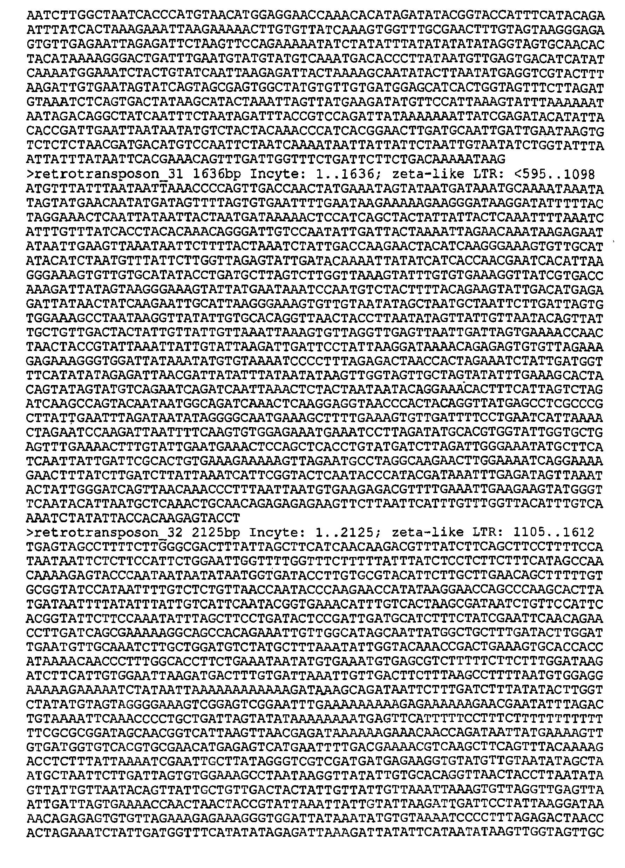

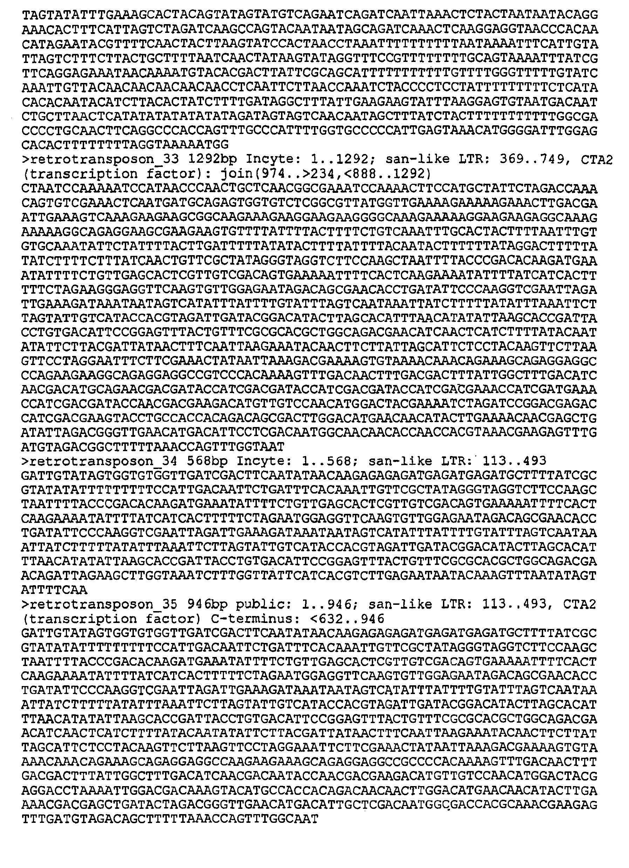

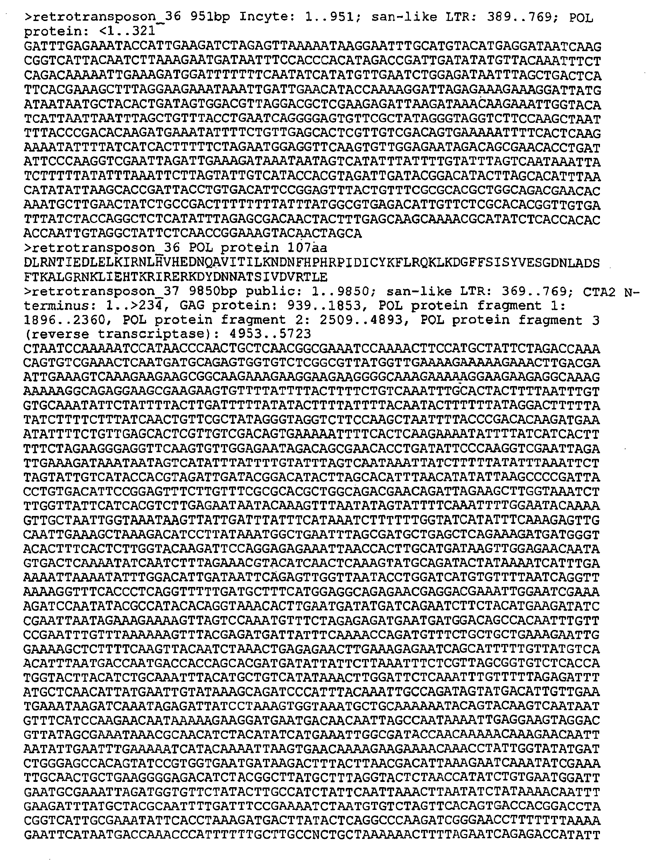

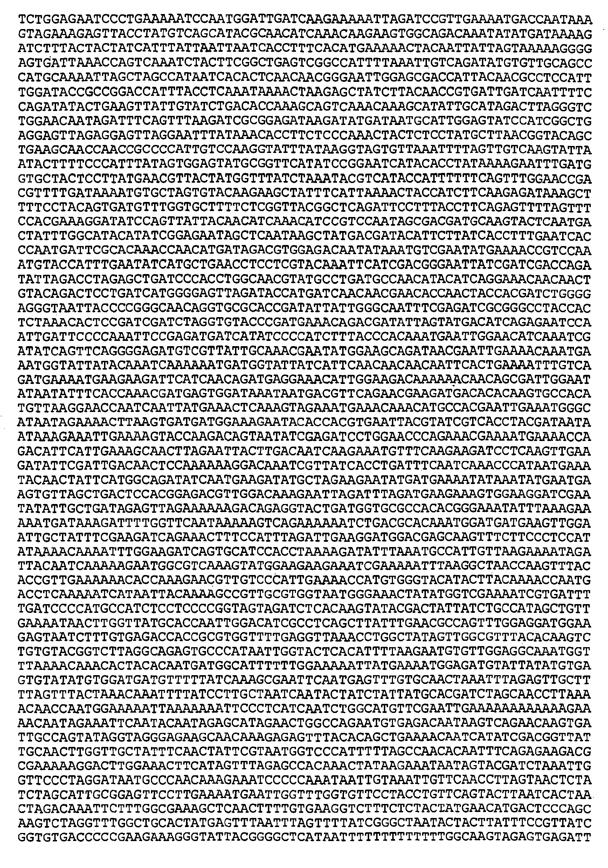

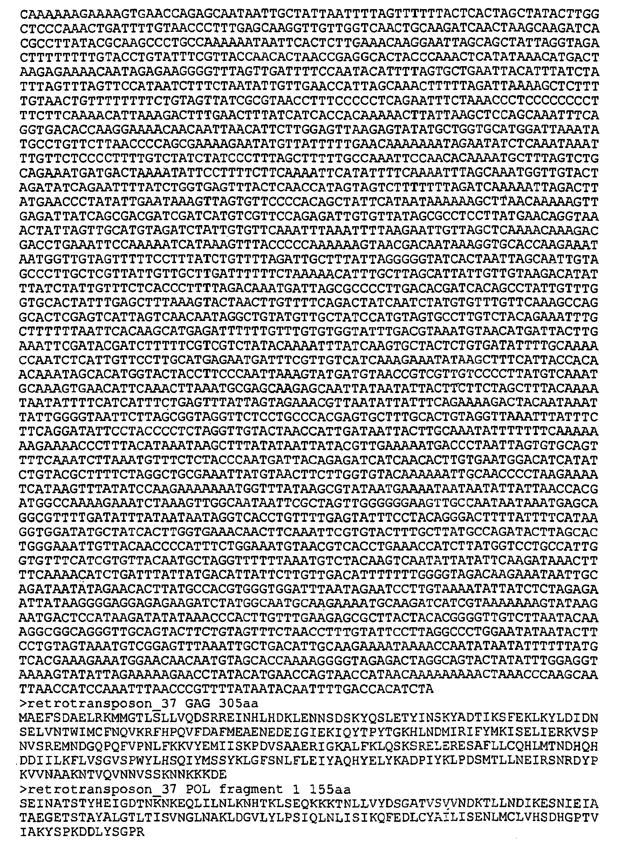

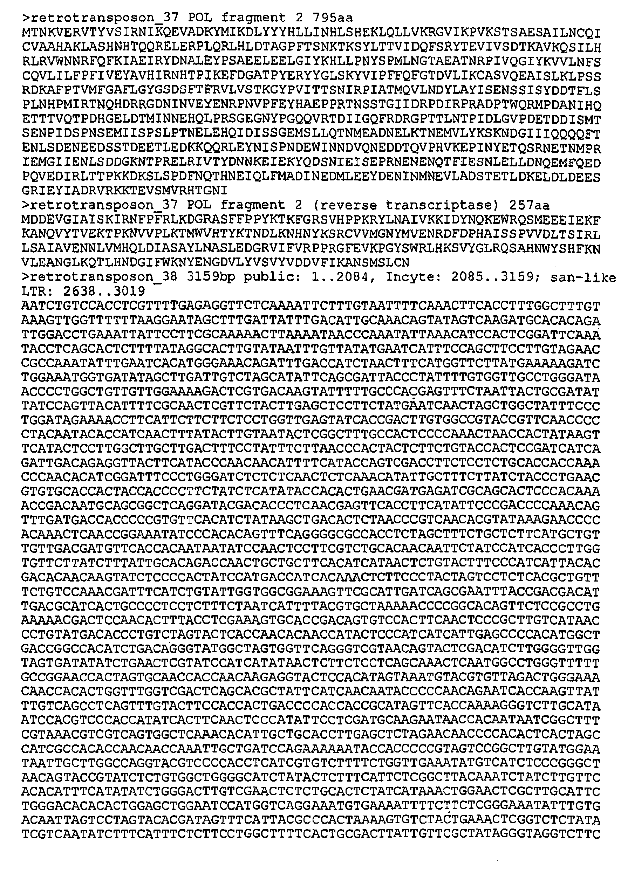

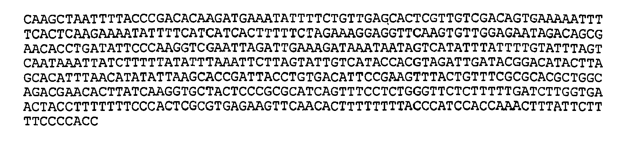

Figure 71 shows the nucleotide sequences of a further 38 retrotransposons.

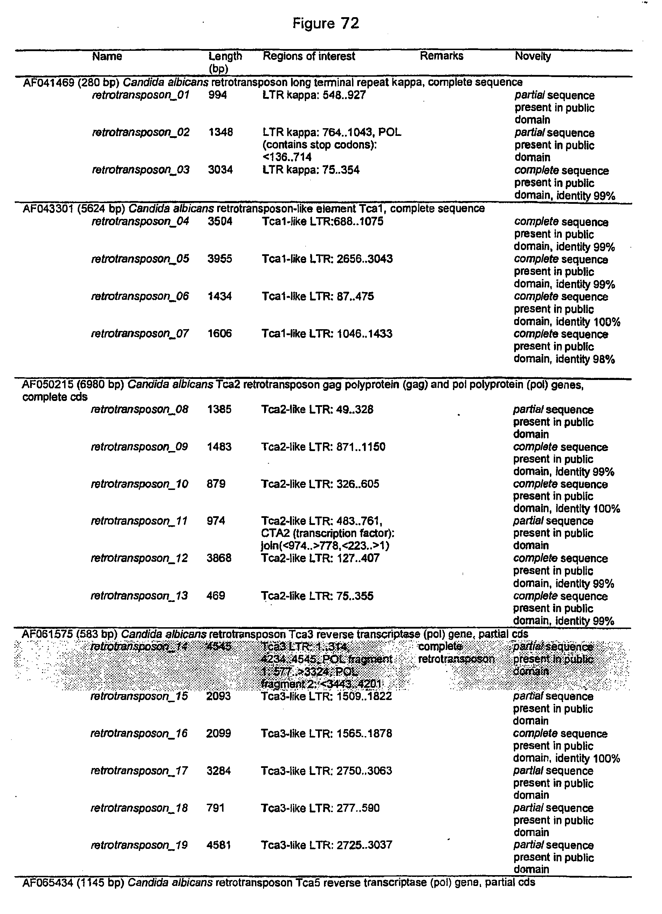

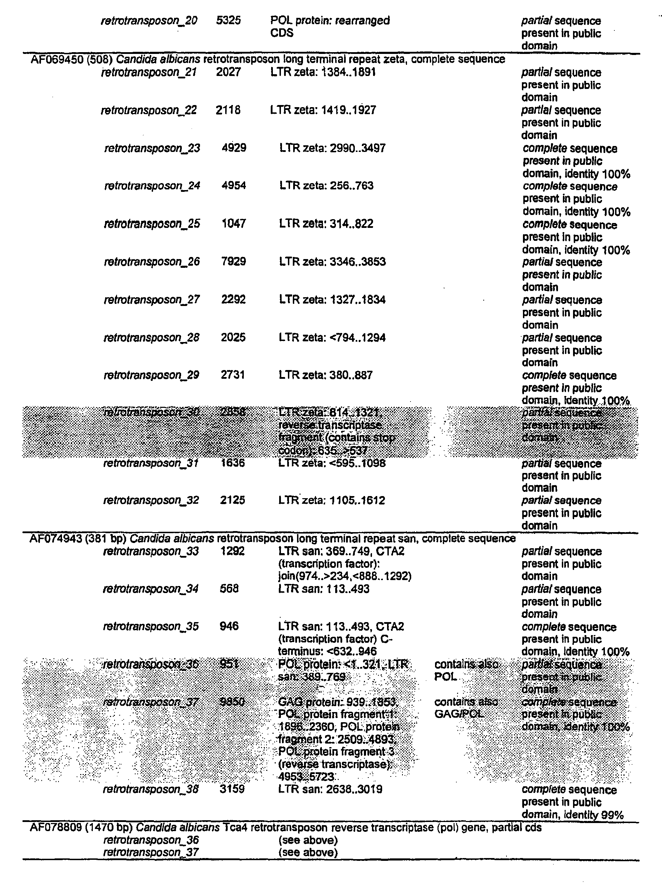

Figure 72 is an overview table of the additional 38 sequences.

### DETAILED DESCRIPTION OF THE INVENTION

Retrotransposons have many uses. Retrotransposons can be used as vectors for expression - either *in vivo* or *in vitro* of exogenous nucleic acid molecules. Retrotransposons thus can also be used for immunological, immunogenic or vaccine compositions, as well as for therapeutic compositions. Further, retrotransposons can be used for eliciting an immunological or immunogenic or protective immunological (vaccine) response, as well as a therapeutic response. Retrotransposons can be used for gene insertion and expression studies in cell culture, gene therapy, for the generation of transgenic animals, and in where traditional RNA retroviral vectors may be used (as well as in instances where such RNA retroviral vectors theoretically may be employed but may be considered unsafe or undesirable).

For instance, reference is made to: Gilbert et al., Biol Chem 380(3):299-303 (March 1999), Plebanski et al. Eur J Immunol 28(12):4345-55 (Dec. 1998), Garcia-Valcarcel et al. Vaccine 15(6-7):709-10 (Apr-May 1997), Poggeler et al. Biochem Biophys Res Commun 219(3):890-9 (Feb 1996); Kingsman et al. Ann NY Acad Sci 754:202-13 (May 1995); Adams et al. Mol Biotechnol 1(2):125-35 (Apr 1994); Adams et al. Int. Rev. lmmunol 11(2):133-41 (1994); Kingsman et al. Trends Biotechnol 9(9):303-9 (Sep 1991); Cook et al. Biotechnology 9(8):748-51 (Aug 1991); Kingsman et al. Vaccine 6(4):304-6 (1988); Malim et al. Nucleic Acids Res 15(18):7571-80 (1987); WO88/03169 WO92/07950; WO94/20608; and U.S. Patents Nos. 5,041,385, 5,354,674, 5,879,933, 5,969,126, 5,925,565, 5,885,971, 5,916,804, and 5,292,662 relate to retrotransposons and uses thereof, such as in introducing nucleotide sequences or nucleic acid molecules of interest into certain cells (expression systems, e.g. 72-kDa mitochondrial polypeptide), gene transfer, position-specific insertion vectors, vaccines (or immunological or immunogenic or therapeutic compositions; *in vivo* presentation of antigen or therapeutic or antigen or therapeutic delivery systems such as for antigens from *Plasmodium,* varicella zoster, HIV antigens, other viral antigens or for therapeutics such as interferon), purification or presentation or targeting vehicles, and in carriers or adjuvants, and the like. Indeed, these documents demonstrate that retrotransposons "can be administered safely in humans" (Plebanski et al., *supra*). Inventive nucleic acid molecules (DNA, RNA), amino acids, and retrotransposons can be used in the same fashion as previous retrotransposons; and thus, can be formulated and used in the fashion that retrotransposons are formulated in herein cited documents.

Thus, for instance, retrotransposons of the invention can be used to express nucleic acid molecules and can be formulated in compositions such as immunogenic, immunological or vaccine compositions. An immunological composition elicits an immunological response - local or systemic. The response can, but need not be, protective. An immunogenic composition likewise elicits a local or systemic immunological response which can, but need not be, protective. A vaccine composition elicits a local or systemic protective response. Accordingly, the terms "immunological composition" and "immunogenic composition" include a "vaccine composition" (as the two former terms can be protective compositions).

With respect to nucleic acid molecules and polypeptides of the invention, the nucleic acid molecules and polypeptides advantageously have at least about 65% or greater homology or identity or similarity with herein disclosed sequences, e.g., at least 70%, such as at least 75%, or at least 80% or advantageously at least 85%, for instance at least 90%, such as at least 95% or even 97% or 100%, similarity or homology or identity with herein disclosed sequences, such as (a) the LTR and/or POL region of Fig. 2B, or (b) the sequence illustrated in Fig. 2B, or (c) a nucleic acid sequence positioned between at least two long terminal repeats of the sequence of pCal as in GenBank accession number AF007776, or (d) a LTR and/or POL region of (c), or (e) any of sequences 1-28, or (f) any of retrotransposons 1-28, or (g) a sequence which hybridizes under standard stringent conditions to any of (a)-(f), or (h) a functional fragment of any of (a)-(g) (including subsequences discussed below).

Nucleotide sequence homology or identity or similarity can be determined using the "Align" program of Myers and Miller, ("Optimal Alignments in Linear Space", CABIOS 4, 11-17, 1988, incorporated herein by reference) and available at NCBI. Alternatively or additionally, the term "homology" or "identity", for instance, with respect to a nucleotide or amino acid sequence, can indicate a quantitative measure of homology between two sequences. The percent sequence homology can be calculated as (N*_{ref} -* N*_{dif}*)*100/N*_{ref}* , wherein N*_{dif}* is the total number of non-identical residues in the two sequences when aligned and wherein N*_{ref}* is the number of residues in one of the sequences. Hence, the DNA sequence AGTCAGTC will have a sequence similarity of 75% with the sequence AATCAATC (N*_{ref}* = 8; N*_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman, 1983 PNAS USA 80:726, incorporated herein by reference), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. RNA sequences within the scope of the invention can be derived from DNA sequences, by thymidine (T) in the DNA sequence being considered equal to uracil (U) in RNA sequences.

Additionally or alternatively, nucleotide and/or amino acid sequence similarity or identity or homology can be determined using the BlastP program (Altschul et al., Nucl. Acids Res. 25, 3389-3402, incorporated herein by reference) and available at NCBI. The following references (each incorporated herein by reference) also provide algorithms for comparing the relative identity or homology or similarity of amino acid residues of two proteins, and additionally or alternatively with respect to the foregoing, the teachings in these references can be used for determining percent homology or identity: Needleman SB and Wunsch CD, "A general method applicable to the search for similarities in the amino acid sequences of two proteins," J. Mol. Biol. 48:444-453 (1970); Smith TF and Waterman MS, "Comparison of Bio-sequences," Advances in Applied Mathematics 2:482-489 (1981); Smith TF, Waterman MS and Sadler JR, "Statistical characterization of nucleic acid sequence functional domains," Nucleic Acids Res., 11:2205-2220 (1983); Feng DF and Dolittle RF, "Progressive sequence alignment as a prerequisite to correct phylogenetic trees," J. of Molec. Evol., 25:351-360 (1987); Higgins DG and Sharp PM, "Fast and sensitive multiple sequence alignment on a microcomputer," CABIOS, 5: 151-153 (1989); Thompson JD, Higgins DG and Gibson TJ, "ClusterW: improving the sensitivity of progressive multiple sequence alignment through sequence weighing, positions-specific gap penalties and weight matrix choice, Nucleic Acid Res., 22:4673-480 (1994); and, Devereux J, Haeberlie P and Smithies O, "A comprehensive set of sequence analysis program for the VAX," Nucl. Acids Res., 12: 387-395 (1984).

Furthermore, as to inventive nucleic acid molecules, the invention comprehends codon equivalent nucleic acid molecules. For instance, if the invention comprehends "X" protein having amino acid sequence "A" and nucleic acid molecule "N" encoding protein X, the invention comprehends nucleic acid molecules that also encode protein X via one or more different codons than in nucleic acid molecule N.

In addition, as to inventive nucleic acid molecules, the invention comprehends nucleic acid molecules that hybridize under stringent conditions to herein disclosed nucleic acid molecules.

As to herein disclosed amino acid sequences, the invention comprehends nucleic acid molecules encoding the herein disclosed amino acid sequences, as well as nucleic acid molecules that hybridize under stringent conditions to nucleic acid molecules encoding herein disclosed amino acid sequences, as these nucleic acid molecules that hybridize under stringent conditions to nucleic acid molecules encoding herein disclosed amino acid sequences can provide proteins having similarity, homology or identity as herein discussed.

The disclosed nucleic acid sequences or portions or fragments thereof, e.g., subsequences comprising at least about 12 nucleotides in length, for instance, at least about 15, about 18, about 21, about 24 or about 27 nucleotides in length, such as at least about 30, about 33, about 36, about 39 or about 42 nucleotides in length, for example, a nucleic acid molecule of at least about 12 nucleotides in length such as about 12 to about 30, about 12 to about 50 or about 12 to about 60, or about 12 to about 75 or about 12 to about 100 or more nucleotides in length may be useful in hybridization, e.g., as probes or primers; for instance, to detect the presence or absence of *Candida albicans* in a sample or to determine the presence or absence of retrotransposons of the invention in a sample (amplification or detection of *Candida albicans* and/or inventive retrotransposons). The diagnostic applicability of nucleic acid molecules of the invention is a very real world use of the inventive nucleic acid molecules.

Further, the invention comprehends use of nucleic acid molecules and/or retrotransposons as vectors e.g., containing and/or expressing such an exogenous or heterologous (as to *Candida albicans* or as to the cell) or homologous (e.g., as to an organism or animal or cell) nucleic acid molecule, e.g., the use of a recombinant retrotransposon of the invention as a vector for delivery of a nucleic acid molecule that is exogenous or heterologous or even homologous to a cell, organism or animal, for instance, to elicit an immunogenic, immunological or protective immune response (e.g., from expression of an exogenous or heterologous nucleic acid molecule encoding an epitope of interest or an antigen) or as a therapeutic (e.g., to express a homologous nucleic acid molecule such as interferon or a gene that may need to be expressed in a particular individual).

Even further still, the invention comprehends use of the retrotransposons to contain and/or express a nucleic acid molecule deleterious to *Candida albicans,* e.g., so that the retrotransposon can become integrated into the *Candida albicans* genome and be lethal to *Candida albicans*; for instance, as a form of treatment against *Candida albicans.* The therapeutic, immunogenic, immunological or vaccine compositions can contain the retrotransposon in amounts and in carriers or vehicles analogous to those employed in herein cited documents.

The nucleic acids used for hybridization can, of course, be conveniently labelled by incorporating or attaching a marker, e.g., a radioactive or other marker. Such markers are well known in the art. The labelling of said nucleic acid molecules can be effected by conventional methods. The presence or expression of *Candida albicans* or of retrotransposons thereof (such as inventive retrotransposons) can be monitored by using a primer pair that specifically hybridizes and by carrying out a PCR reaction according to standard procedures. Specific hybridization of the above mentioned probes or primers preferably occurs at stringent hybridization conditions. A probe or primer can be any stretch of at least 8, preferably at least 10, more preferably at least 12, 13, 14, or 15, such as at least 20, e.g., at least 23 or 25, for instance at least 27 or 30 nucleotides in a herein defined nucleic acid molecule which are unique thereto. As to PCR or hybridization primers or probes and optimal lengths therefor, reference is also made to Kajimura et al., GATA 7(4):71-79 (1990), incorporated herein by reference.

with respect to hybridization, it is advantageously under high stringency conditions; and, hybridizing or hybridization under high stringency conditions can be synonymous with stringent hybridization conditions, terms which are well known in the art; see, for example, Sambrook, "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985; both incorporated herein by reference.

With respect to therapeutic, immunogenic, immunological and vaccine formulations, in addition and/or as an alternative to employing compositions and amounts of retrotransposon and routes of administration as in herein cited documents, it is noted that in classical formulations, e.g., classical immunogenic, immunological or vaccine or therapeutic formulations containing an antigen or epitope of interest (e.g., subunit formulations) or containing a biologically active therapeutic, typically contain the active ingredient in in an amount on the order of micrograms to milligrams, such as 5 micrograms to 500 milligrams, or, about 0.001 to about 20 wt%, preferably about 0.01 to about 10 wt%, and most preferably about 0.05 to about 5 wt%; and, in compositions involving a recombinant such as a recombinant viral vector expressing an antigen, epitope of interest or biologically active molecule, the vector is administered in an amount of about at least 10³ pfu; more preferably about 10⁴ pfu to about 10¹⁰ pfu, e.g., about 10⁵ pfu to about 10⁸ pfu, for instance about 10⁶ pfu to about 10⁸ pfu; and, in DNA plasmid compositions, suitable quantities of plasmid DNA such compositions can be 1 ug to 100 mg, preferably 0.1 to 10 mg, e.g., 500 micrograms, but lower levels such as 0.1 to 2 mg or preferably 1-10 ug may be employed. Accordingly, the recombinant retrotransposons of the invention can be administered in dosages sufficient to elicit a response analogous to compositions wherein the antigen, epitope of interest or biologically active molecule are directly present; or to have expression analogous to dosages in such compositions; or to have expression analogous to expression obtained *in vivo* by recombinant viral or DNA plasmid compositions.

Of course, for any composition to be administered to an animal or human, including the components thereof, and for any particular method of administration, it is preferred to determine therefor: toxicity, such as by determining the lethal dose (LD) and LD₅₀ in a suitable animal model e.g., rodent such as mouse; and, the dosage of the composition(s), concentration of components therein and timing of administering the composition(s), which elicit a suitable response, e.g., a suitable immunological or therapeutic response, such as by titrations of sera and analysis thereof, e.g., for antibodies or antigens or epitopes of interest or the therapeutic molecule. Such determinations do not require undue experimentation from the knowledge of the skilled artisan, this disclosure and the documents cited herein. And, the time for sequential administrations can be ascertained without undue experimentation using similar analyses. Thus, the amount of retrotransposon in the inventive compositions and the dosages administered can be determined by techniques well known to those skilled in the medical or veterinary arts and taking into consideration such factors as the particular antigen, eptitope of interest or therapeutic being expressed, the carrier, or diluent, any adjuvant (if present), the age, sex, weight, species and condition of the particular patient, and the route of administration.

Examples of compositions of the invention include liquid preparations for orifice, e.g., oral, nasal, anal, vaginal, peroral, intragastric, mucosal (e.g., perlingual, alveolar, gingival, olfactory or respiratory mucosa) etc., administration such as suspensions, syrups or elixirs; and, preparations for parenteral, subcutaneous, intradermal, intramuscular or intravenous administration (e.g., injectable administration), such as sterile suspensions or emulsions. Such compositions may be in admixture with a suitable carrier, diluent, or excipient such as sterile water, physiological saline, glucose or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, incorporated herein by reference, may be consulted to prepare suitable preparations, without undue experimentation.

Compositions of the invention, are conveniently provided as liquid preparations, e.g., isotonic aqueous solutions, suspensions, emulsions or viscous compositions which may be buffered to a selected pH. If digestive tract absorption is preferred, compositions of the invention can be in the "solid" form of pills, tablets, capsules, caplets and the like, including "solid" preparations which are time-released or which have a liquid filling, e.g., gelatin covered liquid, whereby the gelatin is dissolved in the stomach for delivery to the gut. If nasal or respiratory (mucosal) administration is desired, compositions may be in a form and dispensed by a squeeze spray dispenser, pump dispenser or aerosol dispenser. Aerosols are usually under pressure by means of a hydrocarbon. Pump dispensers can preferably dispense a metered dose or, a dose having a particular particle size.

Compositions of the invention can contain pharmaceutically acceptable flavors and/or colors for rendering them more appealing, especially if they are administered orally. The viscous compositions may be in the form of gels, lotions, ointments, creams and the like and will typically contain a sufficient amount of a thickening agent so that the viscosity is from about 2500 to 6500 cps, although more viscous compositions, even up to 10,000 cps may be employed. Viscous compositions have a viscosity preferably of 2500 to 5000 cps, since above that range they become more difficult to administer. However, above that range, the compositions can approach solid or gelatin forms which are then easily administered as a swallowed pill for oral ingestion.

Liquid preparations are normally easier to prepare than gels, other viscous compositions, and solid compositions. Additionally, liquid compositions are somewhat more convenient to administer, especially by injection or orally, to animals, children, particularly small children, and others who may have difficulty swallowing a pill, tablet, capsule or the like, or in multi-dose situations. Viscous compositions, on the other hand, can be formulated' within the appropriate viscosity range to provide longer contact periods with mucosa, such as the lining of the stomach or nasal mucosa.

Obviously, the choice of suitable carriers and other additives will depend on the exact route of administration and the nature of the particular dosage form, e.g., liquid dosage form (e.g., whether the composition is to be formulated into a solution, a suspension, gel or another liquid form), or solid dosage form (e.g., whether the composition is to be formulated into a pill, tablet, capsule, caplet, time release form or liquid-filled form).

Solutions, suspensions and gels, normally contain a major amount of water (preferably purified water) in addition to the retrotransposon. Minor amounts of other ingredients such as pH adjusters (e.g., a base such as NaOH), emulsifiers or dispersing agents, buffering agents, preservatives, wetting agents, jelling agents, (e.g., methylcellulose), colors and/or flavors may also be present. The compositions can be isotonic, i.e., it can have the same osmotic pressure as blood and lacrimal fluid.

The desired isotonicity of the compositions of this invention may be accomplished using sodium chloride, or other pharmaceutically acceptable agents such as dextrose, boric acid, sodium tartrate, propylene glycol or other inorganic or organic solutes. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl ceNulose, carbomer, and the like. The preferred concentration of the thickener will depend upon the agent selected. The important point is to use an amount which will achieve the selected viscosity. Viscous compositions are normally prepared from solutions by the addition of such thickening agents.

A pharmaceutically acceptable preservative can be employed to increase the shelf-life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the compositions must be selected to be chemically inert with respect to the retrotransposon. This will present no problem to those skilled in chemical and pharmaceutical principles, or problems can be readily avoided by reference to standard texts or by simple experiments (not involving undue experimentation), from this disclosure and the documents cited herein.

The compositions of this invention are prepared by mixing the ingredients following generally accepted procedures. For example the selected components may be simply mixed in a blender, or other standard device to produce a concentrated mixture which may then be adjusted to the final concentration and viscosity by the addition of water or thickening agent and possibly a buffer to control pH or an additional solute to control tonicity. Generally the pH may be from about 3 to 7.5. Compositions can be administered in dosages and by techniques well known to those skilled in the medical and veterinary arts taking into consideration such factors as the age, sex, weight, and condition of the particular patient or animal, and the composition form used for administration (e.g., solid vs. liquid). Dosages for humans or other mammals can be determined without undue experimentation by the skilled artisan, from this disclosure, the documents cited herein, the Examples below

The inventive retrotransposons can contain and preferably express at least one nucleic acid molecule encoding an antigen or epitope of interest. An epitope of interest is an immunologically relevant region of an antigen or immunogen or immunologically active fragment thereof, e.g., from a pathogen or toxin of veterinary or human interest. An epitope of interest can be from an antigen of a pathogen or toxin, or from another antigen or toxin which elicits a response with respect to the pathogen or toxin, e.g., from an antigen of a first human or veterinary pathogen or toxin that elicits a response with respect to the pathogen or toxin in question (such as a measles virus antigen or epitope of interest eliciting an immunological response against canine distemper). Thus, for instance, an epitope of interest can be from: a Morbillivirus antigen, e.g., a canine distemper virus or measles or rinderpest antigen such as HA or F; a rabies glycoprotein, e.g., rabies glycoprotein G; an avian influenza antigen, e.g., turkey influenza HA, Chicken/Pennsylvania/1/83 influenza antigen such as a nucleoprotein (NP) or influenza A/Jalisco/95 H5 hemagglutinin; a human influenza antigen such as HA and/or NA; a bovine leukemia virus antigen, e.g., gp51, 30 envelope; a Newcastle Disease Virus (NDV) antigen, e.g., HN or F; a feline leukemia virus antigen (FeLV), e.g., FeLV envelope protein; a rous associated virus antigen such as RAV-1 env; matrix and/or preplomer of infectious bronchitis virus; a Herpesvirus glycoprotein, e.g., a glycoprotein, for instance from feline herpesvirus, equine herpesvirus, bovine herpesvirus, pseudorabies virus, canine herpesvirus, HSV, Marek's Disease Virus, herpesvirus of turkeys (HVT) or cytomegalovirus; a flavivirus antigen, e.g., a Japanese encephalitis virus (JEV) antigen, a Yellow Fever antigen, or a Dengue virus antigen; a malaria (*Plasmodium*) antigen, an immunodeficiency virus antigen, e.g., a feline immunodeficiency virus (FIV) antigen or a simian immunodeficiency virus (SIV) antigen or a human immunodeficiency virus antigen (HIV) such as gp120, gp160; a parvovirus antigen, e.g., canine parvovirus; an equine influenza antigen; a poxvirus antigen, e.g., an ectromelia antigen, a canary pox virus antigen or a fowl pox virus antigen; an infectious bursal disease virus antigen, e.g., VP2, VP3, VP4; a Hepatitis virus antigen, e.g., HBsAg; a Hantaan virus antigen; a *C. tetani* antigen; a mumps antigen; a pneumococcal antigen, e.g., PspA; a *Borrelia* antigen, e.g., OspA, OspB, OspC of *Borrelia* associated with Lyme disease such as *Borrelia burgdorferi, Borrelia afzelli* and *Borrelia garinii;* a chicken pox (varicella zoster) antigen. Of course, this is intended as exemplary, as the epitope of interest expressed by an inventive retrotransposon can be derived from any antigen of any veterinary or human pathogen or toxin; and, the recombinant retrotransposon can express express an antigen of any veterinary or human pathogen or toxin. Thus, it is envisioned that the inventive recombinant retrotransposon contain at least one nucleic acid molecule encoding at least one antigen or epitope of interest.

With respect to DNA encoding epitopes of interest, antigens and/or therapeutics, attention is directed to documents cited herein, see, e.g., documents cited *supra* and documents cited *infra,* for instance: U.S. Patents Nos. 5,174,993 and 5,505,941 (e.g., rabies glycoprotein (G), gene, turkey influenza hemagglutinin gene, gp51,30 envelope gene of bovine leukemia virus, Newcastle Disease Virus (NDV) antigen, FeLV envelope gene, RAV-1 env gene, NP (nucleoprotein gene of Chicken/Pennsylvania/1/83 influenza virus), matrix and preplomer gene of infectious bronchitis virus; HSV gD); U.S. Patent No. 5,338,683 (e.g., DNA encoding Herpesvirus glycoproteins, *inter alia);* U.S. Patents Nos. 5,494,807, 5,756,103, 5,762,938 and 5,766,599 (e.g., DNA encoding antigens from rabies, Hepatitis B, JEV, YF, Dengue, measles, pseudorabies, Epstein-Barr, HSV, HIV, SIV, EHV, BHV, HCMV, canine parvovirus, equine influenza, FeLV, FHV, Hantaan, *C. tetani,* avian influenza, mumps, NDV, *inter alia*); U.S. Patents Nos. 5,503,834 and 5,759,841 (e.g., Morbillivirus, e.g., measles F, hemagglutinin, *inter alia*); U.S. Patent No. 4,722,848 (e.g., HSV tk, HSV glycoproteins, e.g., gB, gD, influenza HA, Hepatitis B, e.g., HBsAg, *inter alia*); U.S. Patents Nos. 5,514,375, 5,744,140 and 5,744,141 (e.g., flavivirus structural proteins); U.S. Patents Nos. 5,766,598 and 5,863,542 (e.g., Lentivirus antigens such as immunodeficiency virus antigens, *inter alia*); U.S. Patents Nos. 5,658,572 and 5,641,490 (e.g., IBDV antigens, *inter alia*); U.S. Patent No. 5,833,975 (e.g., cytokine and/or tumor associated antigens, *inter alia*); U.S. Patents Nos. 5,688,920, and 5,529,780 (e.g., canine herpesvirus antigens), PCT publication WO 96/3941 (e.g., cytomegalovirus antigens); and U.S. Patents Nos. 5,756,101 and 5,766,597 (*Plasmodium* antigens). Thus, the skilled artisan can obtain DNA or a nucleic acid molecule for including in an inventive retrotransposon, without any undue experimentation.

As to epitopes of interest, one skilled in the art can determine an epitope or immunodominant region of a peptide or polypeptide and ergo the coding nucleic acid molecule or DNA therefor from knowledge in the art, without undue experimentation, for instance, from the amino acid of the peptide or polypeptide and corresponding nucleic acid molecule or DNA sequences coding for the peptide or polypeptide, as well as from the nature of particular amino acids (e.g., size, charge, etc.) and the codon dictionary, *inter alia;* and, in respect to this, attention directed to documents cited herein, including U.S. Patent No. 5,955,089. Accordingly, one skilled in the art can obtain an epitope of interest and a nucleic acid molecule coding therefor without any undue experimentation.

Thus, the invention comprehends an immunogenic, immunological, vaccine or therapeutic composition comprising an inventive retrotransposon of the invention wherein the retrotransposon includes a nucleic acid molecule encoding at least one antigen or epitope of interest or therapeutic molecule. The invention further comprehends a method for inducing an immunological or immune or protective immune or therapeutic response comprising administering to a host such as an animal or human an inventive retrotransposon of the invention wherein the retrotransposon includes a nucleic acid molecule encoding at least one antigen or epitope of interest or therapeutic molecule.

The retrotransposon can have expression in any suitable cell, such as a eukaryotic cell; for instance, fungus or yeast cells such as *Saccharamyces cerevisiae* cells, *Saccharamyces pastorianus* cells, *Candida albicans* cells, vertebrate cells such as fish cells (e.g., shark, salmon, rainbow trout, zebrafish, herring, mackerel cells), amphibian cells (e.g. frog, toad, salamander cells), bird or avian cells (e.g. chicken, turkey, duck, pigeon, dove cells), reptile cells (e.g. snake such as cobra), and mammalian cells (e.g., human, rabbit, hamster, mouse, rat, primate, cells such as VERO, HeLa cells, Chinese hamster ovary (CHO) cell lines, W138, BHK, COS-7 293, MDCK; invertebrate cells such as land invertebrate cells, for instance, insect cells, e.g., lepidopteran cells such as *Spodoptera* (e.g., *Spodoptera frugiperda, Trichoplusia* (e.g., *Trichoplusia ni*), dipteran such as mosquito (e.g. *Culicidae*) cells, fly cells (e.g. *Drosophila*); e.g., typical cells that are used with eukaryotic replicable expression vectors such a *S. frugiperda* cells, VERO cells, MRC-5 cells, SCV-1 cells COS-1 Cells, NIH3T3 cells, mouse L cells, HeLa cells and the like.

The invention further comprehends methods for treating *Candida albicans* comprising administering a recombinant retrotransposon of the invention that includes a nucleic acid molecule that is lethal or deleterious to *Candida albicans,* as well as recombinant retrotransposons that include a nucleic acid molecule that is lethat or deleterious to *Candida albicans.* For instance, a retrotransposon of the invention can disrupt or interfere with a gene essential to the viability of *Candida albicans*; for instance, an inventive retrotransposon can disrupt or interfere with CaSNF1 (Petter et al. Infect Immun 65(12):4909-17 (1997)) and/or H(+)-ATPase (Perlin et al. Ann NY Acad Sci 834:609-17 (1997)) and/or the *Candida albicans* 37 kDa polypeptide that appears to be a ribosomal protein (Montero et al. Microbiology 144(Pt4):839-47 (1998) and/or a *Candida albicans* topoisomerase gene (Keller et al. Biochem J 324(Pt1):329-39 (1997) and/or a yeast essential gene (*cf.* Hanes et al. Yeast 5:55-72 (1989); and/or an inventive retrotransposon can express a candidacidal antibody (Conti et al. J Infect Dis 177(3):807-11 (1998)) and/or an antifungal (Ben-Josef et al. J Antibiot (Tokyo) 50(11):937-43 (1997)) and/or an antibody-like molecule (Tournay et al. DNA Cell Biol 15(8):617-24 (1996)).

Furthermore, in view of the foregoing and the documents cited herein, the invention comprehends a process for the transfer and expression of at least one gene into a cell *in vitro* or *in vivo* comprising the steps of: (a) isolating the gene; (b) introducing the gene into an inventive retrotransposon (a retrotransposon as herein described); (c) introducing said hybrid retrotransposon into a donor cell and allowing the donor cell to package and transmit said hybrid retrotransposon into a virion; (d) transferring said virion to a recipient cell wherein said hybrid retrotransposon replicates by reverse transcription and may also be integrated into the recipient cell's genome; (e) expressing said hybrid retrotransposon as RNA and/or protein from either at least one internal promoter and/or from said retrotransposon long terminal repeat promoter or both (or a promoter as herein described); and (f) screening or selecting for the phenotype of said hybrid retrotransposon. The retrotransposon can contain genetic material encoding at least one dominant selectable marker; e.g., a selectable marker is selected from the group consisting of aminoglycoside phosphotransferase (neo, G418, APH), dihydrofolate reductase (DHFR), hygromycin-B-phosphotransferase (HPH), thymidine kinase (TK), xanthine-guanine phosphoribosyltransferase (XGPRT, gpt), chloramphenicol acetyltransferase (CAT) and luciferase. In the process multiple cellular movable genetic elements can be introduced and expressed as RNA; for instance, the multiple cellular movable genetic elements can be introduced and expressed in tandem in RNA; or, the multiple cellular movable genetic elements can be introduced and expressed as separate transcriptional units within a single cell or organism. And, the gene can encode a peptide, antibody, antigen, hormone, or drug not normally expressed in the cell, at biologically significant levels. (*Cf.* U.S. Patent 5,354,674.)

Similarly, the invention can comprehend polycistronic vector for the expression of one or more or a plurality, e.g., at least two or three polynucleotide sequences comprising a promoter operably linked to a nucleotide sequence comprising elements encoding one, or two or three, or more proteins, and an inventive retrotransposon or portion thereof; the retrotransposon or portion thereof can act as an internal ribosome entry site. The invention thus further comprehends a method of incorporating a DNA encoding a protein of interest into a cell in vitro comprising transforming said cell with this vector. The vector can be a plasmid vector or a viral vector; for instance, a vector from a virus selected from the group consisting of poxvirus, adenovirus, baculovirus, herpesvirus, adeno-associated virus, and retrovirus. The vector can include an an encapsidation sequence. A viral particle can comprise the vector. An isolated cell can comprise the vector. And, the vector can be in a composition. (*Cf.* U.S. Patent No. 5,925,565.) Likewise, the invention comprehends other methods, products, compositions and the like that are analogous to those in documents cited herein, but wherein retrotransposons, nucleic acid molecules, amino acid molecules (proteins, polypeptides) and promoters disclosed herein are employed.

Further, as discussed, the invention can include an immunological, or immunogenic, or vaccine or therapeutic composition comprisng a carrier or diluent and an inventive expression vector wherein the vector expresses an antigen, or an epitope of interest or a therapeutic. The composition can be an immunological, immunogenic or vaccine composition when the vector expresses an antigen or an epitope of interest (*see supra*). The composition can be a therapeutic composition when the vector expresses a therapeutic (e.g., interferon, a cytokine, a tumor associated antigen, etc.; *see supra*). And, the invention can include a method for inducing an immunological response in a host including an animal (e.g., mammal) or a human comprising administering to the host the immunological, immunogenic or vaccine composition; as well as a method for inducing a therapeutic response in a host including an animal (e.g., mammal) or human comprising administering to the host the therapeutic composition. As noted in many documents cited herein, an immunological or immunogenic response can be useful; for instance, in generating antibodies which are themselves useful in diagnostic and other uses.

Accordingly the invention has many embodiments and uses that can be practised without undue experimentation from this disclosure and the knowledge in the art, for instance as exemplified by documents cited and incorporated herein by reference.

A better understanding of the present invention and of its many advantages will be had from the following non-limiting Examples, given as a further description of the invention and as illustration of it.
Plasmids carrying both the retrotransposon and other genetic elements can be assembled by in vitro molecular genetic manipulations. Such plasmids should, for ease of manipulation, be capable of growing both in *E. coli* and in yeasts. Such plasmids should carry some suitable marker (such as ADE2) which can be selected for following yeast transformation. The presence of such plasmids can be detected and selected for following transformation into an Ade-(Adenine auxotrophic) yeast. Detection or selection consists of allowing the yeasts to attempt to grow on media without say adenine. The parental auxotrophic yeast will not grow whereas a transformant carrying say a plasmid with the ADE2 gene will grow. The transformed culture can be maintained on a medium without adenine and this will select for the retention of the plasmid strains carrying the plasmid (maintained by say selection on medium without adenine) can be used to perform the various activities described in this patent. For example they could be plated on a medium which would select for integration events (say by selecting for URA3 +).

### EXAMPLES

### MATERIALS AND METHODS

### Strains and culture conditions

The isolate iB65, precursor to the *Candida albicans* strain currently under investigation (hOG1042), was isolated as a *met2* heterozygote from an Otago University intermediate biology student in 1983. It was subsequently mutagenised with UV radiation (2) and N-methyl-N-nitro-N nitrosoguanidine (Poulter *et al* 1981) to produce five strains - hOG758, hOG759, hOG760, hOG761 and hOG762 - which are all *met2* homozygotes and also auxotrophic for adenine. hOG1042 is an *ade2*/*ade2 MET2*/*met2* revertant of hOG762. The strains were grown at 27°C or 37°C in YPD medium (1% yeast extract, 2% peptone and 2% glucose).

Other *Candida albicans* strains analyzed were F16932 (Poulter, unpublished), SA40 (Agatensi *et al* 1991), SC5314 (Gillum *et al* 1984), and SGY269 (Kelly *et al* 1987). Other *Candida* species analyzed were *C. pseudotropicalis* (CDC B2455), *C. tropicalis* (CDC B397), *C. parapsilosis* (CDC MCC 499), all from the National Health Institute, Porirua, New Zealand, and *C. maltosa* (CHAU1).

### Enzymes

Agarase (GELase™) and phosphatase (HK™ Phosphatase) were purchased from Epicentre Technologies, USA. T4 DNA ligase, Expand high fidelity PCR system, RNase A, and DNase I, Proteinase K, Klenow, and restriction endonucleases were purchased from Boehringer Mannheim GmbH, Biochemica, West Germany. Vent® polymerase was purchased from New England Biolabs, USA. Zymolyase 100T was from Seikagaku Corporation, Tokyo.

### Nucleic acid manipulations

*C. albicans* genomic DNA was prepared essentially by the method of Cryer *et al.* (1975). DNA was separated on 1% agarose gels using TAE buffer. Gel purification of DNA was from low melting point agarose using agarase. Bacterial plasmids were prepared by a modified alkaline lysis/PEG precipitation from Applied Biosystems, Inc. Polymerase chain reactions were performed using an Autogene II Programmable cycling water bath from Grant Instruments (Cambridge) Ltd. Temperature cycling consisted of 35 cycles of 95°C for 1 min, 45°C for 1 min and 72°C for 1 min. PCR products were purified for sequencing using the QlAquick PCR Purification Kit from QIAGEN GmbH, Hilden.

### Sequencing and nucleotide analysis

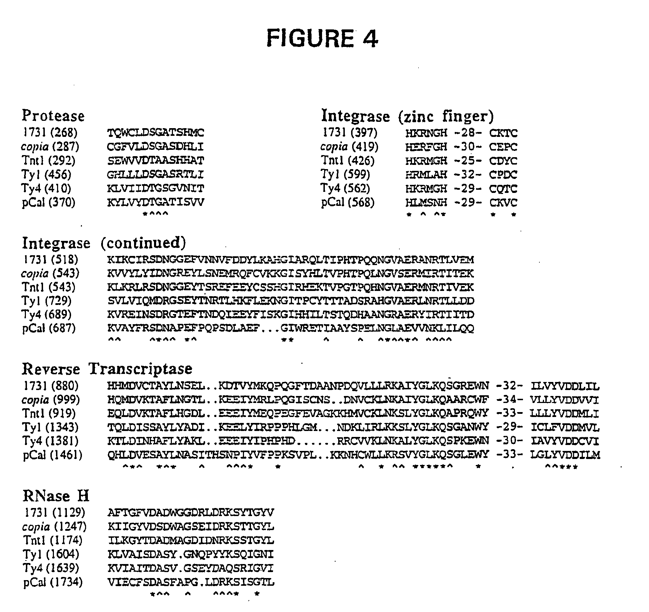

Sequencing was performed using a combination of subcloning and specifically designed oligonucleotide primers. The sequences were determined on an automated DNA sequencer (Applied Biosystems 373A DNA sequencer). Oligonucleotides were purchased from Macromolecular Resources, Fort Collins or from the DNA Synthesiser, Dunedin. Sequences were edited using SeqEd 1.0.3 (Applied Biosystems). Sequence contigs were assembled using VTUTIN 5.21 (Stockwell 1985) and HOMED 5.14 (Stockwell and Petersen 1987). Other sequence analysis was carried out using version 8 of the University of Wisconsin GCG Sequence Analysis Package (Devereux *et al* 1984). The open reading frames were translated using the non-standard *C. albicans* genetic code (CUG codes for serine instead of leucine) (Santos and Tuite 1995 and White *et al* 1995). Sequences for the alignments in figure 4 and for the phylogenetic analysis were obtained from the Genbank database using the following accession numbers: 17.6 - A03971, 1731 - S00954, CfT-I - Z11866, copia - A03324, dong - L08889, gypsy - B25666, HIV1 - K02013, Hopscotch - U12626, jockey - JT0396, MMLV - A03956, Osser - S32437, RSV - S26418, Ta1 - S05465, Tf1 - A36373, Tnt1 - S04273, Tom S34639, Tst1 - X52387, Tx1 - B32494, Ty1 - B28097, Ty2 - S45842, Ty3 - S53577, Ty4 - P47024 and Ty5 - U19263. The trees were constructed using the UPGMA (unweighted pair group method using arithmetic averages), Neighbor-Joining and Parsimony methods available in the PHYLIP package (Felsenstein 1989). Bootstrapping was performed using SEQBOOT and consensus trees were derived using CONSENSE, both programs also from PHYLIP.

The nucleotide sequence of pCal has been submitted to Genbank and assigned the accession number AF007776.

***Candida* nucleic acid isolations.** For DNA isolations, cells were grown at 27°C or 37°C to late log/early stationary phase. DNA for the hOG759 library was then prepared essentially as in Cryer et al. 1975. DNA for the Southern blots and PCRs was prepared as described by Philippsen et al. 1991. To determine the copy number of TCa2 in hOG759 and hOG1042 it was found to be necessary to purify the chromosomal DNA away from the abundant pCal molecules. To do this DNA samples from cells grown at 27°C were electrophoresed on 0.7% agarose gels. The high molecular weight chromosomal DNA was then cut out of the gel under long wavelength UV light. The DNA was then extracted from the gel by spinning through siliconized glass wool in microcentrifuge tubes for 5 min at 6500rpm and 2 min at 8000rpm. DNA was precipitated by adding an equal volume of 5M ammonium acetate and 2 volumes of cold 96% ethanol. The tubes were mixed and then centrifuged at 13000rpm for 30 min. Pellets were washed in 70% ethanol, dried, resuspended in 10mM Tris-Cl, pH 7.5; 1mM EDTA and stored at - 80°C.

RNA extractions were performed as follows. Cells were grown in YPD medium overnight at either 27°C or 37°C then a volume of culture containing ~2.5X10⁸ cells was transferred to Falcon tubes. The cells were spun down, washed once in 1ml RNA buffer [0.5M NaCl; 200mM Tris-Cl, pH 7.5; 10mM EDTA - treated with diethyl pyrocarbonate (DEPC)], then resuspended in 300µl RNA buffer and transferred to eppendorf tubes. To these tubes was added 200µl RNase-free glass beads (425 to 600 µm diameter), 150µl phenol equilibrated with RNA buffer and 150µl chloroform-isoamylalcohol (24:1). The tubes were then vortexed in 30 sec bursts, with intervals on ice, for a total of 5 min vortexing. 30µl of 10% SDS was then added and the tubes were vortexed for a further 2 min. The organic and aqueous phases were then separated by centrifuging for 1 min at 13000rpm. The aqueous phase was then extracted once more from 150µl phenol, 150µl chloroform-isoamylalcohol. RNA was precipitated by adding 2 volumes of cold absolute ethanol and holding at -80°C for 20 min. The tubes were then centrifuged for 10 min at 13000rpm; the resulting RNA was washed in 70% ethanol, dried, resuspended in 50µl DEPC-treated H₂O and stored at -80°C.

RNA preparations were tested for RNase-sensitivity by treating them with 0.2mg.ml⁻¹ RNase A for 30 min at 37°C.

Southern blotting. DNA was electrophoresed in 0.75% agarose with TAE buffer in the presence of 0.5µg.ml⁻¹ ethidium bromide. When the DNA fragments were sufficiently separated, the gels were photographed under UV light followed by a 5 min wash in sterile H₂O. The DNA was then capillary transferred to Hybond-N + nylon membranes (Amersham) using 0.4M NaOH as the transfer solution. Following transfer the membranes were rinsed in 2XSSC and stored at 4°C until hybridization. DNA fragments to be used as probes were isolated by restriction digestion of plasmid clones followed by gel purification of the appropriate fragment as described above for genomic DNA. The locations of the probes used are shown in Fig. 11A. Probes were radiolabelled with λ³²PdCTP by random-primed labelling using Hexanucleotide Mix from Boehringer Mannheim. Prior to hybridization, probes were denatured by heating in a boiling water bath for 10 min. Hybridization was carried out in sealed plastic bags in a shaking water bath. Most hybridizations were performed at 65°C, but some lower stringency ones were at 55°C. The hybridization buffer was similar to that of Church and Gilbert 1984, but without the BSA (0.36M Na₂HPO₄, 0.12M NaH₂PO₄, 1mM EDTA, 7% SDS). Membranes were prehybridized in this buffer for 2 hours, the denatured probe was then added in 5ml of fresh buffer and hybridization was allowed to proceed for 16-20 hours. Post-hybridization washes consisted of two rinses in 2XSSC at room temperature followed by stringency washes in 0.2XSSC (or 0.4XSSC for low stringency), 0.1% SDS at the hybridization temperature. Finally membranes were rinsed in 2XSSC then exposed to Kodak X-Omat AR film at -80°C using an intensifying screen. Membranes were stripped for reprobing by rinsing in H₂O for 1 min, followed by two washes in 0.2M NaOH, 0.1 % SDS at 37°C, and then a final rinse in 2XSSC.

Northern blotting. Briefly, approximately equal amounts of total RNA were denatured in formamide-formaldehyde at 65°C then separated on 1% agarose, 2.2M formaldehyde gels in MOPS running buffer (40mM 3-[N-Morpholino]propanesulfonic acid, pH 7.0; 10mM sodium acetate; 1mM EDTA). Following electrophoresis, gels were washed twice, 20 min per wash, in RNase-free H₂O. RNA was then capillary transferred for 5 hours to Hybond-N+ membranes using 8mM NaOH as the transfer solution. The membranes were then rinsed in 2XSSC, 0.1% SDS for 5 min. The RNA sides of the membranes were then exposed to UV light for 45-60 sec and the membranes were stored at 4°C until hybridization. Probes were radiolabelled double-stranded DNAs prepared as described above for Southern blotting. Hybridization was performed at 42°C in FPH buffer (5XSSC, 5°Denhardt's solution, 50% formamide and 1% SDS). Membranes were prehybridized for 2 hours in this buffer; the denatured probe was then added in 5ml of fresh FPH buffer and hybridization was left to proceed for about 20 hours. After hybridization the membranes were washed twice, 5 min per wash, in 2XSSC at room temperature, twice, 5 min per wash, in 0.2XSSC, 0.1% SDS at room temperature and twice, 15 min per wash, in 0.2XSSC, 0.1 % SDS at 42°C. Finally, the membranes were rinsed in 2XSSC and exposed to x-ray film at - 80°C.

The films from the Southerns and Northerns were scanned using a Bio-Rad GS-670 imaging densitometer. Relative band intensities were determined using Molecular Analyst version 2.1. The brightness/contrast of the scans was adjusted for printing using Adobe Photoshop 3.0.

**Recombinant DNA manipulations.** A λ-library of *Bam*Hl-digested hOG759 DNA was constructed using the LambdaGEM-11 *Bam*Hl Arms Cloning System from Promega, according to the manufacturer's instructions. The library was screened using the DIG DNA Labelling and Detection Kit from Boehringer Mannheim. Probes were derived from clones of pCal. Recombinant λ DNA was prepared according to the protocol accompanying the lambda cloning system from Promega. Bacterial plasmids were prepared using an alkaline lysis-polyethylene glycol precipitation method from Applied BioSystems. Sequencing was performed using a combination of subcloning and specifically designed oligonucleotide primers. Sequences were determined on an ABI 373A DNA Sequencer and edited using SeqEd 1.0.3. Sequences were aligned and assembled into contigs using the programs available in the University of Wisconsin GCG package and HOMED 5. PCRs were performed on an Autogene II programmable cycling water bath from Grant Instruments, Cambridge. Primers were synthesized on an ABI 380B oligonucleotide synthesizer. Primers used for the amplification of the 5' regions of TCa2 retrotransposons from various *C. albicans* strains were as follows: Cal1.2 5'-AGTGAGCTCTGTTGGTTTGTGCACT-3'; Cal2.2 5'-GCGTCTAGAAATTCTGTACCTTC-3'. Together these primers can amplify the first 400 bp of the retrotransposon including the complete left LTR. Primers from the genomic regions flanking the integrated copy of TCa2 were: TGFS-L, 5'-CTACATAGGATGACTCAC-3'; and TGFS-R, 5'-ATCCAAGTCTGAAAGATC-3'. Temperature cycling consisted of 35 cycles of 95°C for 1 min, 45°C for 1 min, and 72°C for 1 min. PCR products were purified prior to cloning using Strataclean resin (Stratagene, La Jolla, CA.).

**Nucleotide sequence accession numbers.** The nucleotide sequence of the TCa2 fragment from hOG759 with the perfect 32-bp minus-strand primer-binding site, and that of the integrated TCa2 element, have been submitted to GenBank and assigned accession numbers AF030556 and AF050215, respectively.

### EXAMPLE 1

### Cloning and mapping

Some uncut genomic DNA prepared from *Candida albicans* strain hOG1042 was analysed on an agarose gel and a distinct band running at about 6.5kb was found (Figure 1). Such a band had never previously been reported from any *Candida* strain or species. To analyse this feature the band was extracted from an agarose gel and tested to see if it could be cut with restriction enzymes. A number of enzymes cut the band into smaller fragments which indicated that it was made up of double-stranded DNA. At this point the band was named pCal (plasmid of *Candida albicans*). The restriction digests allowed the construction of a simple restriction map of pCal. This work revealed that pCal was linear, with a *Pst*1 site about 1kb from one end, an *Eco*R1 site about 1kb from the opposite end and an *Asp*718 site near the middle. To permit further analysis the fragments of pCal produced with *Asp*718 were cloned into the *Asp*718 site of pUC19. Five clones were isolated and each was found to contain just a single *Asp*718 site, the other apparently destroyed during the cloning, as expected. Three of the clones contained a *Pst*1 site and two contained an *Eco*R1 site.

### EXAMPLE 2

### Nucleotide sequence of pCal

The five plasmids containing the pCal fragments were all sequenced from both ends in the hope of finding an identifiable feature which would provide an insight into the nature of pCal. The first remarkable features to be found were 280bp direct repeats. The existence of these direct repeats suggested that pCal was likely to be a retrotransposon. As no other retrotransposon had ever been found existing at a high copy number in a free, linear, dsDNA form we determined the complete sequence of pCal. Therefore, the three clones of pCal carrying the *Pst*1 site and one of the two clones carrying the *Eco*R1 site were completely sequenced. In addition a region of pCal spanning the central *Asp*718 site used in the cloning was amplified by PCR and each strand was sequenced. This analysis confirmed that there was only one *Asp*718 site and that therefore the clones that we had of each half of pCal truly represented adjacent fragments.

Assembly of the 6426bp pCal sequence revealed many characteristics typical of a retrotransposon. An obvious feature was the identical 280bp long terminal direct repeats (LTRs). The borders of these LTRs are short, imperfect, inverted repeats 6bp long - 5'-TGTTGG....CCATCA-3'. This repeat is very similar to that found in the LTRs of TCa1 (TGTTCG), Ty3 (TGTTGTAT), 1731 (TGTTG) and copia (TGTTGGAAT). Within the LTRs putative TATA boxes and a polyadenylation signal were identified. These and other features are highlighted on the sequence of pCal in Figure 2.

The minus-strand primer-binding [(-)PBS] was found adjacent to the left LTR and consists of the sequence GATTAGAAGTC. This is very similar to the (-)PBS of TCa1, GATTAGAAG, but complements 11 bases, rather than 9 of a possible tRNA^{Arg} cleavage product. The *S. cerevisiae* retrotransposons Ty1, Ty2 and Ty3 have been found to contain additional sequences 3'. to the (-)PBS which complement additional regions of the primer tRNA. These additional sequences are likely to be involved in the packaging of the primer tRNA within the VLP, An additional region of complementarity is also apparent in pCal - the sequence GCGTTG, approximately 30 nucleotides 3' of the (-)PBS, perfectly complements the sequence CAACGC (bases 19-24) in the primer tRNA^{Arg} fragment (Figure 3).

A plus-strand priming site or polypurine tract (PPT) was found immediately upstream of the right LTR. It is very similar to the PPT described for TCa1. A second sequence very similar to the 3' PPT was found near the middle of pCal (bases 3455 - 3465). Internal PPTs which function as plus-strand priming sites have been identified in Ty1 and HIV1 and may serve to speed up the reverse transcription process. The two pCal PPTs and that of TCa1 are compared in Figure 3. We believe that the internal PPT of pCal may also be serving as a site for plus-strand initiation during the reverse transcription process.

TCa1 and pCal have very similar (-)PBSs and PPTs and very similar borders to their LTRs. A comparison of the remainder of the LTRs, however, revealed that the similarity did not extend beyond these regions.

### EXAMPLE 3

### The open reading frames

Two long open reading frames were found in pCal, the first 972bp (324aa) and the second 4728bp (1576aa) long. Conserved motifs from the four *pol* proteins - protease, integrase, reverse transcriptase and RNase H - were identified in the second ORF. The order of these motifs (as listed above) places pCal within the Ty1/*copia* group of retrotransposons.The pCal motifs are shown compared to those of other Ty1/*copia* elements in Figure 4. No conserved motifs were found in the first ORF but it is similar in size and position to the *gag* genes of other retroelements. Retroelement *gag* genes in general are known to be extremely variable and it is not uncommon for no identifiable conserved features to be present.

Unlike other retrotransposons, the *gag* and *pol* ORFs of pCal are in the same phase separated only by a UGA termination codon. This arrangement is similar to what has been found for mammalian type C retroviruses such as Moloney murine leukemia virus (MMLV). In MMLV a UAG termination codon separates the *gag* and *pol* ORFs. Translation of the *pol* ORF occurs via the occasional read-through suppression of the UAG codon. This suppression requires an 8bp purine-rich sequence immediately downstream of the stop codon and an adjacent pseudoknot (a pseudoknot being a structural element of RNA formed upon the annealing of the nucleotides of a loop region with nucleotides outside of that loop) (ten Dam et al 1982). In pCal, an 8bp purine-rich sequence, AAAACAGG, lies immediately downstream of the UGA codon and this is followed immediately by a potential pseudoknot. These features are illustrated in Figure 5. A further unusual feature is apparent slightly upstream of the UGA codon. It consists of four tandem repeats of the sequence GAAAAA. The role, if any, of this distinctive sequence in the ribosomal *gag-pol* transition is unclear.

### EXAMPLE 4

### Copy number of pCal

The copy numbers of other extrachromosomal elements from lower eukaryotes have been determined. For instance, the 2 micron circle plasmids of *Saccharomyces* species exist at 50-100 copies per cell and the Ddp elements of *Dictyostelium discoideum* exist at 50-300 copies per cell. When uncut genomic DNA from the *Saccharomyces* and *Dictyostelium* species containing these elements is run out on agarose gels the extrachromosomal elements appear as distinct bands running ahead of the chromosomal DNA. The intensity of the bands relative to that of the chromosomal DNA is indicative of the elements' copy numbers. These elements are comparable in size to pCal and the host genomes are similar in size to that of *C. albicans.* Therefore, using the relative intensity of extrachromosomal and chromosomal DNA in *Saccharomyces* and *Dictyostelium* as a guide, we estimated, from the relative intensity of pCal and hOG1042 chromosomal DNA, that pCal exists at 50-100 copies per cell.

### EXAMPLE 5

### Phylogenetic analysis

In an attempt to gain a better understanding of the relationship of pCal to other retroelements a phylogenetic tree of a number of retrotransposons and retroviruses was constructed. The data used in the analysis were the predicted amino acids of the seven conserved domains of reverse transcriptase identified by Xiong and Eickbush. The tree was constructed using the UPGMA method within the PHYLIP package and is shown in Figure 6. It is generally consistent with the trees constructed earlier by Xiong and Eickbush. For instance, the retroviruses and the gypsy-type retrotransposons are closer to each other than to the Ty1/*copia* retrotransposons. Within the retroviral group HIV1 and RSV are closer to each other than to MMLV and within the Ty3/*gypsy* group CfT-1 and Tf1 form a group as do the *Drosophila* elements 17.6, Tom and *gypsy.* The tree placed pCal with the Ty1/*copia* elements. This placement of pCal is in agreement with the fact that pcal has the *pol* gene order protease - integrase - reverse transcriptase - RNase H. Such an order is diagnostic for Ty1/*copia* elements. Within the Ty1/*copia* division two broad groups are apparent. One group contains the *Saccharomyces* elements Ty1, Ty2 and Ty4 and the other contains *copia* and 1731 of *Drosophila,* Ty5 of *Saccharomyces,* the plant elements Hopscotch, Tst1, Ta1 and Tnt1, Osser from the green alga *Volvox carteri* and pCal. Within this second group pCal is the most divergent element. Similar results were obtained using Neighbor-Joining and Parsimony methods of tree construction.

### EXAMPLE 6

### Partial sequencing of additional clones of pCal

At the start of this work all five of the clones of pcal were partially sequenced. When the partial sequences of the three clones carrying the *Pst*1 site, which represent the left half of pCal, were compared it was found that one clone differed from the other two at a small number of sites. To determine the full extent of these differences, it was decided to completely sequence each of these three clones. When the sequences were compared it was found that two of the clones were identical, but differed from the third clone at twelve sites. The differences were all base substitutions. This finding suggested the possibility that the total population of pCal within a cell might be made up of a number of subpopulations with different sequences. Such a situation could arise in a number ways. For instance, there could be a number of integrated retrotransposons, varying in sequence, each contributing to the pCal population. Alternatively, pCal could be a self-sustaining molecule (ie. independent of any integrated copies) and the inherent inaccuracy of reverse transcriptase could be introducing variation into the system. To investigate this idea further we obtained four additional clones of pCal from a region which differed among the original clones (from the 5' border of the 5' LTR to the *Pst*1 site at position 905). The region of greatest variability was then sequenced in each of these new clones. Analysis of the sequences revealed that the four new clones were identical in sequence to each other and to the two original clones which had been found to be identical. This result suggests that the majority of the pCal molecules in the total pCal population are likely to be very similar, if not identical, in sequence. One cannot, however, rule out the possibility that more than one integrated retrotransposon is contributing to the pCal population or that pCal is a self-sustaining system.

### EXAMPLE 7

### Expression of pCal extrachromosomal DNA.

The TCa2 retrotransposon was originally found as an abundant, linear, extrachromosomal DNA molecule, referred to as pCal, in *C. albicans* strain hOG1042. The level of expression of pCal was so high that it could be seen as a distinct band of about 6.5 kb when uncut hOG1042 DNA was analyzed by agarose gel electrophoresis. The fact that such a band had not been reported in any other *C. albicans* strains suggested that the level of expression of pCal extrachromosomal DNA is much higher in hOG1042 than in any other strain. To examine this idea further we used Southern analysis to compare the level of expression of pCal amongst a variety of *C. albicans* strains. The strains examined included hOG1042 and its close relative hOG759, two recent clinical isolates (SA40 and F16932), and three common laboratory strains (SGY269, SC5314, and ATCC10261). In addition, to see if pCal expression exhibits any temperature-dependence, pCal levels were compared between cells grown at 27°C and cells grown at 37°C. The results are shown in Figure 7. The upper bands in the figure, running at >20 kb, represent the integrated forms of the retrotransposon (TCa2). The differences in hybridization intensity of these bands reflect the differences in the copy number of the integrated form (see below). Also, the extrachromosomal, pcal molecules are seen as a band at about 6.5 kb with a smear trailing off below.

On other blots distinct bands can be seen in the smears, suggesting that the smears represent incomplete or subgenomic reverse transcripts rather than them being the result of degradation during the DNA isolation procedure. A broad range of fragment sizes, as well as molecules of discrete lengths, have similarly been reported for reverse transcripts isolated from Ty1 particles (Garfinkel et al 1985). With these points in mind it can be seen that pCal expression varies greatly amongst the various strains and that it is strongly dependent on temperature. As expected the highest levels of pCal were found in hOG1042 and the closely related strain hOG759. An abundance of pCal molecules was also found in two other strains, SA40 and F16932. Densitometric analysis indicated that the level of expression in these two strains is approximately a fifth that in hOG1042 and hOG759. A low level of pCal expression was found in two strains, SGY269 and SC5314 (about 50- to 100-fold lower than in hOG1042 and hOG759). The majority of pCal molecules in SC5314 appear to be less than full-length. This seems to be a characteristic of this strain, rather than being the result of degradation of this particular sample, as it was seen consistently with different DNA preparations. The last strain, ATCC10261, produced no detectable extrachromosomal pCal molecules at all. In each strain that produces pCal, a much higher level of pCal expression was found at 37° than at 27°. Densitometric analysis indicated a 10- to 20-fold difference in expression between the two temperatures.

### EXAMPLE 8

### TCa2 RNA expression.

The results showed that the number of pCal molecules per cell varies greatly amongst different strains. This strain-dependent expression could arise in a number of different ways. It could result from strain-specific differences in the efficiency of reverse transcription of the retrotransposon RNA molecules. Alternatively, each of the strains could have a similar potential for reverse-transcription, but there could be widely varying amounts of RNA for the reverse transcriptases to act upon. A combination of these two possibilities could also be responsible. In an attempt to distinguish between these three scenarios, RNA was extracted from each of the seven *C. albicans* strains using cells grown at either 27°C or 37°C.

The RNA was then subjected to Northern analysis using the same probe as in the Southern shown in Figure 7. The results are presented in Figure 8. It can be seen, by comparing Figure 7 and Figure 8, that the pattern of TCa2 RNA expression is very *similar* to the pattern of pCal DNA expression. In each strain there is a greater amount of TCa2 RNA in cells grown at 37°C than in cells grown at 27°C. Densitometric analysis indicates a 5- to 10-fold difference between the two temperatures. Also the strains which produce the largest amounts of pCal DNA, in general, also have the largest amounts of TCa2 RNA. This finding that the observed patterns of pCal DNA and TCa2 RNA expression are very similar, and the fact that pCal is a small, linear, extrachromosomal DNA molecule, however, suggests the possibility that the signals seen on the Northern blot in Figure 8 may not represent the RNA at all; instead, they might be the result of hybridization to some pCal DNA contaminating the RNA preparations. To test this possibility, RNA samples were treated with DNase-free RNase A for 30 minutes and then compared to untreated RNA samples by Northern blotting using the TCa2 probe (not shown). We found that after the RNase A treatment less than 10% of the hybridization signal remained, indicating that the great majority of the signals seen in Figure 8 does truly represent hybridization to RNA. In addition, pCal DNA samples were denatured under the same conditions as the RNA, and then also examined by an identical Northern blotting procedure (not shown). We found that, under the Northern blotting conditions, pCal DNA gave only a very weak signal. This suggests that even the hybridization signal that remains after RNase A treatment of the RNA samples is unlikely to be due to contaminating DNA, but rather, is likely to represent incompletely digested RNA.

The similarity in the patterns of TCa2 RNA and pCal DNA expression suggests that the strain-dependent variations in the levels of pCal DNA are largely the result of similar inter-strain variations in the levels of TCa2 RNA. Or put another way, the inter-strain variations in the levels of pCal DNA are introduced mainly at the level of transcription rather than reverse transcription. The inter-strain variations in pCal expression, however, are unlikely to be produced exclusively at the transcriptional stage. It can be seen from Figures 7 and 8 that the patterns of TCa2 RNA and pCal DNA expression, though very similar, are not exactly the same. For instance, SGY269 and SC5314 produce significantly more pCal than ATCC10261 yet both of these strains have lower levels of TCa2 RNA than ATCC10261. In addition, F16932 and SA40 have similar amounts of pCal, but F16932 has approximately 5-fold more TCa2 RNA. These differences probably are the result of variations introduced at the level of reverse transcription.

### EXAMPLE 9

### Comparison of TCa2 LTRs from various strains.

It is possible that the differences in the levels of TCa2 RNA seen in the different strains result from differences in the promoters of the retrotransposons in those strains. As an initial means of testing this possibility we cloned and sequenced the first 400 bp, including the entire left LTR, of TCa2 retrotransposons from each of the various strains. By analogy with other retrotransposons, this region should contain all the major sequences regulating transcription. The sequences are shown compared to each other in Figure 9. It can be seen that the sequences are all remarkably similar to one another, there being no insertions or deletions and very few base substitutions. The few differences that there are do not seem to fall into a pattern that can be easily explained by relatedness of the various elements. The variations appear to be located in a non-random manner, some sites seeming more prone to variation than others. These variable sites may represent hotspots for mutation during reverse transcription. Within the LTRs, the sequences are identical at 275 out of 280 sites and there is no obvious correlation between the differences and the abundance of TCa2 RNA in the host strains. It therefore seems unlikely that differences in the promoters of the TCa2 retrotransposons in the various strains could account for the observed differences in RNA expression.

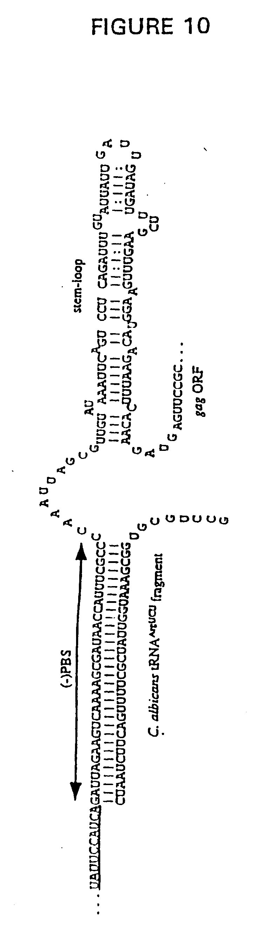

An interesting finding that did emerge from this work, though, is that there is variation in the sequence of the minus-strand primer-binding site (PBS). The PBS is a short sequence adjacent to the left LTR which is complementary to part of a cytoplasmic tRNA. The tRNA binds to the retrotransposon RNA at this site and its 3'OH can then be used by RT to prime minus-strand DNA synthesis. In most retrotransposons and retroviruses, the PBS complements the 3' end of the primer tRNA. TCa2, and a few other Ty1/*copia* retrotransposons, for example Ty5 and *copia* are exceptions to this general rule in that their PBSs complement an internal region of the primer tRNA and the primer is not a complete tRNA, but rather, a 39-or 40-nucleotide fragment of one. In the original description of the pCal sequence, the PBS was predicted to be 11 bases long, by comparison to tRNA^{Arg(UCU)} of *S. cerevisiae.* Since then the sequence of tRNA^{Arg(UCU)} of *C. albicans* has become available. A comparison of the pCal sequence to this tRNA showed that the homology between the pCal RNA and the tRNA primer extends over 32 bp, although there would be a number of unpaired bases in the PBS-tRNA primer duplex. Comparison of the sequences obtained here, however, shows that the variations found in the PBS region actually give some clones a better match to the tRNA primer fragment than that found in the original pCal sequence. One LTR in particular, isolated from hOG759, has 5 base substitutions relative to the original pCal sequence and these result in a perfect 32-bp match to the primer tRNA. In addition, the region between the PBS and the start of the *gag* ORF was found to have the potential to form into a stem-loop. The possible secondary structure of the tRNA primer fragment and the 5' region of the TCa2 RNA, as they might appear in the minus-strand priming complex, is depicted in Figure 10.

### EXAMPLE 10

### TCa2 is a moderately repetitive element and may still be active.

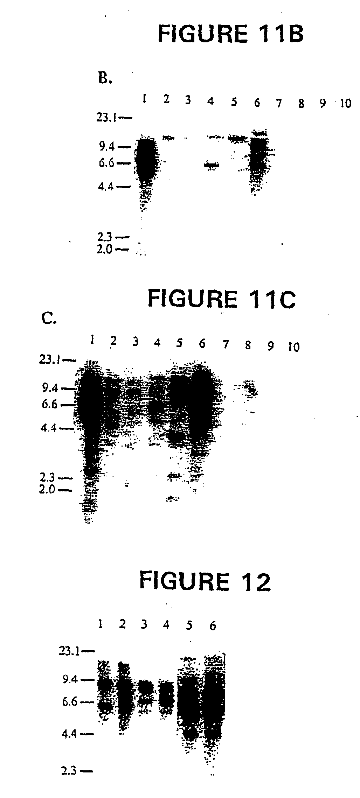

An important unanswered question, which may have implications for the regulation of this system, is: how abundant are the integrated chromosomal copies in the various strains? To answer this question we subjected genomic DNA samples from each strain to Southern analysis using either the internal TCa2 fragment, or the LTR, as a probe (Figure 11). The DNA samples used were isolated from cells grown at 27°C to minimize interference from the extrachromosomal copies. Also, to see if TCa2 is specific to *C. albicans* or whether it is also found in other *Candida* species, we analyzed the closely related species *C. maltosa, C. parapsilosis,* and *C. tropicalis* and the more distantly related *C. pseudotropicalis.* The locations of the internal and LTR probes and some important restriction sites are shown in Figure 11A. In Figure 11B it can be seen that in SGY269, SC5314, ATCC10261 and SA40 the element TCa2 is present at a low copy number - just one or two copies per cell. In F16932 five bands were found that hybridized to TCa2, indicating a moderate copy number in this strain. No hybridization to DNA from any of the other *Candida* species analyzed was detected suggesting that TCa2 is specific to *C. albicans.* This was true even when the blot was reprobed at lower stringency and exposed for a long period of time (not shown). In Figure 11C it can be seen that the TCa2 LTR is more abundant in SGY269, SC5314, ATCC10261, and SA40 (5 to 7 copies per cell) than the full-length retrotransposon. The number of LTRs in F16932 is hard to tell from this exposure because the bands are close together. Analysis of a variety of different exposures, however, revealed about 12 bands hybridizing to the LTR in this strain (not shown).

Determining the copy number of TCa2 in hOG1042 proved to be more problematic. Even though the DNA used was isolated from cells grown at 27°C (in which the expression of pCal is 10- to 20-fold lower than in cells grown at 37°C - Figure 7), it was found that the signal from the extrachromosomal copies overwhelmed any signal from the integrated copies to such an extent that no bands could be distinguished (lanes 1, Figure 11B and 11C). To get around this problem we purified the intact chromosomal DNA away from the extrachromosomal copies of pCal by separating the two on agarose gels, then extracting the chromosomal DNA from the gels. This was done for both hOG1042 and the closely related strain hOG759. The copy number of TCa2 in each strain was then determined by Southern analysis (Figure 12). Three different enzymes, *Pst*I*, Eco*RI*,* and *Cla*I*,* were used to cleave the DNA prior to electrophoresis. The number of bands detected varied depending on which enzyme had been used. Four or five bands were detected in *Pst*I-cut DNA. Four bands were found when *Eco*RI had been used and eight or nine were detected in *Cla*I*-*cut DNA. Each of these enzymes cuts TCa2 on just one side of the probe so the bands detected should represent DNA molecules containing a fragment of TCa2 and the flanking DNA out to the nearest cleavage site for each enzyme. These fragments will generally be of different sizes and so will appear as separate bands. However, in the situation where the distance out to the nearest flanking restriction site is similar for retrotransposons at two different genomic loci, then the resulting fragments will comigrate in the gel and give a single band of increased intensity. The finding that the TCa2 probe hybridizes to different numbers of bands depending on the enzyme used, as shown in Figure 12, and that the bands vary in intensity (for example, Figure 12, lane 1) suggests that the brighter bands represent more than one integrated TCa2 retrotransposon. In such a situation the digest giving the greatest number of bands, and bands the most similar in intensity, is the most reliable indicator of copy number. Here this is the *Cla*I digests. Even in the *Cla*I digests, however, some bands appear at greater intensity than others suggesting that they may also represent more than one integrated copy of TCa2. Taking this into account, and given that the *Cla*I digests give 8 or 9 bands, we estimate that there are 10 to 12 integrated copies of TCa2 in hOG759 and hOG1042. Overall, the hyridization patterns found for hOG759 and hOG1042 are very similar. Interestingly, however, they are not identical. In the *Pst*l digests (Figure 12, lanes 1 and 2) hOG1042 has a band at about 7.5 kb that is not found in hOG759. In the *Eco*RI digests (lanes 3 and 4) the two strains give the same bands, but the band at about 7 kb is brighter in hOG1042. Again, in the *Cla*I digests (lanes 5 and 6) hOG1042 has a band at about 11 kb that is not found in hOG759. Together, these findings suggest that there is at least one more copy of TCa2 in hOG1042 than in its close relative hOG759. Given the abundance of full-length copies of the retrotransposon in these strains, the most likely explanation for this finding is that a copy of TCa2 has integrated into the hOG1042 genome in the short time since the divergence of this strain from hOG759.

It is interesting to note that the number of integrated copies of TCa2 in each strain correlates fairly well with the levels of TCa2 RNA produced by each strain. For instance, the highest amounts of TCa2 RNA are found in hOG759 and hOG1042, which also have the greatest number of integrated copies. F16932, with about 5 integrated copies of TCa2, has the next highest amount of RNA, and SGY269, SC5314, ATCC10261, and SA40, with 1 or 2 TCa2 elements apiece, have only low levels of TCa2 RNA. It is not a simple, or linear, correlation however: hOG759 and hOG1042 produce at least 50 times as much TCa2 RNA as SGY269, SC5314, etc. but they have just 10 times as many integrated copies. This indicates that additional factors, as well as TCa2 genomic copy number, are involved in generating the variable levels of TCa2 transcripts.

### EXAMPLE 11

### An integrated copy of TCa2.

The sequence of pCal was primarily based on two clones that were derived from the pool of extrachromosomal copies in hOG1042. To determine if this sequence is typical of the TCa2 retrotransposon family, or if it differs in some important way from the integrated copies, we constructed a λ-library of hOG759 DNA and from it we cloned and sequenced a full-length, integrated copy of TCa2. The sequence of this copy of TCa2 (GenBank accession no. AF050215) is very similar to that of pCal. Over their entire length of 6426 bp the two elements differ at only three sites, each of these differences being the substitution of one base for another. Two of these base substitutions occur in the region encoding the RT and the other is in the RNase H coding region. The base changes do result in changes to the predicted amino acid sequence of the RT and RNase H proteins. It is possible that these amino acid alterations result in significant differences in the catalytic properties of the RTs and RNase Hs. Whether or not such changes play a role in the over-production of pCal in some strains is uncertain. It may be instructive to compare the sequences that we have determined of TCa2 and pcal with the sequence of a copy of TCa2 from a strain which produces only low amounts of pCal, such as SGY269 or SC5314. In any case, the finding that an integrated copy of TCa2 has an almost identical sequence to pCal indicates that there are no major sequence differences distinguishing the extrachromosomal forms of this retrotransposon from the integrated copies.

The DNA sequence of the regions flanking the integrated copy of TCa2 was also determined (not shown). Starting about 800 bp upstream of the retrotransposon is sequence virtually identical to that of the 5' regions of the *C. albicans* CDR1 gene (Prasad et al 1995), which has been assigned to chromosome 3 (http://alces.med.umn.edu/candida/maps/3.html). About 100 bp downstream is the start of an ORF that bears a strong resemblance to the 5' regions of cytoplasmic dynein heavy chain genes found in some other fungi. A *C. albicans* sequence containing an ORF that bears a strong resemblance to the central region of other fungal cytoplasmic dynein heavy chain genes has previously been assigned to chromosome 3 (http://alces.med.umn.edu/bin/genelist LDYN1). These findings indicate that the cloned copy of TCa2 is located on chromosome 3, between CDR1 and a gene encoding cytoplasmic dynein heavy chain. Using PCR and primers corresponding to sequences on either side of the TCa2 integration site we were able to amplify and sequence, from hOG759, another allele without an integrated retrotransposon. This work revealed, therefore, that this locus is heterozygous for the presence of TCa2, and it also showed that the insertion of TCa2 resulted in a duplication of 5 bp (ACACG) at the integration site, as is commonly found with other retrotransposons.

### DISCUSSION OF RESULTS OF EXAMPLES 7-11

Expression of pCal DNA is strongly dependent on temperature and varies greatly among *C. albicans* strains. The expression of TCa2 RNA occurs in a similar pattern to that of the pCal DNA, suggesting that the variations in pCal expression are introduced predominantly at the level of transcription. A comparison of the 5' sequences of TCa2 retrotransposons from various strains, however, failed to identify any intrinsic differences which could account for the observed strain variations in expression. Some elements, though, were found to have very long tRNA primer-binding sites, which may predispose them to efficient reverse transcription. The integrated TCa2 form was found to be a moderately repetitive element, present at 1 to about 10 copies per genome. TCa2 copy number correlates well with TCa2 RNA expression, but is insufficient to account for all the strain variation, suggesting the involvement of other factors. Sequence analysis of an integrated copy of TCa2 showed that it is very similar to pCal and is inserted between two closely placed genes. Variation in TCa2 copy number between two closely related strains suggests that the element is still transpositionally active.

### EXAMPLE 12

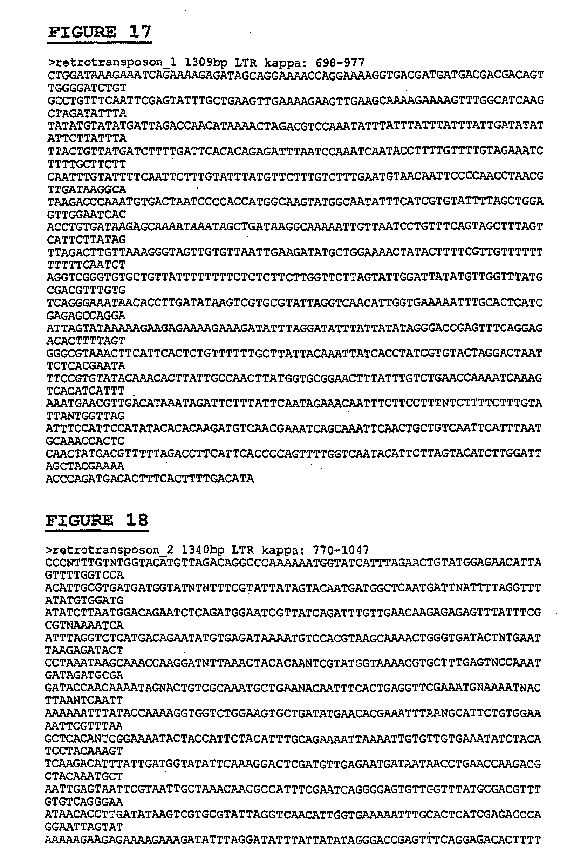

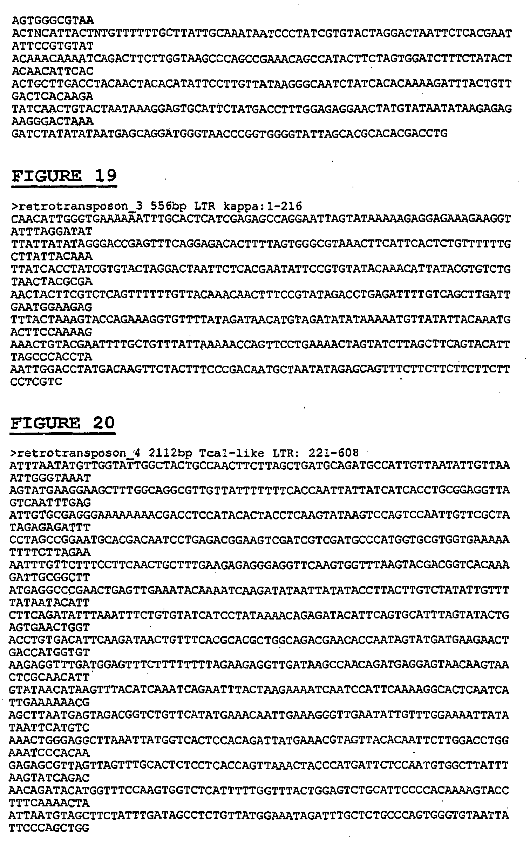

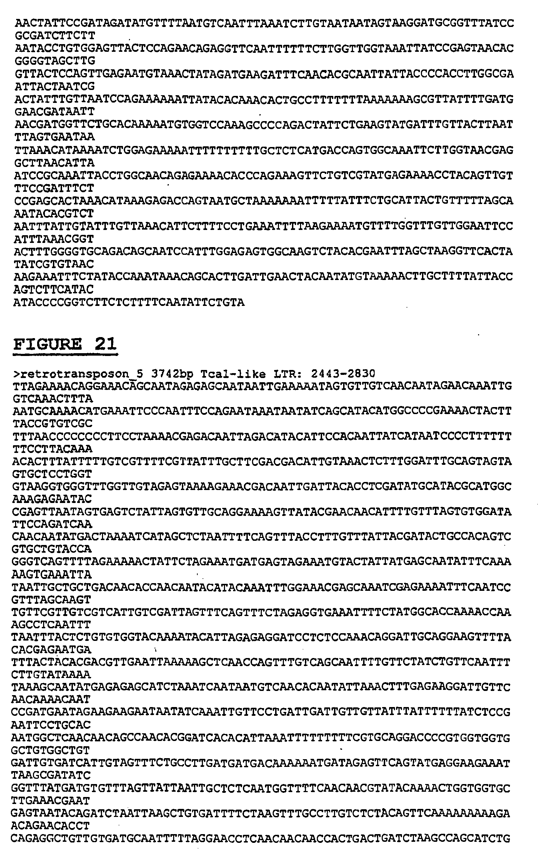

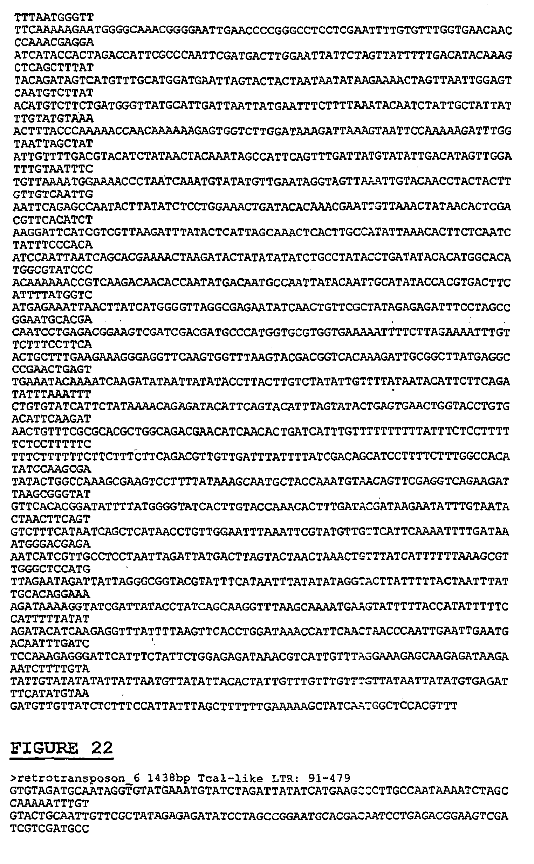

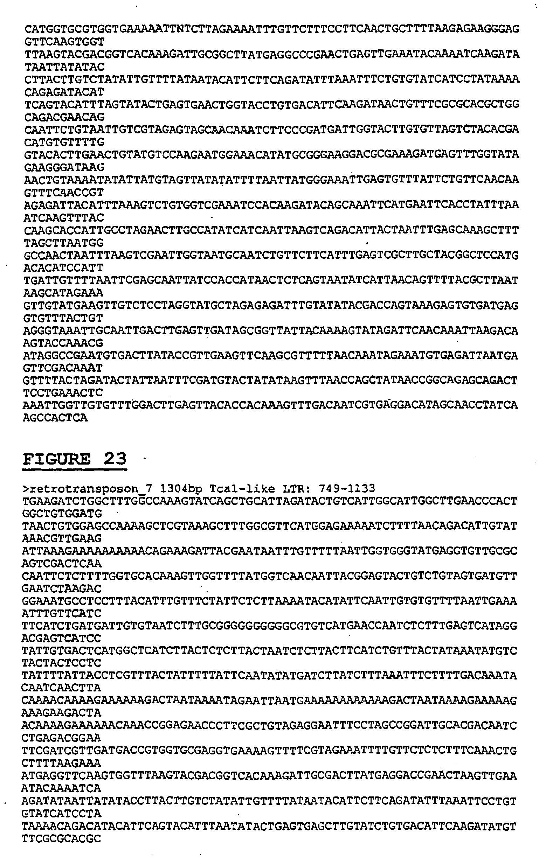

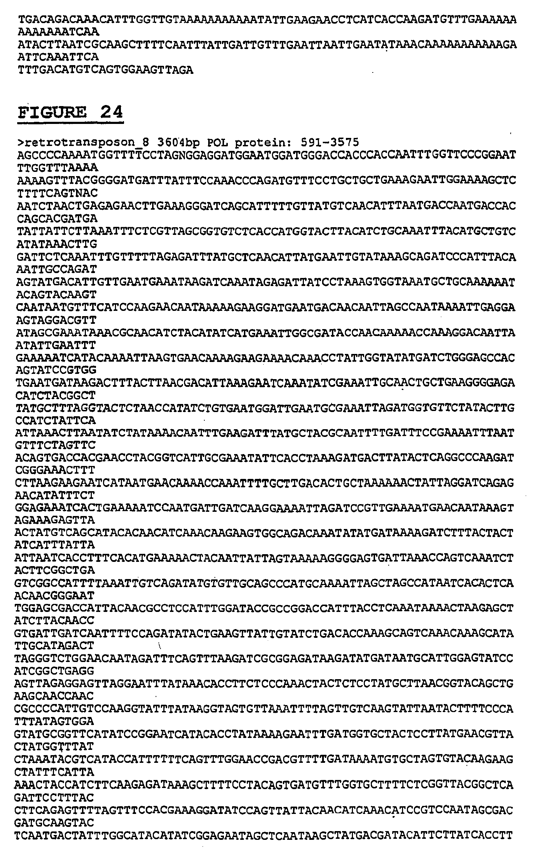

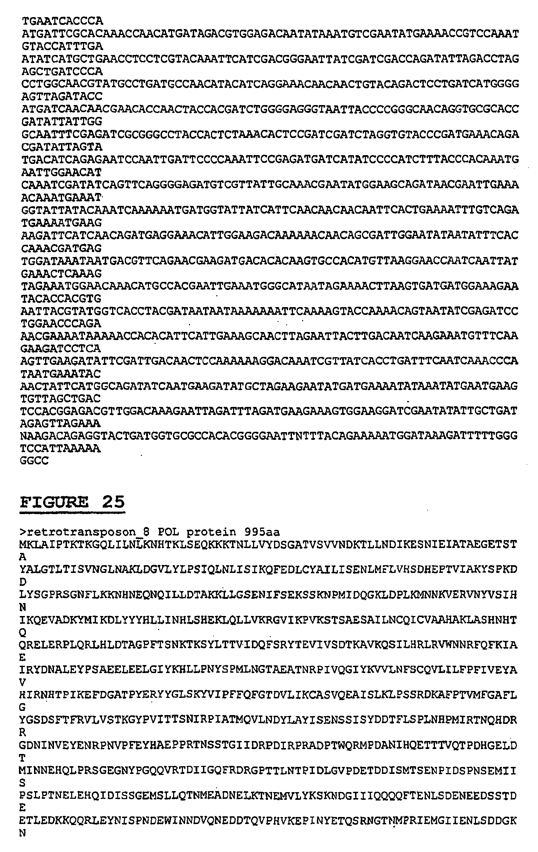

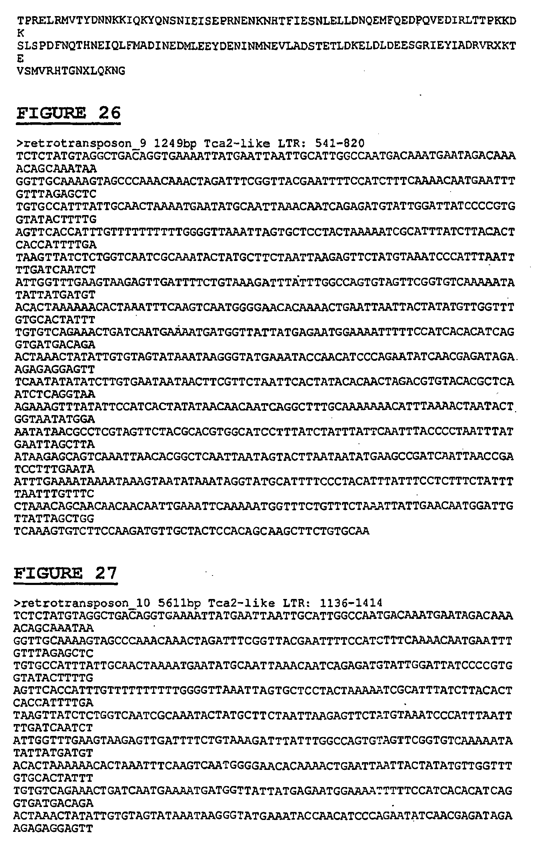

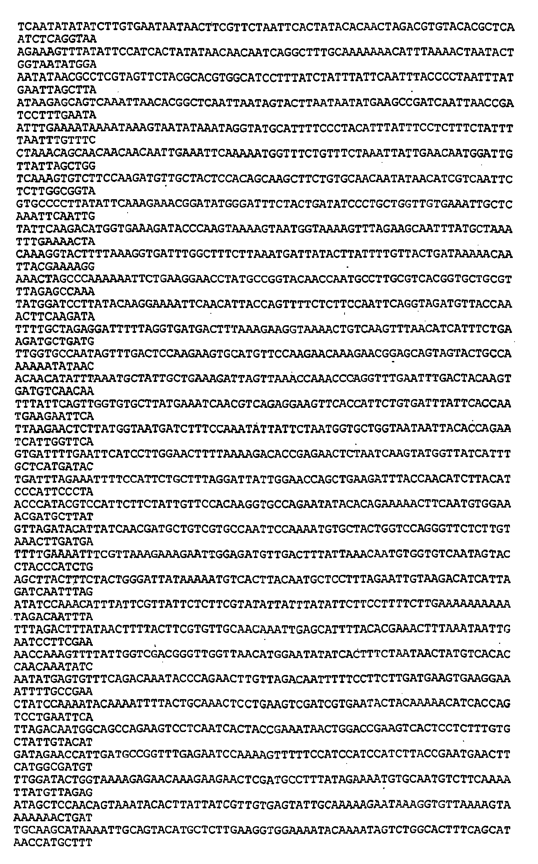

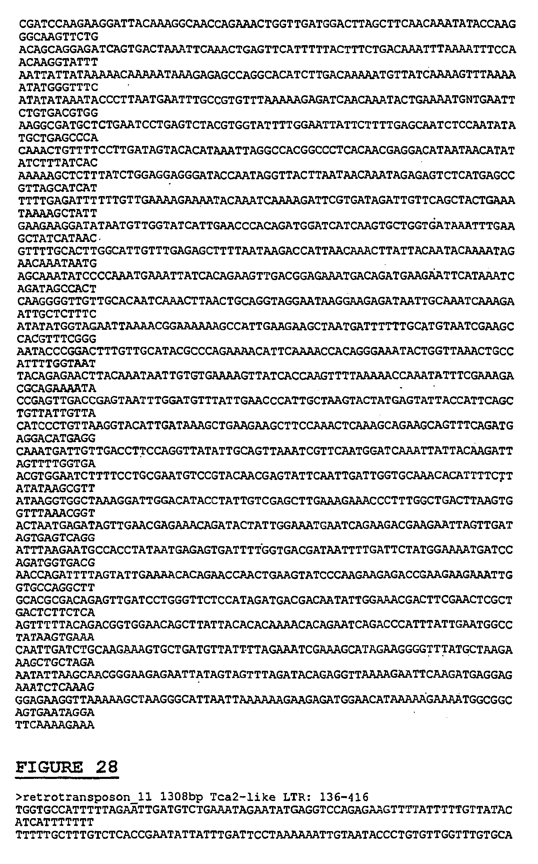

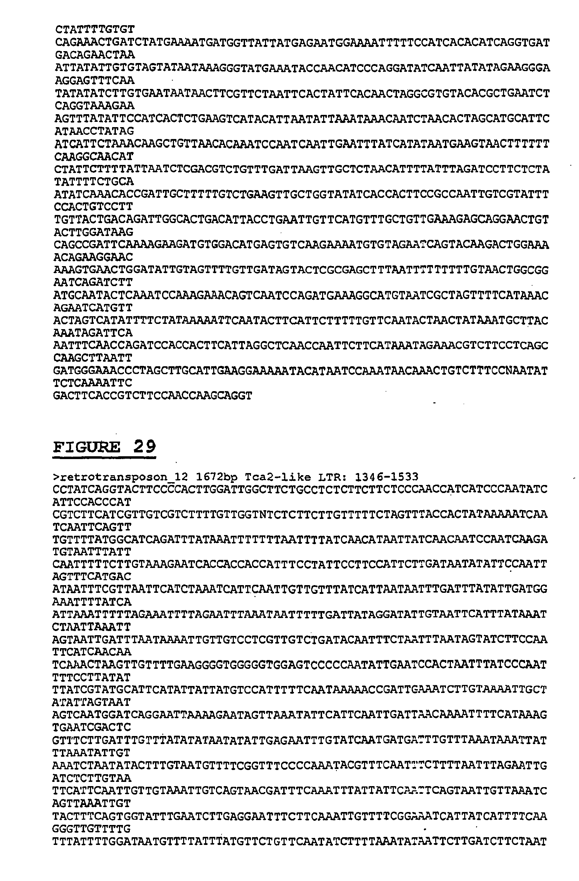

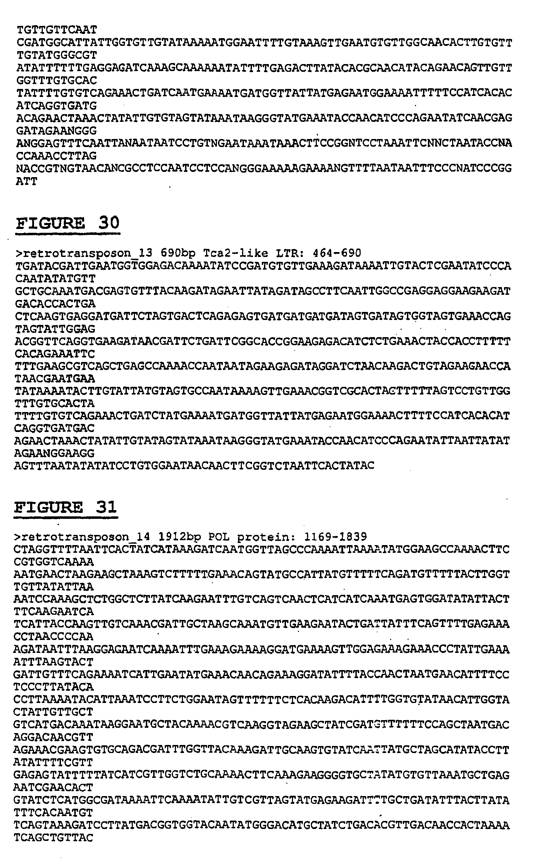

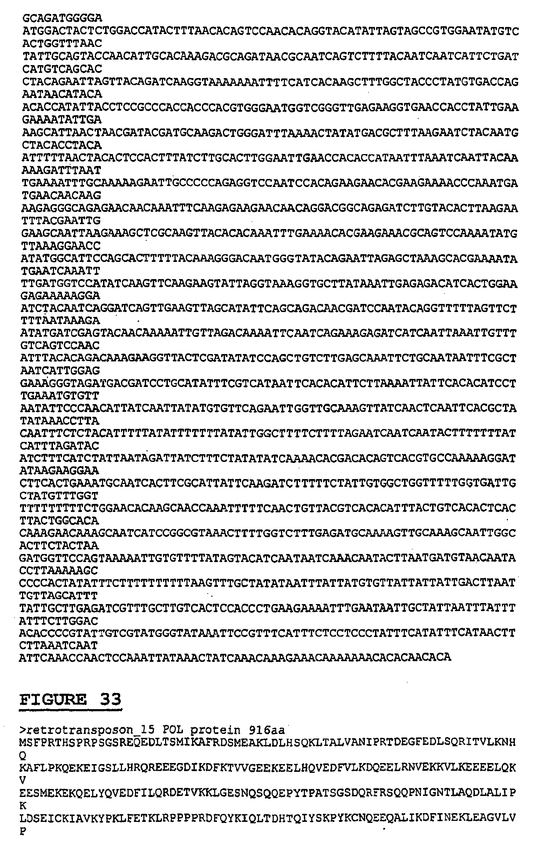

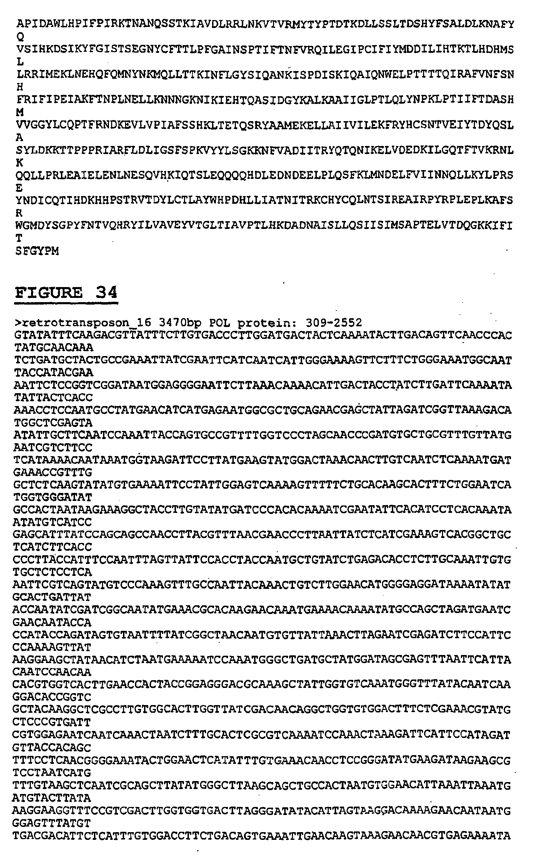

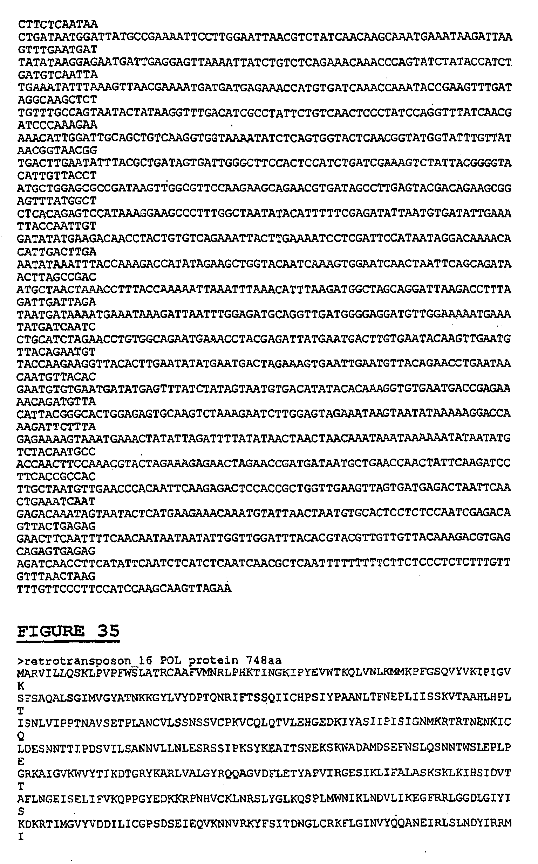

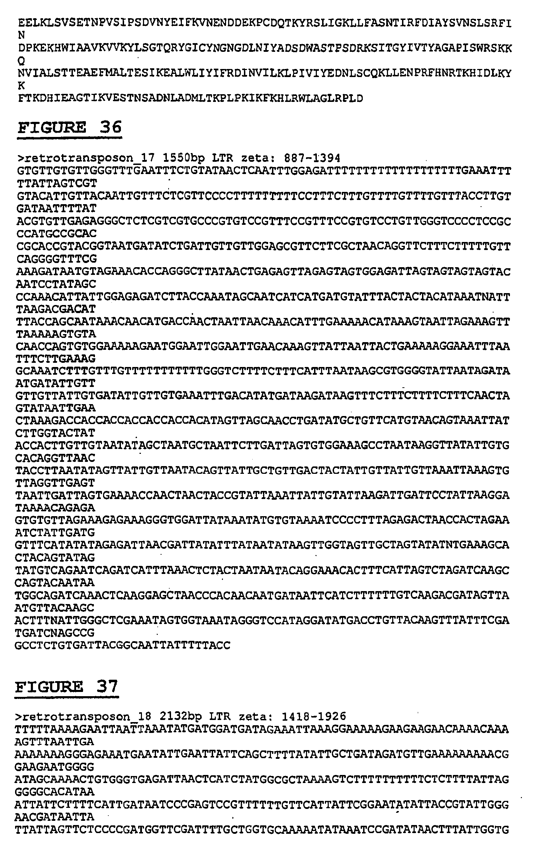

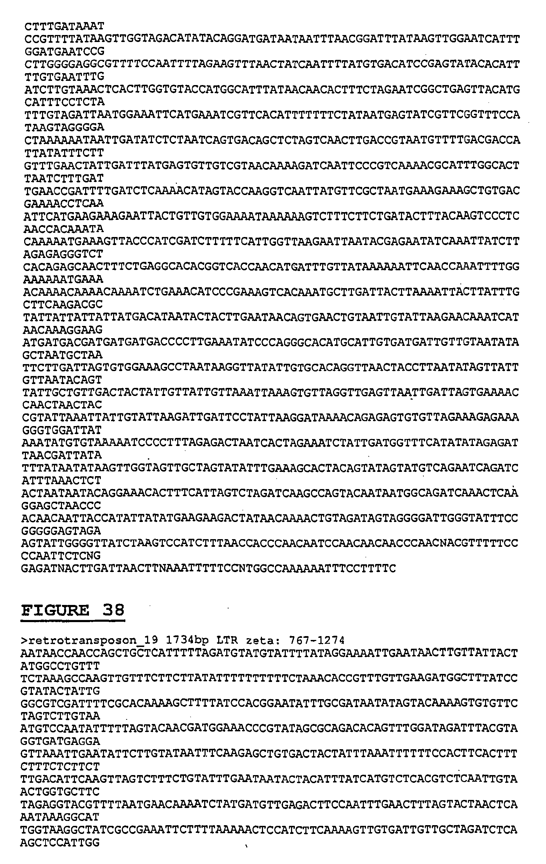

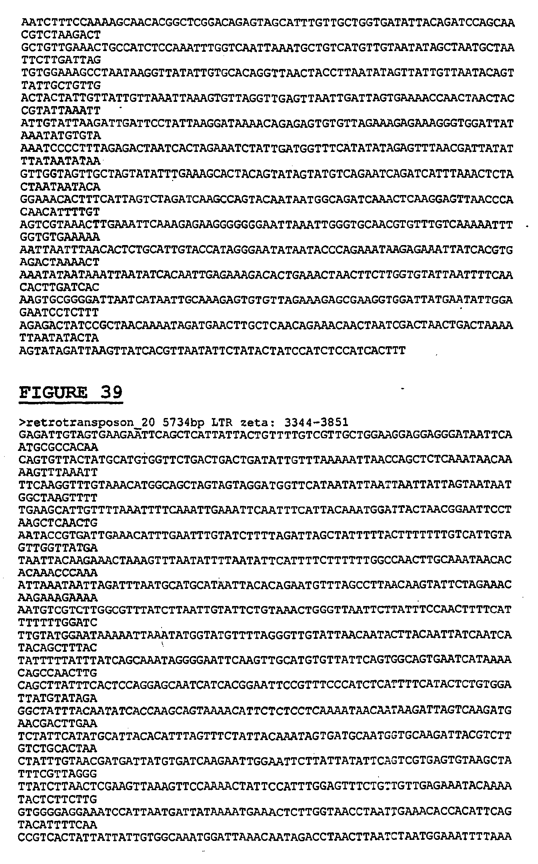

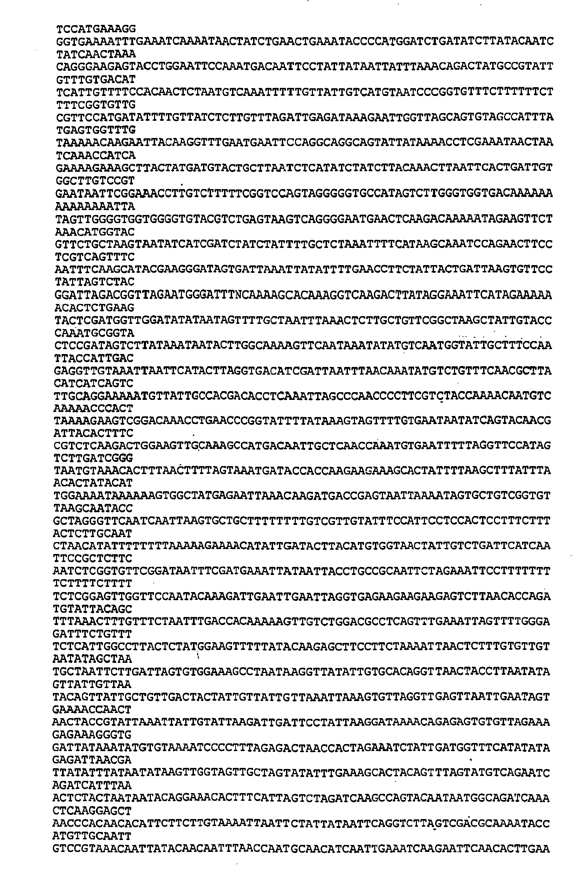

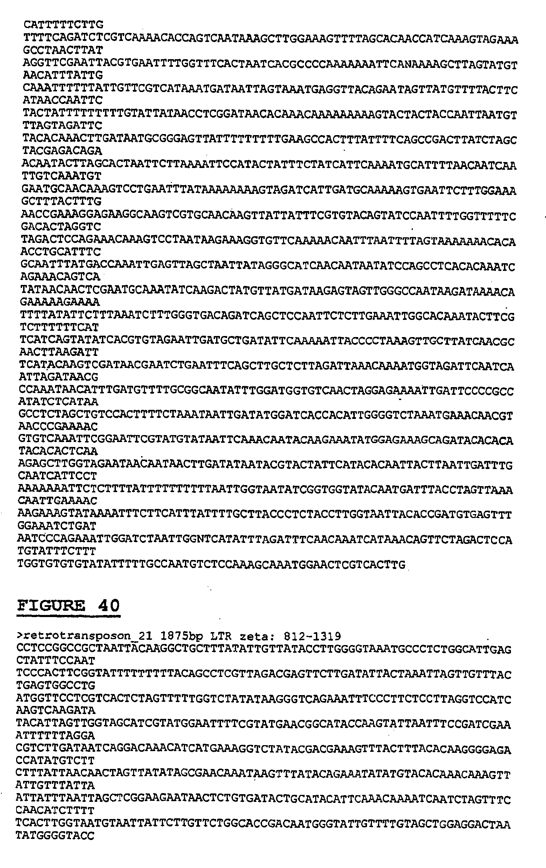

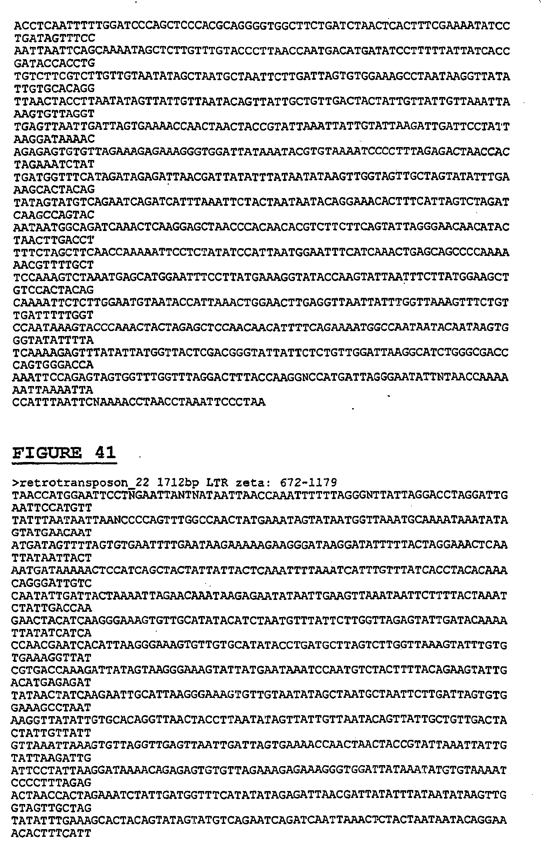

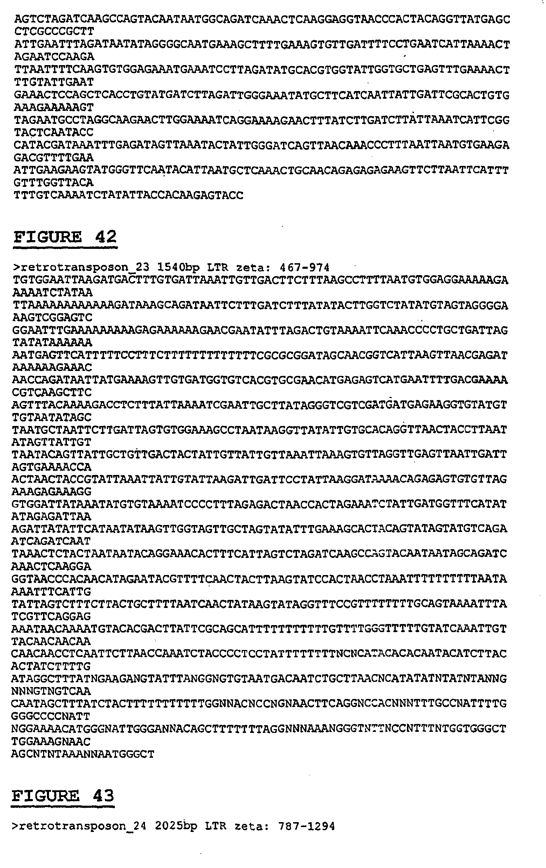

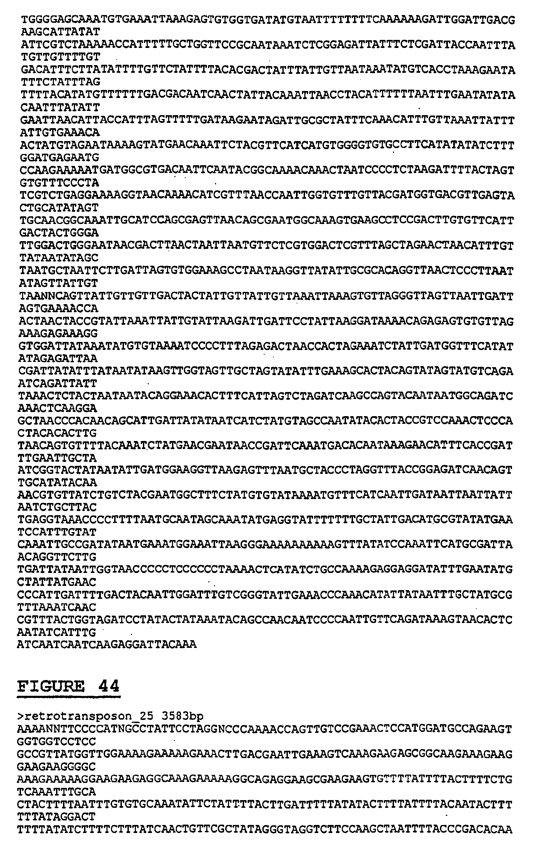

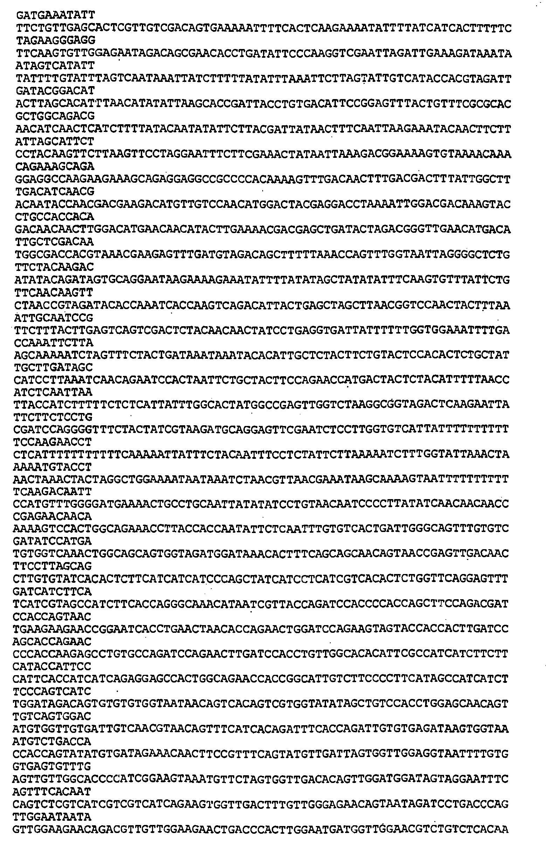

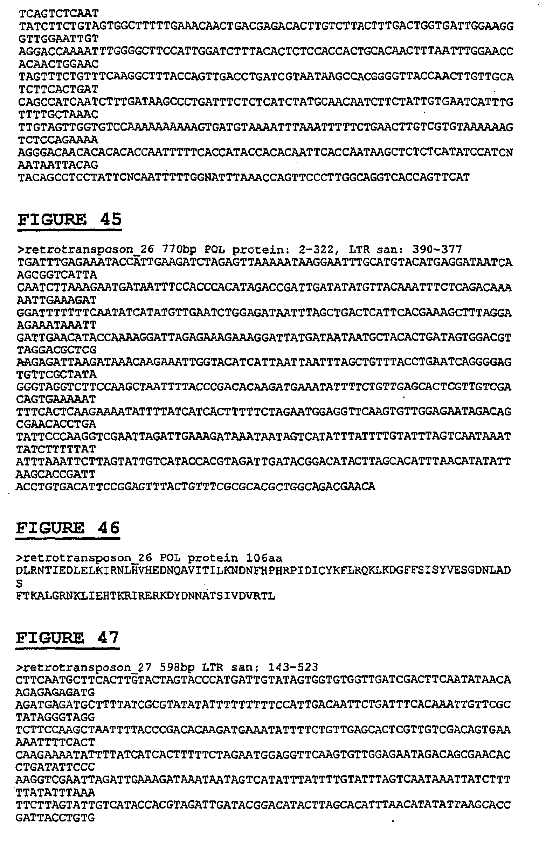

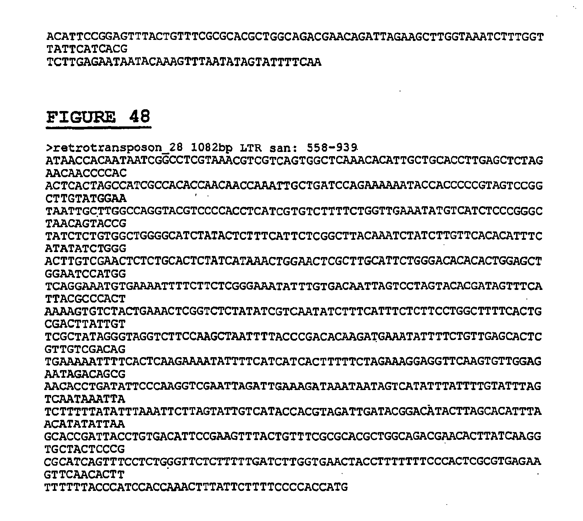

Further retrotransposons have been found. These are shown in Figures 17-48.

Isolation of the *C. albicans* retrotransposon sequences began with a search for sequences similar to *C. albicans* retrotransposon sequences present in the Embl Nucleotide Sequence Database (Stroger et al 1988) release 56, using the BLASTN program (Altschul et al 1990, 1997) version 2.0.4. A total of 28 similar sequences were identified in the proprietary Pathoseq™ database (Incyte Pharmaceuticals Inc Palto Alto CA, USA). These are different from the complete retrotransposon sequences presently available, or extend the partial retrotransposon sequences presently available.

The majority of the retrotransposons are not complete. However these partial retrotransposons can, for example, be usefully used as probes to identify the full sequences.

The partial sequences can be used as probes for the complete sequence if one was screening a DNA library. The full length retrotransposon sequences are themselves potentially useful as variants of the described TCa2. As an example the LTR promoter of TCa2 shows a different activity pattern (eg, temperature inducibility) to another unrelated retrotransposon TCa1. The retrotransposon TCa1 is less transcriptionally active at 37° than 27° while TCa2 is more active at 37° than 27°.

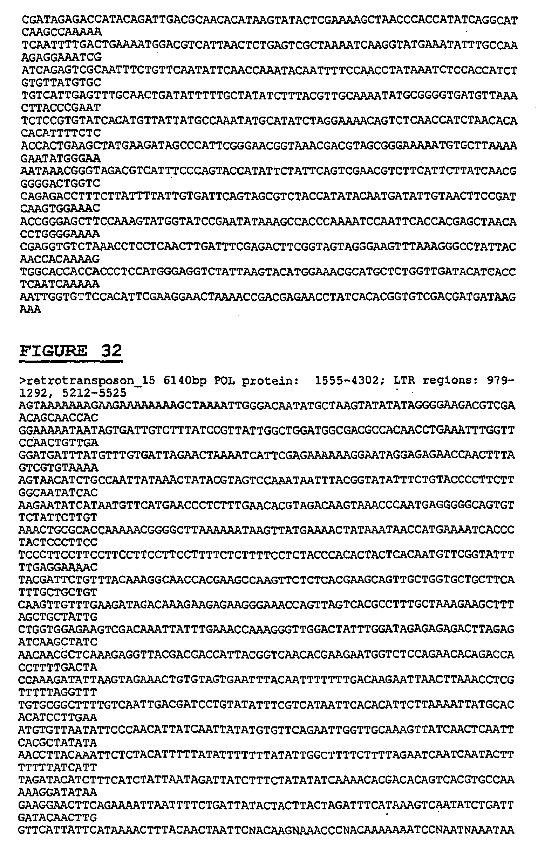

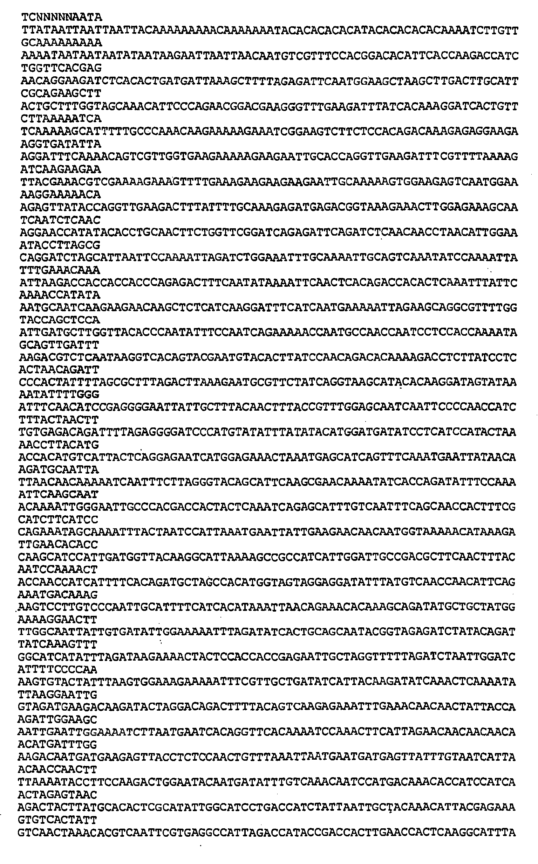

Retrotransposon 15 (Figure 32) is complete and can be used in an expression and disruption system. For example, it can be used to provide an expression vector which includes retrotransposon 15, and could be used in a gene disruption system in *Candida.*

It may also be used as a transformation and expression system for *Candida* comprising the retrotransposon.

### EXAMPLE 13

The Production of auxotrophic mutants from a strain iB65 (the original strain from which all the pCal carrying strains were derived) and its derivatives. This example shows the appearance of an auxotrophic mutant allele in the strains derived from iB65.
i) The strain was isolated from an undergraduate mouthwash (iB= intermediate Biology) in 1984. iB65 was heterozygous for a methionine auxotrophy and gave rise (following UV irradiation) to a number of homozygous methionine auxotrophs in 1984 including hOGMet5;
ii) hOGMet5: (Met). This strain was exposed to N-methyl-N-nitro-nitrosoguanidine mutagenesis and gave rise to numerous red adenine auxotrophs (some termed hOG 758-hOG762). Some of these were ade1 and some ade2. An unusual feature was that some (for example hOG759:Ade1Met) were completely non-revertible.
iii) Strain hOG762 (Ade2Met) was exposed to a further round of UV irradiation in 1988 and gave rise to numerous auxotrophs of a unique type. These auxotrophs required either aspartic acid or proline or alpha keto-glutarate. They are some kind of glyoxylate/TCA cycle mutant. We have never encountered TCA cycle mutants before or since. These auxotrophic mutants, like the ade1 mutant described above, were absolutely non-revertible even after mutagenesis. This is most unusual for Candida mutants. Strain hOG762 must have become heterozygous for the Asp/Pro mutant allele. It therefore acquired the characteristic of producing 'Asp/Pro' auxotrophic homozygous derivatives.

We believe that the non-revertible ade1 and asp/pro mutant alleles produced in these strains were generated by insertions of TCa2. Such an insertion would give a non-revertible mutant allele.

The pCal carrying strains gave rise to non-revertible mutants (as would be expected given the abundance of linear retrotransposon DNA).

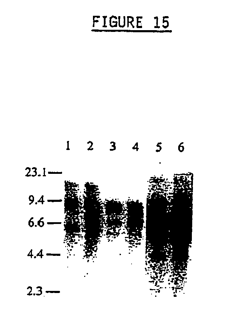

We have tested this hypothesis by comparing the Southerns of hOG1042 (a strain carrying the asp/pro mutant allele heterozygously) and hOG759 (Figure 15). There is an additional band present in hOG1042 (EcoR1 Lane 4, Cla1 Lane 6) which is what would be expected if hOG1042 carries an additional copy of TCA2 integrated into the mutant asp/pro allele.

We have also tried to find evidence for TCA2 retrotransposition in strains of this family in the absence of any mutagenesis or phenotypic change. This is shown in Figure 16, most obviously in Lanes 6 and 11. In general the strains that show extra bands following the EcoR1 digest also show bands following a Cla1 digest. This helps confirm the strains are carrying extra copies of the TCa2 retrotransposon.

These Southerns demonstrate that TCa2/pCal is retrotranspositionally active. If the element is transposing at this frequency in the absence of selection then in the presence of selection it should be relatively easy to isolate strains carrying disrupted alleles.

There are several ways of applying selection but the simplest would be to include a selectable gene within the retrotransposon.

The asp/pro allele is an example of gene disruption by the retrotransposon.

Examples 14-18 show the characterisation of the integrated form of TCa2 and a comparative analysis of its expression.

### EXAMPLE 14

The use of TCa2 as an expression system and as a transformation system: construction of a Vector System with the *Candida albicans* Retrotransposon pCAL The aim was to create a vector system based around the *C. albicans* retrotransposon-like element. The plasmid pRPU3 was constructed in which a URA3 sequence was placed within the retrotransposon at the very end of the ORF2 coding sequence adjacent to the 3' untranslated region. The URA3 is on its own promoter and it functions to confer prototrophy on ura3 auxotrophs following transformation. This demonstrated that a selectable gene, such as URA3, can be placed in this position and still function.

### Materials and Methods

### Microbial Strains and Plasmids

For *in vitro* plasmid construction and for plasmid amplification the *E. coli* strain DH5∂ was used (Woodcock et al., 1989).

The strain from which pCAL was isolated was hOG1042, a C. *albicans* auxotrophic isolate, derived from an oral isolate by mutagenising the parental strain and selecting for red adenine auxotrophs.

Four other yeast strains were used in the transformation of the retrotransposon based plasmids. These were SGY269, GSY112, MIB1 and CHAU1. They were selected as recipients for the transforming DNA because of their uridine auxotrophies and defined genotypes. SGY269, a C. *albicans* strain derived from the parental strain A81-Pu by directed mutagenesis (Kelly et al., 1987) has the genotype *ade2*/*ade2, ura3::ADE2*/*ura3::ADE2.* GSY112 is a haploid *ura3* and *leu2* auxotrophic S. *cerevisiae* strain, Mat∂ *ura3 pep4 ::HIS3 prb1-* DI.6R *leu2 ::hisG can1* cir° (Wagenbach et al., 1991). MIB1 is a *S. cerevisiae* strain constructed for this work and is auxotrophic for both adenine and uridine. It was created by crossing a1.0 (Woods and Bevan, 1965) with GSY112. The diploids were sporulated and an *ade1*/*ura3* was purified. CHAU1, a C. *maltosa* strain (Ohkuma et al., 1993) has the genotype *his5*/*his5 ade1*/*ade1 ura3*/*ura3.*

Plasmid DNA used in the construction of the retrotransposon based plasmids were the kind donations of various labs. Plasmid pET3 was provided by E. Y. H. Tsay (Gillium et al., 1984), pSM7 was provided by M.B. Kurtz (Kurtz et al., 1987) and pRC2312 was provided by R. Cannon (Jenkinson et al., 1988). The *E. coli* plasmids pUC19 (Yannish-Perron et al., 1985) and pBluescript (Short et al., 1988) were used in the cloning exercises. pK19 and pUCK1 are plasmids in which the kanamycin cassette from M13mp18-19 (Markie et al., 1986) was inserted into the *Sca*I site of pUC19. pUCK1 however lacks some of the restriction sites in the cloning cassette. pNRE1 is a plasmid containing the kanamycin cassette from M13mp18-19 as an *Eco*RI fragment in pUC19 and made ampicillin sensitive by removing the *Pvu*I portion of pUC19.

### Oligonucleotides

Two primers, CaIR and CaIF were designed to create a unique *Nsi*I restriction endonuclease recognition site (ATGCAT) at the very end of the POL of pCAL. The overlapping primers match the pCAL sequence at all but one position to provide a site for the insertion of a selectable marker. The mismatch is a T instead of an A in the fourteenth position of CaIR and the complementary A as the tenth residue of CalL. The sequence of CaIR is 5'GATACAAAATGCATTAACGGCAG3' and the sequence of CalL is 5'CTGCCGTTAATGCATTTTGTATC3'. These primers were used in conjunction with the universal forward and reverse primers complementary to pUC19.

Another pair of primers was designed to amplify the C. *albicans URA3* gene from the plasmid pET3 with *Pst*I restriction sites on the ends. The underlined portion of 5URATT
(5'CGACGGCTGCAGTTCTTCAATGATGATTTCAAC3') is complementary to the upstream region of the gene described by Losberger and Ernst (1989), and the underlined portion of 3URA
(5'CGACGGCTGCAGCCTTCACATTTATAATTGGC3') is complementary to the 3' end of the gene but not including any non-coding regions.

Primers were also designed to amplify the *URA3* gene and the retrotransposon LTR after the two had been cloned adjacent to each other in the plasmid pRPU3 (described later). A primer corresponding to the 5' end of the *URA3* gene, URAXMAS1, and a primer complementary to the 3' end of the right LTR, 3LTR, were synthesised. URAXMAS1 (5'GCGAGATCTAGATATGACAGTCAACACTAAG3') contains a synthetic *Xbal* restriction site and allows a fusion construct to be made in frame with PCR products derived from CAL2 and CAL5 (described below). No promoter sequences are amplified with this primer. 3LTR (5'CGACGCCTGCAGGTGATGGAATATAAACTTTC3') contains a synthetic *Pst*I restriction site. The underlined region is that which is complementary to the 3' end of the retroelements right LTR.

Three primers were designed to amplify portions of the retroelement for further analysis. CAL1 (5'AGTGAGCTCTGTTGGTTTGTGCACT3') contains a synthetic *Sac*I restriction site and the underlined region complements the 5' end of the left LTR. CAL2 (5'GCGTCTAGAAATTCTGTACCTTC3') is complementary to a region of the 5'LTR just upstream of the gag ORF. CAL2 in conjunction with CAL1 allows for the amplification of the left LTR. CAL5 (5'GCGTCTAGAACATTCCAGTGAAGT3') complements the region spanning the UGA stop that separates the gag and pol ORFs. A single base mismatch changes the TGA stop to a TGT codon. Both CAL2 and CAL5 contain *Xba*l restriction sites to allow the fusion of the *URA3* gene (in frame in the case of CAL5). CAL5 in conjunction with 101 F (TCTAAGCTACCAAAGCAC) enables the amplification of a portion of the gag ORF and removal of the stop codon so that the gag and pol ORFs are contiguous.

### DNA manipulations

Plasmid DNA isolation and plasmid subcloning; recombinant plasmid construction; and restriction mapping were all performed according to Maniatis et al., (1982). Transformation of *E coli* DH5∂ was performed according to the method of Maniatis et al., (1982) with some modifications. Instead of recovering in SOC media, 500 µl of TB was used. Cells were plated onto BB plates (10 g/L Tryptone, 8 g/L NaCl) with antibiotic selection. DNA fragments were purified after electrophoresis in low melting point agarose (FMC Bioproducts, USA) using agarase (GELase™, Epicentre Technologies, USA) according to the manufacturers instructions.

### Construction of pRPU3 (a marked element)

The pCAL retrotransposon was originally discovered as a linear extrachromosomal element in *C. albicans* strain hOG1042. It was cloned into pUC19 as two halves using a central *Asp*718 site. The resulting clones each had one *Asp*718 site, the other destroyed during the cloning procedure, as expected.

Two of these clones, p30 and p36, represent the 5' half of pCAL, whilst another two clones, p5 and p45, represent the 3' end of the element. An *Eco*Rl site in the cloning cassette of p30 was subsequently destroyed by digesting the plasmid with *Eco*RI, filling in the ends with Klenow and religating. This plasmid, p30E*, was then digested with *Asp*718 and *Bam*HI and the retrotransposon fragment from a similarly digested p45 was ligated in. The new plasmid, pUCCAL, was sequenced. pUCCAL has the same structure as the native retrotransposon. However further sequencing of p36, p5 and additional clones of pCal revealed that the two fragments used to create pUCCAL differed from all the others, presumably because of point mutations incurred in the reverse transcription.

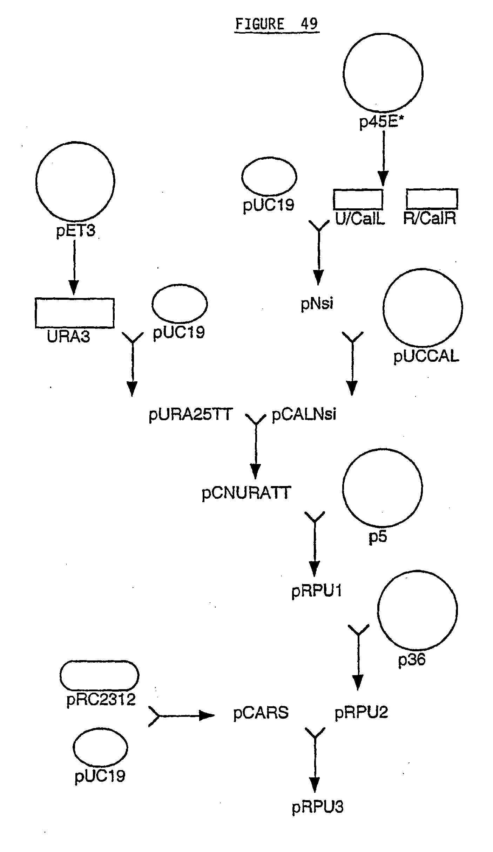

The following describes the construction of a plasmid with DNA sequence that conforms to the most common form of pCAL; construction of a *Nsi*I restriction site within this sequence; and the addition of a selectable marker and a *C. albicans* origin of replication. The cloning strategy is shown in Figure 49. Separate PCR products were generated using the primer CaIL and the universal primer of pUC19, and CaIR and the reverse primer of pUC19. The template was p45E*, a plasmid containing 979bp of the 3' end of the retrotransposon from p45. PCR products were joined using the new *Nsi*I site, cloned into pUC19 and the plasmid was named pNsi. The *EcoR*I/*Hind*III fragment from pUCCAL was replaced with the *Eco*RI/*Hind*III fragment from pNsi. The presence of the *Nsi*I site in the resulting plasmid, pCALNsi, was confirmed by restriction digest.

The plasmid containing the *C. albicans URA*3 gene, pET3, was used as the template for another PCR reaction. The primers 5URATT and 3URA were used to produce a *URA3* gene with synthetic *Pst*I restriction sites at each end. This was cloned into pUC19 and named pURA25TT. The *URA3* gene was cut out of the pURA25TT using *Pst*I and ligated into the *Nsi*I site of pCALNsi creating pCNURATT. The orientation of the *URA3* gene was confirmed by restriction analysis. pCNRUATT represents the complete pCal retrotransposon cloned into pUC19. It has a *C. albicans URA3* gene cloned into a synthetic *Nsi*I restriction site at the 3' end of the pol ORF. The *URA3* gene is expressed off its own promoter.

The intention of this construction was that as the retrotransposon was tagged with a selectable marker it could be analysed in auxotrophic hosts. As analyses of the other clones representing the integrated form of pCAL progressed, some additional steps were required to replace portions of the plasmid represented by, p30 or p45 that were not the most common sequence of pCal. There was four differences over some 3.5kb between p45 and p5 and twelve differences over a similar area between the clones p30 and p36. One of the differences between p5 and p45 was an in frame stop in p45. The following changes were made to render the retrotransposon portion of the plasmid identical to the most common sequence of pCAL. A *Sty*I/*Asp*718 fragment from pCNURATT was replaced with the same fragment from p5 creating pRPU1. All of the retrotransposon sequence from p30 and all of the pUC19 sequence of pRPU1 was replaced with p36 resulting in pRPU2. This was achieved by linearising p36 with *Asp*718 and *Bam*HI and ligating the *Asp*718/*Bam*HI fragment from pRPU1 into this.

The last step in the construction of a plasmid that would be capable of replicating in both *E. coli* and *C. albicans* was to add the Candida Autonomously Replicating Sequence (CARS). This was done by first subcloning the CARS element as a *Sph*l fragment from pRC2312 into pUC19. The CARS element was then transferred to pRPU2 as a *Hind*III/*Bam*HI fragment, creating pRPU3.

### Construction of Reporter Gene Plasmids

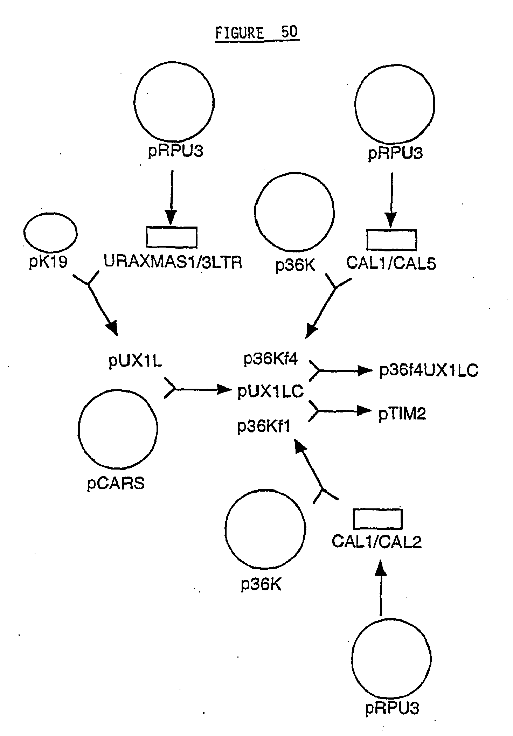

Two plasmids were constructed for use as reporter genes. Both of these contain a CARS element and the *C. albicans URA3* gene (see Figure 50). The *URA3* gene and the right LTR were amplified by PCR using pRPU3 as the template. The primer URAXMAS1 was paired with 3LTR. The resulting PCR product was cloned into *Xba*I/*Pst*I digested pK19 and named pUX1L. The *Xba*I/*Pst*I fragment was then cloned into pCARS creating the plasmid pUX1LC. A fragment of the retroelement was amplified by PCR from p36. The primer CAL2 was used with the primer CAL1 to generate a 0.4 kb product. A kanamycin resistant clone of p36 (p36K) was used as the initial recipient for this PCR product. The product was cloned using the synthetic *Sac*l and *Xba*l restriction sites designed as part of the plasmid. This plasmid was labelled p36Kf1. The Xbal/Sacl fragment from this plasmid was then cloned into pUX1L and labelled pTIM2. Expression of the *URA3* gene in pTIM2 is driven off the LTR promoter.

The plasmid p36f4UX1 LC contains a CARS and the *C. albicans URA3* gene which both function in *S. cerevisiae.* A PCR product was made using the 101F and CAL5 PCR primers and pRPU3 as a template. It was cloned into p36K using the synthetic *Xba*I restriction site of CAL5 and an internal *Bgl*II site. From this plasmid, p36Kf4, the *Sac*I/*Xba*I fragment was cloned into *Sac*I/*Xba*I digested pUX1LC creating p36f4UX1LC. The *URA3* gene in p36f4UX1LC is present as an in frame fusion to the pCAL pol ORF.

### Construction of Plasmids for in vivo Recombination in C. maltosa

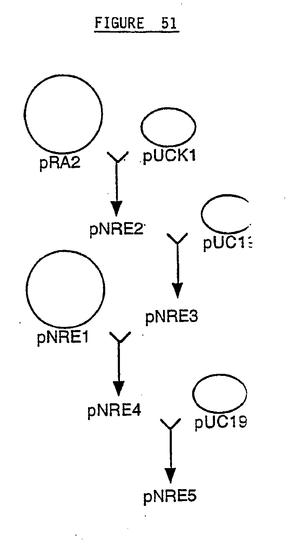

The *C. maltosa ADE1* gene has been cloned in the plasmid pRA2 (Sasnauskas et al., 1991). The gene was cloned from pRA2 into pUCK1 as a *Bam*HI fragment and labelled pNRE2 (see Figure 51). From this plasmid it was cloned as a *Ssp*I fragment into *Hind*II digested pUC19 and named pNRE3. The *Hind*II fragment containing the kanamycin cassette from pNRE1 was ligated into *Sma*I digested pNRE3. The resulting plasmid, pNRE4, was restricted with *Ecl*136/*Dra*I and the fragment containing the kanamycin cassette and the *ADE1* gene was cloned into the *Ssp*I site of pUC19 to create the ampicillin sensitive, kanamycin resistant plasmid pNRE5. Thus pNRE5 is a pUC19 based plasmid containing the adjacent kanamycin resistance cassette and *C. maltosa ADE1* gene inserted into the ampicillin resistance gene.

### Construction of Plasmids for in vivo Recombination in S. cerevisiae

The *C. albicans ADE2* gene from pSM7 was excised using *Eco*RV and blunt-end ligated into *Sma*I digested pBluescript destroying these sites. The resulting plasmid, pBSAde2, was linearised with *Eco*RV and the kanamycin element from pNRE1 was blunt-end ligated in as a *Hind*II fragment. The kanamycin element and the *ADE2* gene are adjacent in this new plasmid, pBSKanAde2. A fragment containing the first 900bp of pCal was cloned into *Sma*I/*Pst*I digested pUC19 and labelled pSP2. The kanamycin element and the *ADE2* gene was excised from pBSKanAde2 as an *Asp*718/*Sac*I fragment and ligated into *Asp718*/*Sac*I restricted pSP2. Thus the adjacent kanamycin resistance cassette and *C. albicans ADE2* gene are flanked by pUC19 on one side and pCal on the other.

### Transformations

The *C. albicans, C. maltosa* and *S. cerevisiae* strains were all transformed using the method of Kelly et al., (1988) with some modifications. A 50mL YPD culture was grown to an OD₆₀₀ of 0.7-1.3 After washing the cells in 1M sorbitol they were resuspended in 20mL SCE, 22µl β-mercaptoethanol and 150µl of 1mg/mL zymolyase 20T (Seikagaku Kogyo Co., Ltd, Tokyo). They were spheroplasted at 27°C until the OD₆₀₀ of 50µl of cells in 1mL of water showed a 50% drop compared to the 1M sorbitol reference. After washing the cells they were suspended in 1mL STC and incubated with the transforming DNA at room temperature for 10 minutes. 1mL of PEG solution was added and the cells were incubated at room temperature for a further 10 minutes. The cells were pelleted and recovered in 1 mL of SOS at 27°C for 90 minutes. This was then plated in an osmotically buffered overlay onto minimal media. Some incubation steps were performed at 37°C for the *C. albicans* and *C. maltosa* strains.

### Plasmid Extraction from Yeast Strains

50mL YNB cultures supplemented with histidine were inoculated with the transformants and incubated at either 27°C (*S. cerevisiae*) or 37°C (*C. maltosa*). Confluent cultures were spun down and the pellet resuspended in 10mL 10mM Tris, 50mM EDTA, pH 7.5. The cells were pelleted again and resuspended in 10mL 50mM EDTA, pH 9.5 and 200 µL β-mercaptoethanol. After incubation for 15 minutes at room temperature the cells were pelleted again and resuspended in 10mL 1M sorbitol, 100mM EDTA, pH 7.5 (SE). To this 50µL 1mg/mL zymolyase 20T was added. After 90 minutes incubation at 37°C the cells were pelleted. The pellet was resuspended in 10mL SE with 100µL 10mg/mL pronase and 1mL 10% SDS. This was incubated at 37°C for 60 minutes. This was then extracted with an equal volume of phenol:chloroform (1:1) twice. Two volumes of 95% ethanol was added and the precipitate spun down. The DNA pellet was resuspended in 100µL TE. This was transformed into *E. coli* from which transformants containing the yeast plasmid were purified according to Maniatis et al., (1982).

### Results

### Site Directed Mutagenesis

Using a plasmid containing the 5' end of the retrotransposon (p45E*) as the template, two PCR reactions were performed. One used the universal forward primer and CalL and the other the reverse primer and CalR. Each of the resultant PCR products were gel purified. The purified universal/CalL product was digested with *Eco*RI and *Nsi*I and the reverse/CaIR product was digested with *Bam*HI and *Nsi*I*.* The digested fragments were ligated into *Eca*RI/*Bam*HI restricted pUC19. The resulting plasmid, pNsi, contained the *Nsi*I restriction site as confirmed by restriction analysis. Sequencing of pNsi confirmed that there were no other changes. The A at position 6135 of pCAL was changed to a T, resulting in the change ATGCAA to ATGCAT.

### In Vitro Plasmid Construction

The construction of pRPU3 was achieved by conventional cloning methods. The intermediate constructs were confirmed as being correct by restriction analysis. Steps in which portions of the new plasmid were derived from PCR products or steps where the insert was replacing a fragment of similar size, were verified as being the desired product by sequencing the relevant region. Similarly the intermediates and final products in the construction of pTIM2, p36f4UX1LC, pNRE5 and pSPKanAde2 were analysed by restriction analysis and sequencing where appropriate.

### Transformation of S.cerevisiae, C. maltosa and C. albicans

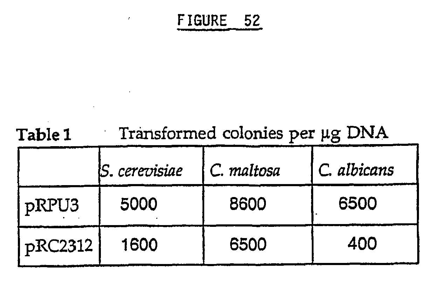

The three yeast strains transformed in this work represent the species from which the retrotransposon was isolated *C. albicans* (SGY269), a closely related species *C. maltosa* (CHAU1) and a more distantly related species *S. cerevisiae* (GSY112). Each of these yeasts were transformed with the newly constructed plasmid, pRPU3, and a plasmid known to transform efficiently both *C. albicans* and *S. cerevisiae,* pRC2312 (Jenkinson et al., 1988). The relative numbers of transformed cells per µg of transforming DNA are shown in Figure 52. The efficiency of transformation was determined for each of the yeasts. One pRPU3 transformant was found for every 400 viable cells in each of the strains. There was more variation in the pRC2312 transformations ranging from 1/1300 successfully transformed cells for *S. cerevisiae* GSY112 down to 1/10 000 for *C. albicans* SGY269. The successful expression of the *URA3* gene required the transcription termination signals from the right LTR of the retroelement. These results suggest that the signals for transcription termination are present in the LTR and function effectively in all three yeasts.

In addition the *C. maltosa* strain CHAU1 was transformed with pTIM2 and linearised pNRE5. When the cells were plated onto minimal media supplemented with histidine they required either exogenous adenine and uridine or the plasmids carrying the genes which enabled the cell to make these products. The *URA3* gene was carried on the plasmid pTIM3 and this plasmid could stably maintain itself as it contained the CARS from pRC2312. The *ADE1* gene however is carried on a plasmid that is not only linearised and hence unstable in yeasts, but also has no CARS and as such cannot be maintained as an independent DNA molecule in the cell. The ways in which a cell transformed with pTIM2 can survive on histidine supplemented media include recombining the plasmids with each other, recombining the linear DNA into the genome such that it is maintained by the hosts origins of replication, or alternatively have the adenine and uridine auxotrophies revert. Transformants were obtained that were able to survive on the histidine supplemented media. All of the transformants when purified onto complete media and grown overnight in 50mL YEP media lost the ability to grow on media lacking uridine or adenine. This indicates that the function of prototrophy was carried by one plasmid which was lost when its maintenance was not required. The natural promoter signals and the transcription termination signals for the *ADE1* gene are contained within the plasmid outside the retroelements LTRs. The *URA3* gene in both pTIM2 and p36f4UX1LC (used in the *S. cerevisiae in vivo* transformation) is not driven off its own promoter as it is in pRPU3. It is driven by the promoter signals in the left LTR and in p36f4UX1 LC it is part of a fusion construction with the gag gene of pCAL.

The *S. cerevisiae* strain MIB1 was transformed with the linearised pSPKanAde2 and p36f4UX1 LC. As with the *C. maltosa* transformation described above, the linearised DNA must recombine with genomic DNA or with a plasmid carrying an origin of replication in order to complement both auxotrophies. pSPKanAde2 has extensive homology to the pCAL and pUC 19 portions of p36f4UX1LC which allows preferential recombination with between the plasmids rather than illegitimate recombination into the chromosomes. p36f4UX1LC transformants were obtained on media supplemented with adenine. Similar numbers were obtained from a p36f4UX1LC/pSPKanAde2 transformation on adenine supplemented media. Of these transformants up to 10% were also able to grow on minimal media, indicating that the *in vivo* recombination occurs with some efficiency even without selection. Growth of these transformants on complete media results in the inability to grow on media lacking either uridine or adenine indicating that recombination has occurred between the plasmids.

### In Vivo Plasmid Construction

The *Ade1*/*Ura3l* auxotrophic yeast *C. maltosa* CHAU1 was transformed with pTIM2 and linearised pNRE5. pTIM2 contains the Candida Autonomously Replicating Sequence (CARS) and the *URA3* gene, and as such is maintained in *Ura*3 auxotrophic yeasts as a multi-copy plasmid. pNRE5 will complement the *Ade1* auxotrophy but is unable to maintain itself as an independent element. To confer the functionality of the gene it must recombine with some other DNA that is stably maintained. After selecting transformants that were able to complement both auxotrophies we passaged colonies on complete media and repurified them on media lacking adenine and/or uridine. The colonies were unable to grow under these conditions indicating that the function conferred by the *ADE1* and *URA3* genes was found on a plasmid or plasmids. Genomic DNA preparations were performed and plasmids rescued by *E. coli* transformation. The plasmids were selected for their ability to confer resistance to kanamycin and replica plating showed that they were ampicillin resistant. Plasmid preparations showed that there was only one plasmid and that it was larger than either of the parental plasmids, pNRE5 or pTIM2. Restriction analysis showed that this new plasmid contained restriction fragments unique to each of the parental plasmids and hence was a chimera of the two.

Similarly the *Ade2*/*Ura3* auxotrophic yeast *S. cerevisiae* MIB1 was transformed with a plasmid containing a CARS and the *C. albicans URA3* gene, p36f4UX1LC, and a linearised plasmid containing the *C. albicans ADE2* gene, pSPKanAde2. Transformants were selected that complemented the *Ura3* auxotrophy and were subsequently purified onto medium lacking adenine. About 10% of the transformants that grew on the medium lacking uridine also grew on medium lacking adenine. After plating the cells on complete medium they lost their ability to grow on media lacking adenine and/or uridine indicating that this ability was conferred by plasmid DNA. Genomic DNA preparations from these cells were made and the plasmids rescued by *E, coli* transformation. Plasmids were selected for their ability to confer resistance to both ampicillin and kanamycin. Plasmid preparations showed that there was a single plasmid larger than either parental plasmid. Restriction analysis showed that the new plasmid, contained restriction fragments unique to both p36f4UX1LC and pSPKanAde2.

### DISCUSSION

### Transformations

By constructing and transforming plasmids with different features we have been able to demonstrate that the new *C. albicans* retrotransposon like element, pCAL, contains promoter and transcription termination signals. In the plasmid pRPU3, a marker gene, *URA3,* was ligated into the 3' end of the pol gene of pCAL. The *URA3* gene contained its own promoter sequence but no transcription termination signals. Thus to be successfully expressed when transformed into the yeasts a message could be driven off either its own promoter or that of the retroelement, but it was reliant on the polyadenylation signal in the right LTR to terminate transcription. The successful transformation of three *Ura3* auxotrophs, *C. albicans* SGY269,
*C. maltosa* CHAU1 and *S. cerevisiae* GSY112, indicates that not only is the polyadenylation signal functional in the host species but that it works in at least two other yeast species. pTIM2 and p36f4UX1LC also contain the *C. albicans URA3* gene, however neither of these plasmids contain the *URA3* promoter sequence. pTIM2 has the left LTR and non-coding sequence of pCAL immediately upstream of the *URA3* gene while p36f4UX1LC has the *URA3* gene as a fusion product with the gag gene of pCAL. pTIM2 and p36f4UX1LC where shown to function in *C. maltosa* CHAU1 and *S. cerevisiae* MIS1 respectively. in addition they both function in *C. albicans* (data not shown).

### In Vivo Recombination

We report the *in vivo* recombination of two plasmids in both *S. cerevisiae* and *C. maltosa* as a method for constructing plasmids too large to be easily constructed in *E. coli* or for constructing plasmids where there are no unique restriction sites available. Selection of recombinant plasmids only requires that one plasmid contain a autonomously replicating sequence and that the other plasmid contains a selectable marker. As both of the plasmids are reliant on each other for expression and maintenance there is positive selection for legitimate recombination. In the *C. maltosa* CHAU1 transformation the homology between the pUC19 derived portions of pNRE5 and pTIM2 was used to direct recombination.

The MIB1 transformation results show that recombination occurs without selection in up to 10% of the transformants. This is significant because it suggests that the recombination machinery preferentially associates with naked DNA rather than chromosomal DNA.

The plasmids constructed by *in vivo* recombination are potentially useful for the analysis of the frequency of transposition under various conditions. By including a marker gene (*URA3*) within the LTRs and one external to the LTRs (ADE) of a complete retrotransposon or a functional portion of it, the frequency of transposition can be determined by analysing the preparation of cells which maintain prototrophy after growth on complete media. The majority of cells will lose the functionality with plasmid loss. Others will become prototrophic for one or both of the defects due either to retrotranspositoin or recombination. Transposition will integrate everything between the LTRs including the *URA3* gene. These colonies will be auxotrohpic for adenine and prototrophic for uridine. Recombination between homologous regions of the plasmid and the genome (such as the LTRs or the marker genes) will result in the incorporation of plasmid information from both within the LTRs and outside of them. The resulting colony would be prototrophic for both adenine and uridine. The possibility of reversion of the phenotypic markers becomes increasingly important when analysing rare events such as retrotransposition. Where transposition occurs there will be an increase in the number of LTRs which can be detected by Southerns, whereas reversion of the phenotypic markers will result in no increase in LTR numbers.

The presence of a strong promoter within the LTRs is not repressing expression of the adjacent *URA3* promoter. Such repression has been encountered in other systems extending over several kilobases (the "Temin" effect). The most effective way to use selection is to have the prototrophic gene (such as *URA3*) placed on its own promoter backwards with respect to the retrotransposon (adjacent to the 3'UTR). The prototrophic gene is disrupted with an intron which is aligned forwards with respect to the retrotransposon. In this situation the *URA3* gene is non-functional (because of the intron) unless the whole element has been transcribed, the intron removed and the retrotranscript reintegrated. In other words all the URA transformants are due to retrotransposition (rather than say random integration of the plasmid). This is the system used in *Saccharomyces* and *Schizosaccharomyces.*

Taken together we believe that these results demonstrate that TCa2 is an active retrotransposon. This is further supported by the observation that the Southern pattern of strains differs - suggesting an active retrotransposon. If TCa2 is active it follows naturally that it should function to disrupt genes at the new integration site. The pRPU3 results indicate that TCa2 can be 'tagged' with a *URA3* gene expressed from its own promoter.

### EXAMPLE 15

### Use of TCa2 as an expression system and as a transformation system

We have demonstrated that there is a very strong, temperature regulated promoter in the LTRs of TCa2. This is established by the abundant RNA as measured by northern blots. This is of considerable value as there is no other strong inducible promoter in *Candida.* Most genes from *S. cerevisiae* do not function in *Candida* and this is probably due to a promoter specificity (the reverse does not hold, most *Candida* genes do work in *S. cerevisiae).* This means that one can not use the *S. cerevisiae* expression systems in *Candida.* In addition we have demonstrated that the LTR promoter will work in *Candida* by placing a *Candida URA3* gene in phase and adjacent (just 5') to the initiator methionine of ORF1. Such plasmids (pTIM1/2) function in Candida and confer *URA3* prototrophy on Ura- auxotrophs. This establishes that the promoter is working. Such transformations are, we think, reasonably efficient and the transformants are reasonably stable. A curious and interesting observation may explain this. Strains transformed with pTIM plasmids show an obvious band on agarose gels. This DNA is not pTIM. It does not hybridise with TCa2. It is in fact circular extrachromosomal copies of the ribosomal repeat element. The Candida replication origin used in pTIM is called CARS. It was derived from Candida. It is a part of the ribosomal repeat structure. We believe that the abundant RNA transcribed from the LTR promoter in pTIM (and similar) is resulting in the cell 'up regulating' the ribosomal system by producing free circular replicating rDNA plasmids. This would explain the circular DNA in pTIM transformants. If the upregulation is also acting on the CARS element carried by pTIM then the system will up regulate itself in a positive feed back loop. That is to say; the LTR driven RNA transcription up regulates the pTIM CARS which results in more replication of pTIM and more copies of pTIM. This will result in more transcripts from the LTRs and therefore even greater up regulation of pTIM. The bottom line is you get an efficient transformation and stable (more or less) transformants.

### EXAMPLE 16

### Use of a pCal construct to induce random mutagenesis

In order to 'tag' the retrotransposon the intention was to use an inverted ('back to front') intron inserted within a reporter gene (URA3). Such an inverted intron would prevent URA3 phenotypic function unless the intron is removed from the transcript.

pURA UR { Inverted Intron}A3 / pRet
/ DNA
Initial DNA construct.

UR { Inverted Intron} A3
Transcript from URA3 promoter (pURA).

The transcript is not able to code for the URA3 gene product because the intron cannot be removed (it is in backwards).

pURA UR { Inverted Intron}A3 /
(before splicing)

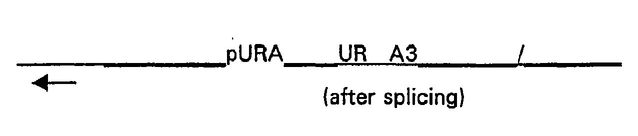
pURA UR A3 /
(after splicing)

Transcript (before and after splicing) from Retrotransposon promoter (pRet).
The transcript is not able to code for the URA3 gene product because, although the intron can be removed (processed or spliced), the URA3 sequence is backwards.

pURA UR A3 /
DNA
Integrated spliced construct.

Reverse transcriptase/integrase functions of the retrotransposon may act on the spliced pRet transcript converting it to a double stranded integrated DNA. Once integrated the copy in the genome will provide a functional pURA3.

There is no experimental work on introns in *Candida.* So we selected one possible candidate, the very small intron (mini-intron) from the peptide transporter gene (Basrai et al 1995). This was amplified by PCR and inserted into the URA3 gene in both the forward and backward direction. The forward was a control to make sure the peptide transporter intron would splice. As expected, it did.

pURA UR {Intron}A3
DNA
URA3 gene with forward Intron

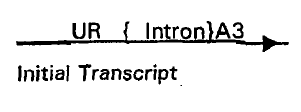

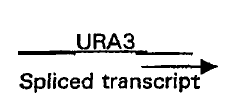

Again, as expected, the backward intron failed to splice, even though it was the identical sequence put into the identical URA3 site.

pURA UR { Inverted Intron}A3
DNA
URA3 gene with Inverted Intron

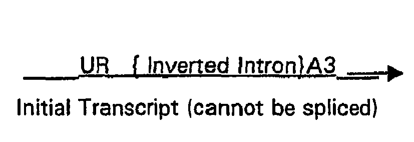

We have now mounted this URA3/inverted intron element onto a retrotransposon plasmid putting the element into a (synthetic) Nsi1 site at the 3' end of the coding sequence. We have also added an ADE2 element between the right LTR and the *Candida* ARS (CARS). This is summarised below.

Left LTR
Retrotransposon
CARS
*NsiI
LTR
Left LTR
Retrotransposon
URA3/Intron
CARS
ADE2
LTR

In theory the retrotransposon will transcribe from the left LTR to the right LTR, the transcript will have the intron spliced out and the spliced elements will be converted into DNA by reverse transcriptase and integrated. The URA3 element will then be transcribed off its own promoter to give a URA⁺ phenotype. There are possible problems to do with the pURA3 element interfering with transcription of the retrotransposon or the reverse transcriptase but these can only be found, and fixed empirically. The ADE2 was added to the plasmid to give positive selection (as the URA3/intron is non-functional in the plasmid).

The plasmid is quite large and therefore not that easy to work with but it has been completed. The plasmid has been transformed into two ADE2 URA strains, one carrying a URA3 point mutation and the other a URA3 deletion (a small deletion). ADE2⁺ transformants were selected and grown at 37°C to encourage retrotransposition. Cultures were then plated on minimal medium + adenine. The plasmid is lost under these conditions and only URA⁺ variants (retrotranspositions?) can grow. Both strains gave URA⁺ derivatives. The URA⁻ point mutation is reasonably stable and the URA⁻ deletion completely so. We, therefore, are sure that these URA⁺ variants are not revertants. They are, we believe, a mixture of retrotransposition and gene conversion. There is very little literature on gene conversion in *Candida.*

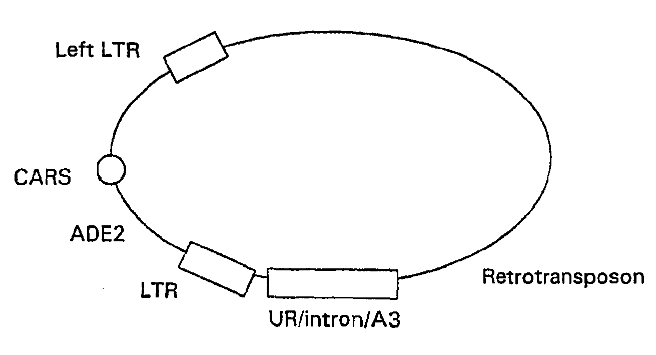
Left LTR
CARS
ADE2
LTR
UR/intron/A3
Retrotransposon

URΔA3 URA3Δ(Deletion) Homozygote
URΔA3 DNA with characteristic ΔSouthern pattern

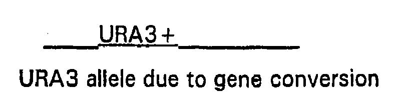

Gene conversion between the URA3Δ and the URA3/inverted /―intron allele can generate a URA⁺ allele that will have the wild-type Southern allele pattern.

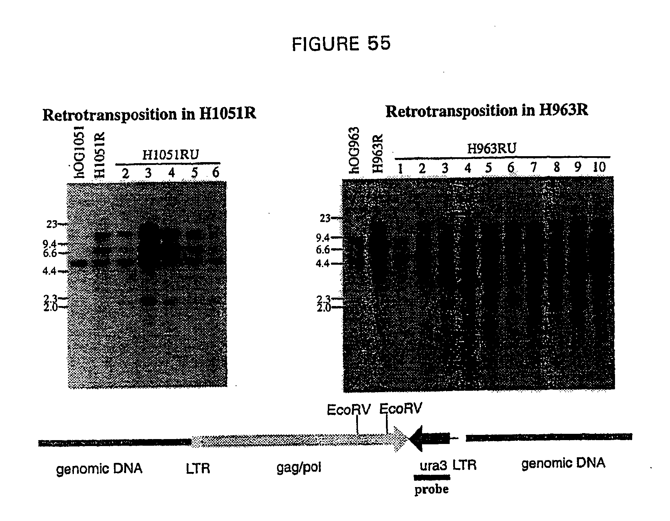

The URA3+ colonies generated in these experiments were analysed by Southern analysis to confirm the presence of a new copy of TCa2 containing the URA3+ gene (Figure 55). The URA3+ colonies derived from L11051R all appear to contain the same putative retrotransposition event. The clones derived from L1963R appear to contain different events, since several different sized bands are observed. However, some of the URA3+ colonies appear not to contain extra bands.

Some of the URA⁺ variants are clearly due to gene conversion. Some are clearly not due to gene conversion. They give new and various bands which we think indicates retrotransposition into random sites.

### EXAMPLE 17

### Further analysis of URA3 +

We have done further analysis of the URA3 + strains thought to be carrying a new retrotransposition (URA3 + and having 'unusual' Southerns when probed with a URA3 probe) (Figure 55).
Specifically we have done 'inverse PCR' (IPCR) after a Taql (4base cutter) digest of the DNA and self-ligation. The IPCR primers correspond to:
i) the URA3 gene (interrupted by the peptide transporter intron); and
ii) the boundary of the URA3 and TCa2 LTR.

These should only give a product following a retrotransposition event since the intron must be removed before primer i) will work.

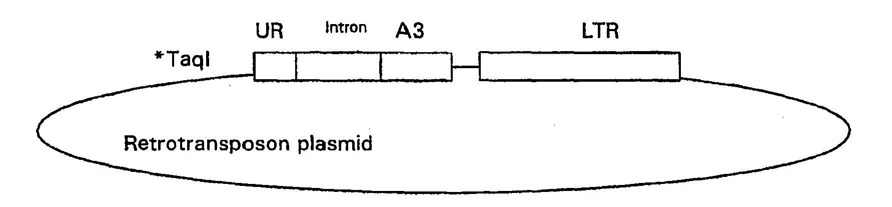
UR Intron A3 LTR
*TaqI
Retrotransposon plasmid

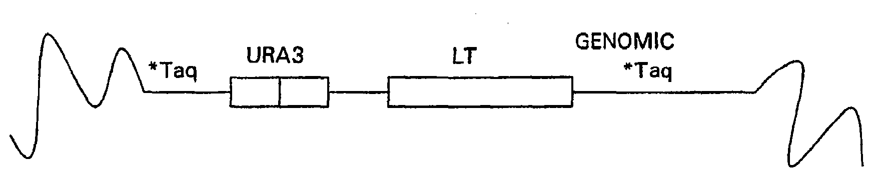
GENOMIC
*Taq    URA3    LT    *Taq

Integrated into genome following retrotransposition

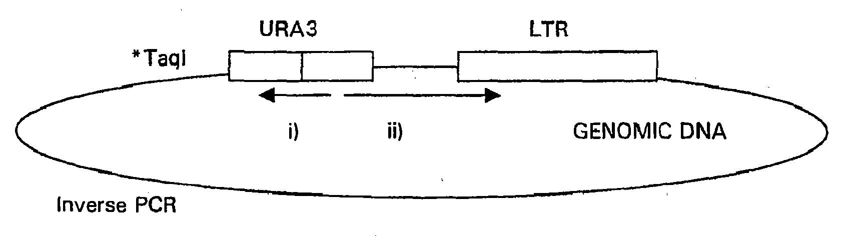
URA3 LTR
*TaqI
i) ii) GENOMIC DNA
Inverse PCR

The inverse PCR products have been sequenced from several independent URA3 + and the sequence confirms that there has been a retrotransposition (the intron has gone) and that there is an additional retrotransposon integrated into a novel site in the genome.

In summary the system works. So far all the integrations are in different sites.

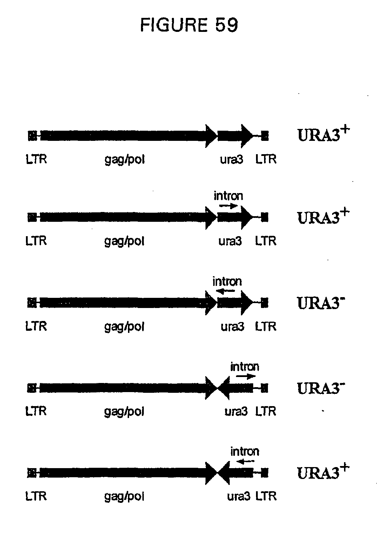

Results are shown in Figures 59-62.

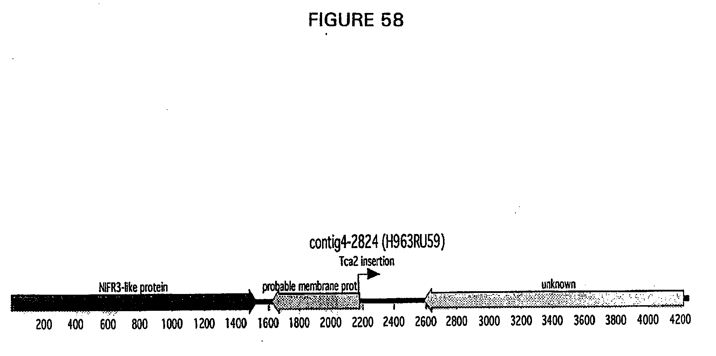

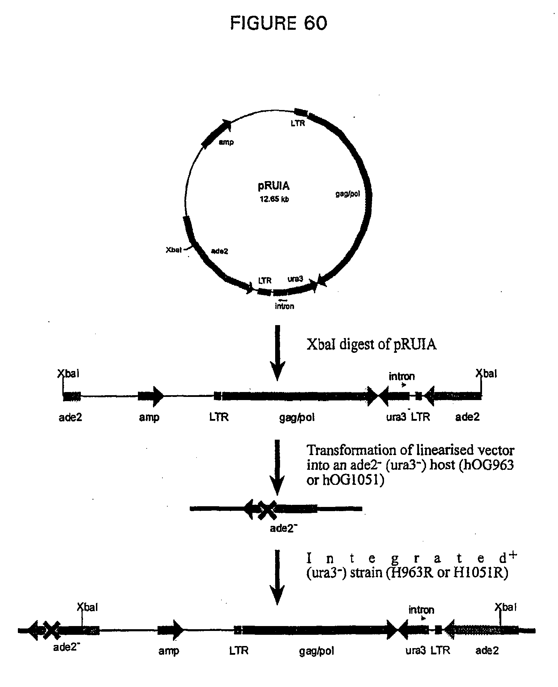

The ABI PRISM DNA sequence chromatograms of Figures 59 and 60 show that the URA+ tagged retrotransposon has undergone retrotransposition and integrated into a new site in the Candida genome. In other words it is an actual example of a random tagged integration/mutation event.
Specifically:
DNA was isolated from the URA+ *C.albicans,* digested with the restriction enzyme Taql, self-ligated and subjected to inverse PCR. The resulting PCR product was cloned and sequenced from the 'universal' forward and reverse primers.
The sequence H963RU59 defines the exact integration site of the retrotransposon.
This integration site falls within the ORF of a membrane protein. This is not a unique event, the table (Figure 58) describes other integration events.
These integration sites do not seem to be associated with tRNA genes or LTR sequences from Tca2 or other retrotransposons. The integrations seem to have occurred at a wide variety of sites. The integration site sequences show no obvious homology to each other, In as far as a generalisation can be made on the present data, the Tca2 integrase seems to prefer to integrate near to the 5 end of coding sequences (ORFs). This may be within the ORF (as in strain H963RU59) or within several hundred base pairs 5 to the ORF. Such integration will potentially inactivate the ORF expression, down-regulate or up-regulate the ORF expression or alter the regulation of expression (for example, make expression of the ORF temperature sensitive).
This pattern of integration is unlike that of any previously described retrotransposon integrase. For example, in Saccharomyces cerevisiae Ty1, Ty2, Ty3 and Ty4 integrate near tRNA sites, while Ty5 integrates into telomeric DNA.
The Tca2 integration pattern is unlike those integrases previously described and therefore could not be predicted. The use of Tca2 as a random integration system is therefore a non-obvious application of this retrotransposon.

### EXAMPLE 18

### Evidence of temperature dependent retrotransposition

Strain hOG 1042, which contains TCa2, was grown in liquid culture (Yeast extract, Peptone, Glucose) at 37°C. Serial subcultures were made every day for 3 weeks. A number of single colonies from this liquid culture were isolated on solid medium and DNA extracted from them. These DNA samples were included in a Southern analysis, where the probe used would hybridise to the 3' region of the POL gene of the integrated retrotransposon.

The results of this Southern (Figure 16) indicate the presence of one or more new bands in many of the strains cultured over the 3 weeks as compared to the original hOG 1042. It is assumed that these new bands represent the presence of TCa2 integrated at new genomic loci. This implies that TCa2 has actively retrotransposed to generate new copies of itself at new positions. The size of the new band(s) vary from strain to strain, indicating that the new integration sites are different in each individual strain.

### EXAMPLE 19

### Vector construction

The initial phase of the project involved the construction of a vector that could be used to characterise retrotransposition events in *C. albicans.* This vector contains the retrotransposon TCa2 and a selectable marker gene with an intron inserted. The *URA3* gene, from *C. albicans,* was chosen as the selectable marker. Since the *URA3* gene does not contain a native intron, a small intron from a *C. albicans* peptide transporter gene was used.

### Insertion of an intron into the ura3 gene

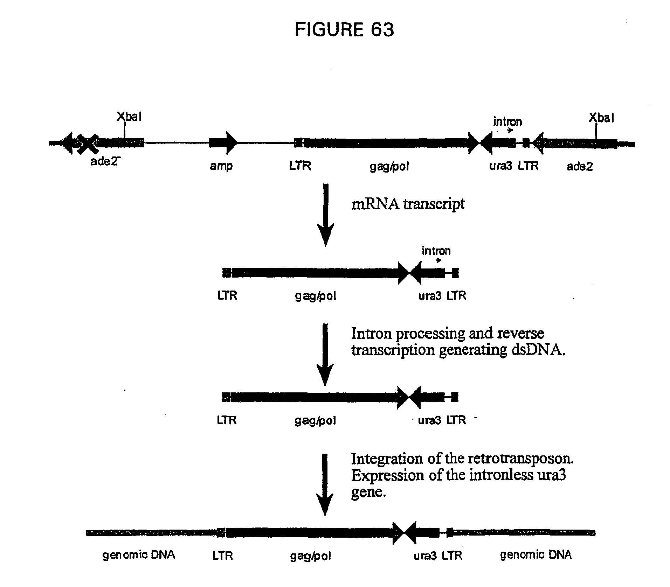

The intron of the peptide transporter gene was inserted into the *URA3* gene, close to the start of the open reading frame (ORF). This location was used since most *C. albicans* introns are located near the front of ORFs. The *URA3* gene used contains only a short promoter region (130 bp) and no transcription termination signal so as not to interfere with the transcription of the retrotransposon. The intron was inserted in both forward and reverse orientations (with respect to the *URA3* gene) to allow analysis of the intron processing. The intron-containing *URA3* gene was placed into TCa2 near the end of the *pol* gene, in both orientations (Figure 59).
It was found that the *URA3* gene in these constructs was functional only when the intron was placed in the normal orientation with respect to the *URA3* gene. In addition the *URA3* gene was functional in either orientation with respect to TCa2. Therefore the intron is capable of being processed correctly.
A construct was then produced which contains the *URA3* gene in the reverse orientation with respect to TCa2 and an intron inserted into this gene in the forward direction with respect to TCa2. In addition an *ADE2* gene and *Candida* autonomously replicating sequence (CARS) were also present on this vector. The resultant vector was transformed into an *ura3⁻ ade2⁻ C. albicans* strain (hOG963). Transformants were selected using the *ADE2* marker. Transformants were grown overnight in minimal media supplemented with uridine and then plated on minimal media containing adenine but lacking uridine. If retrotransposition had occurred then *URA3⁺* colonies would be produced as a result of splicing of the reverse intron from the *URA3* gene and therefore restoration of a functional gene (Figure 63). Several such colonies were produced, however they all appeared to be the result of gene conversion of the plasmid borne *URA3* gene with the native *URA3* gene. It was therefore decided to integrate the vector in the hope that this would reduce the frequency of gene conversion.

### Integration of the retrotransposition vector

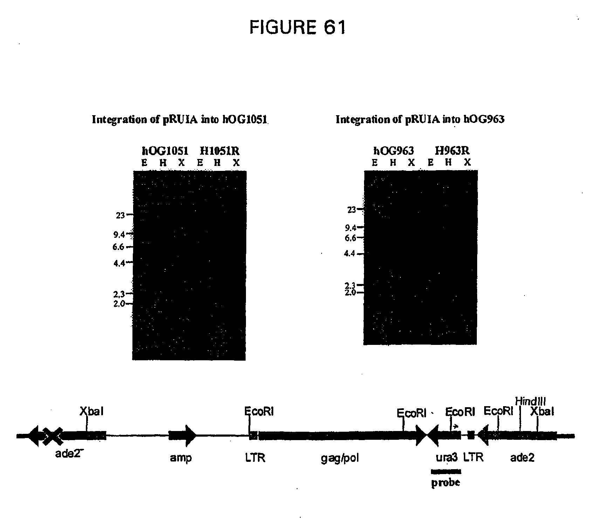

The CARS from the plasmid used in the previous analysis was removed resulting in the plasmid pRUIA (Figure 60). This plasmid was digested at the unique *Xba* I site (within the *ADE2* gene) and transformed into two *ura3⁻ ade2⁻* strains of *C. albicans,* hOG963 and hOG1051, giving rise to the strains H963R and H1051R, respectively. A schematic diagram of the integration is shown in Figure 60. Southern analysis of strains containing the integrated pRUIA is shown in Figure 61.

### Expression of the tagged TCa2 is temperature sensitive

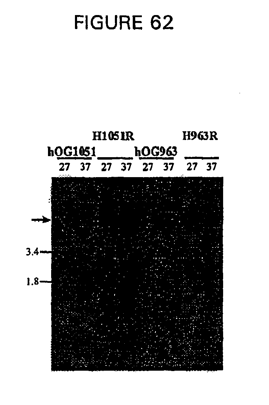

It is known that in some *C. albicans* strains (for example hOG1051) TCa2 is expressed at higher levels at 37°C, as compared to cultures grown at 27°C. To insure that the full tagged TCa2 was being expressed Northern analysis was performed (Figure 62). Results of this analysis indicate that the TCa2 construct containing the *URA3* gene is expressed as one long transcript.

### Retrotransposition in C. albicans

The strains H1051R and H963R (containing the integrated pRUIA) were used to analyse retrotransposition of TCa2. Since retrotransposition occurs via a mRNA intermediate the intron inserted into the *URA3* gene can be processed before reverse transcription of TCa2. The double stranded DNA copy of the retrotransposon is then integrated into the host genome. Since the *URA3* gene has had the intron removed it can produce a functional protein. A diagram of this process is shown in Figure 63.

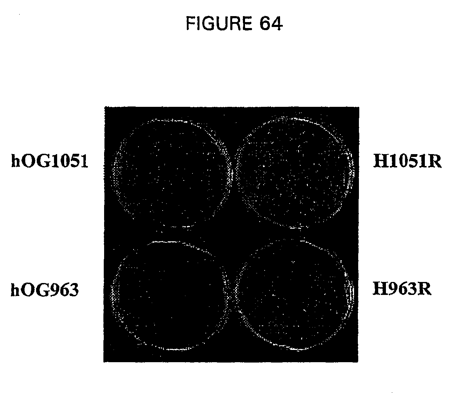

*C. albicans* strains containing integrated pRUIA (H1O51R and H963R) were grown overnight in rich medium (YPD) then plated on minimal media. If retrotransposition has occurred then *URA3*⁺ colonies are produced. An example of a typical experiment is shown in Figure 64.

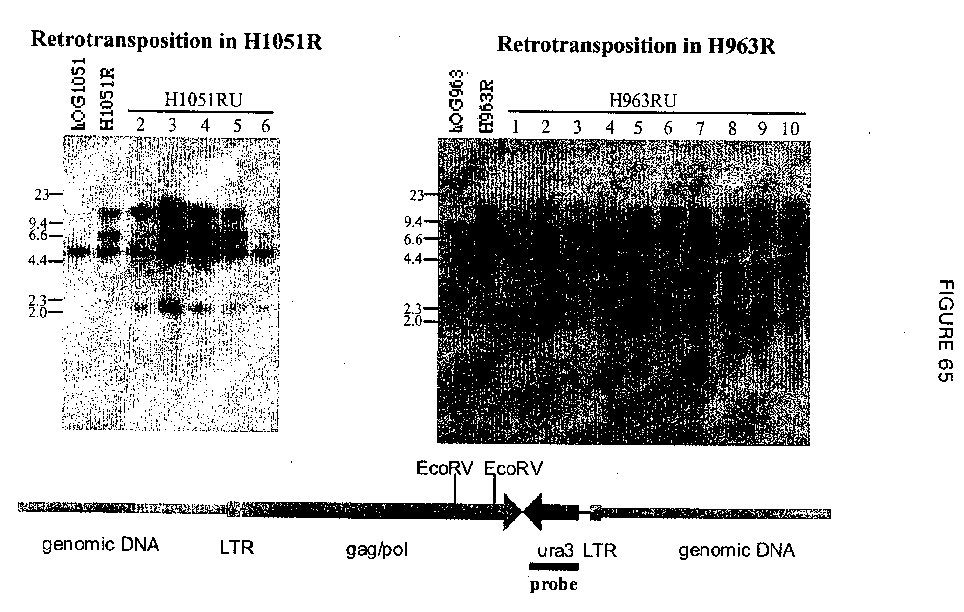

The strain hOG1051 is known to overexpress TCa2 (Figure 62). The derivative H1051R gave rise to approximately 10-fold more *URA3*⁺ colonies than H963R. The estimated rate of *URA3*⁺ production for H1051R is approximately 10⁻⁵ *URA3*⁺ colonies/cell plated.
The *URA3*⁺ colonies generated in these experiments were analysed by Southern analysis to confirm the presence of a new copy of the TCa2 containing the *URA3*⁺ gene (Figure 65).

The *URA3*⁺ colonies derived from H1051R all appear to contain the same putative retrotransposition event. The clones derived from H963R appear to contain different events, since several different sized bands are observed. However, some of the *URA3*⁺ colonies appear not to contain extra bands.

### Inverse PCR of tagged retrotranspositions

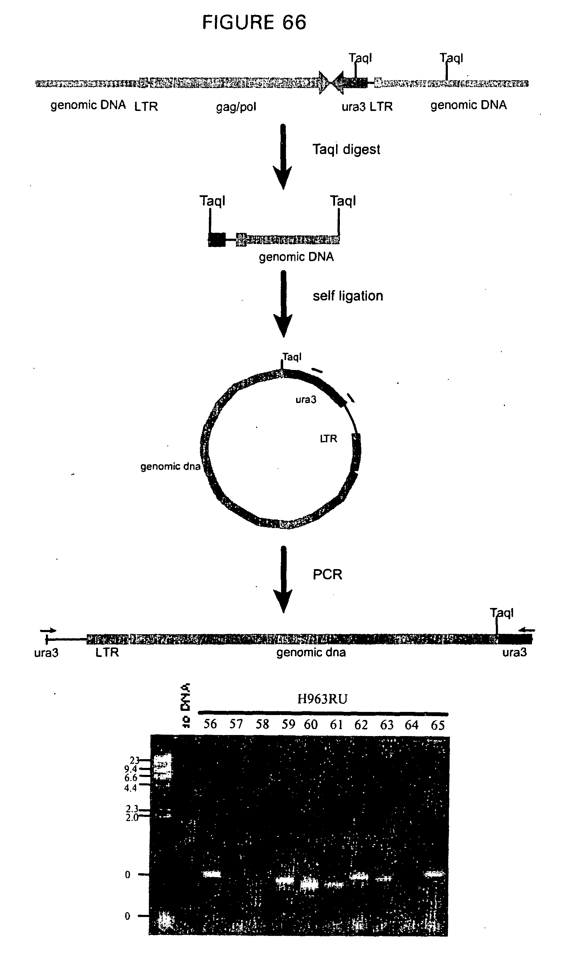

In order to analyse the putative retrotransposition events further inverse PCR was used to determine the sequence flanking the 3' end of the tagged TCa2. A PCR primer was designed to the boundary of the *URA3*/TCa2 and another primer to the site of intron insertion in the *URA3* gene. These two primers are specific for the tagged retrotransposon, since the *URA3*/TCa2 boundary is unique to the integrated vector and any retrotransposon insertions which result. The second primer requires that the intron is removed, thereby only allowing the generation of PCR products from retrotransposition events. Primers were designed so inverse PCR could be performed with the restriction enzymes *Tag* I or *Nla* III. Both of these enzymes have a four base pair recognition sequences. It was expected that this would allow inverse PCR of any integration events since these enzymes cut frequently in the genome. A schematic diagram of the inverse PCR strategy is shown in Figure 66. Initially the inverse PCR products were cloned and sequenced, however once the inverse PCR was optimised the PCR products could be directly sequenced.

### Analysis of insertion sites of the tagged TCa2

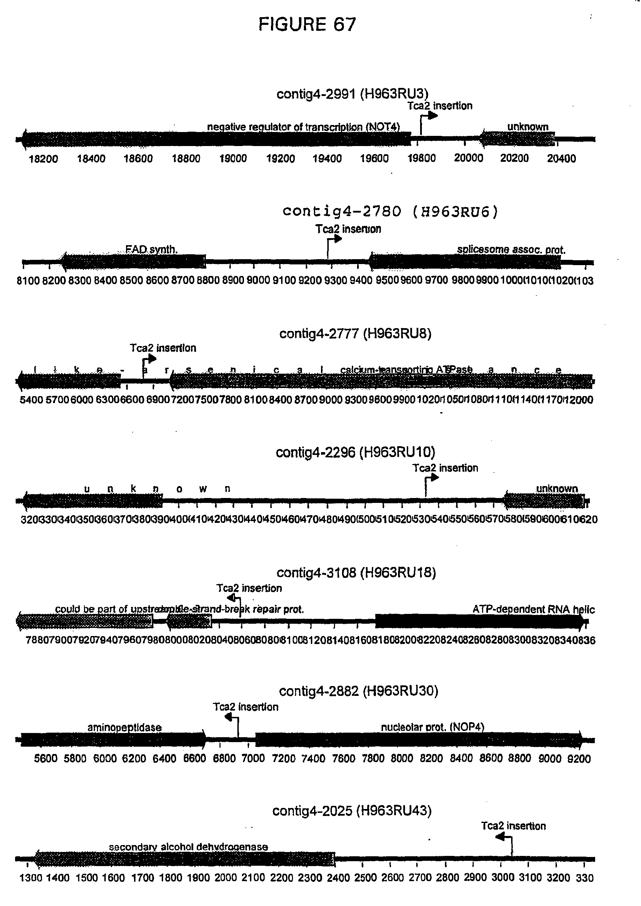

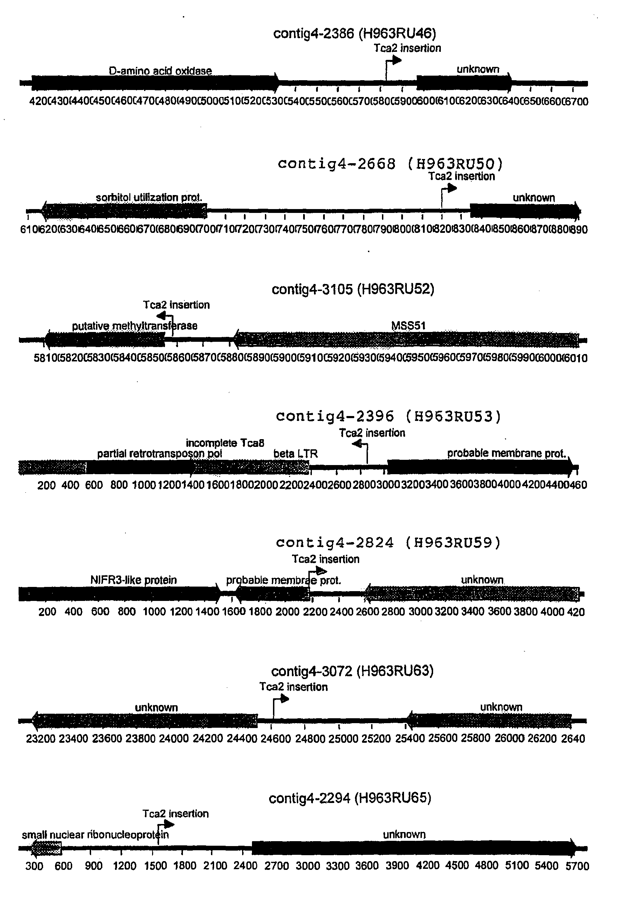

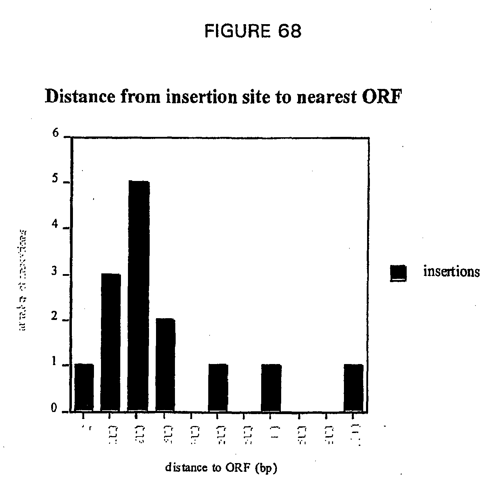

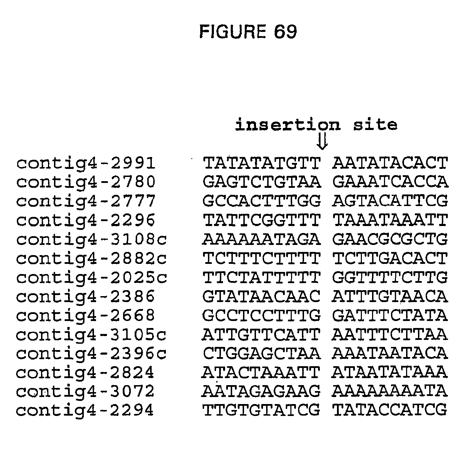

Analysis of the Ty retrotransposons of *S. cerevisiae* indicates the presence of some target site specificity. Ty3, for example integrates 1-4 nucleotides from the start site of RNA polymerase III transcription start sites; Ty1 integrates close to tRNA genes while Ty5 inserts near telomeres. Although Ty1 tends to integrate close to tRNA genes, insertions into coding sequences have also been observed. From analysis of pre-existing TCa2 insertions in the public database a target-site preference similar to those of the Ty elements is not observed. Instead, the data suggest that TCa2 has a preference for inserting into the noncoding DNA adjacent to ORFs.
Analysis of tagged TCa2 retrotranspositions reveals the occurrence of two main types of events, in this system. Insertion site sequences obtained from *URA3*⁺ colonies of H1051R all appear to be the result of homologous recombination with TCa2 LTRs. The parental strain of H 1051 R is known to contain an abundance of TCa2 linear DNA, it is possible therefore that homologous recombination is occurring since there may not be a sufficient level of the retrotransposon integrase.
Target site sequences obtained from H963R *URA3*⁺ colonies again show some events which appear to be the result of homologous recombination into LTRs, however these account for only about 40% of the events analysed. It should be noted that the proportion of recombination events appears to vary between experiments. The remainder of sequences analysed have target site sequences not previously found next to TCa2 elements; these events are thought to be genuine retrotransposition events. To date the genomic location of 14 insertions have been determined by comparison of the flanking sequences with the assembled *C. albicans* genomic sequence from the Stanford sequencing project. In addition one insertion was found in a repeat sequence, and three other insertions could not be assigned to a contig because the sequence obtained was too short, or that region had not been sequenced. These sequences have not been included in the analysis presented.
Open reading frame maps of the regions flanking the TCa2 insertions are shown in Figure 67. With the exception of one insertion into a gene (H963RU59) all other events are in the intergenic regions between ORFs. No evidence could be seen for an association with tRNAs or RNA polymerase III transcription sequences, as is seen for Ty1 and Ty3.
In order to determine the target site preference of TCa2 various analyses have been performed. There appears to be a strong preference for intergenic regions. Figure 68 shows the distribution of insertion sites in relation to the nearest ORF. This may be the result of integration occurring via an interaction with transcription factors. If this were the case then it would be expected that there would be a preference for the control regions of promoters. In support of this argument most insertions are closest to the 5' end of ORFs, rather than in the 3' region (Figure 67).
An attempt was made to determine if there is any sequence specificity for the insertion site. A region 500 bp either side of the insertion site was analysed for sequence patterns, however no consistent pattern was observed, indicating that there is no absolute sequence specificity of the TCa2 integrase. The only sequence pattern that could be determined for the integration site is a preference for AT rich sequences close to the insertion site (Figure 69), however this observation may be biased by the AT richness of *C. albicans* intergenic sequences.
These findings are consistent with the above proposal that TCa2 integration sites are determined by the distribution of transcription factors, rather than by the integrase interacting directly in a sequence-specific manner with the target site DNA.

### Removal of marker genes following retrotransposition

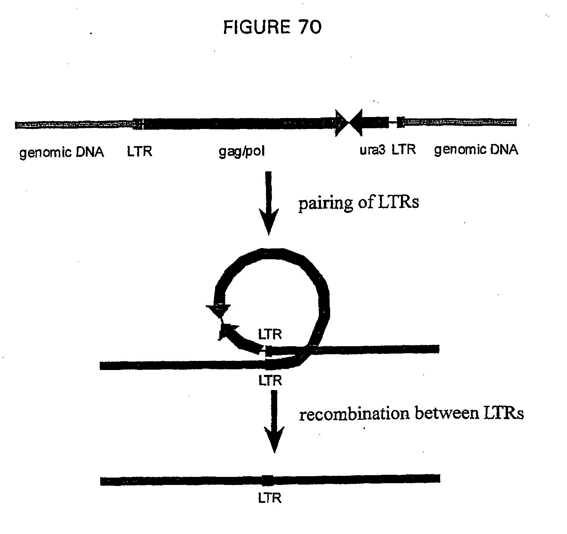

In order to construct further gene disruptions in strains which have undergone tagged retrotransposition it would be desirable to have selectable marker in these strains. Both the *ADE2* and *URA3* genes used as markers in these experiments are able to be removed, allowing reuse of these markers. Removal of the *URA3* gene should be possible through homologous recombination between the LTR sequences. Such an event should result in the presence of a single LTR (solo LTR) at the site of insertion (Figure 70). It has been demonstrated in one of the H963R *URA3*⁺ strains that the *URA3* gene can be removed by selection with 5-fluoroorotic acid (5-FOA). Analysis of these *ura3* revertants is currently in progress.
In a similar way recombination between *ADE2* genes surrounding the integrated pRUIA results in the loss of the vector. These cells are now *ade2⁻* and can be selected by their red colour on selective media. This event can be seen in H963RU1 (Figure 65). Note the loss of the band which corresponds to the integrated pRUIA.

### DISCUSSION

Analysis of the complete 6426bp sequence of pCal revealed that it is a free (i.e. unintegrated), double-stranded DNA form of a new retrotransposon belonging to the Ty1/*copia* group. Initially, no significant similarity at the nucleotide level was found between pCal and any other sequence in the databases. This was not considered surprising, however, because reverse transcriptase has no editing function, so reverse transcriptase-based elements have a higher mutation rate than those utilising other polymerases. A more appropriate and useful analysis was to look for the conserved functional motifs expected to be present. Such areas have tight evolutionary constraints and are often similar, even in highly divergent elements such as *copia* and *gypsy.* A close examination of the sequence revealed that pCal has many of the features commonly found in retrotransposons. Such features include the 280bp long terminal direct repeats (LTRs) with short inverted repeats and putative transcriptional initiation and termination signals, a (-)PBS adjacent to the left LTR, a PPT adjacent to the right LTR and two long ORFs, the first similar in size and position to the *gag* ORFs of other retroelements and the second containing motifs homologous to *pol* ORFs. Within the *gag* ORF of pCal no nucleic acid binding motif could be identified. A CX₂CX₄HX₄C nucleic acid binding motif is found within the *gag* ORF of some retrotransposons of the Ty1/*copia* group, for example Ta1, *copia,* 1731 and Tp1. However, this motif is not found in the functional retrotransposon Ty1. Taken together, all the features required for retrotransposition appear to be intact in pCal suggesting that it is likely to be a functional retrotransposon.

The order of the motifs within the *pol* gene of pCal (protease - integrase - reverse transcriptase - RNase H) suggests that pCal is a member of the Ty1/*copia* group. In agreement with this a phylogenetic analysis, based on the reverse transcriptase genes of a diverse range of retroelements, also placed pCal within the Ty1/*copia* group (Figure 12). This analysis, however, also revealed that pCal has no close relatives within the known set of Ty1/*copia* retrotransposons: pCal was placed as the most divergent element in a large group of retrotransposons containing representatives from plants (Ta1, Tnt1, Hopscotch and Tst1), insects (*copia* and 1731), a green alga (Osser) and yeast (Ty5). It is probable that the reverse transcriptase of pCal is functional and so, therefore, this placement of pCal is probably a genuine reflection of the divergent nature of this element, rather than being the result of the unselected accumulation of random mutations.

Within the LTRs of pCal there was no extended DNA sequence homology to the other *C. albicans* retroelements, TCa1 and beta. TCa1 and pCal do, however, share features such as similar inverted terminal repeats on their LTRs, a very similar PPT sequence and they potentially utilise the same tRNA^{Arg} fragment as a primer. The TCa1 (-)PBS complements nine nucleotides at the 3' end of the tRNA^{Arg} fragment (bases 31-39). The pCal (-)PBS complements eleven nucleotides of the tRNA^{Arg} fragment (bases 29-39) and, similarly to what has been found in Ty1, Ty2 and Ty3, pCal has an additional sequence downstream of the (-)PBS which complements a further 6 bases (19-24) of the tRNA^{Arg} fragment.

Given that pCal and TCa1 are believed to use an internal fragment of the tRNA^{Arg} (nucleotides 1-39), it is of great interest to note that the retrotransposon *copia* uses the first 39 nucleotides of tRNA^{iMet} as a primer. It is not clear if the fragment is the result of normal tRNA degradation. The *copia* primer may be a product of 'hyperprocessing' of tRNA^{iMet} by *Drosophila* RNase P. Hyperprocessing was defined as the processing of a mature tRNA to produce another functional RNA molecule, although, to date, the only assigned function of these tRNA fragments is as primers for retrotransposons. The RNA component of *E. coli* RNase P was shown to cleave a number of sites in the tRNA^{iMet}, one of these being between nucleotides 39 and 40. The *Drosophila* tRNA^{iMet} and yeast tRNA^{Arg3} have a very similar physical structure in terms of numbers and positions of loops and stems, residues in each loop, number of base pairs in each stem and total number of nucleotides in the tRNA. It is therefore possible that a similar hyperprocessing reaction is occurring with a tRNA^{Arg} in *C. albicans* to produce the primers for pCal and TCa1.

If pCal is using a tRNA fragment for priming, there are implications for control of replication. An element using a whole tRNA as a primer has a pool of normal, functional tRNAs to draw on, even if the tRNA in question is a rare one. Elements using a fragment, however, have to contend with the stability of tRNAs and the possibility that once a tRNA starts degrading, it may be rapidly further degraded. The elements using a fragment as a primer will have to bind the tRNA after only partial degradation. This process could be a limiting step in the reverse transcription process and consequently control copy number of pCal.

Most retrotransposons and retroviruses have been found to have their *gag* and *pol* ORFs lying in different phases on the mRNA. The necessary down-regulation of the *pol* gene with respect to the *gag* gene is thus brought about by the fairly low frequency of ribosomal frameshifting from the *gag* reading frame to the *pol* reading frame. There are, however, exceptions to this finding. For instance, the *gypsy*-type retrotransposon Tf1 from *Schizosaccharomyces pombe* has its *gag* and *pol* ORFs fused into one long ORF. The *gag* and *pol* gene products are thus produced in equal amounts. The required excess of *gag* protein to *pol* enzyme is produced post-translationally, via an enhanced rate of degradation of the *pol* enzymes. Some insect and plant retrotransposons of the Ty1/*copia* group, for example *copia,* Ta1 and Tnt1 also have their *gag* and *pol* ORFs fused into one long ORF. In *copia,* at least, the down-regulation of *pol* occurs by the frequent splicing of the mRNA to remove most of the *pol* ORF. The fact that the *gag* and *pol* ORFs of pCal are in the same phase implies that pCal is another retrotransposon that doesn't use frameshifting to down-regulate *pol.* Instead it seems likely that some form of stop codon suppression is required for translation of the *pol* ORF and this would also be likely to result in the down-regulation of *pol* relative to *gag.* It is therefore interesting to note that pCal has some structural similarities with mammalian type C retroviruses, such as Moloney murine leukemia virus (MMLV), in the vicinity of the *gaglpol* boundary. In MMLV a UAG stop codon which separates the *gag* and *pol* ORFs is suppressed with an efficiency of about 5%, being translated as glutamine. An 8bp purine-rich sequence immediately 3' to the stop codon and an adjacent pseudoknot structure are both necessary and sufficient for stop codon suppression. Mutations disrupting the stems of the pseudoknot impaired suppression and compensatory mutations restored suppression. Also the sequence of the purine-rich tract between the stop codon and the pseudoknot was found to be critical and it is likely that the length of this sequence is important. The MMLV read-through mechanism is not yet fully understood, but a pseudoknot-induced ribosomal pause at the suppressed UAG codon is likely to be involved. Similarly to MMLV, pCal has an 8bp purine-rich sequence immediately 3' to the UGA stop codon, although not the same sequence as in MMLV, and it has a putative pseudoknot (Figure 5). There is only the 8bp purine-rich sequence between the termination signal and the start of the putative pseudoknot. It is therefore likely that a similar form of read-through suppression is occurring in pCal and MMLV.

It has been reported that *C. albicans* and some other closely related *Candida* species contain a tRNA capable of suppressing UAG and UGA stop codons. This tRNA, tRNA^{SerCAG}, was originally identified as being responsible for the translation of the universal CUG-leucine codon as serine in certain *Candida* species. The tRNA^{SerCAG} has some unusual structural features and a recent report has even shown that tRNA^{SerCAG} can be charged to a low degree (about 3%) with leucine and can incorporate this leucine into proteins during translation. This is one of the first examples of the assignment of a single tRNA species to two amino acids. This strange tRNA was also implicated in some aberrant translational events. It was found that when C. *albicans* tRNAs were added to *in vitro* translation systems, proteins which migrated more slowly than expected on SDS-PAGE gels were produced. These results were interpreted as evidence that *C. albicans* contains a tRNA capable of suppressing UAG and UGA stop codons. The tRNA responsible for the unusual translational events has been identified as tRNA^{SerCAG}. However, results could not be simply explained by tRNA^{SerCAG} being an omnipotent nonsense suppressor: The amino-terminal regions of proteins synthesised in the presence of tRNA^{SerCAG} also migrated more slowly than expected with SDS-PAGE. At present it is unclear what the actual effects of tRNA^{SerCAG} are, aside from incorporation of serine at CUG codons. This leaves open the question of what molecule it is that mediates the suppression of the UGA termination codon at the *gaglpol* boundary of pCal. Sequencing the *gag* and the *gag*/*pol* fusion proteins and mutational analyses of the regions surrounding the stop codon could be used to determine the mechanism by which the *pol* genes of pCal are translated.

The pCal system is producing much more free dsDNA - estimated at 50-100 copies per cell - than any other reported retrotransposon system. This is true even of the system in which Ty1 of *S. cerevisiae* is expressed off a high copy number plasmid under the control of the highly inducible *GAL1* promoter. Such a *GAL* promoter system is capable of producing about 10 dsDNA copies per cell and the DNA requires Southern blotting before it can be detected. We have detected integrated retrotransposons, similar in sequence to pCal, which we have named TCa2. This integrated form has been detected in a diverse range of *C. albicans* strains. Extremely high levels of the free, linear, dsDNA form (pCal), however, have only been detected in hOG1042 and its close relatives (descendants of iB65).

Overall, pCal presents itself as a highly unusual retrotransposon. While having many of the features conserved among retrotransposons, it has a number of features which set it apart from other elements of its class. For instance, the translation of the *pol* ORF seems to be dependent upon the pseudoknot-assisted read-through of a UGA stop codon. This is similar to the mechanism used by mammalian type C retroviruses, but has not been previously reported in retrotransposons. A phylogenetic analysis of the reverse transcriptase sequences of a number of LTR-retroelements showed that, while pCal lies within the Ty1/*copia* group of retrotransposons, it is one of the most divergent elements within this group. The most distinctive feature of pCal, however, is that it exists at a high copy number as a free, linear, double-stranded DNA molecule.

The TCa2 retrotransposon was originally discovered due to its appearance as an abundant, extrachromosomal DNA molecule in *Candida albicans* strain hOG1042. Sequence analysis of some clones of this extrachromosomal form of TCa2 (referred to as pCal) showed it to be basal member of the Ty1/*copia* class of retrotransposon. Here we have extended the characterization of this element to include an analysis of its integrated forms, and a comparison of the expression of its RNA and extrachromosomal DNA forms, in a variety of *C. albicans* strains.

An important finding to emerge from this work is that there is a large amount of variation amongst different *C. albicans* strains, in both the amount of TCa2 RNA and extrachromosomal pCal DNA produced, and in the genomic copy number of TCa2. It is of interest that the number of integrated copies of TCa2 in the different strains correlates with the amount of TCa2 RNA produced by each strain, and again, that the amount of TCa2 RNA in each strain is related to the amount of extrachromosomal pCal DNA. The greatest numbers of integrated copies of TCa2, 10 to 12, occur in the closely related strains hOG759 and hOG1042. About 5 copies are found in F16932, and the other strains examined, SGY269, SC5314, ATCC10261, and SA40 each have 1 or 2 copies. The highest levels of TCa2 RNA are also found in hOG759 and hOG1042. The next highest level occurs in F16932, and the other four strains each have a relatively low level. The greatest amounts of pCal extrachromosomal DNA are, once again, found in hOG759 and hOG1042. Moderate levels of pCal are found in F16932, and also in SA40. Low levels occur in SGY269 and SC5314, and, lastly, no extrachromosomal copies of pCal, at all, were detected in ATCC10261. These correlations between genomic copy number and abundance of RNA, and between the abundance of RNA and the abundance of extrachromosomal DNA, suggest that a large amount of the variation seen among strains, in the amount of pCal DNA and TCa2 RNA that they produce, is simply a consequence of variations in the number of integrated copies. Or, to put this another way, the genomic copy number of TCa2 is a major determinant of TCa2 RNA levels, and the TCa2 RNA levels are a major determinant of pCal DNA levels. As mentioned in the results, however, the correlations are not perfect which suggests that other factors are also involved. To reiterate: hOG759 and hOG1042 have roughly twice as many integrated TCa2 copies as F16932 and ten times as many as the other four strains, yet they produce about 5 times and 50 to 100 times as much RNA, respectively; SA40 has about a fifth the TCa2 RNA found in F16932 and only slightly more than SGY269 and SC5314, yet it produces similar quantities of pCal to F16932 and 10 to 20 times as much as the other two strains; and ATCC10261 produces a slightly larger amount of TCa2 RNA than SGY269 and SC5314, and a similar amount to SA40, yet it doesn't produce any detectable extrachromosomal copies of pCal.

A simple explanation for the result with ATCC10261 is that the TCa2 elements in this strain have suffered mutations that corrupt their RT gene or render inactive other sequences required for reverse transcription, for example the polypurine tract. Such an occurrence would account for the lack of extrachromosomal pCal molecules in this strain. Accounting for the relative overproduction of TCa2 RNA in hOG759 and hOG1042, and the relative overproduction of pCal DNA in SA40 is, however, not so simple. In hOG759 and hOG1042 there is roughly five times as much TCa2 RNA as would have been expected from a comparison with TCa2 copy number and RNA expression in other strains. This suggests that one or more TCa2 elements in these strains are being transcribed at a very high rate. There are a number of possible explanations for this. Firstly, it is possible that an element in these strains has suffered an alteration to its promoter region such that it becomes hyperactive and produces an abundance of transcripts. A comparison of the 5' regions of TCa2 elements from various strains (Fig. 9), however, failed to identify any significant differences between the LTRs of hOG759 and hOG1042 and the LTRs of other strains, although this does not rule out the possibility that such an element exists. Another possible factor that could be involved is the genomic location of the TCa2 elements. It is possible, for instance, that TCa2 retrotransposons are normally integrated in regions of silent chromatin, as is the case with the Ty5 element of *Saccharomyces.* If, for some reason, a copy of the retrotransposon became integrated at an open or transcriptionally active region of the genome then this might result in the overexpression of its RNA. Strain variation in proteins involved in regulating transcription could also be involved in the overproduction of TCa2 RNA in hOG759 and hOG1042. These strains have been subjected to mutagenesis with UV radiation and N-methyl-N-nitro-N-nitrosoguanidine. It is possible that in the course of this mutagenesis these strains have, for instance, lost some repressor of TCa2 transcription or suffered a mutation in some other transcription factor, with the result that the TCa2 retrotransposons are subsequently transcribed at a higher than normal rate. Finally, it is conceivable that the higher copy number in hOG759 and hOG1042 acts to titrate out a repressor molecule, with the result that there are unrepressed elements which are then transcribed at a high rate. As can be seen, further experiments will be required to determine which, if any, of these factors are involved.

Strain SA40 produces about 5 to 6 times as much pCal DNA as might have been predicted from a comparison of TCa2 RNA and pCal DNA levels in the other strains. This suggests that reverse transcription of TCa2 RNA is proceeding more efficiently in this strain than in other strains. Again, there are a number of possible explanations. For instance, the retrotransposon in this strain could have a superior RT or the genomic RNA may be more efficiently packaged into the virus-like particle where reverse transcription occurs. Alternatively, it could result from some host factor, such as increased availability of the primer tRNA fragment, which may be limiting for reverse transcription. Whatever the cause, it is interesting that strain SA40 manages to produce abundant amounts of pCal DNA from, apparently, just one integrated copy of the element. This may make it a useful strain for further dissection of this system.

Determination of the number of integrated elements in the closely related strains hOG759 and hOG1042 revealed that hOG1042 has at least one more copy than hOG759. There are at least three possible explanations for this: (1) a recombination between the two LTRs of a retrotransposon in hOG759 resulting in the deletion of an element, (2) a non-homologous chromosomal recombination resulting in either the duplication of an element in hOG1042 or in the deletion of an element in hOG759, and (3) a transposition event in hOG1042 resulting in an additional copy in this strain. Intra-element recombination and non-homologous recombinations are both likely to be relatively rare events and so, given the abundance of full-length pCal molecules in hOG1042, and the fact that the elements encode a potential integrase enzyme, the most likely explanation of the extra copy in hOG1042 is that it is the result of a transposition event since the divergence of this strain from hOG759. Since the divergence of hOG1042 from hOG759, the strains have spent most of their time stored at -80°C, with no more than a week or two of active growth. The discovery of what is likely to be a transposition in hOG1042, in just a short period of time since its divergence from hOG759, suggests that the retrotransposon may be transposing at a high rate, which is perhaps not surprising given the abundance of apparently full-length reverse transcripts. If this element is still actively transposing then it may make a useful system for insertional mutagenesis in *C. albicans,* as has been the case with Ty1 and *Saccharomyces.* Regarding this last point, it is of interest that hOG1042, not only has more integrated copies of TCa2 than hOG759, but also has suffered a *de novo* auxotrophic mutation (resulting in a requirement for aspartate or proline when brought to homozygosity) that is not found in hOG759. It is possible that this spontaneous mutation is the result of a TCa2 transposition event.

The expression of TCa2 RNA was found to be 5 to 10 times higher at 37°C than at 27°C. This contrasts with the expression of the *C. albicans* retrotransposon-like element TCa1, in which the RNA was found to be 20- to 30-fold more abundant at 25°C than at 37°C. The temperature-dependent expression of these retrotransposons does not appear to be the result of a general temperature-dependent variation in transcription rate, so it is probably a specific retrotransposon effect. It is not clear what advantage it confers on the retrotransposons to regulate their expression in this manner. It has been suggested that TCa1 could play a role in, for instance, up-regulating genes which improve the chances of the survival outside of the host, or, alternatively, down-regulating genes which trigger host defences. Similar effects could be proposed for TCa2. For example, transposition of TCa2 could up-regulate genes required for maintaining an infection, or could down-regulate genes not required outside the host. It would be interesting to identify the sequences within TCa1 and TCa2 that are responsible for their temperature-dependent expression. Such sequences may be widely used in *C. albicans* as a means of regulating the expression of specific genes. The TCa1 and TCa2 promoters may also make useful temperature-inducible promoters in transformation studies analyzing other *C. albicans* genes.

In our original description of pCal we estimated that it appears at 50-100 extrachromosomal copies per cell in hOG1042 (30). In Figure 7, however, the TCa2 probe can be seen to hybridize to the extrachromosomal and chromosomal DNA from hOG1042 (37°C) to a similar degree. The number of integrated copies in hOG1042 is 10 to 12, suggesting that, at 37°C, pCal is also present at 10 to 12 copies per cell. This estimate may be misleading, however, because at least some of the pCal molecules are likely to be located in the interior of a large, proteinaceous particle, and therefore may be lost during the DNA isolation procedure. In agreement with this, we have found that the amount of pCal obtained, relative to chromosomal DNA, varies with different DNA extraction protocols (not shown). The method used to isolate the DNA for the Southern shown in Figure 7 gives a lower amount of pCal than some other methods. An unbiased technique will be required to accurately determine the absolute number of extrachromosomal pCal molecules per cell. The technique that we have used in Figure 7 should, however, be a reliable indicator of the relative amounts of pCal in the different strains and at the different temperatures.

An analysis of the 5' regions of TCa2 retrotranposons from the various strains showed that some of these elements have minus-strand primer-binding sites which are very long, One clone from hOG759 has a perfect 32 bp match to the primer tRNA^{Arg(UCU)} fragment. The other clone from hOG759 and the two clones from SC5314 also have 32-bp matches to the tRNA primer, allowing for 2 G-U base pairs. The p30 clone of pCal from hOG1042 also has a 32-bp match but with 3 G-U base pairs. All the other clones have 31 out of 32-bp matches to the tRNA primer with 4 G-U base pairs. To the best of our knowledge, these 32-base PBSs are the longest described. Most retrotransposons have PBSs that are 10 to 12 nucleotides long, for example Ty1 (10 nucleotides). Retroviruses, for example, Moloney murine leukemia virus have 18 nucleotide PBSs. After the TCa2 PBS, the next longest PBS of which we are aware is 24 nucleotides long and is found in the *magellan* element of maize. It has been shown that introducing a mismatch into the Ty1 PBS reduces the Ty1 transposition frequency at higher temperatures while increasing the length of the PBS results in an increase in the transposition frequency at higher temperatures. These differences in transposition frequency are most likely due to differences in the efficiency of the initiation of the reverse transcription process. This suggests that long PBSs are more efficient than short PBSs at high temperatures. The very long PBSs found in TCa2 elements, may thus predispose these retrotransposons to high levels of reverse transcription at 37°C. On the other hand, it has recently been shown that there are regions, in addition to the PBS, where Ty1 binds to its tRNA primer, such that 30 bases of Ty1 RNA are paired with primer tRNA. Disruption of as few as two of these base pairs was found to have a drastic effect on transposition frequency. It may be that a long PBS is necessary for efficient reverse transcription at 37°C, especially for elements, such as TCa2, utilising a tRNA fragment that is just 40 nucleotides long and to which there would be little opportunity for additional regions of base-pairing.

No hybridization of either the TCa2 internal or LTR probes was observed to DNA of *C. maltosa, C. tropicalis,* or *C. parapsilosis* which are all close relatives of *C. albicans,* nor to DNA of the more distantly related *C. pseudotropicalis.* This suggests that TCa2 is specific to *C. albicans.* Given the apparent ubiquity of retrotransposons in the eukaryotes, it is likely that these species have retrotransposons, but that these retrotransposons have diyerged sufficiently since speciation that they are no longer detectable by hybridization to TCa2.

In most of the *C. albicans* strains that we have examined here, there is a fairly low number of integrated copies of TCa2 (5 or fewer per genome). The full-lerigth TCa1 element is also present at low copy numbers (just 1 or 2 per genome) and all the retroelement LTRs found in *C. albicans* to date, and those of TCa1 and TCa2, appear at a similar low copy number of about 5 to15 per genome. These low copy numbers are suggestive of a mechanism whereby transposition of retroelements in *C. albicans* is held in check. In hOG759 and hOG1042, however, the copy number of TCa2 is higher (about 10 full-length elements per genome) and appears to be capable of increase. It may be that in these strains the TCa2 retrotransposons have escaped the normal constraints on their replication and are thus transposing at rates much above normal. If, as is most likely, the majority of newly transposed copies are themselves capable of transposition they may serve to increase the rate of transposition still further. It would therefore be interesting to see what would happen in these strains if they were continuously grown for an extended period.

### INDUSTRIAL APPLICABILITY

The invention relates to a novel retrotransposon from *Candida* which could be used to assign a function to a nucleotide sequence in *Candida.* It could also be useful in a gene disruption system and in discovering a gene. The retrotransposon may also be useful in detecting the presence of *Candida.*

### REFERENCES

Agatensi, L., F. Franchi, F. Mondello, R.L Bevilacqua, T. Ceddia, F. De Bernardis, and A. Cassone 1991. Vaginopathic and proteolytic Candida species in outpatients attending a gynaecology clinic. J. Clin. Pathol. 44:826-830.
Altschul, S.F., Gish, W., Miller W., Myers, E.W., Lipman, D.J. 1990. Basic local alignment search tool. J. Mol. Biol, 215:403-410.
Altschul, S.F., Madden, T.L., Schaffter, A.A., Zhang, J., Zhang, Z., Miller, W., Lipman, D.J. 1997. Gapped BLAST and PSI-BLAST: a new generation of protein database search programs. Nucleic Acids Res. 25:3389-3402.
Boeke, J.D., and S. Sandmeyer 1991. Yeast transposable elements. In the molecular and cellular biology of the yeast Saccharomyces cerevisiae. (eds. J.R. Broach, E.W. Jones, and J. Pringle), pp193-261. Cold Spring Harbor Laboratory Press, Cold Spring Harbor, New York.
Basrai, M.A., Lubkowitz, M.A., Perry, J.R., Miller, D., Krainer, E., Naider, F. and Becker, J.M. (1995) Cloning of a Candida albicans peptide transport gene. Microbiology 141:1147-1156.
Chen, J-Y., and W. A. Fonzi. 1992. A Temperature-Regulated, Retrotransposon-Like Element from Candida albicans. J. Bacteriol. 174:5624-5632.
Church, G.M., and W. Gilbert. 1984. Genomic sequencing. Proc. Natl. Acad. Sci. USA 81:1991-1995.
Cryer, D. R., R. Eccleshall, and J. Marmur. 1975. Isolation of Yeast DNA. Methods Cell Biol. 12:39-44.
Devereux, J., P. Haerberli, and O. Smithies. 1984. A comprehensive set of sequence analysis programs for the VAX. Nucleic Acids Res. 12:387-395.
Felsenstein, J. 1989. PHYLIP-Phylogeny inference package (version 3.2). Cladistics 5:164-166
Fourcade-Peronnet, F., L. d'Auriol, J. Becker, F. Galibert, and M. Best-Belpomme. 1988. Primary structure and functional organization of Drosophila 1731 retrotranposon. Nucleic Acids Res. 16:6113-6125.
Garfinkel, D.J., J.D. Boeke, and G.R. Fink. 1985. Ty element transposition: reverse transcriptase and virus-like particles. Cell 42:507-517.
Gillium, A.M., E.Y.H. Tsay and D.R. Kirsch. 1984. Isolation of the Candida albicans gene for orotidine-5'-phosphate decarbosylase by complementation of S.cerevisiae ura3 and E. coli pyrF mutations. Mol. Gen. Genet. 198: 179-182.
Hansen, L.J., D.L. Chalker, and S.B. Sandmeyer. 1988. Ty3, a Yeast Retrotransposon Associated with tRNA Genes, Has Homology to Animal Retroviruses. Mol. Cell. Biol. 8:5245-5256.
Jenkinson, H.F., Schep, G.P., and M. G. Shepherd. 1988. Cloning and expression of the 3-isopropylmalate dehydrogenase gene from Candida albicans . FEMS Microbiol. Lett. 49: 285-288.
Kelly, R., S.M. Miller, M.B. Kurtz and D.R. Kirsch. 1987. Directed mutagenesis in Candid albicans: one-step gene disruption to isolate ura3 mutants. Mol. Cell. Biol. 7: 199-208.
Kelly, R., S.M. Miller, M.B. Kurtz and D.R. Kirsch. 1988. One-step gene disruption by cotransformation to isolate double auxotrophs in Candida albicans. Mol. Gen. Genet. 214:24-31.
Kurtz, M.B, Cortelyou, M.W, Miller, S.M., Lai, M and D.R.Kirsch. 1987. Development of Autonomously Replicating Plasmids for Candida albicans Mol Cell Biol 7:209-217.
Kelly, R., Miller, S.M and M.B. Kurts. 1988. One-step gene disruption by cotransformation to isolate double auxotrophs in Candida albicans. Mol Gen. Genet. 214:24-31.
Losberger, C., and Ernst, J.F. 1989. Sequence and transcript analysis of the C.albicans URA3 gene encoding orotidine-t'-phosphate decarboxylase. Current Genetics. 16: 153-157
Markie, D., Hill, D.F and R Poulter. 1986. The Construction of a Modified Drug Resistance Cassette Proc. Univ. Otago Med Sch 64:69-70.
Maniatis, T., Fritsch, E.F., and J. Sambrook. 1982. Molecular Cloning: a laboratory manual, Cola Spring Harbor Laboratory, Cola Spring Harbor, New York.
Matthews, G.D., T.J.D Goodwin, M.I. Butler, T.A. Berryman, and R.T.M Poulter. 1997. PCal, a highly unusual Ty1/copia retrotransposon from the pathogenic yeast Candida albicans. J. Bacteriol 179: 7118-7128.
Mount, S. M., and G. M. Rubin. 1985. Complete Nucleotide Sequence of the Drosophila Transposable Element Copia: Homology Between Copia and Retroviral Proteins. Mol. Cell. Biol. 5:1630-1638.
Odds, F.C. 1988. Candida and candidosis. A review and bibliography. Balliere Tindall, London, UK
Ohkuma, M., Muraoka, S., Hwang, C.W., Ohta, A. and Takagi, M. 1993. Cloning of the C-URA3 gene and construction of a triple auxotroph (his5, ade1, ura3) as a useful host for the genetic engineering of Candida maltosa. Current Genetics 23:205-210.
Perreau, V.M., Santos M.A., Tuite, M.F. 1997. Beta, a novel repetitive DNA element associated with tRNA genes in the pathogenic yeast Candida albicans . Mol. Microbiol July 25(2):229-236.
Philippsen, P., A. Stotz and C. Scherf. 1991. DNA of Saccharomyces cerevisiae. Methods enzymol. 194: 169-182.
Poulter, R., K. Jeffrey, M. J. Hubbard, M. G. Shepherd, and P. A . Sullivan. 1981. Parasexual Genetic Analysis of Candida albicans by Spheroplast Fusion. J. Bacteriol. 146:833-840.
Prasad, R., P. De Wergifosse, A. Goffeau, and E. Balzi. 1995. Molecular cloning and characterization of a novel gene of Candida Albicans, CDR1, conferring multiple resistance to drugs and antifungals. Curr. Genet. 27:230-329.
Santos MA, Tuite MF (1995) The CUG codon is decoded in vivo as serine and not leucine in Candida albicans. Nucleic Acids Res 23:1481-6
Sasnauskas. K., Jomantiene, R., Geneviciute, E., Januska, A. and J. Lebedys. 1991. Molecular cloning of the Candida maltosa ADE1 gene. Gene 107: 161-164.
Short, J.M., Fernandez, J.M. Sorge, J.A. and W.D Huse. 1988. λZAP: a bacteriophage λ expression vector with in vivo excision properties. Nucl. Acids Res 16: 7583-7600.
Stockwell PA (1985) VTUTIN: a full screen gel management editor. Comput Appl Biosci. 1:253-9.
Stockwell, P.A., and G.B. Petersen. 1987. HOMED: a homologous sequence editor. Comp. Appl. Biosci. 3:37-43.
Stoesser, G., Moseley, M.A. , Sleep, J., McGowran, M., Garcia-Pa stor, M., Sterk, P. 1998. The EMBL nucleotide sequence database. Nucleic Acids Res. 26:8-15.
ten Dam, E., Pleij, K., and Draper, D. 1992. Structural and functional aspects of RNA pseudoknots. Biochemistry 31:11665-11676.
Wagenbach, M. O'Rourke, K., Vitez, L., Wieczorek, A., Hoffman, S., Durfee, S., Tedesco, J. and G Stetier. 1991. Synthesis of Wild Type and Mutant Human Hemoglobins in Saccharomyces cerevisiae Bio Tech 9:57-61.
Woodcock, D.M., Crowther, P.J., Doherty, J., DeCruz, E., Noyer-Weidner, M., Smith, S.S., Michael, M.Z., and M.W. Graham. 1989. Quantitative evaluation of Escherichia coli lest strains for tolerance to cytosine methylation in plasmid and phage recombinants Nucleic Acid Res 17:3469-3478.
Woods, R.A. and E.A Bevan. 1965. Interallelic Complementation at the ad-2 Locus of Saccaromyces cerevisiae. Heredity 21: 121-130.
Xiong, Y., and T. H. Eickbush. 1990. Origin and evolution of retroelements based upon their reverse transcriptase sequences. EMBO J. 9:3353-3362.
Yanish-Perron, C., Viera, J. and J. Messing. 1985. Improved M13 phage cloning vectors and host strains: nucleotide sequences of M13mp 18 and pUC19 vectors. Gene 33: 103-119.

## Claims

1. A method of gene disruption or altered expression comprising integrating a retrotransposon, comprising a nucleotide sequence selected from the group consisting of:
(a) the sequence illustrated in Figure 2B;
(b) a nucleotide sequence with at least 65% similarity with the LTR and POL region of Figure 2B;
(c) a nucleotide sequence that hybridizes under conditions of standard stringency to the nucleotide sequence shown in Figure 2B;
into a site or sites in a yeast or fungus or *Candida* wherein the retrotransposon contains elements that cause gene disruption or altered expression at the site or sites; and, optionally the gene disruption or altered expression is non-revertible.

2. A gene discovery method comprising integrating a retrotransposon, comprising a nucleotide sequence selected from the group consisting of:
(a) the sequence illustrated in Figure 2B;
(b) a nucleotide sequence with at least 65% similarity with the LTR and POL region of Figure 2B;
(c) a nucleotide sequence that hybridizes under conditions of standard stringency to the nucleotide sequence shown in Figure 2B;
into a site or sites in a yeast or fungus or *Candida* wherein the retrotransposon contains elements that cause gene disruption or altered expression at the site or sites, and, optionally the gene disruption or altered expression is non-revertible; and, mapping the gene or genes disrupted or whose expression has been altered, by the retrotransposon.

3. A method according to claim 1 or 2 wherein the retrotransposon integrates preferentially closer to the 5' end of an ORF than to the 3' end of the ORF.

4. A method of preferentially integrating a retrotransposon closer to the 5' end of an ORF than the 3' end of the ORF, comprising the use of Tca2 integrase to preferentially direct the integration of the retrotransposon to a position closer to the 5' end of the ORF than the 3' end.
